# EUROPEAN PATENT APPLICATION

(11) **EP 1 469 073 A2**
(43) Date of publication of application: **20.10.2004**
(21) Application number: 04017461.7
(22) Date of filing: 01.06.2000
(51) Int. Cl.: C12N 15/12, C07K 14/47, C12N 15/63, C07K 16/18, G01N 33/68, C12Q 1/68, G01N 33/53, A61K 38/17, A61K 48/00, A61K 39/395

(54) **Polynucleotides and membrane-bound polypeptides encoded thereby**

(30) Priority: 03.06.1999 US 137322 P; 16.03.2000 US 189810 P; 22.03.2000 US 191158 P; 30.03.2000 US 193086 P; 03.05.2000 US 201388 P; 31.05.2000 US 584411
(62) Divisional of application: 00942669.3
(71) Applicant: Curagen Corporation, New Haven, CT 06511 (US)
(72) Inventor: Shimkets, Richard A., West Haven, CT 06516 (US); Fernandes, Elma, Brandford, CT 06505 (US); Herrman, John, Guildford, CT 06437 (US); Vernet, Corrine, Gainesville, FL 32060 (US)
(74) Representative: MacLean, Martin Robert

(57) **Abstract**

The present invention provides isolated NOVX polypeptides, as well as polynucleotides encoding them and antibodies that immunospecifically bind to NOVX or any derivative, variant, mutant, or fragment of NOVX polypeptides, polynucleotides or antibodies. The invention additionally provides methods in which the NOVX polypeptides, polynucleotides and antibodies are used in detection and treatment of a broad range of pathological states, as well as other uses.

## Description

### FIELD OF THE INVENTION

The invention relates to nucleic acids and polypeptides encoded thereby, and methods of using these nucleic acids and polypeptides.

### BACKGROUND OF THE INVENTION

Eukaryotic cells are subdivided by membranes into multiple functionally distinct compartments that are referred to as organelles. Each organelle includes proteins essential for its proper function. These proteins can include sequence motifs often referred to as sorting signals. The sorting signals can aid in targeting the proteins to their appropriate cellular organelle. In addition, sorting signals can direct some proteins to be exported, or secreted, from the cell.

One type of sorting signal is a signal sequence, which is also referred to as a signal peptide or leader sequence. The signal sequence is present as an ammo-terminal extension on a newly synthesized polypeptide chain A signal sequence can target proteins to an intracellular organelle called the endoplasmic reticulum (ER).

The signal sequence takes part in an array of protein-protein and protein-lipid interactions that result in translocation of a polypeptide containing the signal sequence through a channel in the ER. After translocation, a membrane-bound enzyme, named a signal peptidase, liberates the mature protein from the signal sequence.

The ER functions to separate membrane-bound proteins and secreted proteins from proteins that remain in the cytoplasm. Once targeted to the ER, both secreted and membrane-bound proteins can be further distributed to another cellular organelle called the Golgi apparatus. The Golgi directs the proteins to other cellular organelles such as vesicles, lysosomes, the plasma membrane, mitochondria and microbodies.

Secreted and membrane-bound proteins are involved in many biologically diverse activities. Examples of known secreted proteins include human insulin, interferon, interleukins, transforming growth factor-beta, human growth hormone, erythropoietin, and lymphokines. Only a limited number of genes encoding human membrane-bound and secreted proteins have been identified.

### SUMMARY OF THE INVENTION

The invention is based in part on the discovery of nucleic acids that include open reading frames encoding novel polypeptides, including secreted and membrane-bound polypeptides, and on the polypeptides encoded thereby. The nucleic acids and polypeptides are collectively referred to herein as "NOVX".

Accordingly, in one aspect, the invention provides an isolated nucleic acid molecule (*e*.*g*., SEQ ID NO:1, 3, 5, 7, 9 11, 13, 15, 17, 19, 21, 23, 25, 27, 29, 31, 33, 35, 37, 39, 41, 43, or 45) that encodes novel polypeptide, or a fragment, homolog, analog or derivative thereof. The nucleic acid can also include, *e.g.*, a nucleic acid sequence encoding a polypeptide at least 85% identical to a polypeptide comprising the amino acid sequence of SEQ ID NO:2, 4, 6, 8, 10, 12, 14, 16, 18, 20, 22, 24, 26, 28, 30, 32, 34, 36, 38, 40, 42, 44, and 46. The nucleic acid can be, *e*.*g*., a genomic DNA fragment, or a cDNA molecule.

Also included in the invention is a vector containing one or more of the nucleic acids described herein, and a cell containing the vectors or nucleic acids described herein.

The invention is also directed to host cells transformed with a recombinant expression vector comprising any of the nucleic acid molecules described above.

In another aspect, the invention includes a pharmaceutical composition that includes a NOVX nucleic acid and a pharmaceutically acceptable carrier or diluent.

In a further aspect, the invention includes a substantially purified NOVX polypeptide, *e.g.*, any of the NOVX polypeptides encoded by a NOVX nucleic acid, and fragments, homologs, analogs, and derivatives thereof. The invention also includes a pharmaceutical composition that includes a NOVX polypeptide and a pharmaceutically acceptable carrier or diluent.

In a still a further aspect, the invention provides an antibody that binds specifically to a NOVX polypeptide. The antibody can be, *e.g.*, a monoclonal or polyclonal antibody, and fragments, homologs, analogs, and derivatives thereof. The invention also includes a pharmaceutical composition including NOVX antibody and a pharmaceutically acceptable carrier or diluent. The invention is also directed to isolated antibodies that bind to an epitope on a polypeptide encoded by any of the nucleic acid molecules described above.

The invention also includes kits comprising any of the pharmaceutical compositions described above.

The invention further provides a method for producing a NOVX polypeptide by providing a cell containing a NOVX nucleic acid, *e.g.,* a vector that includes a NOVX nucleic acid, and culturing the cell under conditions sufficient to express the NOVX polypeptide encoded by the nucleic acid. The expressed NOVX polypeptide is then recovered from the cell. Preferably, the cell produces little or no endogenous NOVX polypeptide. The cell can be, *e*.*g*., a prokaryotic cell or eukaryotic cell.

The invention is also directed to methods of identifying a NOVX polypeptide or nucleic acid in a sample by contacting the sample with a compound that specifically binds to the polypeptide or nucleic acid, and detecting complex formation, if present.

The invention further provides methods of identifying a compound that modulates the activity of a NOVX polypeptide by contacting NOVX polypeptide with a compound and determining whether the NOVX polypeptide activity is modified.

The invention is also directed to compounds that modulate NOVX polypeptide activity identified by contacting a NOVX polypeptide with the compound and determining whether the compound modifies activity of the NOVX polypeptide, binds to the NOVX polypeptide, or binds to a nucleic acid molecule encoding a NOVX polypeptide.

In another aspect, the invention provides a method of determining the presence of or predisposition of a NOVX-associated disorder in a subject. The method includes providing a sample from the subject and measuring the amount of NOVX polypeptide in the subject sample. The amount of NOVX polypeptide in the subject sample is then compared to the amount of NOVX polypeptide in a control sample. An alteration in the amount of NOVX polypeptide in the subject protein sample relative to the amount of NOVX polypeptide in the control protein sample indicates the subject has a tissue proliferation-associated condition. A control sample is preferably taken from a matched individual, *i*.*e*., an individual of similar age, sex, or other general condition but who is not suspected of having a tissue proliferation-associated condition. Alternatively, the control sample may be taken from the subject at a time when the subject is not suspected of having a tissue proliferation-associated disorder. In some embodiments, the NOVX is detected using a NOVX antibody.

In a further aspect, the invention provides a method of determining the presence of or predisposition to a NOVX-associated disorder in a subject. The method includes providing a nucleic acid sample, *e*.*g*., RNA or DNA, or both, from the subject and measuring the amount of the NOVX nucleic acid in the subject nucleic acid sample. The amount of NOVX nucleic acid sample in the subject nucleic acid is then compared to the amount of a NOVX nucleic acid in a control sample. An alteration in the amount of NOVX nucleic acid in the sample relative to the amount of NOVX in the control sample indicates the subject has a tissue proliferation-associated disorder.

In a still further aspect, the invention provides method of treating or preventing or delaying a NOVX-associated disorder. The method includes administering to a subject in which such treatment or prevention or delay is desired a NOVX nucleic acid, a NOVX polypeptide, or a NOVX antibody in an amount sufficient to treat, prevent, or delay a tissue proliferation-associated disorder in the subject.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. All publications, patent applications, patents, and other references mentioned herein are incorporated by reference in their entirety. In the case of conflict, the present specification, including definitions, will control. In addition, the materials, methods, and examples are illustrative only and not intended to be limiting.

Other features and advantages of the invention will be apparent from the following detailed description and claims.

### BRIEF DESCRIPTION OF THE DRAWINGS

Figure 1 depicts the expression of a secreted NOV5 protein by human embryonic kidney 293 cells.
Figure 2 depicts the expression of a secreted NOV5 protein by *E. coli* cells.
Figure 3 depicts the expression of an NOV6 protein in human embryonic kidney 293 cells.

### DETAILED DESCRIPTION OF THE INVENTION

The invention provides novel polynucleotides and polypeptides encoded thereby. The polynucleotides and their encoded polypeptides can be grouped according to the functions played by their gene products. Such functions include structural proteins and proteins, which are associated with metabolic pathways fatty acid metabolism, glycolysis, intermediary metabolism, calcium metabolism, proteases, and amino acid metabolism, etc.

Included in the invention are novel nucleic acid sequences and their encoded polypeptides. The sequences are collectively referred to as "NOVX nucleic acids" or "NOVX polynucleotides" and the corresponding encoded polypeptide is referred to as a "NOVX polypeptide" or "NOVX protein". For example, one NOVX nucleic acid according to the invention is a nucleic acid that includes a NOV1 nucleic acid, and one NOVX polypeptide according to the invention is a polypeptide that includes the amino acid sequence of a NOV1 polypeptide. Unless indicated otherwise, ''NOVX'' is meant to refer to any of the NOV1-23 sequences disclosed herein.

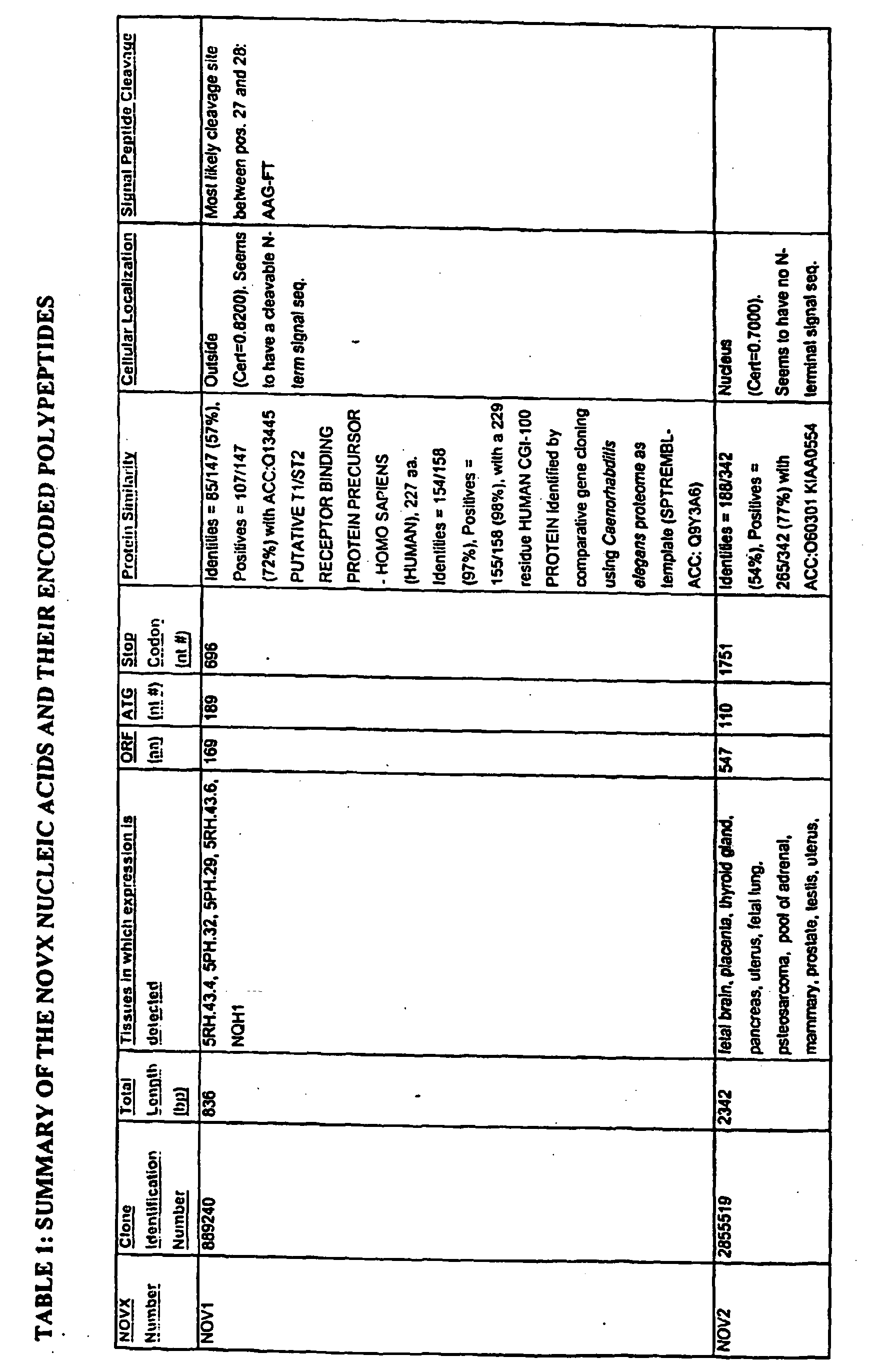

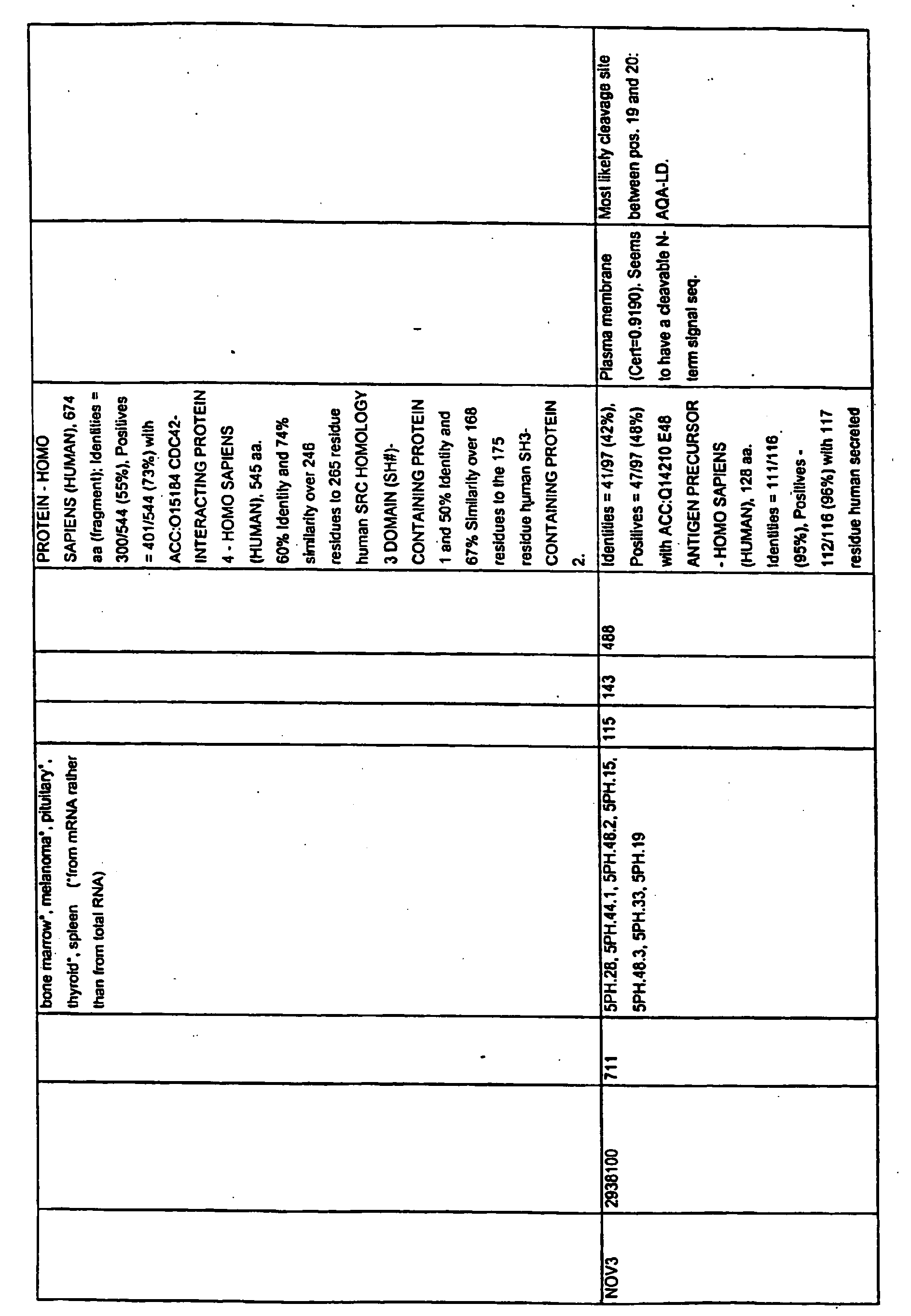

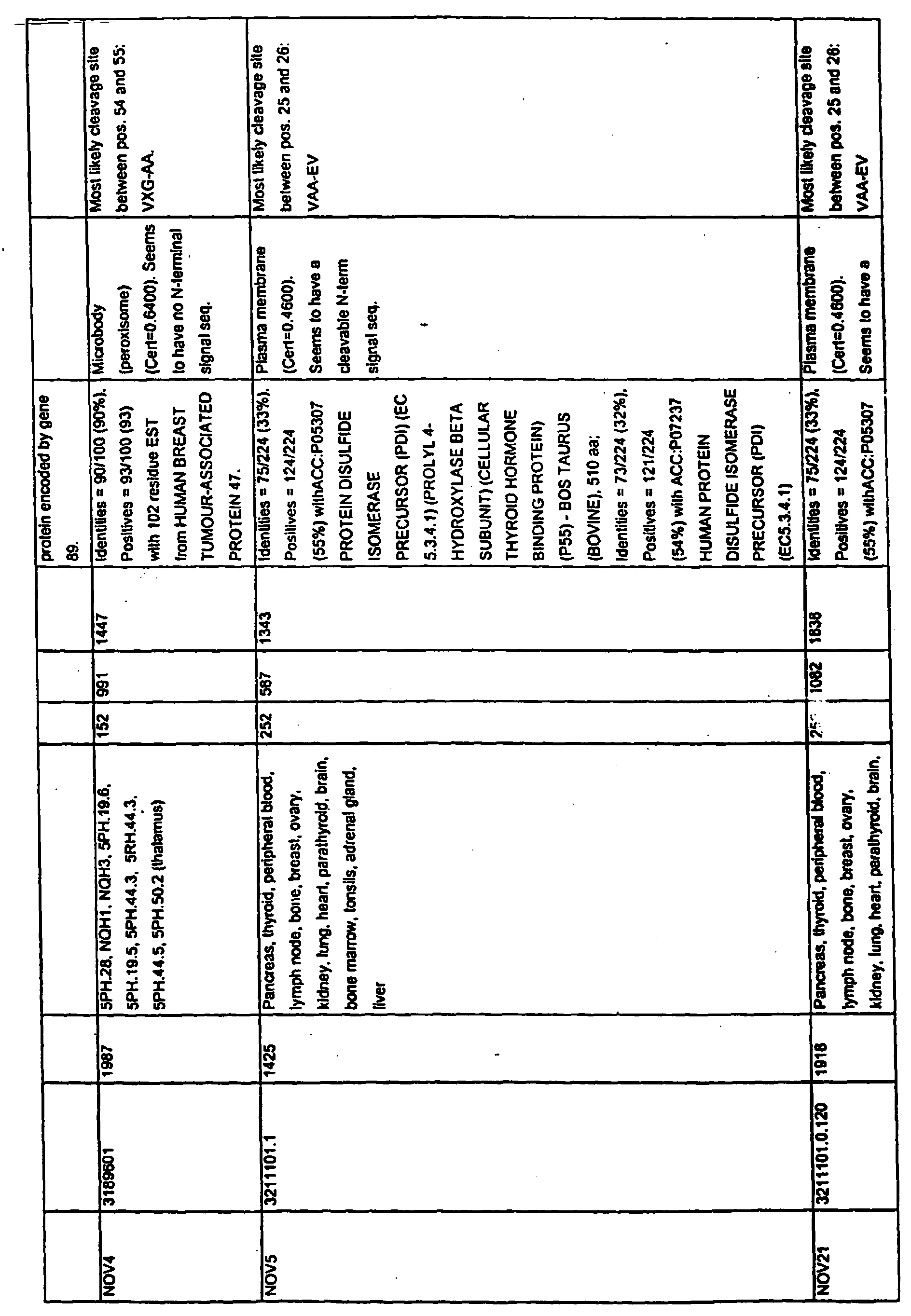

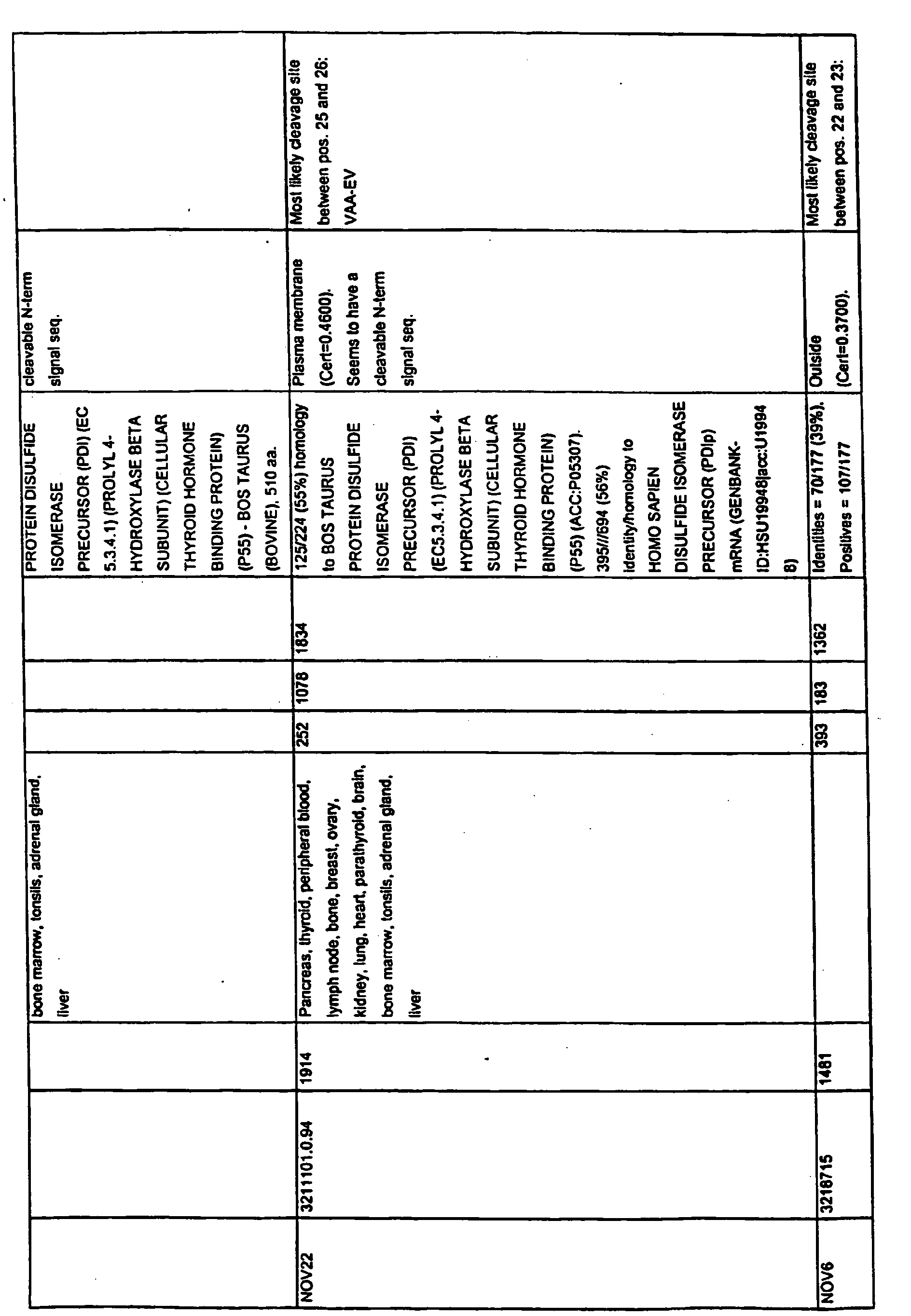

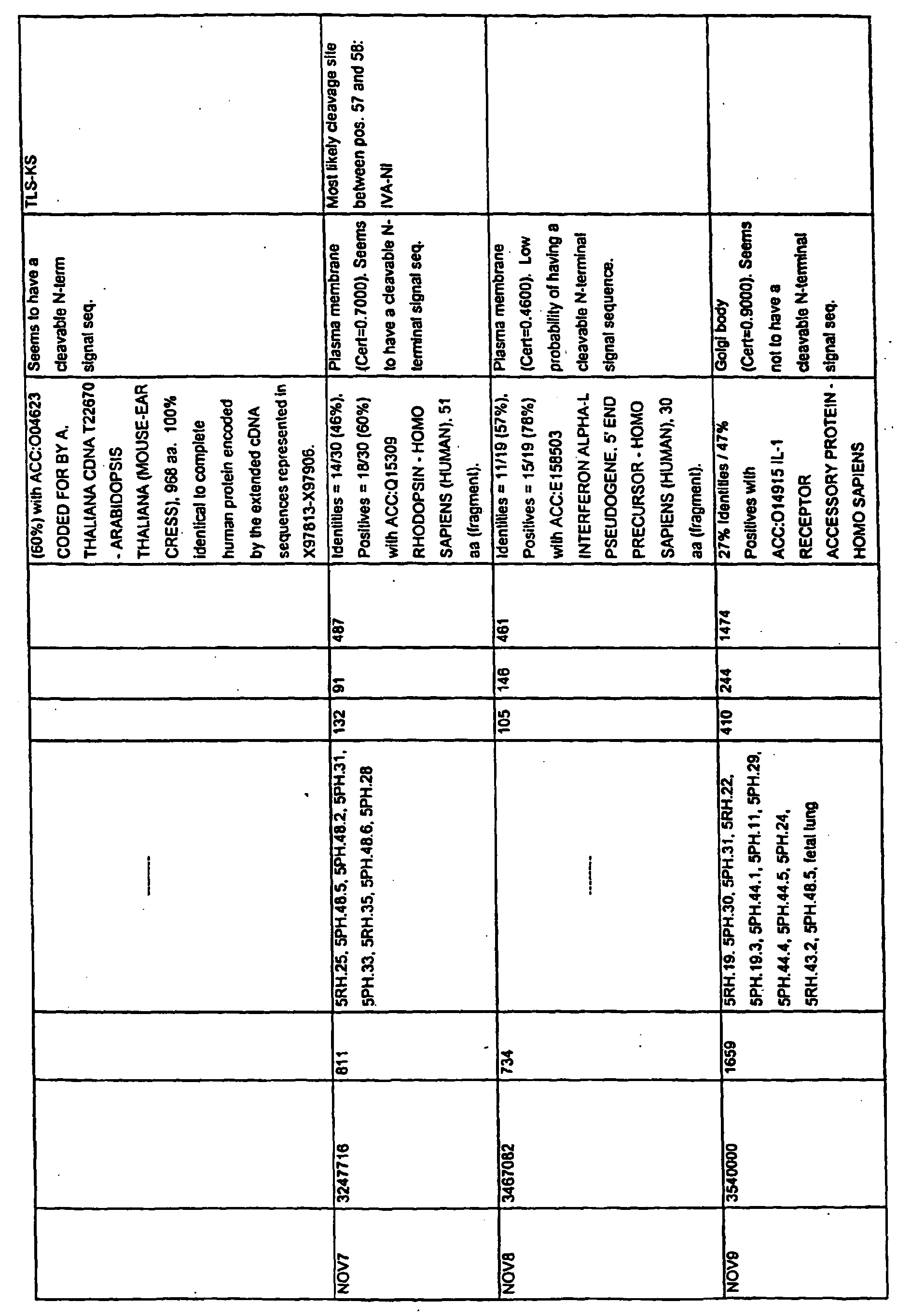

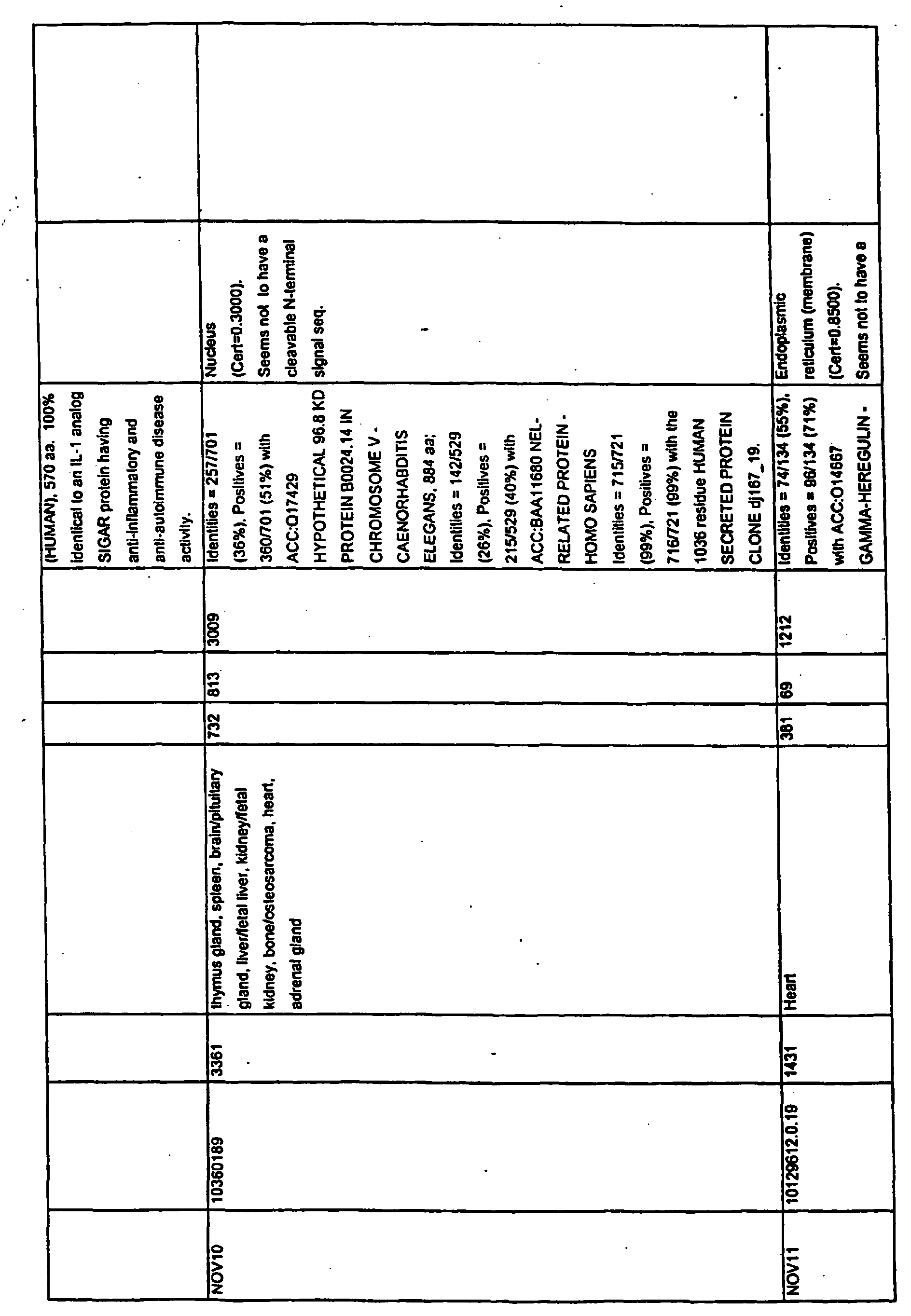

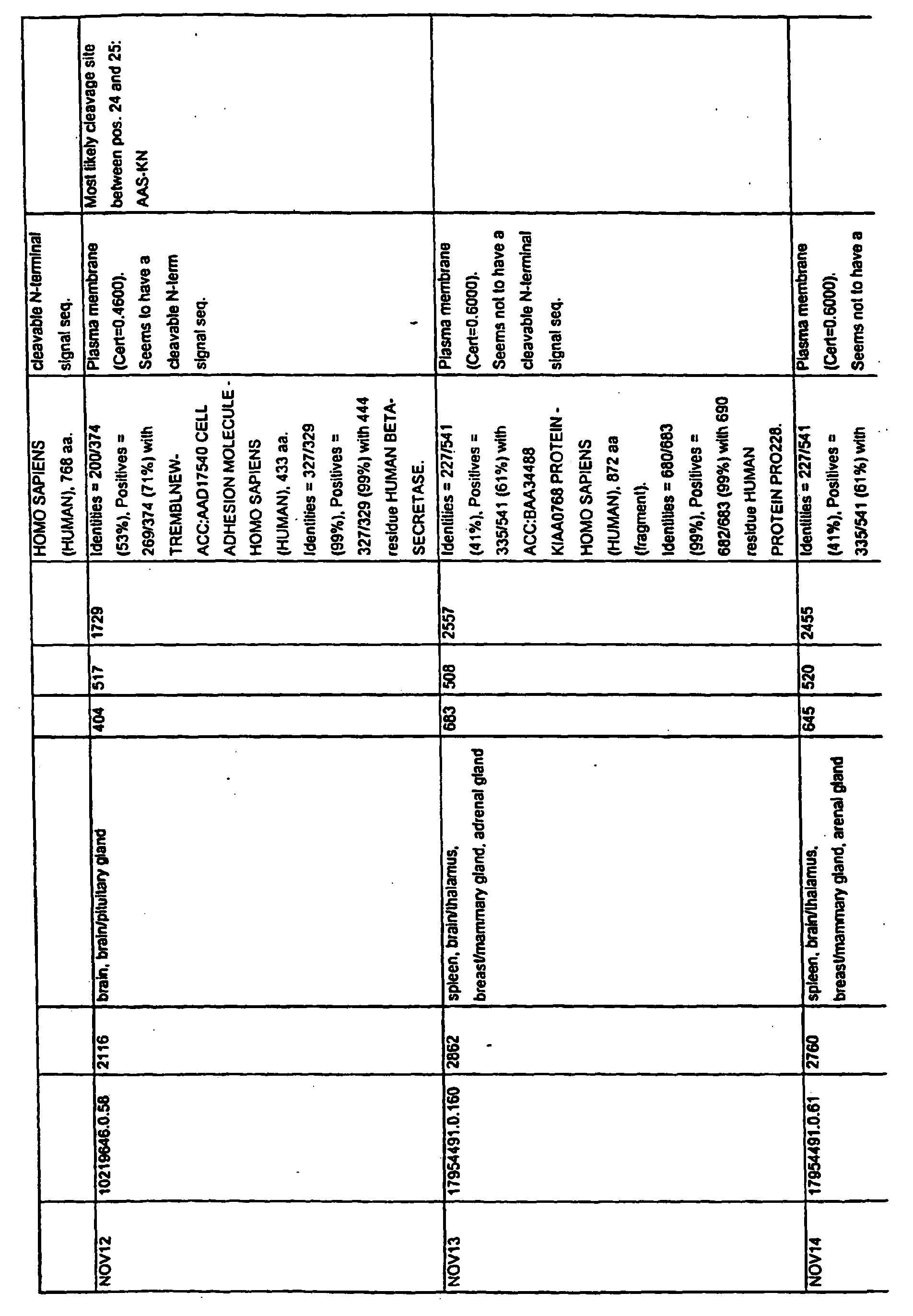

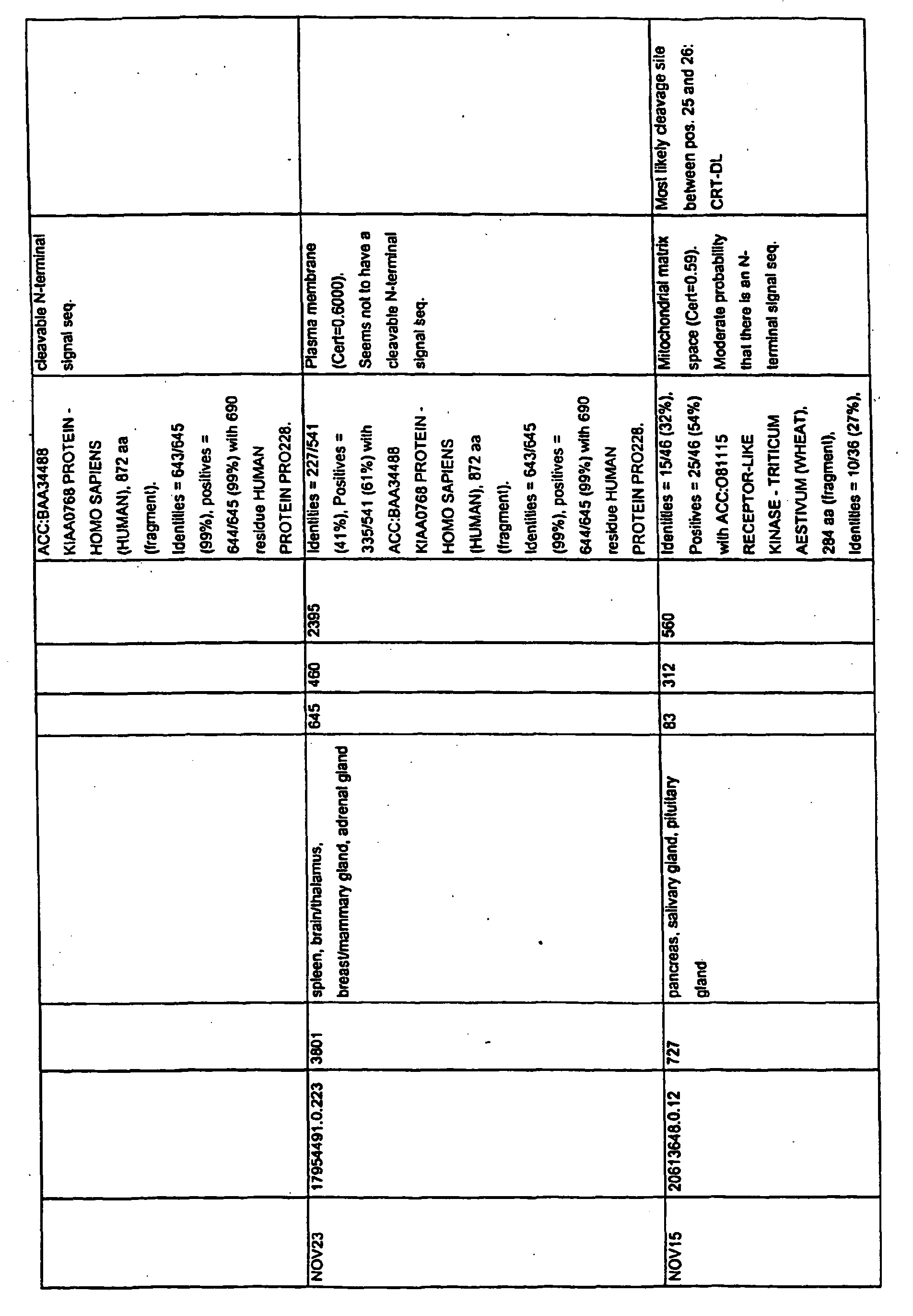

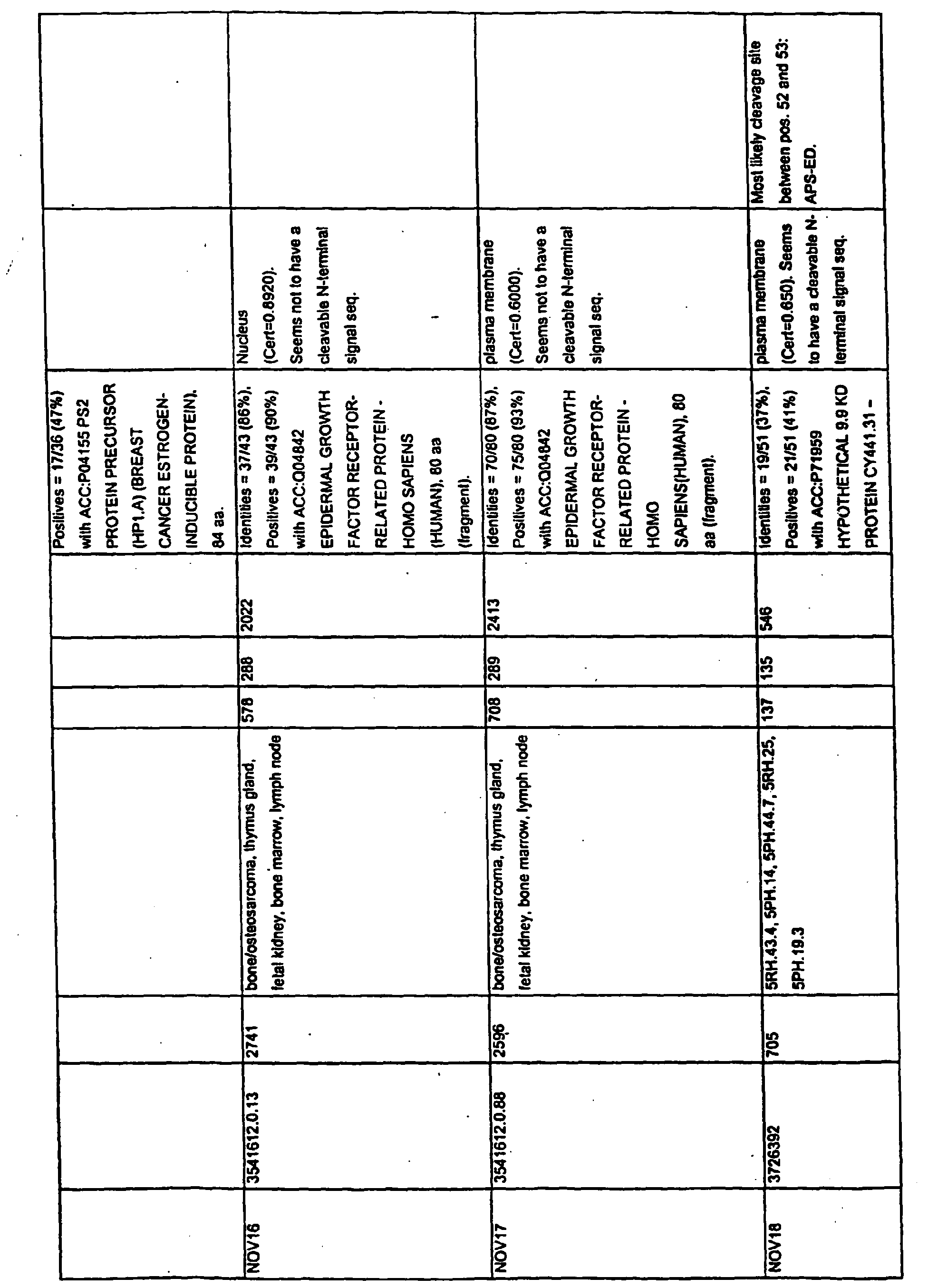

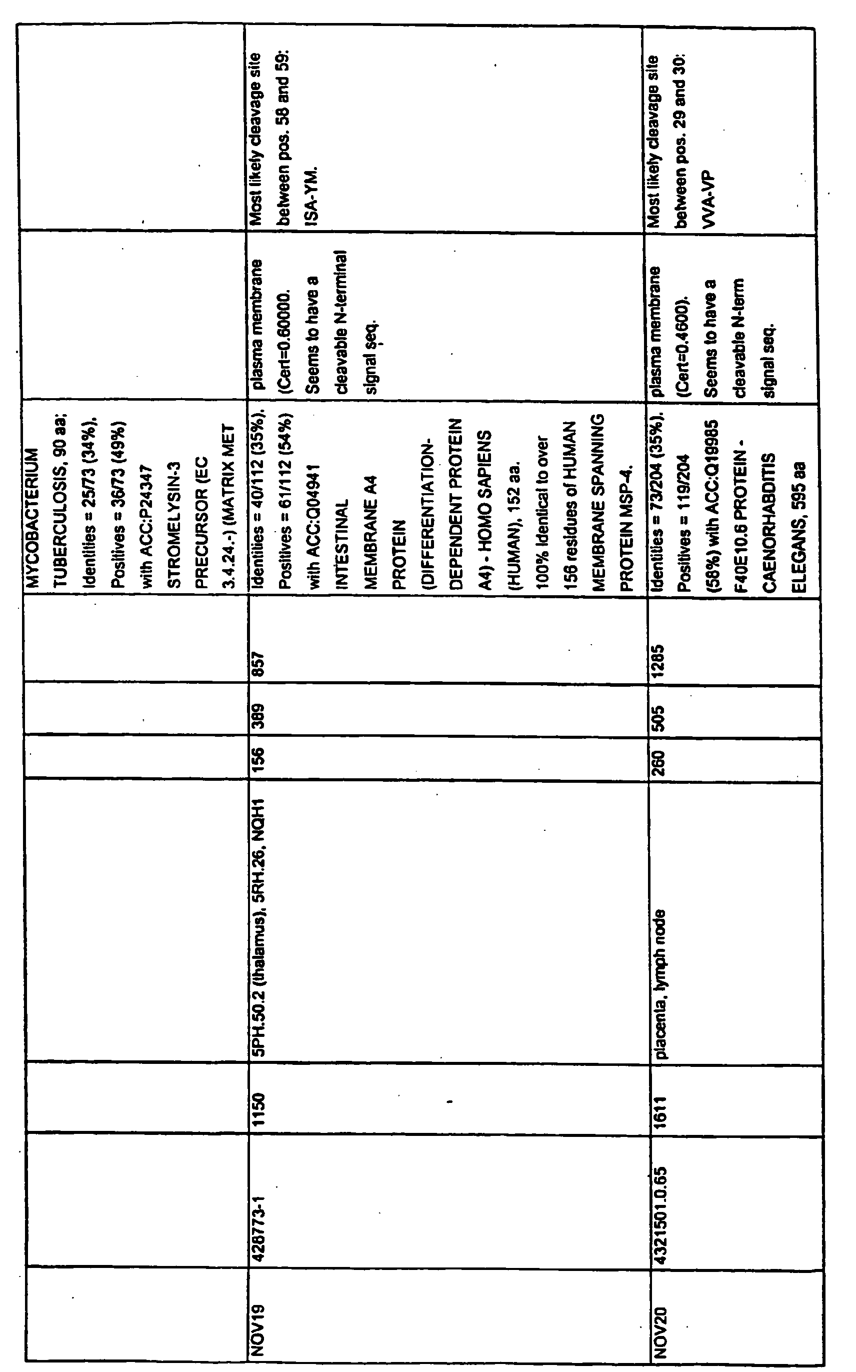

**Table 2:**

| **Tissue Type/Disease Association Information** | | |
|---|---|---|
| **Tissue Name** | **Tissue Information** | **Disease Association** |
| 5PH.11 (Placenta) | Placenta | Infertility, birth defects |
| 5PH.14 (Bone Marrow) | Bone Marrow | Hemophilia, hypercoagulation, Idiopathic thrombocytopenic purpura, autoimmume disease, allergies, immunodeficiencies, transplantation, Graft versus host, |
| 5PH.15 (Bone Marrow) | Bone Marrow | Hemophilia, hypercoagulation, Idiopathic thrombocytopenic purpura, autoimmume disease, allergies, immunodeficiencies, transplantation, Graft versus host, |
| 5PH.19 (One Fetal tissue and two cell lines) | Mixed | |
| 5PH.19.3 (osteogenic sarcoma cell lines -HTB96) | Osteogenic Sarcoma | Sarcomas, osteoporosis, osteopctrosis |
| 5PH.19.5 (Heart) | Heart | Cardiomyopathy, Atherosclerosis, Hypertension, Congenital heart defects, Aortic stenosis, Atrial septal defect (ASD), Atrioventricular (A-V) canal defect, Ductus arteriosus, Pulmonary stenosis, Subaortic stenosis, Ventricular septal defect (VSD), valve diseases, Tuberous sclerosis, Scleroderma, Obesity, Transplantation |
| 5PH.19.6 (Spleen) | Spleen | Hemophilia, Hypercoagulation, Idiopathic thrombocytopenic purpura, Immunodeficiencies, Graft versus host |
| 5PH.24 (Pancreas) | Pancreas | Pancreatitis, diabetes, pancreatic cancer |
| 5PH.28 (Heart) | Heart | Cardiomyopathy, Atherosclerosis, Hypertension, Congenital heart defects, Aortic stenosis ,Atrial septal defect (ASD),Atrioventricular (A-V) canal defect, Ductus arteriosus , Pulmonary stenosis , Subaortic stenosis, Ventricular septal defect (VSD), valve diseases, Tuberous sclerosis, Scleroderma, Obesity, Transplantation |
| SPH.29 (Fetal Kidney) | Fetal Kidney | Diabetes, Autoimmune disease, Renal artery stenosis, Interstitial nephritis, Glomerulonephrtis, Polycystic kidney disease, Systemic lupus erythematosus, Renal tubular acidosis, IgA nephropathy, Hypercalceimia, Lesch-Nyhan syndrome |
| 5PH.30 (Lymph Node) | Lymph Node | Lymphedema, Allergies |
| 5PH.31 (P)ancreas) | Pancreas | Pancreatitis, diabetes, pancreatic cancer |
| 5PH.32 (Thyroid) | Thyroid | Hyperthyroidism and Hypothyroidism |
| SPH.33 (Fetal Brain) | Fetal brain | Von Hippel-Lindau (VHL) syndrome, Alzheimer's disease, Stroke, Tuberous sclerosis, hypercalceimia, Parkinson's disease, Huntington's disease, Cerebral palsy, Epilepsy, Lesch-Nyhan syndrome, Multiple sclerosis, Ataxia-telangiectasia, Leukodystrophies, Behavioral disorders, Addiction, Anxiety, Pain, Neuroprotection |
| 5PH.44.1 (Kidney) | Kidney | Diabetes, Autoimmune disease, Renal artery stenosis, Interstitial nephritis, Glomerulonephritis, Polycystic kidney disease, Systemic lupus erythematosus, Renal tubular acidosis, IgA nephropathy, Hypercatceimia, Lesch-Nyhan syndrome |
| 5PH.443 (Heart) | Heart | Cardiomyopathy, Atherosclerosis, Hypertension, Congenital heart defects, Aortic stenosis ,Atrial septal defect (ASD),Atrioventricular (A-V) canal defect, Ductus arteriosus , Pulmonary stenosis , Subaortic stenosis, Ventricular septal defect (VSD), valve diseases, Tuberous sclerosis, Scleroderma, Obesity, Transplantation |
| 5PH.44.4 (Prostate) | Prostate | Prostate Cancer |
| 5PH.44.5 (Spleen) | Spleen | Hemophilia, Hypercoagulation, Idiopathic thrombocytopenic purpura , Immunodeficiencies, Graft versus host |
| 5PH.44.7 (Uterus) | Uterus | Infertility, birth defects |
| 5PH.48.2 (Thalamus-Brain) | Thalamus | Von Hippel-Lindau (VHL) syndrome , Alzheimer's disease, Stroke, Tuberous sclerosis, hypercalceimia, Parkinson's disease, Huntington's disease, Cerebral palsy, Epilepsy, Lesch-Nyhan syndrome, Multiple sclerosis, Ataxia-telangiectasia, Leukodystrophies, Behavioral disorders, Addiction, Anxiety, Pain, Neuroprotection |
| 5PH.48.3 (Adrenal Gland) | Adrenal Gland/Suprarenal gland | Adrenoleukodystrophy , Congenital Adrenal Hyperplasia, |
| 5PH.48.5 (Salivary Gland) | Salivary Gland | Dry mouth, infection |
| 5PH.48.6 (Mammary Gland) | Mammary Gland | Lactation disorders, breast cancer |
| 5PH.50.2 (thalamus) | Thalamus | Von Hippel-Lindau (VHL) syndrome , Alzheimer's disease, Stroke, Tuberous sclerosis, hypercalceimia, Parkinson's disease, Huntington's disease, Cerebral palsy, Epilepsy, Lesch-Nyhan syndrome, Multiple sclerosis, Ataxia-telangiectasia, Leukodystrophies, Behavioral disorders, Addiction, Anxiety, Pain, Neuroprotection |
| 5RH.19 (Fetal Brain) | Fetal brain | Von Hippel-Lindau (VHL) syndrome, Alzheimer's disease, Stroke, Tuberous sclerosis, hypercalceimia, Parkinson's disease, Huntington's disease, Cerebral palsy, Epilepsy, Lesch-Nyhan syndrome, Multiple sclerosis, Ataxia-telangiectasia, Leukodystrophies, Behavioral disorders, Addiction, Anxiety, Pain, Neuroprotection |
| 5RH.22 (Placenta) | Placenta | Infertility, birth defects |
| 5RH.25 (Fetal Brain) | Fetal brain | Von Hippel-Lindau (VHL) syndrome , Alzheimer's disease, Stroke, Tuberous sclerosis, hypercalceimia, Parkinson's disease, Huntington's disease, Cerebral palsy, Epilepsy, Lesch-Nyhan syndrome, Multiple sclerosis, Ataxia-telangiectasia, Leukodystrophies, Behavioral disorders, Addiction, Anxiety, Pain, Neuroprotection |
| 5RH.26 (Bone Marrow) | Bone Marrow | Hemophilia, Hypercoagulation, Idiopathic thrombocytopenic purpura, autoimmume disease, allergies, immunodeficiencies, transplantation, Graft versus host, |
| SRH.35 (Pancreas) | Pancreas | Pancreatitis, diabetes, pancreatic cancer |
| 5RH.43.2 (hematopoetic stem cells - CRL2043) | Hematopoeitic stem cells | Leukemia, osteoporosis, post-chemotherapeutic stem cell repopulation |
| 5RH.43.4 (Fetal Liver) | Fetal Liver | Von Hippel-Lindau (VHL) syndrome, Cirrhosis, Transplantation |
| SRH.43.6 (Spleen) | Spleen | Hemophilia, Hypercoagulation, Idiopathic thrombocytopenic purpura, Immunodeficiencies, Graft versus host |
| 5RH.44.3 (Heart) | Heart | Cardiomyopathy, Atherosclerosis, Hypertension, Congenital heart defects, Aortic stenosis ,Atrial septal defect (ASD),Atrioventricular (A-V) canal defect, Ductus arteriosus, Pulmonary stenosis , Subaortic stenosis, Ventricular septal defect (VSD), valve diseases, Tuberous sclerosis, Scleroderma, Obesity, Transplantation |
| NQH 1 (Mixture of eight adult & two fetal tissues) | | |
| NQH3 (Bone Marrow) | Bone Marrow | Hemophilia, hypercoagulation, Idiopathic thrombocytopenic purpura, autoimmume disease, allergies, immunodeficiencies, transplantation, Graft versus host, |

Column 1 of Table 1 provides the NOVX assignment for the novel nucleic acids and encoded polypeptides of this invention. Column 2 provides a clone identification number for disclosed sequences corresponding to various NOVX sequences. Column 3 shows the length of a disclosed NOVX nucleic acid. Column 4 provides information about the tissues in which NOVX sequences are expressed. Column 5 shows the length of the polypeptide (in amino acids) encoded by an open reading frame ("ORF") found in disclosed NOVX nucleic acid sequences. Columns 6 and 7 show the nucleotide position of the start (ATG) and stop codons, respectively, of the ORF. Column 8 contains protein similarity information for each of the polypeptides of the invention. Column 9 provides the predicted cellular localization of each polypeptide, and column 10 shows the most likely site for signal peptide cleavage.

NOVX nucleic acids, and their encoded polypeptides, according to the invention are useful in a variety of applications and contexts. For example, various NOVX nucleic acids and polypeptides according to the invention are useful based on their relatedness to previously described proteins, as summarized in Column 8 of Table 1.

NOVX nucleic acids can also be used to identify a cell in a cell sample. For example, identification of an RNA species homologous to a given NOVX nucleic acid indicates the tissue is one of those identified in Table 1, column 4, for the given NOVX. Similarly, detection of a NOVX polypeptide in a cell sample indicates that the sample includes one or more of the cell types indicated in Table 1, column 4, for the particular NOVX polypeptide.

For each polypeptide listed in Table 1, the noncoding regions are those regions of the polypeptide that do not fall within the ORF. For example, for the disclosed NOV1 nucleic acid sequence, noncoding regions extend from nucleotides 1-168 and nucleotides 696-836. Similarly, for the disclsoed NOV2 nucleic acid sequence, the noncoding regions extend from nucleotides 1-110 and 1751-2342. From these examples, along with the information presented in Table 1, a person of ordinary skill in this art can determine the locations of the noncoding regions for each of NOV1-23.

Table 2 provides explanatory information for some of the tissue types provided in Column 4 of Table 1. Column 1 of Table 2 identifies the tissue name. Specifically, Column 1 of Table 2 corresponds to the tissue name abbreviations used in Column 4 of Table 1. Column 2 of Table 2 identifies the origin of the particular tissue type. Finally, Column 3 of Table 2 provides information about any disease association connected with a particular tissue type.

**TABLE 3:**

| **SEQ ID NO ASSIGNMENTS** | | | |
|---|---|---|---|
| **NOVX ASSIGNMENT** | **CLONE IDENTIFICATION NUMBER** | **NUCLEOTIDE SEQ ID NO:** | **POLYPEPTIDE SEQ ID NO:** |
| NOV1 | 889240 | SEQ ID NO:1 | SEQ ID NO:2 |
| NOV2 | 2855519 | SEQ ID NO:3 | SEQ ID NO:4 |
| NOV3 | 2938100 | SEQ ID NO:5 | SEQ ID NO:6 |
| NOV4 | 3189601 | SEQ ID NO:7 | SEQ ID NO:8 |
| NOV5 | 3211101 | SEQ ID NO:9 | SEQ ID NO:10 |
| NOV6 | 3218715 | SEQ ID NO:11 | SEQ ID NO:12 |
| NOV7 | 3247716 | SEQ ID NO:13 | SEQ ID NO:14 |
| NOV8 | 3467082 | SEQ ID NO:15 | SEQ ID NO:16 |
| NOV9 | 3540000 | SEQ ID NO:17 | SEQ ID NO:18 |
| NOV10 | 10360189 | SEQ ID NO:19 | SEQ ID NO:20 |
| NOV11 | 10129616.0.19 | SEQ ID NO:21 | SEQ ID NO:22 |
| NOV12 | 10219646.0.58 | SEQ ID NO:23 | SEQ ID NO:24 |
| NOV13 | 17954491.0.160 | SEQ ID NO:25 | SEQ ID NO:26 |
| NOV14 | 17954491.0.61 | SEQ ID NO:27 | SEQ ID NO:28 |
| NOV15 | 20613648.0.12 | SEQ ID NO:29 | SEQ ID NO:30 |
| NOV16 | 3541612.0.13 | SEQ ID NO:31 | SEQ ID NO:32 |
| NOV17 | 3541612.0.88 | SEQ ID NO:33 | SEQ ID NO:34 |
| NOV18 | 3726392 | SEQ ID NO:35 | SEQ ID NO:36 |
| NOV19 | 428773-1 | SEQ ID NO:37 | SEQ ID NO:38 |
| NOV20 | 4321501.0.65 | SEQ ID NO:39 | SEQ ID NO:40 |
| NOV21 | 3211101.0.120 | SEQ ID NO:41 | SEQ ID NO:42 |
| NOV22 | 3211101.0.94 | SEQ ID NO:43 | SEQ ID NO:44 |
| NOV23 | 17954491.0.223 | SEQ ID NO:45 | SEQ ID NO:46 |

Table 3 provides the SEQ ID NOs for disclosed NOVX nucleic acid sequences and encoded polypeptide sequences according to the invention. Column 1 of Table 3 provides the NOVX assignment of each of the identified sequences, while column 2 shows a clone identification number for each NOVX sequence. Column 3 displays the SEQ ID NOs assigned for the disclosed NOV:1-23 nucleic acid sequences. Finally, Column 4 displays the SEQ ID NOs assigned to the encoded polypeptides.

The sequence of various NOVX nucleic acids and encoded polypeptides according to the invention are as follows:

NOV1 (SEQ ID NOS:1&2)

Translated Protein - Nucleotide 189 to 695

1      CAGAGTCCCTACCCTTTGGAGAACTGCGCTTCTCTTTCGGAGGGA
46     GTGTTCGCCGCCGCCGCGGCCGCCACCTGGAGTTTCTTCAGACTC
91     CAGATTTCCCTGTCAACCACGAGGAGTCCAGAGAGGAAACGCGGA
136    GCGGAGACAACAGTACCTGACGCCTCTTTCAGCCCGGGATCGCCC
181    CAGCAGGGATGGGCGACAAGATCTGGCTGCCCTTCCCCGTGCTCC
               MetGlyAspLysIleTrpLeuProPheProValLeuL
226    TTCTGGCCGCTCTGCTTCGGGTGCTGCTGCCTGGGGCGGCCGGCT
       euLeuAlaAlaLeuLeuArgValLeuLeuProGlyAlaAlaGlyP
271    TCACACCTTCCCTCGATAGCGACTTCACCTTTACCCTTCCCGCCG
       heThrProSerLeuAspSerAspPheThrPheThrLeuProAlaG
316    GCCAGAAGGAGTGCTTCTACCAGCCCATGCCCCTGAAGGCCTCGC
       lyGlnLysGluCysPheTyrGlnProMetProLeuLysAlaSerL
361    TGGAGATCGAGTACCAAGTTTTAGATGGAGCAGGATTAGATATTG
       euGluIleGluTyrGlnValLeuAspGlyAlaGlyLeuAspIleA
406    ATTTCCATCTTACCTCTCCAGAAGGCAAAACCTTAGTTTTTGAAC
       spPheHisLeuThrSerProGluGlyLysThrLeuValPheGluG
451    AAAGAAAATCAGATGGAGTTCACACTGTAGAGACTGAAGTTGGTG
       lnArgLysSerAspGlyValHisThrValGluThrGluValGlyA
496    ATTACATGTTCTGCTTTGACAATACATTCAGCACCATTTCTGAGA
       spTyrMetPheCysPheAspAsnThrPheSerThrIleSerGluL

541 AGGTGATTTTCTTTGAATTAATCCTGGATAATATGGAGAACAGG
ysValIlePhePheGluLeuIleLeuAspAsnMetGlyGluGlnA
586 CACAAGAACAAGAAGATTGGAAGAAATATATTACTGGCACAGATA
laGlnGluGlnGluAspTrpLysLysTyrIleThrGlyThrAspI
631 TATTGGATATGAAACTGGAAGACATCCTGGACCTGCCCGGCGGC
leLeuAspMetLysLeuGluAspIleLeuAspLeuProGlyArgP
676 CGCTCGAGCCCTATAGTGAGTAAGTCTGGAGGCCCGGCGGCCGC
roLeuGluProTyrSerGlu (SEQ ID NO:2)
721 TCCTGCAGTAGGGTACCGAGCTCGTCGACGCATGCTGATCTAGAT
766 CTTAATTAACACGTGGTGCCAAGCTTTGGAAGACTCAGCTTTTGT
811 TCCCTTTAGTGAGGGTTAATTTCGAG (SEQ ID NO:1)

NOV2(SEQ ID NOS:3&4)
Translated Protein - Nucleotide 110 to 1750

1 AGACCCGCTGAGCTGCTAGCCCGCCGGCCAGCGAGTGAGAGGTCG
46 GACAGACTGTGGAGCCGACAGACTGAAGGACAGCGGCACCGCCAG
91 ACGGCCAGAAAGTTCCGCCATGAGCTGGGGCACGGAGCTGTGGA
MetSerTrpGlyThrGluLeuTrpAs
136 TCAGTTCGACAGCTTAGACAAGCATACACAATGGGGAATTGACTT
pGlnPheAspSerLeuAspLysHisThrGlnTrpGlyIleAspPh
181 CTTGGAAAGATATGCCAAATTTGTTAAAGAGAGGATAGAAATTGA
eLeuGluArgTyrAlaLysPheValLysGluArgIleGluIleGl
226 ACAGAACTATGCGAAACAATTGAGAAATCTGGTTAAGAAGTACTG
uGlnAsnTyrAlaLysGlnLeuArgAsnLeuValLysLysTyrCy
271 CCCCAAACGTTCATCCAAAGATGAAGAGCCACGGTTTACCTCGTG
sProLysArgSerSerLysAspGluGluProArgPheThrSerCy
316 TGTAGCCTTTTTTAATATCCTTAATGAGTTAAATGACTATGCAGG
sValAlaPhePheAsnIleLeuAsnGluLeuAsnAspTyrAlaGl
361 ACAGCGAGAAGTTGTAGCAGAAGAAATGGCGCACAGAGTGTATGG
yGlnArgGluValValAlaGluGluMetAlaHisArgValTyrGl
406 TGAATTAATGAGACATGCTCATGATCTGAAAACTGAAAGAAAAAT
yGluLeuMetArgHisAlaHisAspLeuLysThrGluArgLysMe
451 GCATCTGCAAGAAGGCCGAAAAGCTCACCAATCTCTTGCCATGTG

tHisLeuGlnGluGlyArgLysAlaHisGlnSerLeuAlaMetCy
496 CTGGAACCAGATGGATAATAGTAAAAAGAAGTTTGAAAGAGAATG
sTrpAsnGlnMetAspAsnSerLysLysLysPheGluArgGluCy
541 TAGAGAGGCAGAAAAGGCCCACCAGAGTTATGAAAGATTGGATAA
sArgGluAlaGluLysAlaHisGlnSerTyrGluArgLeuAspAs
586 TGATACTAATGCAACCAAGGCAGATGTTGAAAATGCCAAACAGCA
nAspThrAsnAlaThrLysAlaAspValGluAsnAlaLysGlnGl
631 GTTGAATCTGCGTACGCATATGGCCGATGAAAATAAAAATGCATA
nLeuAsnLeuArgThrHisMetAlaAspGluAsnLysAsnAlaTy
676 TGCTGCACAATTACAAAACTTTAATGGAGAACAACATAAACATTT
rAlaAlaGlnLeuGlnAsnPheAsnGlyGluGlnHisLysHisPh
721 TTATGTAGTGATTCCTCAGATTTACAAGCAACTACAAGAAATGGA
eTyrValValIleProGlnIleTyrLysGlnLeuGlnGluMetAs
766 CGAACGAAGGACTATTAAACTCAGTGAGTGTTACAGAGGATTTGC
pGluArgArgThrIleLysLeuSerGluCysTyrArgGlyPheAl
811 TGACTCAGAACGCAAAGTTATTCCCATCATTTCAAAATGTTTGGA
aAspSerGluArgLysValIleProIleIleSerLysCysLeuGl
856 AGGAATGATTCTTGCAGCAAAATCAGTTGATGAAAGAAGAGACTC
uGlyMetIleLeuAlaAlaLysSerValAspGluArgArgAspSe
901 TCAAATGGTGGTAGACTCCTTCAAATCTGGTTTTGAACCTCCAGG
rGlnMetValValAspSerPheLysSerGlyPheGluProProGl
946 AGACTTTCCATTTGAAGATTACAGTCAACATATATATAGAACCAT
yAspPheProPheGluAspTyrSerGlnHisIleTyrArgThrIl
991 TTCTGATGGGACTATCAGTGCATCCAAACAGGAGAGTGGGAAGAT
eSerAspGlyThrIleSerAlaSerLysGlnGluSerGlyLysMe
1036 GGATGCCAAAACCCCAGTAGGAAAGGCCAAGGGCAAATTGTGGCT
tAspAlaLysThrProValGlyLysAlaLysGlyLysLeuTrpLe
1081 CTTTGGAAAGAAGCCAAAGGGCCCAGCACTAGAAGATTTCAGTCA
uPheGlyLysLysProLysGlyProAlaLeuGluAspPheSerHi
1126 TCTGCCACCAGAACAGAGACGTAAAAAACTACAGCAGCGCATTGA
sLeuProProGluGlnArgArgLysLysLeuGlnGlnArgIleAs
1171 TGAACTTAACAGAGAACTACAGAAAGAATCAGACCAAAAAGATGC
pGluLeuAsnArgGluLeuGlnLysGluSerAspGlnLysAspAl
1216 ACTCAACAAAATGAAAGATGTATATGAGAAGGATCCACAAATGGG

aLeuAsnLysMetLysAspValTyrGluLysAspProGlnMetGl
1261 GGATCCAGGGAGTTTGCAGCCTAAATTAGCAGAGACCATGAATAA
yAspProGlySerLeuGlnProLysLeuAlaGluThrMetAsnAs
1306 CATTGACCGCCTACGAATGGAAATCCATAAGAATGAGGCTTGGCT
nIleAspArgLeuArgMetGluIleHisLysAsnGluAlaTrpLe
1351 CTCTGAAGTCGAAGGCAAAACAGGTGGGAGAGGAGACAGAAGACA
uSerGluValGluGlyLysThrGlyGlyArgGlyAspArgArgHi
1396 TAGCAGTGACATAAATCATCTTGTAACACAGGGACGAGAAAGTCC
sSerSerAspIleAsnHisLeuValThrGlnGlyArgGluSerPr
1441 TGAGGGAAGTTACACTGATGATGCAAACCAGGAAGTCCGTGGGCC
oGluGlySerTyrThrAspAspAlaAsnGlnGluValArgGlyPr
1486 ACCCCAGCAGCATGGTCACCACAATGAGTTTGATGATGAATTTGA
oProGlnGlnHisGlyHisHisAsnGluPheAspAspGluPheGl
1531 GGATGATGATCCCTTGCCTGCTATTGGACACTGCAAAGCTATCTA
uAspAspAspProLeuProAlaIleGlyHisCysLysAlaIleTy
1576 CCCTTTTGATGGACATAATGAAGGTACTCTAGCAATGAAAGAAGG
rProPheAspGlyHisAsnGluGlyThrLeuAlaMetLysGluGl
1621 TGAAGTTCTCTACATTATAGAGGAGGACAAAGGTGACGGATGGAC
yGluValLeuTyrIleIleGluGluAspLysGlyAspGlyTrpTh
1666 AAGAGCTCGGAGACAGAACGGTGAAGAAGGCTACGTTCCCACGTC
rArgAlaArgArgGlnAsnGlyGluGluGlyTyrValProThrSe
1711 ATACATAGATGTAACTCTAGAGAAAAACAGTAAAGGTTCCTGAAG
rTyrIleAspValThrLeuGluLysAsnSerLysGlySer (SEQ ID NO:4)
1756 AGGGTTTCTGAGGAAATGGGCAAGATGTTGAAGGAGGTTACATGC
1801 AGCTGCTTTTGGGGGAGGGTATTAGAGTTGTCAGGCTCAAAGAGA
1846 GTGAGAGAAGCAAGTTGCATGAGTGCATGCAGACATGATTTTTTT
1891 TTTACTAACTTCATTAGCATTTCCATACATTGTTTTTAAAAATCA
1936 TAATACCAACCCTTAAGTTCCTAGTTCACAGTTATTCCCACAAAA
1981 GAAAAAGCCAACAATAGTGTACCATTTTTCTATTTTATTTTATTG
2026 CTGTCTAATCAATAAAGAATGCAGAGCTGTCAAAAAATGTGTCTT
2071 ACATTTAGCTGTCCCAACAGGATTGTCTTCCCTCCCAGCTCTGGT
2116 TTTAATTGGCTTTTAGACCCACTATCTGTCAGATCCTTGCCATCT
2161 GTCAGTGTCTGCCTGCGCCACCTCCGTGCTTGCCTAACATCCTGT
2206 TGCATGTCTAGCGTGATTGAGCNAGATTTTCAGGCATGTCTTTAG

2251 AATCCCCTGGTNCTGTCAAAGCCTGGTTTGGTTTACATTGGTNGT
2296 GCAATCNCTTTGTCAACATCTCCAGCACTATNGTTCCNTCTTAGG
2341 TN (SEQ ID NO:3)

NOV3 (SEQ ID NOS:5&6)
Translated Protein - Nucleotide 143 to 487

1 GCGAATTGGCTTCCGAGTGAAAATCACCAGCCGGCCCCAGTCTTT
46 GGCCCCCTGAGTTGGATCCTTTGCGCGCCACCCTGAGTTGGATCC
91 AGGGTAGCTGCTGTTGACCTCCCCACTCCCACGCTGCCTCCTGCC
136 TGCAGCCATGACGCCCTGCTCACCTGATCTGGTGGTCCTCATGGG
MetThrProCysSerProAspLeuValValLeuMetGl
181 CTTACCTCTGGCCCAGGCCTTGGACTGCCACGTGTGTGCCTACAA
yLeuProLeuAlaGlnAlaLeuAspCysHisValCysAlaTyrAs
226 CGGAGACAACTGCTTCAACCCCATGCGCTGCCCGGCTATGGTTGC
nGlyAspAsnCysPheAsnProMetArgCysProAlaMetValAl
271 CTACTGCATGACCACGCGCACCTACTACACCCCCACCAGGATGAA
aTyrCysMetThrThrArgThrTyrTyrThrProThrArgMetLy
316 GGTCAGTAAGTCCTGCGTGCCCCGCTGCTTCGAGACTGTGTATGA
sValSerLysSerCysValProArgCysPheGluThrValTyrAs
361 TGGCTACTCCAAGCACGCGTCCACCACCTCCTGCTGCCAGTACGA
pGlyTyrSerLysHisAlaSerThrThrSerCysCysGlnTyrAs
406 CCTCTGCAACGGCACCGGCCTTGCCACCCCGGCCACCCTGGCCCT
pLeuCysAsnGlyThrGlyLeuAlaThrProAlaThrLeuAlaLe
451 GGCCCCCATCCTCCTGGCCACCCTCTGGGGTCTCCTCTAAAGCCC
uAlaProIleLeuLeuAlaThrLeuTrpGlyLeuLeu (SEQ ID NO:6)
496 CCGAGGCAGACCCACTCAAGAACAAAGCTCTCGAGACACACTGCT
541 ACACCCTCGCACCCAGCTCACCCTGCCTCACCCTCCACACTCCCT
586 GCGACCTCCTCAGCCATGCCCAGGGTCAGGACTGTGGGCAAGAAG
631 ACACCCGACCTCCCCCAACCACCACACGACCTCACTTCGAGGCCT
676 TGACCTTTAAATAAAAAAAAAAAAAAAAAAAAAA (SEQ ID NO:5)

NOV4 (SEQ ID NOS:7&8)
Translated Protein - Nucleotide 991 to 1446

1 TGAATGAAGGCCTGTCCATGCCTCGTCAAAGAGCCCATCCCAACC
46 AAGCCCCGGTGGGCAGGCAACATCAAGACCCTAGGAGACGCCTAT
91 GAGTTTGCGGTGGACGTGAGAGACTTCTCACCTGAAGACATCATT
136 GTCACCACCTCCAAACAAACCAACATCGAGGTGCGGGGCTGAGAA
181 AGCTGGCGGCTGAACGGACACTGTACATGAAACACCTTACGACTA
226 CACAAGTGCCAGACTGCCGGAGGACGTGGACCCCGAGCGTGCGGT
271 GACCTCGGCTACTGTCGGGAGGACGGACAGCCTCACTAGTCCGGG
316 CACGCGTCACCCGCATACAGAACACGTCCAGCAGACCTTCCGGAC
361 GGAGATCAAAATCTGAGTGCCTCTCCCTTCCCTTTCCCTGTCCCC
406 CCGCCCCACGCCTGCCAGCAAAGCCTCGCTAACCCCATTACAACA
451 GCTCCAGGACATCTCAGCCCAGGTTCTAGCCCCCACGCACCCCAG
496 ACCCCAGGTGGACCATCCTCCCAAACTAGGGCCCTCCACTCTATC
541 CAGGGCAGGCCAGGGACTCCCTGGCCTGACACATGATGCCCAGAT
586 TTCAGATTTGGCCTCCGTCACTTAATCCAGAGTACAGGGGCTGGG
631 GTCAGGGAAGGAAGATCTAAAGAACCCACTGTGGGTCAGGGGAAT
676 GGGACCAGCAGGACATATGGGCAAGCTCTGCAGGACAGACAGACA
721 GACAAACCCTCTGATCTATGAAGTCTCTGCAGGGCAAGGGACCA
766 GGGACCTGGAACCCTCTTGGCCAAGGGGAGTGGAGAGACAGAGG
811 GAAGGTCACAGGCAAGGGTGCCTATCTAAGTGGAACTAATTGCCC
856 GAGGGCTCAGCAAGGCCAAGAGGAGACAGCCGTGACGGTAAACTT
901 CCCCTCTACCAGCCTCCAAGCCCCACGCCAGCGAGCAGGCTGCCT
946 GCCCACCCCGTGCCCCCAGCCAGCTGGCTGTGCCAGGGCAGAGCC
991 ATGCCACATCTGTATATAGATGGGGTTTTTCCAATACAGCTGGTT
MetProHisLeuTyrIleAspGlyValPheProIleGlnLeuVal
1036 CGTGAAAAACTGCATGAAACTCCTGCCGTCCTGCGCCTGCTGGGG
ArgGluLysLeuHisGluThrProAlaValLeuArgLeuLeuGly
1081 CCTCCAGGCAAGGCCAAGTGGGGTTGGGGGTGGGGCTGGTCCTTC
ProProGlyLysAlaLysTrpGlyTrpGlyTrpGlyTrpSerPhe
1126 TCCCTCCCACAGGCCTGTGTTCNTGGGGCTGCTCCCATGCAGACA
SerLeuProGlnAlaCysVal---GlyAlaAlaProMetGlnThr
1171 GGATCACCTAACAGAGATGGAAGCCAGGGCATGGATGGGGCTTTG
GlySerProAsnArgAspGlySerGlnGlyMetAspGlyAlaLeu
1216 GGTCCTCGAGGTTGGACCCCAGCTTCTTGCCACCTTCCCCTCCGG

GlyProArgGlyTrpThrProAlaSerCysHisLeuProLeuArg
1261 CAGTCAGCTCTCCATCCATCCCCCTCTTTAATCTATGAATCTATA
GlnSerAlaLeuHisProSerProSerLeuIleTyrGluSerIle
1306 GGCTCGGTGTGTGTAACAACACACCCCTATCGTTGTCCTTCAAAT
GlySerValCysValThrThrHisProTyrArgCysProSerAsn
1351 ACTCAGCATTACCATTGGTTGAGGCCAAATTCAGAGCTTTCTCAA
ThrGlnHisTyrHisTrpLeuArgProAsnSerGluLeuSerGln
1396 ATCAGATTTACAATCTCCATTTTCATTAACGGGGAAACATCCCCG
IleArgPheThrIleSerIlePheIleAsnGlyGluThrSerPro
1441 AGCCACTGAGTGCTGTGCTTTGTCACTGAAGGTTAGATCTGAACC
SerHis (SEQ ID NO:8)
1486 CAGGGTGTCAACNGCTGCTCTCAACTCCCCACCTCTGGGCACTGA
1531 GGAGTATTTCCCCTCATTCTACCTCTCTAAGGCTATGCACCCCTC
1576 CCCACGTCTTCCAGCTGGGGGATGGGGGAGTCATAGGAAAAGCC
1621 CCCATCTCCCATCTGGGATAGGGACCTTCCATCAGCCTTAACCCT
1666 GGGAAATGCCTGCTGCCCCCAGTGACTCTTGGTTTCGTCTCCCAC
1711 ATACAGAAGCAGGGTGGAGGGGAAGGGTGGGTCTCAGTTAGCAGG
1756 GGTCCCCAGGGCAAGTCAGCCTCCTCCCTCCATGCCTCTCTGGTC
1801 AGTGTGCCTTAGGGTGGCCTCTCACTCCCACCACTCTGGGCCCTT
1846 GGGGGAGGACTGGGGAGGGGGCCGTGGGAGAGCCCTGACGCTGGA
1891 ACCTGTATACACAATAAAGGACAGTCTCACAGACNTCTGGAGGCC
1936 GCCTGCCNGGAGTTCTCAAACTTAGGGCAGGGCNNNNCTTACTTG
1981 AGAGAAA (SEQ ID NO:7)

NOV5 (SEQ ID NOS:9&10)
Translated Protein - Nucleotide 587 to 1342

1 TGTGTGTGTGTGTGTGTGTGTGCGCGTGTGTGTGTGCACGCGTGC
46 GTGCGTGTGTGCACGTGCNNGTGTGTGTGTGGTTGGCAGGCCTAG
91 TGATCCTGTTGTTTAGTGTCTCTGAGATTTGAGTTGTGCCTTTTT
136 ACTTTGCATAAAGTAGATACTTGGCCATATGTAGTTCCAAGGAGA
181 AGTCAGAGTTCCACCTTTGGAGTCTTTCCTTCTGATTCACGATTT
226 TCTTTCAACAATTTTCCACTTAGGAATCCATCACAAAAGTTTTGC
271 ACATGCTCTACGGAAACTTCTGCTGTGGGCAGTGTATCCCACTCG

316 TCATCTAGAGTCTGGTAAATTGCCAAAGCTGGCAGTTGAGACTCC
361 TTTAGTTTGAAAAATGATATCACCTTCCCATTTTCTTTCATACCA
406 CTGTCCACCAGAATAAAGAGAATCTTCCCCTGGAAGAGCTTGGCT
451 GCCTTCTGGTATCTGTGCATGTTCTCTCCATACTCTGGGGAGGCC
496 TTGTTCATTATCAGGAGGAGATGATTCTGAATTACGCTGTTGAAT
541 AACCCAATCACAGTCACAGGGTTGGAGCAGGAGCAGGAGAGGGAC
586 AATGGAAGCTGCCCCGTCCAGGTTCATGTTCCTCTTATTTCTCCT
MetGluAlaAlaProSerArgPheMetPheLeuLeuPheLeuLe
631 CACGTGTGAGCTGGCTGCAGAAGTTGCTGCAGAAGTTGAGAAATC
uThrCysGluLeuAlaAlaGluValAlaAlaGluValGluLysSe
676 CTCAGATGGTCCTGGTGCTGCCCAGGAACCCACGTGGCTCACAGA
rSerAspGlyProGlyAlaAlaGlnGluProThrTrpLeuThrAs
721 TGTCCCAGCTGCCATGGAATTCATTGCTGCCACTGAGGTGGCTGT
pValProAlaAlaMetGluPheIleAlaAlaThrGluValAlaVa
766 CATAGGCTTCTTCCAGGATTTAGAAATACCAGCAGTGCCCATACT
lIleGlyPhePheGlnAspLeuGluIleProAlaValProIleLe
811 CCATAGCATGGTGCAAAAATTCCCAGGCGTGTCATTTGGGATCAG
uHisSerMetValGlnLysPheProGlyValSerPheGlyIleSe
856 CACTGATTCTGAGGTTCTGACACACTACAACATCACTGGGAACAC
rThrAspSerGluValLeuThrHisTyrAsnIleThrGlyAsnTh
901 CATCTGCCTCTTTCGCCTGGTAGACAATGAACAACTGAATTTAGA
rIleCysLeuPheArgLeuValAspAsnGluGlnLeuAsnLeuGl
946 GGACGAAGACATTGAAAGCATTGATGCCACCAAATTGAGCCGTTT
uAspGluAspIleGluSerIleAspAlaThrLysLeuSerArgPh
991 CATTGAGATCAACAGCCTCCACATGGTGACAGAGTACAACCCTGT
eIleGluIleAsnSerLeuHisMetValThrGluTyrAsnProVa
1036 GACTGTGATTGGGTTATTCAACAGCGTAATTCAGATTCATCTCCT
lThrValIleGlyLeuPheAsnSerValIleGlnIleHisLeuLe
1081 CCTGATAATGAACAAGGCCTCCCCAGAGTATGAAGAGAACATGCA
uLeuIleMetAsnLysAlaSerProGluTyrGluGluAsnMetHi
1126 CAGATACCAGAAGGCAGCCAAGCTCTTCCAGGGGAAGATTCTCTT
sArgTyrGlnLysAlaAlaLysLeuPheGlnGlyLysIleLeuPh
1171 TATTCTGGTGGACAGTGGTATGAAAGAAAATGGGAAGGTGATATC
eIleLeuValAspSerGlyMetLysGluAsnGlyLysValIleSe

1216 ATTTTTCAAACTAAAGGAGTCTCGACTGCCAGCTTTGGGAATTTA
rPhePheLysLeuLysGluSerArgLeuProAlaLeuGlyIleTy
1261 CCAGACTCTAGATGACGAGTGGGATACACTGCCCACAGCAGAAGT
rGlnThrLeuAspAspGluTrpAspThrLeuProThrAlaGluVa
1306 TTCCGTAGAGCATGTGCAAAACTTTTGTGATGGATTCTAAGTGGG
lSerValGluHisValGlnAsnPheCysAspGlyPhe (SEQ ID NO: 10)
1351 AAATTGTTGAAAGAAAATCGTGAATCAGGAAGGGGAAAAGGAC
1396 TCCCAAAAAGGGGTTGGGGGAAAAACCT (SEQ ID NO:9)

NOV21 (SEQ ID NOS:41&42)
Translated Protein - Frame: 2 - Nucleotide 1082 to 1837

1 TTATTATGCAGGTTGTTGATTTACATAGGGAGTTGGAGATGCTAACCAAGCATGGAGTTTCACATGGTCTATTTCTGCTG

81 AGTTCAGGGACTTGGAGACAGCCTTTAACTTCTGGCAAAAAGACAATTTCACAAAGGTGTTTAAAACCATCCTTTGGTTT

161 TTGATCCTGAGTCAGAGACGGACATGTGCTTATGAAAGAAGGTAGAGTTTCAACCCTTAGGTAACCTTAAAAGAGCAGGA

241 ACTATGTTGTGTGTAAGTCATGTGCAGTATACAAACTTGATATTAAATGACAAATTGGAACAATCTTTCTCTAGGAATGC

321 CTCTCTTTCATAGAGGCATCACAGTGAGTCTCTTAAAGCCTTGATCTAGGTGTGTTACAGATGGGCTTACAGAGTATGAA

401 TGCACGATAAGAAGGAAATTGGATAGGGAGTGAGGATATGAAATTTAAAAGAAGGAAGAAGAGAAAACGAGATTTTAAGA

481 CAGGAAATGAAGCTCTGTGTGTGTGTGTGTGTGTGTGTGCGCGTGTGTGTGTGCACGCGTGCGTGCGTGTGTGCACGTGC

561 GTGTGTGTGTGGTTGGCAGGCCTAGTGATCCTGTTGTTTAGTGTCTCTGAGATTTGAGTTGTGCCTTTTTACTTTGCATA

641 AAGTAGATACTTGGCCATATGTAGTTCCAAGGAGAAGTCAGAGTTCCACCTTTGGAGTCTTTCCTTCTGATTCACGATTT

721 TCTTTCAACAATTTTCCACTTAGGAATCCATCACAAAAGTTTTGCACATGCTCTACGGAAACTTCTGCTGTGGGCAGTGT

801 ATCCCACTCGTCATCTAGAGTCTGGTAAATTGCCAAAGCTGGCAGTTGAGACTCCTTTAGTTTGAAAAATGATATCACCT

881 TCCCATTTTCTTTCATACCACTGTCCACCAGAATAAAGAGAATCTTCCCCTGGAAGAGCTTGGCTGCCTTCTGGTATCTG

961 TGCATGTTCTCTCCATACTCTGGGGAGGCCTTGTTCATTATCAGGAGGAGATGATTCTGAATTACGCTGTTGAATAACCC

1041 AATCACAGTCACAGGGTTGGAGCAGGAGCAGGAGAGGGACAATGGAAGCTGCCCCGTCCAGGTTCATGTTCCTCTTATTT
MetGluAlaAlaProSerArgPheMetPheLeuLeuPhe

1121 CTCCTCACGTGTGAGCTGGCTGCAGAAGTTGCTGCAGAAGTTGAGAAATCCTCAGATGGTCCTGGTGCTGCCCAGGAACC
LeuLeuThrCysGluLeuAlaAlaGluValAlaAlaGluValGluLysSerSerAspGlyProGlyAlaAlaGlnGluPr

1201 CACGTGGCTCACAGATGTCCCAGCTGCCATGGAATTCATTGCTGCCACTGAGGTGGCTGTCATAGGCTTCTTCCAGGATT
oThrTrpLeuThrAspValProAlaAlaMetGluPheIleAlaAlaThrGluValAlaValIleGlyPhePheGlnAspL

1281 TAGAAATACCAGCAGTGCCCATACTCCATAGCATGGTGCAAAAATTCCCAGGCGTGTCATTTGGGATCAGCACTGATTCT
euGluIleProAlaValProIleLeuHisSerMetValGlnLysPheProGlyValSerPheGlyIleSerThrAspSer

1361 GAGGTTCTGACACACTACAACATCACTGGGAACACCATCTGCCTCTTTCGCCTGGTAGACAATGAACAACTGAATTTAGA
GluValLeuThrHisTyrAsnIleThrGlyAsnThrIleCysLeuPheArgLeuValAspAsnGluGlnLeuAsnLeuGl

1441 GGACGAAGACATTGAAAGCATTGATGCCACCAAATTGAGCCGTTTCATTGAGATCAACAGCCTCCACATGGTGACAGAGT
uAspGluAspIleGluSerIleAspAlaThrLysLeuSerArgPheIleGluIleAsnSerLeuHisMetValThrGluT

1521 ACAACCCTGTGACTGTGATTGGGTTATTCAACAGCGTAATTCAGATTCATCTCCTCCTGATAATGAACAAGGCCTCCCCA
yrAsnProValThrValIleGlyLeuPheAsnSerValIleGlnIleHisLeuLeuLeuIleMetAsnLysAlaSerPro

1601 GAGTATGAAGAGAACATGCACAGATACCAGAAGGCAGCCAAGCTCTTCCAGGGGAAGATTCTCTTTATTCTGGTGGACAG
GluTyrGluGluAsnMetHisArgTyrGlnLysAlaAlaLysLeuPheGlnGlyLysIleLeuPheIleLeuValAspSe

1681 TGGTATGAAAGAAAATGGGAAGGTGATATCATTTTTCAAACTAAAGGAGTCTCAACTGCCAGCTTTGGCAATTTACCAGA
rGlyMetLysGluAsnGlyLysValIleSerPhePheLysLeuLysGluSerGlnLeuProAlaLeuAlaIleTyrGlnT

1761 CTCTAGATGACGAGTGGGATACACTGCCCACAGCAGAAGTTTCCGTAGAGCATGTGCAAAACTTTTGTGATGGATTCTAA
hrLeuAspAspGluTrpAspThrLeuProThrAlaGluValSerValGluHisValGlnAsnPheCysAspGlyPhe (SEQ ID NO:42)

1841 GTGGCAAATTGTTGAAAGAAAATCGTGAATCAGGAAGGGGGAAAAGGACTCCCAAAAAGGGGTTGGGGGAAAAACCT (SEQ ID NO:41)

NOV22(SEQ ID NOS:43&44)
1 aggtttttcc cccaacccct ttttgggagt cccttttccc ccttcctgat tcacgatttt
61 ctttcaacaa tttgccactt agaatccatc acaaaagttt tgcacatgct ctacggaaac
121 ttctgctgtg ggcagtgtat cccactcgtc atctagagtc tggtaaattg ccaaagctgg
181 cagttgagac tcctttagtt tgaaaaatga tatcaccttc ccattttctt tcataccact
241 gtccaccaga ataaagagaa tcttcccctg gaagagcttg gctgccttct ggtatctgtg
301 catgttctct tcatactctg gggaggcctt gttcattatc aggaggagat gaatctgaat
361 tacgctgttg aataacccaa tcacagtcac agggttgtac tctgtcacca tgtggaggct
421 gttgatctca atgaaacggc tcaatttggt ggcatcaatg ctttcaatgt cttcgtcctc
481 taaattcagt tgttcattgt ctaccaggcg aaagaggcag atggtgttcc cagtgatgtt
541 gtagtgtgtc agaacctcag aatcagtgct gatcccaaat gacacgcctg ggaatttttg
601 caccatgcta tggagtatgg gcactgctgg tatttctaaa tcctggaaga agcctatgac
661 agccacctca gtggcagcaa tgaattccat ggcagctggg acatctgtga gccacgtggg
721 ttcctgggca gcaccaggac catctgagga tttctcaact tctgcagcaa cttctgcagc
781 cagctcacac gtgaggagaa ataagaggaa catgaacctg gacggggcag cttccattgt
841 ccctctcctg ctcctgctcc aaccctgtga ctgtgattgg gttattcaac agcgtaattc
901 agaatcatct cctcctgata atgaacaagg cctccccaga gtatggagag aacatgcaca
961 gataccagaa ggcagccaag ctcttccagg ggaagattct ctttattctg gtggacagtg
1021 gtatgaaaga aaatgggaag gtgatatcat ttttcaaact aaaggagtct caactgccag
1081 ctttggcaat ttaccagact ctagatgacg agtgggatac actgcccaca gcagaagttt
1141 ccgtagagca tgtgcaaaac ttttgtgatg gattcctaag tggaaaattg ttgaaagaaa
1201 atcgtgaatc agaaggaaag actccaaagg tggaactctg acttctcctt ggaactacat
1261 atggccaagt atctacttta tgcaaagtaa aaaggcacaa ctcaaatctc agagacacta
1321 aacaacagga tcactaggcc tgccaaccac acacacacgc acgtgcacac acgcacgcac
1381 gcgtgcacac acacacgcgc acacacacac acacacacag agcttcattt cctgtcttaa
1441 aatctcgttt tctcttcttc cttcttttaa atttcatatc ctcactccct atccaatttc
1501 cttcttatcg tgcattcata ctctgtaagc ccatctgtaa cacacctaga tcaaggcttt
1561 aagagactca ctgtgatgcc tctatgaaag agaggcattc ctagagaaag attgttccaa
1621 tttgtcattt aatatcaagt ttgtatactg cacatgactt acacacaaca tagttcctgc
1681 tcttttaagg ttacctaagg gttgaaactc taccttcttt cataagcaca tgtccgtctc
1741 tgactcagga tcaaaaacca aaggatggtt ttaaacacct ttgtgaaatt gtctttttgc
1801 cagaagttaa aggctgtctc caagtccctg aactcagcag aaatagacca tgtgaaactc
1861 catgcttggt tagcatctcc aactccctat gtaaatcaac aacctgcata ataa
(SEQ ID NO:43)

MEAAPSRFMFLLFLLTCELAAEVAAEVEKSSDGPGAAQEPTWLTDVPAAMEFIAATEVAVIGFFQDLEIPAVPILH
SMVQKFPGVSFGISTDSEVLTHYNITGNTICLFRLVDNEQLNLEDEDIESIDATKLSRFIEINSLHMVTEYNPVTV
IGLFNSVIQIHLLLIMNKASPEYEENMHRYQKAAKLFQGKILFILVDSGMKENGKVISFFKLKESQLPALAIYQTL
DDEWDTLPTAEVSVEHVQNFCDGF (SEQ ID NO:44)

NOV6 (SEQ ID NOS:11&12)
Translated Protein - Nucleotide 183 to 1361

1 GGCGTTTGTGGCCGTCCGGCTNCCCTGACATGCAGATTTCCACCC
46 AGAAGACAGAGAAGGAGCCAGTGGTCATGGAATGGGCTGGGGTCA
91 AAGACTGGGTGCCTGGGAGCTGAGGCAGCCACCGTTTCAGCCTGG
136 CCAGCCCTCTGGACCCCGAGGTTGGACCCTACTGTGACACACCTA
181 CCATGCGGACACTCTTCAACCTCCTCTGGCTTGCCCTGGCCTGCA
MetArgThrLeuPheAsnLeuLeuTrpLeuAlaLeuAlaCysS
226 GCCCTGTTCACACTACCCTGTCAAAGTCAGATGCCAAAAAGCCG
erProValHisThrThrLeuSerLysSerAspAlaLysLysAlaA
271 CCTCAAAGACGCTGCTGGAGAAGAGTCAGTTTTCAGATAAGCCGG
laSerLysThrLeuLeuGluLysSerGlnPheSerAspLysProV
316 TGCAAGACCGGGGTTTGGTGGTGACGGACCTCAAAGCTGAGAGTG
alGlnAspArgGlyLeuValValThrAspLeuLysAlaGluSerV
361 TGGTTCTTGAGCATCGCAGCTACTGCTCGGCAAAGGCCCGGGACA
alValLeuGluHisArgSerTyrCysSerAlaLysAlaArgAspA
406 GACACTTTGCTGGGGATGTACTGGGCTATGTCACTCCATGGAACA
rgHisPheAlaGlyAspValLeuGlyTyrValThrProTrpAsnS
451 GCCATGGCTACGATGTCACCAAGGTCTTTGGGAGCAAGTTCACAC
erHisGlyTyrAspValThrLysValPheGlySerLysPheThrG
496 AGATCTCACCCGTCTGGCTGCAGCTGAAGAGACGTGGCCGTGAGA
lnIleSerProValTrpLeuGlnLeuLysArgArgGlyArgGluM
541 TGTTTGAGGTCACGGGCCTCCACGACGTGGACCAAGGGTGGATGC
etPheGluValThrGlyLeuHisAspValAspGlnGlyTrpMetA
586 GAGCTGTCAGGAAGCATGCCAAGGGCCTGCACATAGTGCCTCGGC
rgAlaValArgLysHisAlaLysGlyLeuHisIleValProArgL
631 TCCTATTTGAGGACTGGACTTACGATGATTTCCGGAACGTCTTAG
euLeuPheGluAspTrpThrTyrAspAspPheArgAsnValLeuA
676 ACAGTGAGGATGAGATAGAGGAGCTGAGCAAGACCGTGGTCCAGG
spSerGluAspGluIleGluGluLeuSerLysThrValValGlnV
721 TGGCAAAGAACCAGCATTTCGATGGCTTCGTGGTGGAGGTCTGGA
alAlaLysAsnGlnHisPheAspGlyPheValValGluValTrpA

766 ACCAGCTGCTAAGCCAGAAGCGCGTGGGCCTCATCCACATGCTCA
snGlnLeuLeuSerGlnLysArgValGlyLeuIleHisMetLeuT
811 CCCACTTGGCCGAGGCTCTGCACCAGGCCCGGCTGCTGGCCCTCC
hrHisLeuAlaGluAlaLeuHisGlnAlaArgLeuLeuAlaLeuL
856 TGGTCATCCCGCCTGCAATCACCCCCGGGACCGACCAGCTGGGCA
euValIleProProAlaIleThrProGlyThrAspGlnLeuGlyM
901 TGTTCACGCACAAGGAGTTTGAGCAGCTGGCCCCCGTGCTGGATG
etPheThrHisLysGluPheGluGlnLeuAlaProValLeuAspG
946 GTTTCAGCCTCATGACCTACGACTACTCTACAGCGCATCAGCCTG
lyPheSerLeuMetThrTyrAspTyrSerThrAlaHisGlnProG
991 GCCCTAATGCACCCCTGTCCTGGGTTCGAGCCTGCGTCCAGGTCC
lyProAsnAlaProLeuSerTrpValArgAlaCysValGlnValL
1036 TGGACCCGAAGTCCAAGTGGCGAAGCAAAATCCTCCTGGGGCTCA
euAspProLysSerLysTrpArgSerLysIleLeuLeuGlyLeuA
1081 ACTTTTATGGTATGGACTACGCGACCTCCAAGGATGCCCGTGAGC
snPheTyrGlyMetAspTyrAlaThrSerLysAspAlaArgGluP
1126 CTGTTGTCGGGGCCAGGTACATCCAGACACTGAAGGACCACAGGC
roValValGlyAlaArgTyrIleGlnThrLeuLysAspHisArgP
1171 CCCGGATGGTGTGGGACAGCCAGGCCTCAGAGCACTTCTTCGAGT
roArgMetValTrpAspSerGlnAlaSerGluHisPhePheGluT
1216 ACAAGAAGAGCCGCAGTGGGAGGCACGTCGTCTTCTACCCAACCC
yrLysLysSerArgSerGlyArgHisValValPheTyrProThrL
1261 TGAAGTCCCTGCAGGTGCGGCTGGAGCTGGCCCGGGAGCTGGGCG
euLysSerLeuGlnValArgLeuGluLeuAlaArgGluLeuGlyV
1306 TTGGGGTCTCTATCTGGGAGCTGGGCCAGGGCCTGGACTACTTCT
alGlyValSerIleTrpGluLeuGlyGlnGlyLeuAspTyrPheT
1351 ACGACCTGCTCTAGGTGGGCATTGCGGCCTCCGCGGTGGACGTGT
yrAspLeuLeu (SEQ ID NO:12)
1396 TCTTTTCTAAGCCATGGAGTGAGTGAGCAGGTGTGAAATACAGGC
1441 CTCCACTCCGTTTGCTGTGAAAAAAAAAAAAAAAAAAAA SEQ ID NO:11)

NOV7 (SEQ ID NOS:13&14)
Translated Protein - Nucleotide 91 to 486

1 CCCGCCAGCGGGTGGAACTCGGGTTAGCCCACTCCAGCTTTTTCC
46 GAAGGCCGCCAGGGCCTACCCCCAAGCCCCCCCAGGGCGCGGC
91 ATGCTCATGGGTTGCGCTGGGCCCGGAAAGCATGCGCAGCGGCTG

136 GCCTGCCTTCCGCCCCGGGCCCTTTGCCCAGAAGGATGGAGCCCT

181 TTGCATTCTTTTCGTCACCTCATTTATTTAATTTTTTTTTTTATG

226 TTGGATGTAGTTTTTTTTCTGTTGCAATTGTGGCAAATATACAT

271 GTCCGCTGTCCCCAGTTCCAGCGACAACAAAAGACAACCCCAAC

316 CTCCTCCAGATGCACAGTGTGTGTCACGCGTGTCTGGACTGTGAA

361 GACATGCACATAGCGAGCCTATACGGTTCTAAAGGTCACTGGAGG

406 GCGTGGTTTCTGTACCAGGGCCAAATCCCAGCACCCAGTACCCTG

451 CACACCCACCGCCCTGTGCCCTGCATGTGGAAATGCTGAGAGAAC
 (SEQ ID NO:14)
496 GTGCTCCAGTTCGGGCCTCCCCAGCCCCTCCCCACTGGAAGGGCA
541 GGTCTGGTCCCCTTTGTCATTGCTCCTCACCCACTGCTGTCTCCA
586 ACCCCAAATAGGAGAGTGACGGCCACCTGGGCAGCTCTTCTTTGG
631 AGCATGCATCCTGCTTGGCCGGCTCCTCCTCCTCCAGCCAGT
676 GGGAGCACTTTACTTGCTGTATTTTCCTGTGACCTCCCATGACCG
721 CAGGGATGAAGTCAATGACGCAGTTCCTCCAATTGCTACTAAGCC
766 AAAACCCAGTCCCAGCCTTGCTCAGATCCCTGGAACACAGTTAGT
811 G (SEQ ID NO:13)

NOV8 (SEQ ID NOS:15&16)
Translated Protein - Nucleotide 146 to 460

1 ACGAGTACAGCCTACCAGTGGGTCTCAGATCCCTCCTCTTTTTGC
46 CCCCCACAGTNTTCTCGAAGNGCCCCATGGGGGGGATNTTACGGA

91 AAACTAATAAGATNCAAAGAANNATTACCTACCTTGATAAAATTN
136 CCTTTAATGAATGAACACCACACTCAGGATAAAATCCAAACTCCC
MetAsnThrThrLeuArgIleLysSerLysLeuPr
181 TACTTCTGCATATGTGGCTTTCTGTGACCTGGCTTGTGCCCGTTC
oThrSerAlaTyrValAlaPheCysAspLeuAlaCysAlaArgSe
226 CCCTAGCTGCTCCCCAATCTGGTCTCCTACCATGTCACTCTCTGT
rProSerCysSerProIleTrpSerProThrMetSerLeuSerVa
271 GTGCCACCCATGCTGGTCTCTTTCCAGTTCAAGCCCATCCAGCCT
lCysHisProCysTrpSerLeuSerSerSerSerProSerSerLe
316 CTGGGCTTTTCTCTGCCTTTTTTGTTGTTCCCTCCTCCCGGAAT
uTrpAlaPheLeuCysLeuPheLeuLeuPheProProProGlyMe
361 GCTTTTCCCAGGGTCTCCCATGGCTGACTTCTCTGGCCTGAGGGC
tLeuPheProGlySerProMetAlaAspPheSerGlyLeuArgAl
406 TCCATTCAAATGTCACCTCCTTACAGGAGCCTTCTCTGATGATCT
aProPheLysCysHisLeuLeuThrGlyAlaPheSerAspAspLe
451 AAAAGGTCTCTAGGAACTTTTAGTGTCTTCCTGTAATTCTCTGTA
uLysGlyLeu (SEQ ID NO:16)
496 CATTTCCTGTGTTTCCTTATTTATTTACTGTTTGAAACATAGTCA
541 TAGTAGACAATAAATATTAAACTACGTGAAACTAGTTTAGTATTT
586 ATAATATTATAACTTATTTAGATATAATTATGTTATTATAATAAA
631 ATATGTGAAACAGCTGCTTTTGTAGGGGAAAAAGTTGAATATTGG
676 CCATTCCACATGGTTCACTGAAGAAATAATAATGTTATCATTAAG
721 TGTACTTATTGGCA (SEQ ID NO:15)

NOV9 (SEQ ID NOS:17&18)
Translated Protein - Nucleotide 244 to 1473

1 CTAGAATTCAGCGGCCGCTGAATTCTAGTTTGCTCCCAAAGGCGC
46 ACCAATGACCAACATTTGCCCCCCGGAGGAAAGAACTGGAACCAG
91 CCTCTGACCTGTCCAGGTGCCCTGTCCAGCTGACTGCAAGGACAG
136 AGAGGAGTCCTGCCCAGCTCTTGGATCAGTCTGCTGGCCGAGGAG
181 CCCGGTGGAGCCAGGGGTGACCCTGGAGCCCAGCCTGCCCCGAGG
226 AGGCCCCGGCTCAGAGCCATGCCAGGTGTCTGTGATAGGGCCCCT
MetProGlyValCysAspArgAlaPro

271 GACTTCCTCTCCCCGTCTGAAGACCAGGTGCTGAGGCCTGCCTTG
AspPheLeuSerProSerGluAspGlnValLeuArgProAlaLeu
316 GGCAGCTCAGTGGCTCTGAACTGCACGGCTTGGGTAGTCTCTGGG
GlySerSerValAlaLeuAsnCysThrAlaTrpValValSerGly
361 CCCCACTGCTCCCTGCCTTCAGTCCAGTGGCTGAAAGACGGGCTT
ProHisCysSerLeuProSerValGlnTrpLeuLysAspGlyLeu
406 CCATTGGGAATTGGGGGCCACTACAGCCTCCACGAGTACTCCTGG
ProLeuGlyIleGlyGlyHisTyrSerLeuHisGluTyrSerTrp
451 GTCAAGGCCAACCTGTCAGAGGTGCTTGTGTCCAGTGTCCTGGGG
ValLysAlaAsnLeuSerGluValLeuValSerSerValLeuGly
496 GTCAACGTGACCAGCACTGAAGTCTATGGGGCCTTCACCTGCTCC
ValAsnValThrSerThrGluValTyrGlyAlaPheThrCysSer
541 ATCCAGAACATCAGCTTCTCCTCCTTCACTCTTCAGAGAGCTGGC
IleGlnAsnIleSerPheSerSerPheThrLeuGlnArgAlaGly
586 CCTACAAGCCACGTGGCTGCGGTGCTGGCCTCCCTCCTGGTCCTG
ProThrSerHisValAlaAlaValLeuAlaSerLeuLeuValLeu
631 CTGGCCCTGCTGCTGGCCGCCCTGCTCTATGTCAAGTGCCGTCTC
LeuAlaLeuLeuLeuAlaAlaLeuLeuTyrValLysCysArgLeu
676 AACGTGCTGCTCTGGTACCAGGACGCGTATGGGGAGGTGGAGATA
AsnValLeuLeuTrpTyrGlnAspAlaTyrGlyGluValGluIle
721 AACGACGGGAAGCTCTACGACGCCTACGTCTCCTACAGCGACTGC
AsnAspGlyLysLeuTyrAspAlaTyrValSerTyrSerAspCys
766 CCCGAGGACCGCAAGTTCGTGAACTTCATCCTAAAGCCGCAGCTG
ProGluAspArgLysPheValAsnPheIleLeuLysProGlnLeu
811 GAGCGGCGTCGGGGCTACAAGCTCTTCCTGGACGACCGCGACCTC
GluArgArgArgGlyTyrLysLeuPheLeuAspAspArgAspLeu
856 CTGCCCCGCGCTGAGCCCTCCGCCGACCTCTTGGTGAACCTGAGC
LeuProArgAlaGluProSerAlaAspLeuLeuValAsnLeuSer
901 CGCTGCCGACGCCTCATCGTGGTGCTTTCGGACGCCTTCCTGAGC
ArgCysArgArgLeuIleValValLeuSerAspAlaPheLeuSer
946 CGGGCCTGGTGCAGCCACAGCTTCCGGGAGGGCCTGTGCCGGCTG
ArgAlaTrpCysSerHisSerPheArgGluGlyLeuCysArgLeu
991 CTGGAGCTCACCCGCAGACCCATCTTCATCACCTTCGAGGGCCAG
LeuGluLeuThrArgArgProIlePheIleThrPheGluGlyGln

1036 AGGCGCGACCCCGCGCACCCGGCGCTCCGCCTGCTGCGCCAGCAC
ArgArgAspProAlaHisProAlaLeuArgLeuLeuArgGlnHis
1081 CGCCACCTGGTGACCTTGCTGCTCTGGAGGCCCGGCTCCGTGACT
ArgHisLeuValThrLeuLeuLeuTrpArgProGlySerValThr
1126 CCTTCCTCCGATTTTTGGAAAGAAGTGCAGCTGGCGCTGCCGCGG
ProSerSerAspPheTrpLysGluValGlnLeuAlaLeuProArg
1171 AAGGTGCGGTACAGGCCGGTGGAAGGAGACCCCCAGACGCAGCTG
LysValArgTyrArgProValGluGlyAspProGlnThrGlnLeu
1216 CAGGACGACAAGGACCCCATGCTGATTCTTCGAGGCCGAGTCCCT
GlnAspAspLysAspProMetLeuIleLeuArgGlyArgValPro
1261 GAGGGCCGGGCCCTGGACTCAGAGGTGGACCCGGACCCTGAGGGC
GluGlyArgAlaLeuAspSerGluValAspProAspProGluGly
1306 GACCTGGGTGTCCGGGGGCCTGTTTTTGGAGAGCCATCAGCTCCA
AspLeuGlyValArgGlyProValPheGlyGluProSerAlaPro
1351 CCGCACACCAGTGGGGTCTCGCTGGGAGAGAGCCGGAGCAGCGAA
ProHisThrSerGlyValSerLeuGlyGluSerArgSerSerGlu
1396 GTGGACGTCTCGGATCTCGGCTCGCGAAACTACAGTGCCCGCACA
ValAspValSerAspLeuGlySerArgAsnTyrSerAlaArgThr
1441 GACTTCTACTGCCTGGTGTCCAAGGATGATATGTAGCTCCCACCC
AspPheTyrCysLeuValSerLysAspAspMet (SEQ ID NO:18)
1486 CAGAGTGCAGGATCATAGGGACAGCGGGGCCAGGGCAGCGGCGTC
1531 GCTCCTCTGCTCAACAGGACCACAACCCCTGCCAGCAGCCCTGGG
1576 ACCCTGCCAGCAGCCCTGGGAAAAGGCTGTGGCCTCAGGGCGCCT
1621 CCCAGTGCCAGAAAATAAAGTCCTTTTGGATTCTGAAAA (SEQ ID NO:17)

NOV10 (SEQ ID NOS:19&20)
Translated Protein - Nucleotide 813 to 3008

1 AAAGAGAGTCTCACCCTGTTTCCCAGACCGGAATGCAGTGGCGTG
46 ATCAACCTCGTGGGCTCAAGTGATCCTCCCACCTCAAACTCCTGA
91 GTGCTGGGACCACAGGCATGCACAACCATTCCCAGCTAATTTTT
136 GTTTTGTTTTTGTAGAGACTGGGTCTCACTGTGTTGCCCAGGCTG
181 GTCATGAACTCCTGGGCTCAAGTAATCCCCGTGCCTTGGTCTCTG
226 AAAGTGTTGGGATTACAGGCATGAGCCACTGTGCCTGGCCAAAAA

271 AGAGCTCTTTAAAAAATAATTTTGTAGATTGACAAATGTGACTCT
316 TGTAATTTTATTGAACATGAAAAACCCAGGAATCTTTATTTGAT
361 ATTAAACATTTTTAAAGGCATCTCAGTTGTTGTTGTAATAACACA
406 TTAAGAGAAGTAGTGGTTTTTTATTTCCAACCTTTGTGCATATAG
451 CTATTTAATGCCTACATGGATGGCTATTATTTCACTTTTTTCAGT
496 TATTATGAAGAGATTGGGTTTCATTCATTTGTAAAGTTTCAGCCA
541 GACTGCCTTTCACAAATTGATTTGTCAAAATTGAATGTTAATCTT
586 GACATCCCAGTGCGTTTTTGCCCGCGAACAGGCCTTTGAATGAAG
631 CTGCAAACACACATTATCTGGTTGTTAATTGTTTTACAGATGAGA
676 ACTGGACTGATGACCAACTGCTTGGTTTTAAACCATGCAATGAAA
721 ACCTTATTGCTGGCTGCAATATAATCAATGGGAAATGTGAATGTA
766 ACACCATTCGAACCTGCAGCAATCCCTTTGAGTTTCCAAGTCAGG
811 ATATGTGCCTTTCAGCTTTAAAGAGAATTGAAGAAGAGAAGCCAG
MetCysLeuSerAlaLeuLysArgIleGluGluGluLysProA
856 ATTGCTCCAAGGCCCGCTGTGAAGTCCAGTTCTCTCCACGTTGTC
spCysSerLysAlaArgCysGluValGlnPheSerProArgCysP
901 CTGAAGATTCTGTTCTGATCGAGGGTTATGCTCCTCCTGGGGAGT
roGluAspSerValLeuIleGluGlyTyrAlaProProGlyGluC
946 GCTGTCCCTTACCCAGCCGCTGCGTGTGCAACCCCGCAGGCTGTC
ysCysProLeuProSerArgCysValCysAsnProAlaGlyCysL
991 TGCGCAAAGTCTGCCAGCCGGGAAACCTGAACATACTAGTGTCAA
euArgLysValCysGlnProGlyAsnLeuAsnIleLeuValSerL
1036 AAGCCTCAGGGAAGCCGGGAGAGTGCTGTGACCTCTATGAGTGCA
ysAlaSerGlyLysProGlyGluCysCysAspLeuTyrGluCysL
1081 AACCAGTTTTCGGCGTGGACTGCAGGACTGTGGAATGCCCTCCTG
ysProValPheGlyValAspCysArgThrValGluCysProProV
1126 TTCAGCAGACCGCGTGTCCCCCGGACAGCTATGAAACTCAAGTCA
alGlnGlnThrAlaCysProProAspSerTyrGluThrGlnValA
1171 GACTAACTGCAGATGGTTGCTGTACTTTGCCAACAAGATGCGAGT
rgLeuThrAlaAspGlyCysCysThrLeuProThrArgCysGluC
1216 GTCTCTCTGGCTTATGTGGTTTCCCCGTGTGTGAGGTGGGATCCA
ysLeuSerGlyLeuCysGlyPheProValCysGluValGlySerT
1261 CTCCCCGCATAGTCTCTCGTGGCGATGGGACACCTGGAAAGTGCT
hrProArgIleValSerArgGlyAspGlyThrProGlyLysCysC

1306 GTGATGTCTTTGAATGTGTTAATGATACAAAGCCAGCCTGCGTAT
ysAspValPheGluCysValAsnAspThrLysProAlaCysValP
1351 TTAACAATGTGGAATATTATGATGGAGACATGTTTCGAATGGACA
heAsnAsnValGluTyrTyrAspGlyAspMetPheArgMetAspA
1396 ACTGTCGGTTCTGTCGATGCCAAGGGGGCGTTGCCATCTGCTTCA
snCysArgPheCysArgCysGlnGlyGlyValAlaIleCysPheT
1441 CTGCCCAGTGTGGTGAGATAAACTGCGAGAGGTACTACGTGCCCG
hrAlaGlnCysGlyGluIleAsnCysGluArgTyrTyrValProG
1486 AAGGAGAGTGCTGCCCAGTGTGTGAAGATCCAGTGTATCCTTTTA
luGlyGluCysCysProValCysGluAspProValTyrProPheA
1531 ATAATCCCGCTGGCTGCTATGCCAATGGCCTGATCCTTGCCCACG
snAsnProAlaGlyCysTyrAlaAsnGlyLeuIleLeuAlaHisG
1576 GAGACCGGTGGCGGGAAGACGACTGCACATTCTGCCAGTGCGTCA
lyAspArgTrpArgGluAspAspCysThrPheCysGlnCysValA
1621 ACGGTGAACGCCACTGCGTTGCGACCGTCTGCGGACAGACCTGCA
snGlyGluArgHisCysValAlaThrValCysGlyGlnThrCysT
1666 CAAACCCTGTGAAAGTGCCTGGGGAGTGTTGCCCTGTGTGCGAAG
hrAsnProValLysValProGlyGluCysCysProValCysGluG
1711 AACCAACCATCATCACAGTTGATCCACCTGCATGTGGGGAGTTAT
luProThrIleIleThrValAspProProAlaCysGlyGluLeuS
1756 CAAACTGCACTCTGACAGGGAAGGACTGCATTAATGGTTTCAAAC
erAsnCysThrLeuThrGlyLysAspCysIleAsnGlyPheLysA
1801 GCGATCACAATGGTTGTCGGACCTGTCAGTGCATAAACACCGAGG
rgAspHisAsnGlyCysArgThrCysGlnCysIleAsnThrGluG
1846 AACTATGTTCAGAACGTAAACAAGGCTGCACCTTGAACTGTCCCT
luLeuCysSerGluArgLysGlnGlyCysThrLeuAsnCysProP
1891 TCGGTTTCCTTACTGATGCCCAAAACTGTGAGATCTGTGAGTGCC
heGlyPheLeuThrAspAlaGlnAsnCysGluIleCysGluCysA
1936 GCCCAAGGCCCAAGAAGTGCAGACCCATAATCTGTGACAAGTATT
rgProArgProLysLysCysArgProIleIleCysAspLysTyrC
1981 GTCCACTTGGATTGCTGAAGAATAAGCACGGCTGTGACATCTGTC
ysProLeuGlyLeuLeuLysAsnLysHisGlyCysAspIleCysA
2026 GCTGTAAGAAATGTCCAGAGCTCTCATGCAGTAAGATCTGCCCCT
rgCysLysLysCysProGluLeuSerCysSerLysIleCysProL

2071 TGGGTTTCCAGCAGGACAGTCGCGGCTGTCTTATCTGCAAGTGCA
euGlyPheGlnGlnAspSerArgGlyCysLeuIleCysLysCysA
2116 GAGAGGCCTCTGCTTCAGCTGGGCCACCCATCCTGTCGGGCACTT
rgGluAlaSerAlaSerAlaGlyProProIleLeuSerGlyThrC
2161 GTCTCACCGTGGATGGTCATCATCATAAAAATGAGGAGAGCTGGC
ysLeuThrValAspGlyHisHisHisLysAsnGluGluSerTrpH
2206 ACGATGGGTGCCGGGAATGCTACTGTCTCAATGGACGGGAAATGT
isAspGlyCysArgGluCysTyrCysLeuAsnGlyArgGluMetC
2251 GTGCCCTGATCACCTGCCCGGTGCCTGCCTGTGGCAACCCCACCA
ysAlaLeuIleThrCysProValProAlaCysGlyAsnProThrI
2296 TTCACCCTGGACAGTGCTGCCCATCATGTGCAGATGACTTTGTGG
leHisProGlyGlnCysCysProSerCysAlaAspAspPheValV
2341 TGCAGAAGCCAGAGCTCAGTACTCCCTCCATTTGCCACGCCCCTG
alGlnLysProGluLeuSerThrProSerIleCysHisAlaProG
2386 GAGGAGAATACTTTGTGGAAGGAGAAACGTGGAACATTGACTCCT
lyGlyGluTyrPheValGluGlyGluThrTrpAsnIleAspSerC
2431 GTACTCAGTGCACCTGCCACAGCGGACGGGTGCTGTGTGAGACAG
ysThrGlnCysThrCysHisSerGlyArgValLeuCysGluThrG
2476 AGGTGTGCCCACCGCTGCTCTGCCAGAACCCCTCACGCACCCAGG
luValCysProProLeuLeuCysGlnAsnProSerArgThrGlnA
2521 ATTCCTGCTGCCCACAGTGTACAGATCAACCTTTTCGGCCTTCCT
spSerCysCysProGlnCysThrAspGlnProPheArgProSerL
2566 TGTCCCGCAATAACAGCGTACCTAATTACTGCAAAAATGATGAAG
euSerArgAsnAsnSerValProAsnTyrCysLysAsnAspGluG
2611 GGGATATATTCCTGGCAGCTGAGTCCTGGAAGCCTGACGTTTGTA
lyAspIlePheLeuAlaAlaGluSerTrpLysProAspValCysT
2656 CCAGCTGCATCTGCATTGATAGCGTAATTAGCTGTTTCTCTGAGT
hrSerCysIleCysIleAspSerValIleSerCysPheSerGluS
2701 CCTGCCCTTCTGTATCCTGTGAAAGACCTGTCTTGAGAAAAGGCC
erCysProSerValSerCysGluArgProValLeuArgLysGlyG
2746 AGTGTTGTCCCTACTGCATAGAAGACACAATTCCAAAGAAGGTGG
lnCysCysProTyrCysIleGluAspThrIleProLysLysValV
2791 TGTGCCACTTCAGTGGGAAGGCCTATGCCGACGAGGAGCGGTGGG
alCysHisPheSerGlyLysAlaTyrAlaAspGluGluArgTrpA

2836 ACCTTGACAGCTGCACCCACTACTACTGCCTGCAGGGCCAGACCC
spLeuAspSerCysThrHisTyrTyrCysLeuGlnGlyGlnThrL
2881 TCTGCTCGACCGTCAGCTGCCCCCTCTGCCCTGTGTTGAGCCCA
euCysSerThrValSerCysProProLeuProCysValGluProI
2926 TCAACGTGGAAGGAAGTTGCTGCCCAATGTGTCCAGTTTCACCTT
leAsnValGluGlySerCysCysProMetCysProValSerProL
2971 TACCATCTTTGGATATGAGTACAGAACCTATGAGCTGTTAGGTGA
euProSerLeuAspMetSerThrGluProMetSerCys (SEQ ID NO:20)
3016 TTAGCACCTGTCTCTTTACAGAAGAAACTGAGGCTCAGGAAAGAG
3061 CCCCTGTGGGAAGAGGACTCACTGTCATGCCTCAGCTTGGTGGAG
3106 TTTCACCGGAAATCTACCCATATGCAGGGTCAAGGCAAAAGAATT
3151 CCAAAGTTACGTCTCTCCCTCTCACTCAGGAAAAAACCTGAGGTG
3196 GAACTGAATCAATCCCAGCTCTGGGGCCTCTGCAGAAACTTTTAC
3241 TACTTAGCCATTGACATTTACAGTATAATACCTATCTGATCAAAC
3286 TGGATAATGTAAATATATTTACTGAAGATCAGCTTCTAATCTAAA
3331 TGGTTCCAGTGGTAACATAATGGACATCTGA (SEQ ID NO:19)

NOV11(SEQ ID NOS:21&22)
Translated Protein - Nucleotide 69 to 1211

1 AAAAAAGGCGGGGGTGGACTTAGCAGTGTAATTTGAGACCGGTG
46 GTAAGGATTGGAGCGAGCTAGAGATGCTGCACGCTGCTAACAAGG
MetLeuHisAlaAlaAsnLysG
91 GAAGGAAGCCTTCAGCTGAGGCAGGTCGTCCCATTCCACCTACAT
lyArgLysProSerAlaGluAlaGlyArgProIleProProThrS
136 CCTCGCCTAGTCTCCTCCCATCTGCTCAGCTGCCTAGCTCCCATA
erSerProSerLeuLeuProSerAlaGlnLeuProSerSerHisA
181 ATCCTCCACCAGTTAGCTGCCAGATGCCATTGCTAGACAGCAACA
snProProProValSerCysGlnMetProLeuLeuAspSerAsnT
226 CCTCCCATCAAATCATGGACACCAACCCTGATGAGGAATTCTCCC
hrSerHisGlnIleMetAspThrAsnProAspGluGluPheSerP
271 CCAATTCATACCTGCTCAGAGCATGCTCAGGGCCCCAGCAAGCCT
roAsnSerTyrLeuLeuArgAlaCysSerGlyProGlnGlnAlaS
316 CCAGCAGTGGCCCTCCGAACCACCACAGCCAGTCGACTCTGAGGC

erSerSerGlyProProAsnHisHisSerGlnSerThrLeuArgP
361 CCCCTCTCCCACCCCCTCACAACCACACGCTGTCCCATCACCACT
roProLeuProProProHisAsnHisThrLeuSerHisHisHisS
406 CGTCCGCCAACTCCCTCAACAGGAACTCACTGACCAATCGGCGGA
erSerAlaAsnSerLeuAsnArgAsnSerLeuThrAsnArgArgS
451 GTCAGATCCACGCCCCGGCCCCAGCGCCCAATGACCTGGCCACCA
erGlnIleHisAlaProAlaProAlaProAsnAspLeuAlaThrT
496 CACCAGAGTCCGTTCAGCTTCAGGACAGCTGGGTGCTAAACAGCA
hrProGluSerValGlnLeuGlnAspSerTrpValLeuAsnSerA
541 ACGTGCCACTGGAGACCCGGCACTTCCTCTTCAAGACCTCCTCGG
snValProLeuGluThrArgHisPheLeuPheLysThrSerSerG
586 GGAGCACACCCTTGTTCAGCAGCTCTTCCCCGGGATACCCTTTGA
lySerThrProLeuPheSerSerSerSerProGlyTyrProLeuT
631 CCTCAGGAACGGTTTACACGCCCCCGCCCCGCCTGCTGCCCAGGA
hrSerGlyThrValTyrThrProProProArgLeuLeuProArgA
676 ATACTTTCTCCAGGAAGGCTTTCAAGCTGAAGAAGCCCTCCAAAT
snThrPheSerArgLysAlaPheLysLeuLysLysProSerLysT
721 ACTGCAGCTGGAAATGTGCTGCCCTCTCCGCCATTGCCGCGGCCC
yrCysSerTrpLysCysAlaAlaLeuSerAlaIleAlaAlaAlaL
766 TCCTCTTGGCTATTTTGCTGGCGTATTTCATAGTGCCCTGGTCGT
euLeuLeuAlaIleLeuLeuAlaTyrPheIleValProTrpSerL
811 TGAAAAACAGCAGCATAGACAGTGGTGAAGCAGAAGTTGGTCGGC
euLysAsnSerSerIleAspSerGlyGluAlaGluValGlyArgA
856 GGGTAACACAAGAAGTCCCACCAGGGGTGTTTTGGAGGTCACAAA
rgValThrGlnGluValProProGlyValPheTrpArgSerGlnI
901 TTCACATCAGTCAGCCCCAGTTCTTAAAGTTCAACATCTCCCTCG
leHisIleSerGlnProGlnPheLeuLysPheAsnIleSerLeuG
946 GGAAGGACGCTCTCTTTGGTGTTTACATAAGAAGAGGACTTCCAC
lyLysAspAlaLeuPheGlyValTyrIleArgArgGlyLeuProP
991 CATCTCATGCCCAGTATGACTTCATGGAACGTCTGGACGGGAAGG
roSerHisAlaGlnTyrAspPheMetGluArgLeuAspGlyLysG
1036 AGAAGTGGAGTGTGGTTGAGTCTCCCAGGGAACGCCGGAGCATAC
luLysTrpSerValValGluSerProArgGluArgArgSerIleG
1081 AGACCTTGGTTCAGAATGAAGCCGTGTTTGTGCAGTACCTGGATG

lnThrLeuValGlnAsnGluAlaValPheValGlnTyrLeuAspV
1126 TGGGCCTGTGGCATCTGGCCTTCTACAATGATGGAAAGACAAAG
alGlyLeuTrpHisLeuAlaPheTyrAsnAspGlyLysAspLysG
1171 AGATGGTTTCCTTCAATACTGTTGTCCTAGATGGGACCATCTAGT
luMetValSerPheAsnThrValValLeuAspGlyThrIle (SEQ ID NO:22)
1216 TGCAGAAAAACAAGCTCAGGGCGCCCACTGATTTGACATTATGAT
1261 TCAGTGCAGGACTGTCCACGTAACTGCCATGGAATGGTGAANTG
1306 TGTGTCCGGGGTGTGTCACTGTTTCCCAGGATTTCTAGGAGCAGA
1351 CTGTGCTAAAGACCTTCCTGCCTTGACTTTCTGCAAGACAATCAT
1396 TAATAAAGCTGCTCTGTAAATACTAAAAAAAAAACA (SEQ ID NO:21)

NOV12 (SEQ ID NOS. 23&24)
Translated Protein - Nucleotide 517 to 1728

1 CACTATAGGGCTCGAGCGGCCGCCCGGGCAGGTCCAGGACCCCGA
46 GACACCCCGGGCGCGAGCGGCAGTGCTGCTTGCTTGCTCCTCCTC
91 TCCCCCAGCCCTTCCCCTCCGTGACCTACCCACTCCTTGCAGCCC
136 TCGCCCGCACCTTCTCCAACACCCCGGCATCCCTGCACCACCTGC
181 TCGGGCAGCCCCGGCGGGCTCTGGGACTTGCTGTGCGCGCCGAGA
226 GGAAGGCAAGCTCCAAACCCCTGCCTGGAAGACGGGCTGTCGCGG
271 CTGCACCACCAGCAGGAGGAGGAGGAGAAGAAACTATTTCGCGAT
316 ACCCCATTCTGCGGGTGCTTTGCCGCTGCCGCTTCTGCTGCCGCC
361 GATCCGAGTCCGCGGGTTCGAACACCGCAGCGGTGGGGACGGTGG
406 GTCCGGCGGGCGCCGGGAGGAGGACACCAGCGGAGCCCTGCACTC
451 TCGTGCCCCGCTCACCAGCATCTACTTGCCCCCTCGTTCCTTCCC
496 CAGCCCTTTAGAGAAGGGACCATGATTTGGAAACGCAGCGCCGTT
MetIleTrpLysArgSerAlaVal
541 CTCCGCTTCTACAGTGTCTGCGGGCTCCTGCTACAAGCGGCTGCT
LeuArgPheTyrSerValCysGlyLeuLeuLeuGlnAlaAlaAla
586 TCAAAGAATAAAGTTAAAGGCAGCCAAGGGCAGTTTCCACTAACA
SerLysAsnLysValLysGlySerGlnGlyGlnPheProLeuThr
631 CAGAATGTAACCGTTGTTGAAGGTGGAACTGCAATTTTGACCTGC
GlnAsnValThrValValGluGlyGlyThrAlaIleLeuThrCys
676 AGGGTTGATCAAAATGATAACACCTCCCTCCAGTGGTCAAATCCA

ArgValAspGlnAsnAspAsnThrSerLeuGlnTrpSerAsnPrc
721 GCTCAACAGACTCTGTACTTTGACGACAAGAAAGCTTTAAGGGAC
AlaGlnGlnThrLeuTyrPheAspAspLysLysAlaLeuArgAsp
766 AATAGGATCGAGCTGGTTCGCGCTTCCTGGCATGAATTGAGTATT
AsnArgIleGluLeuValArgAlaSerTrpHisGluLeuSerIle
811 AGTGTCAGTGATGTGTCTCTCTCTGATGAAGGACAGTACACCTGT
SerValSerAspValSerLeuSerAspGluGlyGlnTyrThrCys
856 TCTTTATTTACAATGCCTGTCAAAACTTCCAAGGCATATCTCACC
SerLeuPheThrMetProValLysThrSerLysAlaTyrLeuThr
901 GTTCTGGGTGTTCCTGAAAAGCCTCAGATTAGTGGATTCTCATCA
ValLeuGlyValProGluLysProGlnIleSerGlyPheSerSer
946 CCAGTTATGGAGGGTGACTTGATGCAGCTGACTTGCAAAACATCT
ProValMetGluGlyAspLeuMetGlnLeuThrCysLysThrSer
991 GGTAGTAAACCTGCAGCTGATATAAGATGGTTCAAAAATGACAAA
GlySerLysProAlaAlaAspIleArgTrpPheLysAsnAspLys
1036 GAGATTAAAGATGTAAAATATTTAAAAGAAGAGGATGCAAATCGC
GluIleLysAspValLysTyrLeuLysGluGluAspAlaAsnArg
1081 AAGACATTCACTGTCAGCAGCACACTGGACTTCCGAGTGGACCGG
LysThrPheThrValSerSerThrLeuAspPheArgValAspArg
1126 AGTGATGATGGAGTGGCGGTCATCTGCAGAGTAGATCACGAATCC
SerAspAspGlyValAlaValIleCysArgValAspHisGluSer
1171 CTCAATGCCACCCCTCAGGTAGCCATGCAGGTGCTAGAAATACAC
LeuAsnAlaThrProGlnValAlaMetGlnValLeuGluIleHis
1216 TATACACCATCAGTTAAGATTATACCATCGACTCCTTTTCCACAA
TyrThrProSerValLysIleIleProSerThrProPheProGln
1261 GAAGGACAGCCTTTAATTTTGACTTGTGAATCCAAAGGAAAACCA
GluGlyGlnProLeuIleLeuThrCysGluSerLysGlyLysPro
1306 CTGCCAGAACCTGTTTTGTGGACAAAGGATGGCGGAGAATTACCA
LeuProGluProValLeuTrpThrLysAspGlyGlyGluLeuPro
1351 GATCCTGACCGAATGGTTGTGAGTGGTAGGGAGCTAAACATTCTT
AspProAspArgMetValValSerGlyArgGluLeuAsnIleLeu
1396 TTCCTGAACAAAACGGATAATGGTACATATCGATGTGAAGCCACA
PheLeuAsnLysThrAspAsnGlyThrTyrArgCysGluAlaThr
1441 AACACCATTGGCCAAAGCAGTGCGGAATATGTTCTCATTGTGCAT

AsnThrIleGlyGlnSerSerAlaGluTyrValLeuIleValHis
1486 GATCCTAATGCTTTGGCTGGCCAGAATGGCCCTGACCATGCTCTC
AspProAsnAlaLeuAlaGlyGlnAsnGlyProAspHisAlaLeu
1531 ATAGGAGGAATAGTGGCTGTAGTTGTATTTGTCACGCTGTGTTCT
IleGlyGlyIleValAlaValValValPheValThrLeuCysSer
1576 ATCTTTCTGCTTGGTCGATATCTGGCAAGGCATAAAGGAACGTAT
IlePheLeuLeuGlyArgTyrLeuAlaArgHisLysGlyThrTyr
1621 TTAACAAATGAAGCTAAAGGAGCTGAAGATGCACCAGATGCTGAT
LeuThrAsnGluAlaLysGlyAlaGluAspAlaProAspAlaAsp
1666 ACAGCCATTATCAATGCTGAAGGCAGCCAAGTCAATGCTGAAGAG
ThrAlaIleIleAsnAlaGluGlySerGlnValAsnAlaGluGlu
1711 AAAAAAGAGTATTTCATTTAAGATGCAGGCCAAGATTCTGAGTTT
LysLysGluTyrPheIle (SEQ ID NO:24)
1756 TACTACCAGGCTGAATGCTGGAGAAACTGGCTATCATCTTTCAG
1801 AAGTCATTTCTACCATCGTCTGCTACCCTTATTAACTCCCATACT
1846 GTACTGCTATCAGTAGCCAGTGTATACCAACAATCAGCTGTTGAA
1891 AGCATCATTCTTTAATTACTGTACCATCCATAATGCAGGACATTT
1936 CTTACTGCCTAAATTTCACACCATTGCTCTTTTAACATACAGTGC
1981 TTGAATATACAGCCTTAACAATGTTAATCATCTCCTTGGATCATT
2026 ATATTGAGTGGTTTTTATACATTAAAAATGTATGCAGAGTTTTT
2071 TTCCCCCATTTTTTCCCCTTTAAGTCATAGACCTTATCAGTTTGC
2116 C (SEQ ID NO:23)

NOV13 (SEQ ID NOS:25&26)
Translated Protein - Nucleotide 508 to 2556

1 GTGGACTCTTCTCCAAATTTGTACTTAGTAATGACACACATTTGT
46 TTGCCTGATTCCCAGCTCTCTTAGGATAGGTCTTCTTGGGAAATG
91 CTTTCATTTCTAATGCAAAGAAAATTGTGCAGGCAGCCACGTTAA
136 GATGTTTTTCTGACAATAATTGGCCAAGATATTCCACTGTGTCTC
181 GAGGCCACTCCTGAAAAGAGGAAGTTTGTTTTCCTGTTGTTCTGA
226 CAGGAAGAGGTGGATCTACTTCATCAACATGCAGTACCAAATTGT
271 TAGGATACAAGCTAAAAAGGAGGGGATTACTCCCAGAGGAGGGAA
316 ATTGCTTTACAATCAGGCAGTTCCCTTTCAAAGTATCTCACCTCA
361 GAATGAAGGGTAACACTTAATCAACATGCTATACTGATCTGGGAA
406 CACAGTTTTATTATAAAGCTGAGTTGTTTATTACATTTTAGTTT
451 CATTGAGATTTACTTGATAAAGGTTGAAATTGGAACAAAAAAAGC
496 CTTCATCTTAAAATGGTTTTTCCACTTTGTTGAATTGTTCCTAT
MetValPheSerThrLeuLeuAsnCysSerTyr
541 ACTCAAAATTGCACCAAGACACCTTGTCTCCCAAATGCAAAATGT
ThrGlnAsnCysThrLysThrProCysLeuProAsnAlaLysCys
586 GAAATACGCAATGGAATTGAAGCCTGCTATTGCAACATGGGATTT
GluIleArgAsnGlyIleGluAlaCysTyrCysAsnMetGlyPhe
631 TCAGGAAATGGTGTCACAATTTGTGAAGATGATAATGAATGTGGA
SerGlyAsnGlyValThrIleCysGluAspAspAsnGluCysGly
676 AATTTAACTCAGTCCTGTGGCGAAAATGCTAATTGCACTAACACA
AsnLeuThrGlnSerCysGlyGluAsnAlaAsnCysThrAsnThr
721 GAAGGAAGTTATTATTGTATGTGTGTACCTGGCTTCAGATCCAGC
GluGlySerTyrTyrCysMetCysValProGlyPheArgSerSer
766 AGTAACCAAGACAGGTTTATCACTAATGATGGAACCGTCTGTATA
SerAsnGlnAspArgPheIleThrAsnAspGlyThrValCysIle
811 GAAAATGTGAATGCAAACTGCCATTTAGATAATGTCTGTATAGCT
GluAsnValAsnAlaAsnCysHisLeuAspAsnValCysIleAla
856 GCAAATATTAATAAAACTTTAACAAAAATCAGATCCATAAAAGAA
AlaAsnIleAsnLysThrLeuThrLysIleArgSerIleLysGlu
901 CCTGTGGCTTTGCTACAAGAAGTCTATAGAAATTCTGTGACAGAT

ProValAlaLeuLeuGlnGluValTyrArgAsnSerValThrAsp
946 CTTTCACCAACAGATATAATTGCATATATAGAAATATTAGCTGAA
LeuSerProThrAspIleIleAlaTyrIleGluIleLeuAlaGlu
991 TCATCTTCATTACTAGGTTACAAGAACAACACTATCTCAGCCAAG
SerSerSerLeuLeuGlyTyrLysAsnAsnThrIleSerAlaLys
1036 GACACCCTTTCTAACTCAACTCTTACTGAATTTGTAAAAACCGTG
AspThrLeuSerAsnSerThrLeuThrGluPheValLysThrVal
1081 AATAATTTTGTTCAAAGGGATACATTTGTAGTTTGGGACAAGTTA
AsnAsnPheValGlnArgAspThrPheValValTrpAspLysLeu
1126 TCTGTGAATCATAGGAGAACACATCTTACAAAACTCATGCACACT
SerValAsnHisArgArgThrHisLeuThrLysLeuMetHisThr
1171 GTTGAACAAGCTACTTTAAGGATATCCCAGAGCTTCCAAAAGACC
ValGluGlnAlaThrLeuArgIleSerGlnSerPheGlnLysThr
1216 ACAGAGTTTGATACAAATTCAACGGATATAGCTCTCAAAGTTTTC
ThrGluPheAspThrAsnSerThrAspIleAlaLeuLysValPhe
1261 TTTTTTGATTCATATAACATGAAACATATTCATCCTCATATGAAT
PhePheAspSerTyrAsnMetLysHisIleHisProHisMetAsn
1306 ATGGATGGAGACTACATAAATATATTTCCAAAGAGAAAAGCTGCA
MetAspGlyAspTyrIleAsnIlePheProLysArgLysAlaAla
1351 TATGATTCAAATGGCAATGTTGCAGTTGCATTTGTATATTATAAG
TyrAspSerAsnGlyAsnValAlaValAlaPheValTyrTyrLys
1396 AGTATTGGTCCTTTGCTTTCATCATCTGACAACTTCTTATTGAAA
SerIleGlyProLeuLeuSerSerSerAspAsnPheLeuLeuLys
1441 CCTCAAAATTATGATAATTCTGAAGAGGAGGAAAGAGTCATATCT
ProGlnAsnTyrAspAsnSerGluGluGluArgValIleSer
1486 TCAGTAATTTCAGTCTCAATGAGCTCAAACCCACCCACATTATAT
SerValIleSerValSerMetSerSerAsnProProThrLeuTyr
1531 GAACTTGAAAAAATAACATTTACATTAAGTCATCGAAAGGTCACA
GluLeuGluLysIleThrPheThrLeuSerHisArgLysValThr
1576 GATAGGTATAGGAGTCTATGTGCATTTTGGAATTACTCACCTGAT
AspArgTyrArgSerLeuCysAlaPheTrpAsnTyrSerProAsp
1621 ACCATGAATGGCAGCTGGTCTTCAGAGGGCTGTGAGCTGACATAC
ThrMetAsnGlySerTrpSerSerGluGlyCysGluLeuThrTyr
1666 TCAAATGAGACCCACACCTCATGCCGCTGTAATCACCTGACACAT

SerAsnGluThrHisThrSerCysArgCysAsnHisLeuThrHis
1711 TTTGCAATTTTGATGTCCTCTGGTCCTTCCATTGGTATTAAAGAT
PheAlaIleLeuMetSerSerGlyProSerIleGlyIleLysAsp
1756 TATAATATTCTTACAAGGATCACTCAACTAGGAATAATTATTTCA
TyrAsnIleLeuThrArgIleThrGlnLeuGlyIleIleIleSer
1801 CTGATTTGTCTTGCCATATGCATTTTTACCTTCTGGTTCTTCAGT
LeuIleCysLeuAlaIleCysIlePheThrPheTrpPhePheSer
1846 GAAATTCAAAGCACCAGGACAACAATTCACAAAAATCTTTGCTGT
GluIleGlnSerThrArgThrThrIleHisLysAsnLeuCysCys
1891 AGCCTATTTCTTGCTGAACTTGTTTTTCTTGTTGGGATCAATACA
SerLeuPheLeuAlaGluLeuValPheLeuValGlyIleAsnThr
1936 AATACTAATAAGCTCTTCTGTTCAATCATTGCCGGACTGCTACAC
AsnThrAsnLysLeuPheCysSerIleIleAlaGlyLeuLeuHis
1981 TACTTCTTTTTAGCTGCTTTTGCATGGATGTGCATTGAAGGCATA
TyrPhePheLeuAlaAlaPheAlaTrpMetCysIleGluGlyIle
2026 CATCTCTATCTCATTGTTGTGGGTGTCATCTACAACAAGGGATTT
HisLeuTyrLeuIleValValGlyValIleTyrAsnLysGlyPhe
2071 TTGCACAAGAATTTTTATATCTTTGGCTATCTAAGCCCAGCCGTG
LeuHisLysAsnPheTyrIlePheGlyTyrLeuSerProAlaVal
2116 GTAGTTGGATTTTCGGCAGCACTAGGATACAGATATTATGGCACA
ValValGlyPheSerAlaAlaLeuGlyTyrArgTyrTyrGlyThr
2161 ACCAAAGTATGTTGGCTTAGCACCGAAAACAACTTTATTTGGAGT
ThrLysValCysTrpLeuSerThrGluAsnAsnPheIleTrpSer
2206 TTTATAGGACCAGCATGCCTAATCATTCTTGTTAATCTCTTGGCT
PheIleGlyProAlaCysLeuIleIleLeuValAsnLeuLeuAla
2251 TTTGGAGTCATCATATACAAAGTTTTTCGTCACACTGCAGGGTTG
PheGlyValIleIleTyrLysValPheArgHisThrAlaGlyLeu
2296 AAACCAGAAGTTAGTTGCTTTGAGAACATAAGGTCTTGTGCAAGA
LysProGluValSerCysPheGluAsnIleArgSerCysAlaArg
2341 GGAGCCCTCGCTCTTCTGTTCCTTCTCGGCACCACCTGGATCTTT
GlyAlaLeuAlaLeuLeuPheLeuLeuGlyThrThrTrpIlePhe
2386 GGGGTTCTCCATGTTGTGCACGCATCAGTGGTTACAGCTTACCTC
GlyValLeuHisValValHisAlaSerValValThrAlaTyrLeu
2431 TTCACAGTCAGCAATGCTTTCCAGGGGATGTTCATTTTTTTATTC

PheThrValSerAsnAlaPheGlnGlyMetPheIlePheLeuPhe
2476 CTGTGTGTTTATCTAGAAAGATTCAAGAAGAATATTACAGATTG
LeuCysValLeuSerArgLysIleGlnGluGluTyrTyrArgLeu
2521 TTCAAAAATGTCCCCTGTTGTTTTGGATGTTTAAGGTAAACATAG
PheLysAsnValProCysCysPheGlyCysLeuArg (SEQ ID NO:26)
2566 AGAATGGTGGATAATTACAACTGCACAAAAATAAAAATTCCAAGC
2611 TGTGGATGACCAATGTATAAAAATGACTCATCAAATTATCCAATT
2656 ATTAACTACTAGACAAAAAGTATTTTAAATCAGTTTTTCTGTTTA
2701 TGCTATAGGAACTGTAGATAATAAGGTAAAATTATGTATCATATA
2746 GATATACTATGTTTTTCTATGTGAAATAGTTCTGTCAAAAATAGT
2791 ATTGCAGATATTTGGAAAGTAATTGGTTTCTCAGGAGTGATATCA
2836 CTGCACCCAAGGAAAGATTTTCTTTCT (SEQ ID NO:25)

NOV14 (SEQ ID NO:27&28)
Translated Protein - Nucleotide 520 to 2454

1 GAGCTCGGATCCACTAGTAACGGCCGCCAGTGTGCTGGAATTCGG
46 CTTTACGACTCACTATAGGGCTCGAGCGGCTGCCCGGGCAGGTCA
91 CATTTGTTTGCCTGATTCCCAGCTCTCTTAGGATAGGTCTTCTTG
136 GGAAATGCTTTCATTTCTAATGCAAAGAAAATTGTGCAGGCAGCC
181 ACGTTAAGATGTTTTTCTGACAATAATTGGCCAAGATATTCCACT
226 GTGTCTCGAGGCCACTCCTGAAAAGAGGAAGTTTGTTTTCCTGTT
271 GTTCTGACAGGAAGAGGTGGATCTACTTCATCAACATGCAGTACC
316 AAATTGTTAGGATACAAGCTAAAAAGGAGGGCATCCTTCCTTGAA
361 TGTGGGGTAGGAACCTTTCCGAAGTGGGGATCTTATGACCTACAA
406 GTGGTTTTTTCCACTTTGTTGAATTGTTCCTATACTCAAAATTGC
451 ACCAAGACACCTTGTCTCCCAAATGCAAAATGTGAAATACGCAAT
496 GGAATTGAAGCCTGCTATTGCAACATGGGATTTTCAGGAAATGGT
MetGlyPheSerGlyAsnGly
541 GTCACAATTTGTGAAGATGATAATGAATGTGGAAATTTAACTCAG
ValThrIleCysGluAspAspAsnGluCysGlyAsnLeuThrGln
586 TCCTGTGGCGAAAATGCTAATTGCACTAACACAGAAGGAAGTTAT
SerCysGlyGluAsnAlaAsnCysThrAsnThrGluGlySerTyr
631 TATTGTATGTGTGTACCTGGCTTCAGATCCAGCAGTAACCAAGAC

TyrCysMetCysValProGlyPheArgSerSerSerAsnGlnAsp
676 AGGTTTATCACTAATGATGGAACCGTCTGTATAGAAAATGTGAAT
ArgPheIleThrAsnAspGlyThrValCysIleGluAsnValAsn
721 GCAAACTGCCATTTAGATAATGTCTGTATAGCTGCAAATATTAAT
AlaAsnCysHisLeuAspAsnValCysIleAlaAlaAsnIleAsn
766 AAAACTTTAACAAAAATCAGATCCATAAAAGAACCTGTGGCTTTG
LysThrLeuThrLysIleArgSerIleLysGluProValAlaLeu
811 CTACAAGAAGTCTATAGAAATTCTGTGACAGATCTTTCACCAACA
LeuGlnGluValTyrArgAsnSerValThrAspLeuSerProThr
856 GATATAATTGCATATATAGAAATATTAGCTGAATCATCTTCATTA
AspIleIleAlaTyrIleGluIleLeuAlaGluSerSerSerLeu
901 CTAGGTTACAAGAACAACACTATCTCAGCCAAGGACACCCTTTCT
LeuGlyTyrLysAsnAsnThrIleSerAlaLysAspThrLeuSer
946 AACTCAACTCTTACTGAATTTGTAAAAACCGTGAATAATTTTGTT
AsnSerThrLeuThrGluPheValLysThrValAsnAsnPheVal
991 CAAAGGGATACATTTGTAGTTTGGGACAAGTTATCTGTGAATCAT
GlnArgAspThrPheValValTrpAspLysLeuSerValAsnHis
1036 AGGAGAACACATCTTACAAAACTCATGCACACTGTTGAACAAGCT
ArgArgThrHisLeuThrLysLeuMetHisThrValGluGlnAla
1081 ACTTTAAGGATATCCCAGAGCTTCCAAAAGACCACAGAGTTTGAT
ThrLeuArgIleSerGlnSerPheGlnLysThrThrGluPheAsp
1126 ACAAATTCAACGGATATAGCTCTCAAAGTTTTCTTTTTTGATTCA
ThrAsnSerThrAspIleAlaLeuLysValPhePhePheAspSer
1171 TATAACATGAAACATATTCATCCTCATATGAATATGGATGGAGAC
TyrAsnMetLysHisIleHisProHisMetAsnMetAspGlyAsp
1216 TACATAAATATATTTCCAAAGAGAAAAGCTGCATATGATTCAAAT
TyrIleAsnIlePheProLysArgLysAlaAlaTyrAspSerAsn
1261 GGCAATGTTGCAGTTGCATTTGTATATTATAAGAGTATTGGTCCT
GlyAsnValAlaValAlaPheValTyrTyrLysSerIleGlyPro
1306 TTGCTTTCATCATCTGACAACTTCTTATTGAAACCTCAAAATTAT
LeuLeuSerSerSerAspAsnPheLeuLeuLysProGlnAsnTyr
1351 GATAATTCTGAAGAGGAGGAAAGAGTCATATCTTCAGTAATTTCA
AspAsnSerGluGluGluGluArgValIleSerSerValIleSer
1396 GTCTCAATGAGCTCAAACCCACCCACATTATATGAACTTGAAAAA

ValSerMetSerSerAsnProProThrLeuTyrGluLeuGluLys
1441 ATAACATTTACATTAAGTCATCGAAAGGTCACAGATAGGTATAGG
IleThrPheThrLeuSerHisArgLysValThrAspArgTyrArg
1486 AGTCTATGTGCATTTTGGAATTACTCACCTGATACCATGAATGGC
SerLeuCysAlaPheTrpAsnTyrSerProAspThrMetAsnGly
1531 AGCTGGTCTTCAGAGGGCTGTGAGCTGACATACTCAAATGAGACC
SerTrpSerSerGluGlyCysGluLeuThrTyrSerAsnGluThr
1576 CACACCTCATGCCGCTGTAATCACCTGACACATTTTGCAATTTTG
HisThrSerCysArgCysAsnHisLeuThrHisPheAlaIleLeu
1621 ATGTCCTCTGGTCCTTCCATTGGTATTAAAGATTATAATATTCTT
MetSerSerGlyProSerIleGlyIleLysAspTyrAsnIleLeu
1666 ACAAGGATCACTCAACTAGGAATAATTATTTCACTGATTTGTCTT
ThrArgIleThrGlnLeuGlyIleIleIleSerLeuIleCysLeu
1711 GCCATATGCATTTTTACCTTCTGGTTCTTCAGTGAAATTCAAAGC
AlaIleCysIlePheThrPheTrpPhePheSerGluIleGlnSer
1756 ACCAGGACAACAATTCACAAAAATCTTTGCTGTAGCCTATTTCTT
ThrArgThrThrIleHisLysAsnLeuCysCysSerLeuPheLeu
1801 GCTGAACTTGTTTTTCTTGTTGGGATCAATACAAATACTAATAAG
AlaGluLeuValPheLeuValGlyIleAsnThrAsnThrAsnLys
1846 CTCTTCTGTTCAATCATTGCCGGACTGCTACACTACTTCTTTTTA
LeuPheCysSerIleIleAlaGlyLeuLeuHisTyrPhePheLeu
1891 GCTGCTTTTGCATGGATGTGCATTGAAGGCATACATCTCTATCTC
AlaAlaPheAlaTrpMetCysIleGluGlyIleHisLeuTyrLeu
1936 ATTGTTGTGGGTGTCATCTACAACAAGGGATTTTTGCACAAGAAT
IleValValGlyValIleTyrAsnLysGlyPheLeuHisLysAsn
1981 TTTTATATCTTTGGCTATCTAAGCCCAGCCGTGGTAGTTGGATTT
PheTyrIlePheGlyTyrLeuSerProAlaValValValGlyPhe
2026 TCGGCAGCACTAGGATACAGATATTATGGCACAACCAAAGTATGT
SerAlaAlaLeuGlyTyrArgTyrTyrGlyThrThrLysValCys
2071 TGGCTTAGCACCGAAAACAACTTTATTTGGAGTTTTATAGGACCA
TrpLeuSerThrGluAsnAsnPheIleTrpSerPheIleGlyPro
2116 GCATGCCTAATCATTCTTGTTAATCTCTTGGCTTTTGGAGTCATC
AlaCysLeuIleIleLeuValAsnLeuLeuAlaPheGlyValIle
2161 ATATACAAAGTTTTTCGTCACACTGCAGGGTTGAAACCAGAAGTT

IleTyrLysValPheArgHisThrAlaGlyLeuLysProGluVal
2206 AGTTGCTTTGAGAACATAAGGTCTTGTGCAAGAGGAGCCCTCGCT
SerCysPheGluAsnIleArgSerCysAlaArgGlyAlaLeuAla
2251 CTTCTGTTCCTTCTCGGCACCACCTGGATCTTTGGGGTTCTCCAT
LeuLeuPheLeuLeuGlyThrThrTrpIlePheGlyValLeuHis
2296 GTTGTGCACGCATCAGTGGTTACAGCTTACCTCTTCACAGTCAGC
ValValHisAlaSerValValThrAlaTyrLeuPheThrValSer
2341 AATGCTTTCCAGGGGATGTTCATTTTTTTATTCCTGTGTGTTTTA
AsnAlaPheGlnGlyMetPheIlePheLeuPheLeuCysValLeu
2386 TCTAGAAAGATTCAAGAAGAATATTACAGATTGTTCAAAAATGTC
SerArgLysIleGlnGluGluTyrTyrArgLeuPheLysAsnVal
2431 CCCTGTTGTTTTGGATGTTTAAGGTAAACATAGAGAATGGTGGAT
ProCysCysPheGlyCysLeuArg (SEQ ID NO:28)
2476 AATTACAACTGCACAAAAATAAAAATTCCAAGCTGTGGATGACCA
2521 ATGTATAAAAATGACTCATCAAATTATCCAATTATTAACTACTAG
2566 ACAAAAAGTATTTTAAATCAGTTTTTCTGTTTATGCTATAGGAAC
2611 TGTAGATAATAAGGTAAAATTATGTATCATATAGATATACTATGT
2656 TTTTCTATGTGAAATAGTTCTGTCAAAAATAGTATTGCAGATATT
2701 TGGAAAGTAATTGGTTTCTCAGGAGTGATATCACTGCACCCAAGG
2746 AAAGATTTTCTTTCT (SEQ ID NO:27)
NOV23 (SEQ ID NO:45&46)

1 GAGCTCGGATCCACTAGTAACGGCCGCCAGTGTGCTGGAATTCGGCTTTACGACTCACTATAGGGCTCGAGCGGCTGCCC
81 GGGCAGGTCACATTTGTTTGCCTGATTCCCAGCTCTCTTAGGATAGGTCTTCTTGGGAAATGCTTTCATTTCTAATGCAA
161 AGAAAATTGTGCAGGCAGCCACGTTAAGATGTTTTTCTGACAATAATCGGCCAAGATATTCCACTGTGTCTCGAGGCCAC
241 TCCTGAAAAGAGGAAGTTTGTTTTCCTGTTGTTCTGACAGGAAGAGGTGGATCTACTTCATCAACATGCAGTACCAAATT
321 GTTAGGATACAAGCTAAAAAGGAGGGTGGTTTTTTCCACTTTGTTGAATTGTTCCTATACTCAAAATTGCACCAAGACAC
401 CTTGTCTCCCAAATGCAAAATGTGAAATACGCAATGGAATTGAAGCCTGCTATTGCAACATGGGATTTTCAGGAAATGGT
MetGlyPheSerGlyAsnGly
481 GTCACAATTTGTGAAGATGATAATGAATGTGGAAATTTAACTCAGTCCTGTGGCGAAAATGCTAATTGCACTAACACAGA
ValThrIleCysGluAspAspAsnGluCysGlyAsnLeuThrGlnSerCysGlyGluAsnAlaAsnCysThrAsnThrGl
561 AGGAAGTTATTATTGTATGTGTGTACCTGGCTTCAGATCCAGCAGTAACCAAGACAGGTTTATCACTAATGATGGAACCG
uGlySerTyrTyrCysMetCysValProGlyPheArgSerSerSerAsnGlnAspArgPheIleThrAsnAspGlyThrV
641 TCTGTATAGAAAATGTGAATGCAAACTGCCATTTAGATAATGTCTGTATAGCTGCAAATATTAATAAAACTTTAACAAAA
alCysIleGluAsnValAsnAlaAsnCysHisLeuAspAsnValCysIleAlaAlaAsnIleAsnLysThrLeuThrLys
721 ATCAGATCCATAAAAGAACCTGTGGCTTTGCTACAAGAAGTCTATAGAAATTCTGTGACAGATCTTTCACCAACAGATAT
IleArgSerIleLysGluProValAlaLeuLeuGlnGluValTyrArgAsnSerValThrAspLeuSerProThrAspIl
801 AATTGCATATATAGAAATATTAGCTGAATCATCTTCATTACTAGGTTACAAGAACAACACTATCTCAGCCAAGGACACCC

eIleAlaTyrIleGluIleLeuAlaGluSerSerSerLeuLeuGlyTyrLysAsnAsnThrIleSerAlaLysAspThrL
881 TTTCTAACTCAACTCTTACTGAATTTGTAAAAACCGTGAATAATTTTGTTCAAAGGGATACATTTGTAGTTTGGGACAAG
euSerAsnSerThrLeuThrGluPheValLysThrValAsnAsnPheValGlnArgAspThrPheValValTrpAspLys
961 TTATCTGTGAATCATAGGAGAACACATCTTACAAAACTCATGCACACTGTTGAACAAGCTACTTTAAGGATATCCCAGAG
LeuSerValAsnHisArgArgThrHisLeuThrLysLeuMetHisThrValGluGlnAlaThrLeuArgIleSerGlnSe
1041 CTTCCAAAAGACCACAGAGTTTGATACAAATTCAACGGATATAGCTCTCAAAGTTTTCTTTTTTGATTCATATAACATGA
rPheGlnLysThrThrGluPheAspThrAsnSerThrAspIleAlaLeuLysValPhePhePheAspSerTyrAsnMetL
1121 AACATATTCATCCTCATATGAATATGGATGGAGACTACATAAATATATTTCCAAAGAGAAAAGCTGCATATGATTCAAAT
ysHisIleHisProHisMetAsnMetAspGlyAspTyrIleAsnIlePheProLysArgLysAlaAlaTyrAspSerAsn
1201 GGCAATGTTGCAGTTGCATTTGTATATTATAAGAGTATTGGTCCTTTGCTTTCATCATCTGACAACTTCTTATTGAAACC
GlyAsnValAlaValAlaPheValTyrTyrLysSerIleGlyProLeuLeuSerSerSerAspAsnPheLeuLeuLysPr
1281 TCAAAATTATGATAATTCTGAAGAGGAGGAAAGAGTCATATCTTCAGTAATTTCAGTCTCAATGAGCTCAAACCCACCCA
oGlnAsnTyrAspAsnSerGluGluGluGluArgValIleSerSerValIleSerValSerMetSerSerAsnProProT
1361 CATTATATGAACTTGAAAAAATAACATTTACATTAAGTCATCGAAAGGTCACAGATAGGTATAGGAGTCTATGTGCATTT
hrLeuTyrGluLeuGluLysIleThrPheThrLeuSerHisArgLysValThrAspArgTyrArgSerLeuCysAlaPhe
1441 TGGAATTACTCACCTGATACCATGAATGGCAGCTGGTCTTCAGAGGGCTGTGAGCTGACATACTCAAATGAGACCCACAC
TrpAsnTyrSerProAspThrMetAsnGlySerTrpSerSerGluGlyCysGluLeuThrTyrSerAsnGluThrHisTh
1521 CTCATGCCGCTGTAATCACCTGACACATTTTGCAATTTTGATGTCCTCTGGTCCTTCCATTGGTATTAAAGATTATAATA
rSerCysArgCysAsnHisLeuThrHisPheAlaIleLeuMetSerSerGlyProSerIleGlyIleLysAspTyrAsnI
1601 TTCTTACAAGGATCACTCAACTAGGAATAATTATTTCACTGATTTGTCTTGCCATATGCATTTTTACCTTCTGGTTCTTC
leLeuThrArgIleThrGlnLeuGlyIleIleIleSerLeuIleCysLeuAlaIleCysIlePheThrPheTrpPhePhe
1681 AGTGAAATTCAAAGCACCAGGACAACAATTCACAAAAATCTTTGCTGTAGCCTATTTCTTGCTGAACTTGTTTTTCTTGT
SerGluIleGlnSerThrArgThrThrIleHisLysAsnLeuCysCysSerLeuPheLeuAlaGluLeuValPheLeuVa
1761 TGGGATCAATACAAATACTAATAAGCTCTTCTGTTCAATCATTGCCGGACTGCTACACTACTTCTTTTTAGCTGCTTTTG
lGlyIleAsnThrAsnThrAsnLysLeuPheCysSerIleIleAlaGlyLeuLeuHisTyrPhePheLeuAlaAlaPheA
1841 CATGGATGTGCATTGAAGGCATACATCTCTATCTCATTGTTGTGGGTGTCATCTACAACAAGGGATTTTTGCACAAGAAT
laTrpMetCysIleGluGlyIleHisLeuTyrLeuIleValValGlyValIleTyrAsnLysGlyPheLeuHisLysAsn
1921 TTTTATATCTTTGGCTATCTAAGCCCAGCCGTGGTAGTTGGATTTTCGGCAGCACTAGGATACAGATATTATGGCACAAC
PheTyrIlePheGlyTyrLeuSerProAlaValValValGlyPheSerAlaAlaLeuGlyTyrArgTyrTyrGlyThrTh
2001 CAAAGTATGTTGGCTTAGCACCGAAAACAACTTTATTTGGAGTTTTATAGGACCAGCATGCCTAATCATTCTTGTTAATC
rLysValCysTrpLeuSerThrGluAsnAsnPheIleTrpSerPheIleGlyProAlaCysLeuIleIleLeuValAsnL
2081 TCTTGGCTTTTGGAGTCATCATATACAAAGTTTTTCGTCACACTGCAGGGTTGAAACCAGAAGTTAGTTGCTTTGAGAAC
euLeuAlaPheGlyValIleIleTyrLysValPheArgHisThrAlaGlyLeuLysProGluValSerCysPheGluAsn
2161 ATAAGGTCTTGTGCAAGAGGAGCCCTCGCTCTTCTGTTCCTTCTCGGCACCACCTGGATCTTTGGGGTTCTCCATGTTGT
IleArgSerCysAlaArgGlyAlaLeuAlaLeuLeuPheLeuLeuGlyThrThrTrpIlePheGlyValLeuHisValVa
2241 GCACGCATCAGTGGTTACAGCTTACCTCTTCACAGTCAGCAATGCTTTCCAGGGGATGTTCATTTTTTTATTCCTGTGTG
lHisAlaSerValValThrAlaTyrLeuPheThrValSerAsnAlaPheGlnGlyMetPheIlePheLeuPheLeuCysV
2321 TTTTATCTAGAAAGATTCAAGAAGAATATTACAGATTGTTCAAAAATGTCCCCTGTTGTTTTGGATGTTTAAGGTAAACA
alLeuSerArgLysIleGlnGluGluTyrTyrArgLeuPheLysAsnValProCysCysPheGlyCysLeuArg (SEQ ID
NO:46)
2401 TAGAGAATGGTGGATAATTACAACTGCACAAAAATAAAAATTCCAAGCTGTGGATGACCAATGTATAAAAATGACTCATC
2481 AAATTATCCAATTATTAACTACTAGACAAAAAGTATTTTAAATCAGTTTTTCTGTTTATGCTATAGGAACTGTAGATAAT
2561 AAGGTAAAATTATGTATCATATAGATATACTATGTTTTTCTATGTGAAATAGTTCTGTCAAAAATAGTATTGCAGATATT
2641 TGGAAAGTAATTGGTTTCTCAGGAGTGATATCACTGCACCCAAGGAAAGATTTTCTTTCTAACACGAGAAGTATATGAAT
2721 GTCCTGAAGGAAACCACTGGCTTGATATTTCTGTGACTCGTGTTGCCTTTGAAACTAGTCCCCTACCACCTCGGTAATGA
2801 GCTCCATTACAGAAAGTGGAACATAAGAGAATGAAGGGGCAGAATATCAAACAGTGAAAAGGGAATGATAAGATGTATTT
2881 TGAATGAACTGTTTTTTCTGTAGACTAGCTGAGAAATTGTTGACATAAAATAAAGAATTGAAGAAACACATTTTACCATT
2961 TTGTGAATTGTTCTGAACTTAAATGTCCACTAAAACAACTTAGACTTCTGTTTGCTAAATCTGTTTCTTTTTCTAATATT
3041 CTAAAAAAAAAAAAAAGGTTTACCTCCACAAATTGAAAAAAN (SEQ ID NO:45)

NOV15 (SEQ ID NOS:29&30)
Translated Protein - Nucleotide 312 to 560

1 CTCAGTATCCAAGAAGAATTGGTTACAGGATCCCCACAGATACCA
46 AAGTCTGTGGGTATTCAAGTCTCTGATATAAAATGACCCAGTACA
91 GTCAACCTTGCATATCTGCAGATACAGAACCCACTGACTGTGTTT
136 TCACAGAATAGCTTATTGTAAGTTTTCTAGAACTGAACCTGGATG
181 TGCATCTGGCACAGTGTGATGCTGGATTCTGTGTCCTCATTAGTC
226 TAACGAGTCTACTCTGTTGCCCACATCACCTCCCATTAGGACCAC
271 TATGCCCTTTTAAAAAGTGGTCTTTATAAGATGTAAGTATTATGA
MetT
316 CACCCTTCNTGCATACAACTATTCAATGGCTTTTGATTAGCCTTA
hrProPhe---HisThrThrIleGlnTrpLeuLeuIleSerLeuA
361 GGATAAAAATCCCGTCCTGCCGCACCGACTTGTCCATCTTGTGGG
rgIleLysIleProSerCysArgThrAspLeuSerIleLeuTrpV
406 TAGCCACTTGTTACTACCTCTCTCAGTGTCCTTCCCAGACATGCT
alAlaThrCysTyrTyrLeuSerGlnCysProSerGlnThrCysC
451 GCTTCCACTCCTCTCCCCTCAGATCCCCGTTTTGCTTAGTTACTT
ysPheHisSerSerProLeuArgSerProPheCysLeuValThrS
496 CCTCCTCATCTTTCAGGTCTCAAAGTAGTTGTCCCTTCACTGAAT
erSerSerSerPheArgSerGlnSerSerCysProPheThrGluC
541 GCACCGACAACCTGGCCCAGTAAAGTTACTTTGTCGTCTGCGCCC
ysThrAspAsnLeuAlaGln (SEQ ID NO:30)
586 AAATAATCCTACCCTTCCATCTGCTACATTCCTTAAATCTGCAAT
631 CCCTTGTTCAAAGCCTGCAAAAGAGCATGAGCTCCTTGAAGCAAA
676 GACAGTATTAGTCATTTTTAATGTAAAGTATAAAAAAAAAAAAAA
721 AAAAAAA (SEQ ID NO:29)

NOV 16 (SEQ ID NOS:31&32)
Translated Protein - Nucleotide 288 to 2021

1 CAATTGACTTGATATGATTTATTATTTTACTACTTATAAGAATG
46 GAAATAAGTTCTCCTTAGTTTTTTCTTGGAGAAAGTCTGACATG
91 TGAGGCACAGATGAGTTATTAAAGGCAGATGACTTTCCAGCCTTG

136 TCTTAAATGTTCCATTCTTTACCTTAGAAATTATTTAAATTTGTG
181 TCCTGTCCCAGAGCATCCGCAAGGGCGCAGCCCAGTGGTTTGGAG
226 TCAGCGGCGACTGGGAGGGGCAGCGGCAGCAGTGGCAGCGCCGCA
271 GCCTGCACCACTGCAGCATGCGCTACGGCCGCCTGAAGGCCTCGT
MetArgTyrGlyArgLeuLysAlaSerC
316 GCCAGCGTGACCTGGAGCTCCCCAGCCAGGAGGCACCGTCCTTCC
ysGlnArgAspLeuGluLeuProSerGlnGluAlaProSerPheG
361 AGGGCACTGAGTCCCCAAAGCCCTGCAAGATGCCCAAGATTGTGG
lnGlyThrGluSerProLysProCysLysMetProLysIleValA
406 ATCCGCTGGCCCGGGGCCGGGCCTTCCGCCACCCGGAGGAGATGG
spProLeuAlaArgGlyArgAlaPheArgHisProGluGluMetA
451 ACAGGCCCCACGCCCTGCACCCACCGCTGACCCCGGAGTCCTGT
spArgProHisAlaLeuHisProProLeuThrProGlyValLeuS
496 CCCTCACCTCCTTCACCAGTGTCCGTTCTGGCTACTCCCACCTGC
erLeuThrSerPheThrSerValArgSerGlyTyrSerHisLeuP
541 CACGCCGCAAGAGAATGTCTGTGGCCCACATGAGCTTGCAAGCTG
roArgArgLysArgMetSerValAlaHisMetSerLeuGlnAlaA
586 CCGCTGCCCTCCTCAAGGGGCGCTCGGTGCTGGATGCCACCGGAC
laAlaAlaLeuLeuLysGlyArgSerValLeuAspAlaThrGlyG
631 AGCGGTGCCGGGTGGTCAAGCGCAGCTTTGCCTTCCCGAGCTTCC
lnArgCysArgValValLysArgSerPheAlaPheProSerPheL
676 TGGAGGAGGATGTGGTCGATGGGGCAGACACGTTTGACTCCTCCT
euGluGluAspValValAspGlyAlaAspThrPheAspSerSerP
721 TTTTTAGTAAGGAAGAAATGAGCTCCATGCCTGATGATGTCTTTG
hePheSerLysGluGluMetSerSerMetProAspAspValPheG
766 AGTCCCCCCCACTCTCTGCCAGCTACTTCCGAGGGATCCCACACT
luSerProProLeuSerAlaSerTyrPheArgGlyIleProHisS
811 CAGCCTCCCCTGTCTCCCCCGATGGGGTGCAAATCCCTCTGAAGG
erAlaSerProValSerProAspGlyValGlnIleProLeuLysG
856 AGTATGGCCGAGCCCCAGTCCCCGGGCCCCGGCGCGGCAAGCGCA
luTyrGlyArgAlaProValProGlyProArgArgGlyLysArgI
901 TCGCCTCCAAGGTGAAGCACTTTGCCTTTGATCGGAAGAAGCGGC
leAlaSerLysValLysHisPheAlaPheAspArgLysLysArgH
946 ACTACGGCCTCGGCGTGGTGGGCAACTGGCTGAACCGCAGCTACC

isTyrGlyLeuGlyValValGlyAsnTrpLeuAsnArgSerTyrA
991 GCCGCAGCATCAGCAGCACTGTGCAGCGGCAGCTGGAGAGCTTCG
rgArgSerIleSerSerThrValGlnArgGlnLeuGluSerPheA
1036 ACAGCCACCGGCCCTACTTCACCTACTGGCTGACCTTCGTCCATG
spSerHisArgProTyrPheThrTyrTrpLeuThrPheValHisV
1081 TCATCATCACGCTGCTGGTGATTTGCACGTATGGCATCGCACCCG
alIleIleThrLeuLeuValIleCysThrTyrGlyIleAlaProV
1126 TGGGCTTTGCCCAGCACGTCACCACCCAGCTGGTGCTGCGGAACA
alGlyPheAlaGlnHisValThrThrGlnLeuValLeuArgAsnL
1171 AAGGTGTGTACGAGAGCGTGAAGTACATCCAGCAGGAGAACTTCT
ysGlyValTyrGluSerValLysTyrIleGlnGlnGluAsnPheT
1216 GGGTTGGCCCCAGCTCGATTGACCTGATCCACCTGGGGGCCAAGT
rpValGlyProSerSerIleAspLeuIleHisLeuGlyAlaLysP
1261 TCTCACCCTGCATCCGGAAGGACGGGCAGATCGAGCAGCTGGTGC
heSerProCysIleArgLysAspGlyGlnIleGluGlnLeuValL
1306 TGCGCGAGCGAGACCTGGAGCGGGACTCAGGCTGCTGTGTCCAGA
euArgGluArgAspLeuGluArgAspSerGlyCysCysValGlnA
1351 ATGACCACTCCGGCTGCATCCAGACCCAGCGGAAGGACTGCTCGG
snAspHisSerGlyCysIleGlnThrGlnArgLysAspCysSerG
1396 AGACTTTGGCCACTTTTGTCAAGTGGCAGGATGACACTGGCCCCC
luThrLeuAlaThrPheValLysTrpGlnAspAspThrGlyProP
1441 CCATGGACAAGTCTGATCTGGGCCAGAAGCGGACTTCGGGGGCTG
roMetAspLysSerAspLeuGlyGlnLysArgThrSerGlyAlaV
1486 TCTGCCACCAGGACCCCAGGACCTGCGAGGAGCCAGCCTCCAGCG
alCysHisGlnAspProArgThrCysGluGluProAlaSerSerG
1531 GTGCCCACATCTGGCCCGATGACATCACTAAGTGGCCGATCTGCA
lyAlaHisIleTrpProAspAspIleThrLysTrpProIleCysT
1576 CAGAGCAGGCCAGGAGCAACCACACAGGCTTCCTGCACATGGACT
hrGluGlnAlaArgSerAsnHisThrGlyPheLeuHisMetAspC
1621 GCGAGATCAAGGGCCGCCCCTGCTGCATCGGCACCAAGGGCAGCT
ysGluIleLysGlyArgProCysCysIleGlyThrLysGlySerC
1666 GTGAGATCACCACCCGGGAATACTGTGAGTTCATGCACGGCTATT
ysGluIleThrThrArgGluTyrCysGluPheMetHisGlyTyrP
1711 TCCATGAGGAAGCAACACTCTGCTCCCAGGTGAGGCGAGGCAGGC

heHisGluGluAlaThrLeuCysSerGlnValArgArgGlyArgP
1756 CTGGAGTAGTGGAGGAGAGGACGCTGGGCATGGCAGCCTGCTGGG
roGlyValValGluGluArgThrLeuGlyMetAlaAlaCysTrpG
1801 GCCGGGGCTCACGCACTCCCTCCCATGTCGGAGCCTCAGACTCAG
lyArgGlySerArgThrProSerHisValGlyAlaSerAspSerA
1846 CCTGCTTCTGGGGCGCTGAGCACCATATGCCCACTCCCAGGTGCA
laCysPheTrpGlyAlaGluHisHisMetProThrProArgCysT
1891 CTGCTTGGACAAGGTGTGTGGCTGCTGCCCTTCCTCAACCCTGA
hrAlaTrpThrArgCysValGlyCysCysProSerSerThrLeuA
1936 GGTCCCAGATCAGTTCTACAGGCTCTGGCTGTCTCTCTTCCTACA
rgSerGlnIleSerSerThrGlySerGlyCysLeuSerSerTyrM
1981 TGCTGGCGTGGTGCACTGCCTCGTGTCTGTGGTCTTTCAAATGAC
etLeuAlaTrpCysThrAlaSerCysLeuTrpSerPheLys (SEQ ID NO:32)
2026 CATCCTGAGGGACCTGGAGAAGCTGGCCGGCTGGCACCGTATCGC
2071 CATCATCTTCATCCTCAGTGGCATCACAGGCAACCTCGCCAGTGC
2116 CATCTTTCTCCCATACCGGGCAGAGGTGGGCCCGGCCGGCTCACA
2161 GTTCGGCCTCCTCGCCTGCCTCTTCGTGGAGCTCTTCCAGAGCTG
2206 GCCGCTGCTGGAGAGGCCCTGGAAGGCCTTCCTCAACCTCTCGAC
2251 CATCGTGCTCTTCCTGTTCATCTGTGGCCTCCTGCCCTGGATCGA
2296 CAACATCGCCCACATCTTCGGCTTCCTCAGTGGCCTGCTGCTGGC
2341 CTTCGCCTTCCTGCCCTACATCACCTTCGGCACCAGCGACAAGTA
2386 CCGCAAGCGGGCACTCATCCTGGTGTCACTGCTGGCCTTTGCCGG
2431 CCTCTTCGCCGCCCTCGTGCTGTGGCTGTACATCTACCCCATTAA
2476 CTGGCCCTGGATCGAGCACCTCACCTGCTTCCCCTTCACCAGCCG
2521 CTTCTGCGAGAAGTATGAGCTGGACCAGGTGCTGCACTGACCGCT
2566 GGGCCACACGGCTGCCCCTCAGCCCTGCTGGAACAGGGTCTGCCT
2611 GCGAGGGCTGCCCTCTGCAGAGCGCTCTCTGTGTGCCAGAGAGCC
2656 AGAGACCCAAGACAGGGCCCGGGCTCTGGACCTGGGTGCCCCCCT
2701 GCCAGGCGAGGCTGACTCCGCGTGAGATGGTTGGTTAAGGC (SEQ ID NO:31)

NOV17 (SEQ ID NOS:33&34)
Translated Protein - Nucleotide 289 to 2412

1 TCAATTGACTTGATATGATTTATTATTTTTACTACTTATAAGAAT

46 GGAAATAAGTTCTCCTTAGTTTTTTCTTGGAGAAAGTCTGACAT
91 GTGAGGCACAGATGAGTTATTAAAGGCAGATGACTTTCCAGCCTT
136 GTCTTAAATGTTCCATTCTTTACCTTAGAAATTATTTAAATTTGT
181 GTCCTGTCCCAGAGCATCCGCAAGGGCGCAGCCCAGTGGTTTGGA
226 GTCAGCGGCGACTGGAGGGGCAGCGGCAGCAGTGGCAGCGCCGC
271 AGCCTGCACCACTGCAGCATGCGCTACGGCCGCCTGAAGGCCTCG
MetArgTyrGlyArgLeuLysAlaSer
316 TGCCAGCGTGACCTGGAGCTCCCAGCCAGGAGGCACCGTCCTTC
CysGlnArgAspLeuGluLeuProSerGlnGluAlaProSerPhe
361 CAGGGCACTGAGTCCCCAAAGCCCTGCAAGATGCCCAAGATTGTG
GlnGlyThrGluSerProLysProCysLysMetProLysIleVal
406 GATCCGCTGGCCCGGGGCCGGGCCTTCCGCCACCCGGAGGAGATG
AspProLeuAlaArgGlyArgAlaPheArgHisProGluGluMet
451 GACAGGCCCCACGCCCTGCACCCACCGCTGACCCCCGGAGTCCTG
AspArgProHisAlaLeuHisProProLeuThrProGlyValLeu
496 TCCCTCACCTCCTTCACCAGTGTCCGTTCTGGCTACTCCCACCTG
SerLeuThrSerPheThrSerValArgSerGlyTyrSerHisLeu
541 CCACGCCGCAAGAGAATGTCTGTGGCCCACATGAGCTTGCAAGCT
ProArgArgLysArgMetSerValAlaHisMetSerLeuGlnAla
586 GCCGCTGCCCTCCTCAAGGGGCGCTCGGTGCTGGATGCCACCGGA
AlaAlaAlaLeuLeuLysGlyArgSerValLeuAspAlaThrGly
631 CAGCGGTGCCGGGTGGTCAAGCGCAGCTTTGCCTTCCCGAGCTTC
GlnArgCysArgValValLysArgSerPheAlaPheProSerPhe
676 CTGGAGGAGGATGTGGTCGATGGGGCAGACACGTTTGACTCCTCC
LeuGluGluAspValValAspGlyAlaAspThrPheAspSerSer
721 TTTTTTAGTAAGGAAGAAATGAGCTCCATGCCTGATGATGTCTTT
PhePheSerLysGluGluMetSerSerMetProAspAspValPhe
766 GAGTCCCCCCCACTCTCTGCCAGCTACTTCCGAGGGATCCCACAC
GluSerProProLeuSerAlaSerTyrPheArgGlyIleProHis
811 TCAGCCTCCCCTGTCTCCCCCGATGGGGTGCAAATCCCTCTGAAG
SerAlaSerProValSerProAspGlyValGlnIleProLeuLys
856 GAGTATGGCCGAGCCCCAGTCCCCGGGCCCCGGCGCGGCAAGCGC
GluTyrGlyArgAlaProValProGlyProArgArgGlyLysArg
901 ATCGCCTCCAAGGTGAAGCACTTTGCCTTTGATCGGAAGAAGCGG

IleAlaSerLysValLysHisPheAlaPheAspArgLysLysArg
946 CACTACGGCCTCGGCGTGGTGGGCAACTGGCTGAACCGCAGCTAC
HisTyrGlyLeuGlyValValGlyAsnTrpLeuAsnArgSerTyr
991 CGCCGCAGCATCAGCAGCACTGTGCAGCGGCAGCTGGAGAGCTTC
ArgArgSerIleSerSerThrValGlnArgGlnLeuGluSerPhe
1036 GACAGCCACCGGCCCTACTTCACCTACTGGCTGACCTTCGTCCAT
AspSerHisArgProTyrPheThrTyrTrpLeuThrPheValHis
1081 GTCATCATCACGCTGCTGGTGATTTGCACGTATGGCATCGCACCC
ValIleIleThrLeuLeuValIleCysThrTyrGlyIleAlaPro
1126 GTGGGCTTTGCCCAGCACGTCACCACCCAGCTGGTGCTGCGGAAC
ValGlyPheAlaGlnHisValThrThrGlnLeuValLeuArgAsn
1171 AAAGGTGTGTACGAGAGCGTGAAGTACATCCAGCAGGAGAACTTC
LysGlyValTyrGluSerValLysTyrIleGlnGlnGluAsnPhe
1216 TGGGTTGGCCCCAGCTCGATTGACCTGATCCACCTGGGGGCCAAG
TrpValGlyProSerSerIleAspLeuIleHisLeuGlyAlaLys
1261 TTCTCACCCTGCATCCGGAAGGACGGGCAGATCGAGCAGCTGGTG
PheSerProCysIleArgLysAspGlyGlnIleGluGlnLeuVal
1306 CTGCGCGAGCGAGACCTGGAGCGGGACTCAGGCTGCTGTGTCCAG
LeuArgGluArgAspLeuGluArgAspSerGlyCysCysValGln
1351 AATGACCACTCCGGCTGCATCCAGACCCAGCGGAAGGACTGCTCG
AsnAspHisSerGlyCysIleGlnThrGlnArgLysAspCysSer
1396 GAGACTTTGGCCACTTTTGTCAAGTGGCAGGATGACACTGGGCCC
GluThrLeuAlaThrPheValLysTrpGlnAspAspThrGlyPro
1441 CCCATGGACAAGTCTGATCTGGGCCAGAAGCGGACTTCGGGGGCT
ProMetAspLysSerAspLeuGlyGlnLysArgThrSerGlyAla
1486 GTCTGCCACCAGGACCCCAGGACCTGCGAGGAGCCAGCCTCCAGC
ValCysHisGlnAspProArgThrCysGluGluProAlaSerSer
1531 GGTGCCCACATCTGGCCCGATGACATCACTAAGTGGCCGATCTGC
GlyAlaHisIleTrpProAspAspIleThrLysTrpProIleCys
1576 ACAGAGCAGGCCAGGAGCAACCACACAGGCTTCCTGCACATGGAC
ThrGluGlnAlaArgSerAsnHisThrGlyPheLeuHisMetAsp
1621 TGCGAGATCAAGGGCCGCCCCTGCTGCATCGGCACCAAGGGCAGC
CysGluIleLysGlyArgProCysCysIleGlyThrLysGlySer
1666 TGTGAGATCACCACCCGGGAATACTGTGAGTTCATGCACGGCTAT

CysGluIleThrThrArgGluTyrCysGluPheMetHisGlyTyr
1711 TTCCATGAGGAAGCAACACTCTGCTCCCAGGTGCACTGCTTGGAC
PheHisGluGluAlaThrLeuCysSerGlnValHisCysLeuAsp
1756 AAGGTGTGTGGGCTGCTGCCCTTCCTCAACCCTGAGGTCCCAGAT
LysValCysGlyLeuLeuProPheLeuAsnProGluValProAsp
1801 CAGTTCTACAGGCTCTGGCTGTCTCTCTTCCTACATGCTGGCGTG
GlnPheTyrArgLeuTrpLeuSerLeuPheLeuHisAlaGlyVal
1846 GTGCACTGCCTCGTGTCTGTGGTCTTTCAAATGACCATCCTGAGG
ValHisCysLeuValSerValValPheGlnMetThrIleLeuArg
1891 GACCTGGAGAAGCTGGCCGGCTGGCACCGTATCGCCATCATCTTC
AspLeuGluLysLeuAlaGlyTrpHisArgIleAlaIleIlePhe
1936 ATCCTCAGTGGCATCACAGGCAACCTCGCCAGTACCATCTTTCTC
IleLeuSerGlyIleThrGlyAsnLeuAlaSerThrIlePheLeu
1981 CCATACCGGGCAGAGGTGGGCCCGGCCGGCTCACAGTTCGGCCTC
ProTyrArgAlaGluValGlyProAlaGlySerGlnPheGlyLeu
2026 CTCGCCTGCCTCTTCGTGGAGCTCTTCCAGAGCTGGCCGCTGCTG
LeuAlaCysLeuPheValGluLeuPheGlnSerTrpProLeuLeu
2071 GAGAGGCCCTGGAAGGCCTTCCTCAACCTCTCGACCATCGTGCTC
GluArgProTrpLysAlaPheLeuAsnLeuSerThrIleValLeu
2116 TTCCTGTTCATCTGTGGCCTCCTGCCCTGGATCGACAACATCGCC
PheLeuPheIleCysGlyLeuLeuProTrpIleAspAsnIleAla
2161 CACATCTTCGGCTTCCTCAGTGGCCTGCTGCTGGCCTTCGCCTTC
HisIlePheGlyPheLeuSerGlyLeuLeuLeuAlaPheAlaPhe
2206 CTGCCCTACATCACCTTCGGCACCAGCGACAAGTACCGCAAGCGG
LeuProTyrIleThrPheGlyThrSerAspLysTyrArgLysArg
2251 GCACTCATCCTGGTGTCACTGCTGGCCTTTGCCGGCCTCTTCGCC
AlaLeuIleLeuValSerLeuLeuAlaPheAlaGlyLeuPheAla
2296 GCCCTCGTGCTGTGGCTGTACATCTACCCCATTAACTGGCCCTGG
AlaLeuValLeuTrpLeuTyrIleTyrProIleAsnTrpProTrp
2341 ATCGAGCACCTCACCTGCTTCCCCTTCACCAGCCGCTTCTGCGAG
IleGluHisLeuThrCysPheProPheThrSerArgPheCysGlu
2386 AAGTATGAGCTGGACCAGGTGCTGCACTGACCGCTGGGCCACACG
LysTyrGluLeuAspGlnValLeuHis (SEQ ID NO:34)

2431 GCTGCCCCTCAGCCCTGCTGGAACAGGGTCTGCCTGCGAGGGCTG
2476 CCCTCTGCAGAGCGCTCTCTGTGTGCCAGAGAGCCAGAGACCCAA
2521 GACAGGGCCCGGGCTCTGGACCTGGGTGCCCCCTGCCAGGCGAG
2566 GCTGACTCCGCGTGAGATGGTTGGTTAAGGC (SEQ ID NO:33)

NOV18 (SEQ ID NOS:35&36)
Translated Protein -Nucleotide 135 to 545

1 GGACCATCTGGGTGCGTTTTTGTCCAAAAGATGCAATATTCAGA
46 CTGACTGACCCCCTGCGTTATTTCACCAAAGACACGATGCATAGT
91 CACCCCGGCCTTGTTTCTCCAATGGCCGTGATACACTAGTGATCA
M
136 TGTTCAGCCCTGCTTCCACCTGCATAGAATCTTTTCTTCTCAGAC
etPheSerProAlaSerThrCysIleGluSerPheLeuLeuArgG
181 AGGGACAGTGCAGCCTCAACATCTCCTGGAGTCTAGAAGCTGTTT
lnGlyGlnCysSerLeuAsnIleSerTrpSerLeuGluAlaValS
226 CCTTTCCCCTCCTTCCTCCTCTTGCTCTAGCCTTAATACTGGCCT
erPheProLeuLeuProProLeuAlaLeuAlaLeuIleLeuAlaP
271 TTTCCCTCCCTGCCCCAAGTGAAGACAGGGCACTCTGCGCCCACC
heSerLeuProAlaProSerGluAspArgAlaLeuCysAlaHisH
316 ACATGCACAGCTGTGCATGGAGACCTGCAGGTGCACGTGCTGGAA
isMetHisSerCysAlaTrpArgProAlaGlyAlaArgAlaGlyT
361 CACGTGTGGTTCCCCCCTGGCCCAGCCTCCTCTGCAGTGCCCCTC
hrArgValValProProTrpProSerLeuLeuCysSerAlaProL
406 TCCCCTGCCCATCCTCCCCACGGAAGCATGTGCTGGTCACACTGG
euProCysProSerSerProArgLysHisValLeuValThrLeuV
451 TTCTCCAGGGGTCTGTGATGGGGCCCCTGGGGGTCAGCTTCTGTC
alLeuGlnGlySerValMetGlyProLeuGlyValSerPheCysP
496 CCTCTGCCTTCTCACCTCTTTGTTCCTTTCTTTTCATGTATCCAT
roSerAlaPheSerProLeuCysSerPheLeuPheMetTyrProP
541 TCAGTTGATGTTTATTGAGCAACTACAGATGTCAGCACTGTGTTA
heSer (SEQ ID NO:36)

586 GGTGCTGGGGGCCCTGCGTGGGAAGATAAAGTTCCTCCCTCAAGG
631 ACTCCCCATCCAGCTGGGAGACAGACAACTAACTACACTGCACCC
676 TGCGGTTTGCAAGGGGCTCCTGCCTGGCTC (SEQ ID NO:35)

NOV19 (SEQ ID NOS:37&38)
Translated Protein - Frame: 2 - Nucleotide 389 to 856

1 GACTCACTATAGGGCGAATTGGGTCTTCTTCCAGAATTCTGGCCA
46 TGGGGATCCAGACTTACTCACTATAGGGCTCGAGCGGCCGCCCGG
91 GCAGGTCTAAGCAAGGGGAGGGATTAGAGCCTCCTTCCTCTCTGC
136 CCCTCCCCATGGGTCTCTAGGGGGCTGGTGCAGGCAGCAGCAGAG
181 GCACTCTGGGCAGCTGGGTGAGGGCCCATCTGGGCAAGGCCCCCA
226 GCGCCTGCCTTCTCTCCCGGGGCCCTGTGGGCAAGCCTCCTGCTT
271 CACTTTCAGGTTTCTCGAAGTGCCTTCTTGCTCCTGTCTGTTTCC
316 CCATCCTGCCAGATTTCTGTTTCTCTTGCTGGGCTTTTGGCAGTA
361 GGGGGCTGTGTTGGTGGCCCTACGAAGATGCTCAGTGCTCGAGA
MetLeuSerAlaArgAs
406 TCGCCGGGACCGGCACCCTGAGGAGGGGGTAGTTGCAGAGCTCCA
pArgArgAspArgHisProGluGluGlyValValAlaGluLeuGl
451 GGGCTTCGCGGTGGACAAGGCCTTCCTCACCTCCCACAAGGGCAT
nGlyPheAlaValAspLysAlaPheLeuThrSerHisLysGlyIl
496 CCTGCTGGAAACCGAGCTGGCCCTGACCCTCATCATCTTCATCTG
eLeuLeuGluThrGluLeuAlaLeuThrLeuIleIlePheIleCy
541 CTTCACGGCCTCCATCTCTGCCTACATGGCCGCGGCGCTACTGGA
sPheThrAlaSerIleSerAlaTyrMetAlaAlaAlaLeuLeuGl
586 GTTCTTCATCACACTTGCCTTCCTCTTCCTCTATGCCACCCAGTA
uPhePheIleThrLeuAlaPheLeuPheLeuTyrAlaThrGlnTy
631 CTACCAGCGCTTCGACCGAATTAACTGGCCCTGTCTGGACTTCCT
rTyrGlnArgPheAspArgIleAsnTrpProCysLeuAspPheLe
676 GCGCTGTGTCAGTGCCATCATCATCTTCCTGGTGGTCTCCTTTGC
uArgCysValSerAlaIleIleIlePheLeuValValSerPheAl
721 AGCTGTGACCTCCCGGGACGGAGCTGCCATTGCTGCTTTTGTTTT

aAlaValThrSerArgAspGlyAlaAlaIleAlaAlaPheValPh
766 TGGCATCATCCTGGTTTCCATCTTTGCCTATGATGCCTTCAAGAT
eGlyIleIleLeuValSerIlePheAlaTyrAspAlaPheLysIl
811 CTACCGGACTGAGATGGCACCCGGGGCCAGCCAGGGGACCAGCA
eTyrArgThrGluMetAlaProGlyAlaSerGlnGlyAspGlnGl
856 GTGACTCTGGGGCTACCTGGCTCCTAGGCCCAGCCAGCCAGAGAG
n (SEQ ID NO:38)
901 GACAGTGGAGCCCAGACACGTCTCCTTGGGATTCACTAGCCCCCA
946 GCCCGCCAAACCCCACCCCAACCCTACACAGCAGTCTGGCCTGAG
991 ACGTCACTGGGGACTTATCTGTGGAGCCTGGTGCTCCAGGATGTG
1036 GCTTCTCATGAAGCTCTGGCCAGAGGAGGGAACTTATTGGGGG
1081 GGGGGGTGGAGGGGAGGAATCTGGACCTCTAAGTCATTCCCAAA
1126 TTAAAATATTCAAATTCTTAAAAAA (SEQ ID NO:37)

NOV20 (SEQ ID NOS:39&40)
Translated Protein - Nucleotide 505 to 1284.

1 CTGGGGCCTTACCTACTAGCGGAATCGACTGAAGAGACGCCTGCC
46 AGTGCGGGAGGTAGGAAGCTCGATCCCCAAAGAAAAGAGCGAGTG
91 GGCAGGCAGCTGCGAGACAGAACCGGAGTGTGCAGGGTCCCTAGA
136 GGCCGGTTCCTGGTCTGTGCTGCTCTCCTGGAAGCCATGGTACAG
181 GCAGAGCTCAGGGCGATCCCCAGGTGAGGGCAGCGGCTCTGCCTG
226 GGATTCCACCGCAGTACAACCGGGTAGATGCGGGGTGGAGAAGAA
271 AGGATGTTGCCTGCACTGCTCGCCAATAGCACCCTGAGAGGCTAC
316 ATTTGCAGAAGCAGCAGCAGCAGAAGACACAGCGCCGGTCCAGGA
361 GGCGGCTCGAGCTGTTCGTAAAGTCGCCCGACAGCTTTTTCTCCG
406 TAGTATGCGAGTTGACAAAACAGCCAGAGAACAGGGCTCCCCATT
451 ACAATCTTTTCGAGATCTTTTCCCTTGCTAACCGGATCTGATTTG
496 TGCGAAAACATGCCTTGCACTTGTACCTGGAGGAACTGGAGACAG
MetProCysThrCysThrTrpArgAsnTrpArgGln
541 TGGATTCGACCTTTAGTAGCGGTCATCTACCTGGTGTCAATAGTG
TrpIleArgProLeuValAlaValIleTyrLeuValSerIleVal
586 GTTGCGGTTCCCCTATGCGTGTGGGAATTACAGAAACTGGAGGTT

ValAlaValProLeuCysValTrpGluLeuGlnLysLeuGluVal
631 GGAATACACACCAAGGCTTGGTTTATTGCTGGAATCTTTTTGCTG
GlyIleHisThrLysAlaTrpPheIleAlaGlyIlePheLeuLeu
676 TTGACTATTCCTATATCACTGTGGGTGATATTGCAACACTTAGTG
LeuThrIleProIleSerLeuTrpValIleLeuGlnHisLeuVal
721 CATTATACACAACCTGAACTACAAAAACCAATAATAAGGATTCTT
HisTyrThrGlnProGluLeuGlnLysProIleIleArgIleLeu
766 TGGATGGTACCTATTTACAGTTTAGATAGTTGGATAGCTTTGAAA
TrpMetValProIleTyrSerLeuAspSerTrpIleAlaLeuLys
811 TATCCCGGAATTGCAATATATGTGGATACCTGCAGAGAATGCTAT
TyrProGlyIleAlaIleTyrValAspThrCysArgGluCysTyr
856 GAAGCTTATGTAATTTACAACTTTATGGGATTCCTTACCAATTAT
GluAlaTyrValIleTyrAsnPheMetGlyPheLeuThrAsnTyr
901 CTAACTAACCGGTATCCAAATCTGGTATTAATCCTTGAAGCCAAA
LeuThrAsnArgTyrProAsnLeuValLeuIleLeuGluAlaLys
946 GATCAACAGAAACATTTCCCTCCTTTATGTTGCTGTCCACCATGG
AspGlnGlnLysHisPheProProLeuCysCysCysProProTrp
991 GCTATGGGAGAAGTATTGCTGTTTAGGTGCAAACTAGGTGTATTA
AlaMetGlyGluValLeuLeuPheArgCysLysLeuGlyValLeu
1036 CAGTACACAGTTGTCAGACCTTTCACCACCATCGTTGCTTTAATC
GlnTyrThrValValArgProPheThrThrIleValAlaLeuIle
1081 TGTGAGCTGCTTGGTATATATGACGAAGGAACTTTAGCTTTTCA
CysGluLeuLeuGlyIleTyrAspGluGlyAsnPheSerPheSer
1126 AATGCTTGGACTTATTTGGTTATAATAAACAACATGTCACAGTTG
AsnAlaTrpThrTyrLeuValIleIleAsnAsnMetSerGlnLeu
1171 TTTGCCATGTATTGTCTCCTGCTCTTTTATAAAGTACTAAAAGAA
PheAlaMetTyrCysLeuLeuLeuPheTyrLysValLeuLysGlu
1216 GAACTGAGCCCAATCCAACCTGTTGGCAAATTTCTTTGTGTAAAG
GluLeuSerProIleGlnProValGlyLysPheLeuCysValLys
1261 CTGGTGGTTTTTGTTTCTTTTTGGTAAGTGTTACTTTTTTTTAAA
LeuValValPheValSerPheTrp (SEQ ID NO:40)
1306 TGTTCTCATTTTTTTAAGGGCAGTAAAAACCGTTGATTAAGGAGG
1351 ATTTTTAAACAGTCTTAATGCGGAAGATAGATTAAAATGTCTCTA
1396 CTTCTCTTTTTAAAAGTTCATCTTTTTAGCCCTTCTACAATTTTC

1441 AAAAGAAATAATTAGATGGTCGCTGTAACATTTATATGAAGAAAA
1486 TAGTTTGAGACAACCTAAATATGTCAATACTAGAATAATTATTAA
1531 AATAAATCATGGCCCTGTCATATAATAGAATACTATGGAGTTTGG
1576 AAGAAAGCATGATGTAGAATATTTAATTATATGGGA (SEQ ID NO:39)

Below follows a brief description of the NOVX polypeptides and nucleic acids described in Table 1. Additional utilities for NOVX nucleic acids and polypeptides according to the invention are also disclosed herein.

### NOV1

A NOV1 nucleic acid molecule according to the invention includes the nucleic acid sequence (SEQ ID NO:1), which is present in clone 889240. SEQ ID NO:1 includes 836 bp coding for a protein resembling T1/ST2, a receptor binding polypeptide. This nucleotide sequence has an open reading frame encoding a polypeptide of 169 amino acid residues (SEQ ID NO:2) with a predicted molecular weight of 19662.4 Da. The start codon is at nucleotides 189-191 and the stop codon is at nucleotides 696-698. The protein of SEQ ID NO:2 is predicted by the PSORT program to localize extracellularly with a certainty of 0.8200. The program SignalP predicts that there is a signal peptide, with the most likely cleavage site between residues 27 and 28 in the sequence AAG-FT.

In the encoded polypeptide, 85 of 147 residues (57%) are identical to, and 107 of 147 residues (72%) are positive with, the 227 residue human putative T1/ST2 receptor binding protein precursor (ACC:Q13445). The polypeptide also has 154 of 158 residues (97%) identical to, and 155 of 158 residues (98%) positive with, a 229 residue human CGI-100 protein identified by comparative gene cloning using the *Caenorhabditis elegans* proteome as template (SPTREMBL-ACC:Q9Y3A6).

In addition, the protein has 154 of 158 residues (97%) identical to, and 155 of 158 residues (98%) positive with, a 229 residue human protein disclosed as having activities as a cytokine, an immune system regulator, a tissue growth regulator, a T1 receptor-like ligand II and a p24 vesicle-trafficking protein and agonist (WO9836068; WO9807754; WO9946281; and WO9931236).

T1/ST2 is a receptor-like molecule homologous to the type I interleukin-1 receptor. T1/ST2 is expressed constitutively and stably on the surface ofT helper type 2 (Th2) cells, but not on Th1 cells. T1/ST2 is also expressed on mast cells.

NOV1 is found in fetal liver, thyroid, fetal kidney, and spleen. The proteins of the invention encoded by a NOV1 nucleic acid sequence include the full protein disclosed as being encoded by the ORF described herein, as well as any mature protein arising therefrom as a result of posttranslational modifications. Thus the proteins of the invention encompass both a precursor and any active forms of the NOV1 protein.

The similarity of NOV1 to a putative ligand for the Interleukin 1 Receptor-related T1/ST2 gene suggests that this novel sequence may function as a ligand for a receptor that has homology to the interleukin-1 receptor family. These receptors play an important role in the immune response system and, therefore, the novel gene can be implicated in similar receptor-ligand systems in the immune response pathway. The novel gene can be therapeutically used as a diagnostic or prognostic marker, protein therapeutic and antibody target or small molecule drug target to treat disorder in the immune response pathway.

### NOV2

A NOV2 nucleic acid sequence of the invention includes the nucleotide sequence of SEQ ID NO:3. The nucleotide sequence (SEQ ID NO:3) includes an open reading frame encoding a polypeptide of 547 amino acid residues (SEQ ID NO:4). The open reading frame begins with a start codon at nucleotides 110-112 and ends with a stop codon at nucleotides 1751-1753. The protein of SEQ ID NO:4 is predicted by the PSORT program to localize in the nucleus with a certainty of 0.7000. No N-terminal signal sequence is predicted for this protein.

The disclosed polypeptide has 188 of 342 amino acid residues (54%) identical to, and 265 of 342 (77%) residues positive with, the 674 residue protein fragment encoded in human KIAA0554 PROTEIN (ACC:060301). In addition, NOV2 has 300 of 544 residues (55%) identical to, and 401 of 544 residues (73%) positive with, the 545 residue human CDC42-interacting protein 4 (ACC:O15184).

In addition, the protein has 60% identity and 74% similarity over 246 residues to the 265 residue human Src homology 3 domain (SH3)-containing protein 1; and 50% identity and 67% similarity over 168 residues to the 175 residue human SH3-containing protein 2 (US Patent No. 5,916,753, issued June 29, 1999). These proteins can be used for the diagnosis, treatment or prevention of cancer and immune or development disorders.

The results in Example 2, *infra*, indicate that NOV2 is preferentially expressed in various tissues, including several cancer cell lines (*e*.*g*., osteosarcoma, thyroid gland, fetal brain, placenta, pancreas, uterus, fetal lung, and in an RNA pool from adrenal gland, mammary gland, prostate gland, testis, uterus, bone marrow, melanoma, pituitary, thyroid and spleen.

The proteins of the invention encoded by a NOV2 nucleic acid include the full protein disclosed as being encoded by the ORF described herein, as well as any mature protein arising therefrom as a result of posttranslational modifications. Thus the proteins of the invention encompass both a precursor and any active forms of the NOV2 protein.

### NOV3

A NOV3 nucleic acid according to the invention can include the nucleic acid sequence of SEQ ID NO:5. The nucleotide sequence of this clone (SEQ ID NO:5) is 711 bp in length and has an open reading frame encoding a polypeptide of 115 amino acid residues (SEQ ID NO:6) with a predicted molecular weight of 53945.0 Da. The start codon of this open reading frame is at nucleotides 143-145 and the stop codon is at nucleotides 488-490. The protein of SEQ ID NO:6 is predicted by the PSORT program to localize to the plasma membrane with a certainty of 0.9190. The program SignalP predicts that there is probably a signal peptide, with the most likely cleavage site between residues 19 and 20: AQA-LD.

The encoded polypeptide has 41 of 97 residues (42%) identical to, and 47 of 97 residues (48%) positive with, the 128 residue human E48 antigen precursor ACC:Q14210). The encoded polypeptide also has 111 of 116 residues (95%) identical to, and 112 of 116 residues (96%) positive with, the 117 residue human secreted protein encoded by gene 89 (WO9902546).

NOV3 is expressed in the heart. It is also expressed in kidney, thalamus, bone marrow, adrenal gland and/or suprarenal gland, and fetal brain.

Proteins provided by a NOV3 nucleic acid include the full protein disclosed as being encoded by the ORF described herein, as well as any mature protein arising therefrom as a result of posttranslational modifications. Thus the proteins of the invention encompass both the precursors and the active forms of the NOV3 protein.

### NOV4

NOV4 is believed to be expressed in heart, bone marrow, spleen, and thalamus. A NOV4 nucleic acid of the invention can include the nucleotide sequence of SEQ ID NO:7. This clone is 1987 bp in length and includes an open reading frame encoding a polypeptide of 152 amino acid residues (SEQ ID NO:8). The start codon is at nucleotides 991-993 and the stop codon is at nucleotides 1447-1449. The protein of SEQ ID NO:8 is predicted by the PSORT program to localize to the microbody (peroxisome) with a certainty of 0.6400. There most likely is no signal peptide present.

The disclosed NOV4 protein has 90 of 100 residues (90%) identical to, and 93 of 100 residues (93%) positive with, the 102 residue expressed sequence tag from human breast tumour-associated protein 47 (DE19813835).

Proteins encoded by a NOV4 nucleic acid sequence include the full protein disclosed as being encoded by the ORF described herein, as well as any mature protein arising therefrom as a result of posttranslational modifications. Thus, the proteins of the invention encompass both the precursors and the active forms of the NOV4 protein.

### NOV5, NOV21, and NOV22

Also included in the invention are NOV5, NOV21, and NOV22, which include related nucleic acids and their encoded polypeptides.

### NOV5

A NOV5 nucleic acid according to the invention includes 1423 nucleotides of SEQ ID NO:9. This nucleic acid encodes a novel thyroid hormone binding protein-like protein from an open reading frame (ORF) beginning with an ATG initiation codon at nucleotide 587 and ending with a stop codon at nucleotide 1343. The encoded polypeptide has 252 amino acid residues, which have the amino acid sequence of SEQ ID NO:10.

The encoded polypeptide has 75 of 224 residues (33%) identical to, and 124 of 224 residues (55%) positive with, the 510 residue bovine protein disulfide isomerase precursor (PDI) (EC 5.3.4.1) (prolyl 4- hydroxylase beta subunit) (cellular thyroid hormone binding protein) (ACC:P05307). In addition, the encoded polypeptide has 73 of 224 residues (32%), identical to, and 121 of 224 residues (54%) positive with, the 508 residue human protein disulfide isomerase precursor (PDI) (EC 5.3.4.1) (prolyl 4- hydroxylase beta subunit) (cellular thyroid hormone binding protein) (p55) (ACC:P07237). PDI, the beta subunit of prolyl 4-hydroxylase, and the cellular thyroid hormone binding protein are identical (see, for example, Yamauchi K, et al. *Biochem Biophys Res Commun* 146(3):1485-1492 (1987)). The catalytic activity of NOV5 includes the rearrangement of both intrachain and interchain disulfide bonds in proteins to form the native structures. Its subcellular location is in the endoplasmic reticulum lumen. It contains two thioredoxin domains.

PSORT analysis predicts that the disclosed NOV5 polypeptide is localized in the plasma membrane with a certainty of 0.4600. Using SIGNALP analysis, the protein of the invention has a cleavable N-terminal signal sequence with the cleavage site most likely occurring between positions 25 and 26 (VAA-EV). The predicted molecular weight of the protein of the invention is 28141.9 daltons. The NOV5 protein differs at two positions from the proteins encoded by the NOV21 and NOV22 nucleic acid sequences described below. The disclosed NOV21 and NOV22 polypeptides are identical in sequence.

Thyroid hormone receptors (TRs) are members of the steroid honnone/retinoic acid receptor superfamily. Members of this family regulate homeostasis, development, and differentiation. Their transcriptional activity is modulated by the thyroid hormone 3,3',5-triiodo-L-thyronine (T3). Lee et al., Biochem Biophys Res Commun 222(3):839-43 (1996), found that expression of, as well as insulin binding to, cellular thyroid hormone binding protein, but not insulin degrading enzyme, is increased during 3T3-L1 adipocyte differentiation. Thus, cellular thyroid hormone binding protein may play a role in regulating some insulin action, especially the counter-regulation occurring between insulin and other hormones during adipocyte differentiation.

NOV5 is highly expressed in the mammary gland.

Proteins encoded by a NOV5 nucleic acid of the invention include the full protein disclosed as being encoded by the ORF described herein, as well as any mature protein arising therefrom as a result ofposttranslational modifications. Thus the proteins of the invention encompass both the precursors and the active forms of the NOV5 protein.

NOV5 nucleic acids and proteins according to the invention are useful in potential therapeutic applications implicated in the following disorders and pathologies: diabetes, metabolic and endocrine disorders, developmental disorders, and/or other pathologies and disorders. For example, a cDNA encoding the thyroid hormone binding protein-like protein may be useful in thyroid hormone binding protein therapy. Similarly, the thyroid hormone binding protein-like protein may be useful when administered to a subject in need thereof. The novel nucleic acid encoding thyroid hormone binding protein-like protein, as well as the thyroid hormone binding protein-like protein of the invention, or fragments thereof, may further be useful in diagnostic applications, wherein the presence or amount of the nucleic acid or the protein need to be assessed. These materials are further useful in the thyroid hormone binding protein ration of antibodies that immunospecifically bind to the novel substances of the invention for use in therapeutic or diagnostic methods.

### NOV21

A NOV21 nucleic acid sequence according to the invention includes the nucleic acid sequence of SEQ ID NO:41. The nucleotide sequence (SEQ ID NO:41) has 1918 bp and has an open reading frame encoding a polypeptide of 252 amino acid residues (SEQ ID NO:42). The start codon is at nucleotides 1082-1084 and the stop codon is at nucleotides 1838-1840. The protein of SEQ ID NO:42 is predicted by the PSORT program to localize in the plasma membrane with a certainty of 0.4600. The program SignalP predicts that the disclosed NOV21 protein has a cleavable N-terminal signal peptide with the most likely cleavage site between residues 25 and 26: VAA-EV. The dislcosed NOV21 protein differs at two positions from the protein encoded by a NOV5 nucleic acid (see above) and is identical to the protein encoded by the NOV22 nucleic acid sequence (see below).

The disclosed NOV21 polypeptide has 75 of 224 residues (33%) identical to, and 124 of 224 residues (55%) positive with, the 510 residue bovine protein disulfide isomerase precursor (PDI) (EC 5.3.4.1) (prolyl 4-hydroxylase beta subunit) (cellular thyroid hormone binding protein) (ACC:P05307).

NOV21 proteins according to the invention include the full protein disclosed as being encoded by the ORF described herein, as well as any mature protein arising therefrom as a result of posttranslational modifications. Thus the proteins of the invention encompass both the precursors and the active forms of the NOV21 protein.

### NOV22

A NOV22 nucleic acid sequence according to the invention includes the nucleic acid sequence of SEQ ID NO:43. The nucleotide sequence includes 1914 nucleotides. An open reading frame was identified beginning with an ATG initiation codon at nucleotides 1078-1080 and ending with a stop codon at nucleotides 1834-1836. One skilled in this art will recognize that the sequence presented as SEQ ID NO:43 is the reverse complement of the coding strand. The encoded polypeptide has 252 amino acid residues (SEQ ID NO:44). The encoded NOV22 polypeptide differs at two positions from the NOV5 protein (see above) and is identical to the NOV21 protein (see above).

The encoded polypeptide has 125 of 224 amino acid (55 %) homology to *Bos taurus* protein disulfide isomerase precursor (PDI) (EC 5.3.4.1) (prolyl-4-hydroxylase beta subunit)(cellular thyroid hormone binding protein)(p55) (ACC: P05307). The disclosed nucleotide sequence has 395 of 694 nucleotides (56 %) identity/homology to *Homo sapien* disulfide isomerase precursor (PDIp) mRNA (GENBANK ID:HSU19948|acc:U19948).

PSORT analysis predicts the protein of the invention to be localized in the plasma membrane with a certainty of 0.4600. Using the SIGNALP analysis, it is predicted that the protein of the invention seems to have a cleavable N-term signal sequence with most likely cleavage site between positions 25 and 26: VAA-EV. The predicted molecular weight of the protein of the invention is 28141.9 daltons.

The NOV5, NOV21, AND NOV22 nucleic acids and proteins are expressed in primarily in pancreas and thyroid, and additionally in peripheral blood, lymph node, bone, breast, ovary, kidney, lung, heart, parathyroid, brain, bone marrow, tonsils, adrenal gland and liver.

The NOV5, NOV21, AND NOV22 nucleic acids and proteins are useful as protein therapeutics, antibody targets, and small molecule drug targets in potential therapeutic applications to treat immunlological diseases, thyroid and metabolic diseases, bone metabolic disorders, diseases of the pancreas including diabetes and digestive disorders, tissue regeneration and development.

### NOV6

A NOV6 nucleic acid according to the invention includes the nucleotide sequence of (SEQ ID NO:11). This sequence is 1481 bp in length and includes an open reading frame encoding a polypeptide of 393 amino acid residues (SEQ ID NO:12). The open reading frame includes a start codon at nucleotides 183-185 and a stop codon at nucleotides 1362-1364. The encoded protein of SEQ ID NO:12 is predicted by the PSORT program to localize extracellularly with a certainty of 0.3700. The program SignalP predicts that the 3218715 protein has a cleavable N-terminal signal peptide with the most likely cleavage site between residues 22 and 23: TLS-KS.

The encoded protein has 70 of 177 residues (39%) identical to, and 107 of 177 residues (60%) positive with a 968 residue protein of *Arabidopsis thaliana* (mouse-ear cress; ACC:O04623).

NOV6 proteins of the invention include the full protein disclosed as being encoded by the ORF described herein, as well as any mature protein arising therefrom as a result of posttranslational modifications. Thus the proteins of the invention encompass both the precursors and the active forms of the NOV6 protein.

### NOV7

NOV7 was identified in pancreas. In addition, it is found in fetal brain, salivary gland, thalamus, fetal brain, spleen, heart. A NOV7 nucleotide sequence according to the invention includes the nucleic acid sequence of SEQ ID NO:13, which is 811 nucleotides in length. The disclosed nucleotide (SEQ IDNO:13) has an open reading frame encoding a polypeptide of 132 amino acid residues (SEQ ID NO:14). The start codon is at nucleotides 91-93 and the stop codon is at nucleotides 487-489. The protein of SEQ ID NO:14 is predicted by the PSORT program to localize in the plasma membrane with a certainty of 0.7000. The program SignalP predicts that there is probably a signal peptide with the most likely cleavage site between residues 57 and 58: IVA-NI.

The encoded polypeptide has 14 of 30 residues (46%) identical to, and 18 of 30 residues (60%) positive with, a 51 residue fragment of human rhodopsin (ACC:Q15309).

NOV7 was identified in pancreas. It is also found in fetal brain, salivary gland, thalamus, spleen, and heart, and in a number of other normal and cancer cell lines.

NOV7 proteins include the full protein disclosed as being encoded by the ORF described herein, as well as any mature protein arising therefrom as a result of posttranslational modifications. Thus the proteins of the invention encompass both the precursors and the active forms of the NOV7 protein.

### NOV8

A NOV8 nucleic acid according to the invention includes SEQ ID NO:15, which is 734 nucleotides in length and has an open reading frame encoding a polypeptide of 105 amino acid residues (SEQ ID NO:16). The start codon of the open reading frame is at nucleotides 146-148, and the stop codon is at nucleotides 461-463. The encoded polypeptide is predicted by the PSORT program to localize in the plasma membrane with a certainty of 0.4600. The program SignalP predicts that there is a low probability that there is a signal peptide.

The encoded protein has 11 of 19 residues (57%) identical to, and 15 of 19 residues (78%) positive with, the 30 residue fragment from human interferon alpha-1 pseudogene, 5' end precursor (ACC:E158503).

NOV8 is broadly expressed to varying extents in most normal and cancer tissues examined.

NOV8 proteins according to the invention include the full protein disclosed as being encoded by the ORF described herein, as well as any mature protein arising therefrom as a result of posttranslational modifications. Thus, the proteins of the invention encompass both the precursors and the active forms of the NOV8 protein.

### NOV9

A NOV9 nucleic acid sequence of the invention includes the nucleotide sequence of SEQ IDNO:17. SEQ ID NO:17 is 1659 nucleotides in length and has an open reading frame encoding a polypeptide of 410 amino acid residues (SEQ ID NO:18). The start codon is at nucleotides 244-246 and the stop codon is at nucleotides 1474-1476. The protein of SEQ ID NO:18 is predicted by the PSORT program to localize in the Golgi body with a certainty of 0.9000. The program SignalP predicts that there is probably no signal peptide.

The encoded NOV9 protein is 27% identical to, and 47% positive with, the 570 residue human IL-1 receptor accessory protein (ACC:O14915).

NOV9 is found in fetal brain, lymph node, pancreas, placenta, osteogenic sarcoma, kidney, placenta, salivary gland, fetal kidney, prostate, spleen, pancreas, hematopoietic stem cells, and fetal lung.

NOV9 proteins include the full protein disclosed as being encoded by the ORF described herein, as well as any mature protein arising therefrom as a result of posttranslational modifications. Thus the proteins of the invention encompass both the precursors and the active forms of the NOV9 protein.

### NOV10

A NOV10 nucleic acid sequence according to the invention includes the nucleotide sequence (SEQ ID NO:19), which is 2261 nucleotides in length. This nucleic acid sequence includes an open reading frame encoding a polypeptide of 732 amino acid residues (SEQ ID NO:20). The start codon is at nucleotides 813-815 and the stop codon is at nucleotides 3009-3011. The polypeptide of SEQ ID NO:20 is predicted by the PSORT program to localize in the nucleus with a low probability. The program SignalP predicts that there is probably no signal peptide.

The NOV10 protein has 257 of 701 residues (36%) identical to, and 360 of 701 residues (51%) positive with, the 884 residue hypothetical 96.8 kDa protein B0024.14 in chromosome V from *Caenorhabditis elegans*, (ACC:Q17429). In addition it has 142 of 529 residues (26%) identical to, and 215 of 529 residues (40%) positive with, the 810 residue human NEL-related protein (ACC:BAA11680).

The NOV10 protein has 715 of 721 residues (99%) identical to, and 716 of 721 residues (99%) positive with, the 1036 residue human secreted protein clone dj 167_19 ( WO9957132-A1.

Example 2, *infra*, indicates that NOV10 is widely expressed in most cell lines examined, with high levels of expression seen in several tumor cell lines.

NOV10 was isolated from spleen; thymus gland, heart, and adrenal gland. In addition, it is also found in brain/pituitary gland, liver, fetal liver, kidney, fetal kidney, bone, osteosarcoma, and heart.

NOV10 proteins of the invention include the full protein disclosed as being encoded by the ORF described herein, as well as any mature protein arising therefrom as a result of posttranslational modifications. Thus the proteins of the invention encompass both the precursors and the active forms of the NOV10 protein.

### NOV11

A NOV11 nucleic acid according to the invention includes the nucleotide sequence of SEQ ID NO:21, which is 1431 nucleotides in length. This nucleic acid was originally identified in heart tissue and includes an open reading frame encoding a NOV 12 polypeptide of 381 amino acid residues (SEQ ID NO:22) from positions 69-71 to positions 1212-1214 in SEQ ID NO:21.

The encoded protein has 74 of 134 residues (55%) identical to, and 96 of 134 residues (71 %) positives with, the human GAMMA-HEREGULIN protein having 768 residues (ACC:O14667). The protein is predicted to localize in the endoplasmic reticulum (membrane) with a certainty of 0.8500. There appears to be no predicted N-terminal signal peptide in the sequence.

Heregulin, is also known as neu differentiation factor (NDF) or glial growth factor 2 (GGF2). Heregulin shows homology to the protein neurestin. Neurestin, in turn, shows homology to members of the tenascin family of proteins. Heregulin is the ligand for HER-2/ErbB2/NEU, a proto-oncogene receptor tyrosine kinase implicated in breast and prostate cancer progression that was originally identified in rat neuro/glioblastoma cell lines. Ectopic expression of HER-2/ErbB2/NEU in MDA-MB-435 breast adenocarcinoma cells confers chemoresistance to Taxol-induced apoptosis relative to vector transfected control cells (Yu et al., Molec. Cell 2:581-591 (1998)).

The tenascins are a growing family of extracellular matrix proteins that play prominent roles in tissue interactions critical to embryogenesis. Overexpression of tenascins has been described in multiple human solid malignancies. The role of the tenascin family of related proteins is to regulate epithelial-stromal interactions, participate in fibronectin-dependent cell attachment and interaction. Indeed, tenascin-C (TN) is overexpressed in the stroma of malignant ovarian tumours particularly at the interface between epithelia and stroma leading to suggestions that it may be involved in the process of invasion (Wilson et al., Br J Cancer 74: 999-1004(1996)) Tenascin-C is considered a therapeutic target for certain malignant brain tumors. (Gladson, J Neuropathol Exp Neurol 58(10):1029-40(1999)).

Stromal or moderate to strong periductal Tn-C expression in DCIS correlates with tumor cell invasion. (Jahkola et al., Eur J Cancer 34(11):1687-92 (1998)); Jahkola et al., Br J Cancer. 78(11):1507-13 (1998)).

Tenascin (TN) is an extracellular matrix protein found in areas of cell migration during development and expressed at high levels in migratory glioma cells. (Treasurywala et al., Glia 24(2):236-43 (1998)). Phillips et al., J Cell Sci 111( Pt 8):1095-104 (1998)). Tenascin expression in hormone-dependent tissues of breast and endometrium indicate that Tenascin expression reflects malignant progression. (Vollmer et al., Cancer Res 52(17):4642-8 (1992)).

The disclosed NOV11 polypeptide is also related to Neurestin (Otaki JM, et al., Dev Biol 212(1):165-81 (1999)). Neurestin is a putative transmembrane molecule implicated in neuronal development. It shows homology to a neuregulin gene product, human gamma-heregulin, a Drosophila receptor-type pair-rule gene product, Odd Oz (Odz) / Ten(m), and Ten(a). It is putatively involved in synapse formation and morphogenesis. A mouse neurestin homolog, DOC4, has independently been isolated from the NIH-3T3 DOC4 is also known as tenascin M (TNM), Drosophila pair-rule gene homolog-containing extracellular EGF-like repeats.

Based on the bioactivity described in the medical literature for related molecules, aNOV11 nucleic acid or it encoded polypeptide may play a role in one or more aspects of tumor cell biology that alter the interactions of tumor epithelial cells with stromal components. For example, NOV11 may play a role in the following malignant properties: autocrine/paracrine stimulation of tumor cell proliferation; autocrine/paracrine stimulation of tumor cell survival and tumor cell resistance to cytotoxic therapy; local tissue remodeling, paranechmal and basement membrane invasion and motility of tumor cells thereby contributing to metastasis; and tumor-mediated immunosuppression of T-cell mediated immune effector cells and pathways resulting in tumor escape from immune surveillance.

Predicted disease indications from expression profiling include a subset of human gliomas, astrocytomas, mixed glioma/astrocytomas, renal cells carcinoma, breast adenocarcinoma, ovarian cancer, melanomas. Targeting of NOV11 by human or humanized monoclonal antibodies designed to disrupt predicted interactions of NOV11 with its cognate receptor may result in significant anti-tumor/anti-metastatic activity and the amelioration of associated symptomatology. Identification of small molecules that specifically and/or selectively interfere with downstream signaling components engaged by NOV11 receptor interactions would also be expected to result in significant anti-tumor/anti-metastatic activity and the amelioration of associated symptomatology. Likewise, modified antisense ribonucleotides or antisense gene expression constructs (*e.g.*, plasmids, adenovirus, adeno-associated viruses, and "naked" DNA approaches) designed to diminish the expression of NOV11 transcripts/messenger RNA (mRNA) would be anticipated based on predicted properties of NOV11 to have anti-tumor impact.

The neuregulin, glial growth factor 2, diminishes autoimmune demyelination and enhances remyelination in a chronic relapsing model for multiple sclerosis. (Cannella et al., Proc. Nat. Acad. Sci. 95:10100-10105 (1998)).

NOV11 may, in addition, be a protein involved in central nervous system myelination, localization in the extracellular matrix, and induction in neuroblastoma cells. (Notterpek, et al., Dev Neurosci 16(5-6):267-78 (1994)). Otaki et al. (Dev Biol 212(1):165-81 (1999)) reported that, as detected by Northern blot analysis, neurestin is highly expressed in the brain and relatively lowly expressed in other tissues. *In situ* hybridization to tissue sections demonstrates that neurestin is expressed in many types of neurons, including pyramidal cells in the cerebral cortex and tufted cells in the olfactory bulb during development. In adults, neurestin is mainly expressed in olfactory and hippocampal granule cells. Nonetheless, in adults, neurestin expression can be induced in external tufted cells during regeneration of olfactory sensory neurons.

Direct delivery of recombinant purified NOV11 or fragments of NOV11 into brain parenchymal regions may promote the regeneration/repair/remyelination of injured central nervous system cells resulting from ischemia, brain trauma, and various neurodegenerative diseases.

It was found that NOV11 is broadly expressed in brain and central nervous system cells, among others.

NOV11 proteins include the full protein disclosed as being encoded by the ORF described herein, as well as any mature protein arising therefrom as a result of posttranslational modifications. Thus, the proteins of the invention encompass both the precursors and the active forms of NOV11 proteins.

### NOV12

A NOV12 nucleic acid according to the invention includes the nucleotide sequence of SEQ ID NO:23, which is 2116 bp in length. The nucleic acid sequence includes an open reading frame encoding a polypeptide of 404 amino acid residues (SEQ ID NO:24). The start codon ofo this open reading frame is at nucleotides 517-519, and the stop codon is at nucleotides 1729-1731. The protein of SEQ ID NO:24 is predicted by the PSORT program to localize in the plasma membrane with a certainty of 0.4600. The program SignalP predicts that there is probably a signal peptide with the most likely cleavage site between residues 24 and 25: AAS-KN.

The disclosed NOV12 protein has 200 of 374 residues (53%) identical to, and 269 of 374 residues (71%) positive with, the 433 residue human cell adhesion molecule protein (TREMBLNEW-ACC:AAD17540).

In addition, the disclosed NOV12 protein has 327 of 329 residues (99%) identical to, and 327 of 329 residues (99%) positive with, the 444 residue human beta-secretase (US Patent No. 5,942,400, issued August 24, 1999). This enzyme is capable of cleaving the beta-amyloid precursor protein (APP) (Y33742; Swedish mutant APP), which is implicated in Alzheimer's disease.

NOV12 was isolated from brain tissue. NOV12 is highly expressed in brain and large cell lung cancer. NOV12 RNA sequences can be isolated from brain tissue, *e*.*g*., pituatary tissue.

NOV12 proteins provided by this invention include the full protein disclosed as being encoded by the ORF described herein, as well as any mature protein arising therefrom as a result of posttranslational modifications. Thus, the proteins of the invention encompass both the precursors and the active forms of the NOV12 protein.

### NOV13

A NOV13 nucleotide sequence according to the invention includes SEQ ID NO:25, which is 2862 nucleotides in length. SEQ ID NO:25 includes an open reading frame encoding a NOV13 polypeptide of 683 amino acid residues (SEQ ID NO:26). The start codon of this open reading frame is at nucleotides 508-510 and the stop codon is at nucleotides 2557-2559. The polypeptide with the amino acid sequence of SEQ ID NO:26 is predicted by the PSORT program to localize in the plasma membrane with a certainty of 0.6000. The program SignalP predicts that there is probably no signal peptide.

The encoded protein has 227 of 541 residues (41%) identical to, and 335 of 541 residues (61%) positive with, a 872 residue fragment of human KIAA0768 protein (ACC:BAA34488). In addition, the encoded protein has 680 of 683 residues (99%) identical to, and 682 of 683 residues (99%) positive with, the 690 residue human protein PRO228 (WO9914328-A2, published March 25, 1999).

NOV13 proteins include the full protein disclosed as being encoded by the ORF described herein, as well as any mature protein arising therefrom as a result of posttranslational modifications. Thus the proteins of the invention encompass both the precursors and the active forms of the NOV13 protein.

### NOV 14 and NOV23

Also included in the invention are NOV 14 and NOV23 nucleic acids. In some embodiments, NOV14 and NOV23 nucleic acids according to the invention encode identical proteins that are a variant of the protein encoded by a NOV13 nucleic acid sequence. The protein encoded by NOV13 includes sequences in its amino terminal region that are absent in the other two proteins. The disclosed NOV14 nucleic acid sequence and NOV23 nucleic acid sequences differ in their untranslated regions. The encoded NOV13, NOV14 and NOV23 polypeptides have identical amino acid sequences.

A NOV14 nucleic acid sequence according to the invention includes the 2760 nucleotides of (SEQ ID NO:27). This nucleic acid includes an open reading frame encoding a polypeptide of 645 amino acid residues (SEQ ID NO:28). The start codon is at nucleotides 520-522 and the stop codon is at nucleotides 2455-2457.

A NOV23 nucleic acid sequence according to the invention can include the 3081 nucleotides of SEQ ID NO:45). This open reading frame has an open reading frame encoding a polypeptide of 645 amino acid residues (SEQ ID NO:46). The start codon is at nucleotides 460-462 and the stop codon is at nucleotides 2395-2397. This encoded polypeptide has an identical amino acid sequence to the NOV 14 polypeptide encoded by SEQ ID NO:26.

In addition, the NOV14 and NOV23 proteins have 643 of 645 residues (99%) identical to, and 644 of 645 residues (99%) positive with, the 690 residue human protein PRO228 (PN WO9914328.

NOV 14 and NOV23 proteins according to the invention include the full protein disclosed as being encoded by the ORF described herein, as well as any mature protein arising therefrom as a result of posttranslational modifications. Thus the proteins of the invention encompass both the precursors and the active forms of the NOV14 and NOV21 proteins.

### NOV15

A NOV15 nucleic acid sequence according to the invention includes the nucleotide sequence (SEQ ID NO:29), which is 727 bp in length and includes an open reading frame encoding a polypeptide of 83 amino acid residues (SEQ ID NO:30). The start codon of this open reading frame is at nucleotides 312-314, and the stop codon is at nucleotides 560-562. The protein of SEQ ID NO:30 is predicted by the PSORT program to localize in the mitochondrial matrix space with a certainty of 0.59. The program SignalP predicts a moderate probability that there is a signal peptide with the most likely cleavage site between residues 25 and 26: CRT-DL.

This protein has 10 of 36 residues (27%) identical to, and 17 of 36 residues (47%) positive with, the 84 residue human PS2 protein precursor (HP1.A) (breast cancer estrogen-inducible protein) (PNR-2) (ACC:P04155). It also has 15 of 46 residues (32%) identical to, and 25 of 46 residues (54%) positive with, the 284 residue fragment of wheat receptor-like kinase (ACC:O8111).

The disclosed NOV15 sequence was isolated from the pituitary gland. In addition, NOV15 homologous sequences are found in the pancreas and the salivary gland.

NOV15 proteins include the full protein disclosed as being encoded by the ORF described herein, as well as any mature protein arising therefrom as a result of posttranslational modifications. Thus the proteins of the invention encompass both the precursors and the active forms of the NOV15 protein.

### NOV16

A NOV16 nucleic acid sequence according to the invention includes the nucleotide sequence (SEQ ID NO:31), which is 2741 nucleotides in length and contains an open reading frame encoding a polypeptide of 578 amino acid residues (SEQ ID NO:32). The start codon of this open reading frame is at nucleotides 288-290, and the stop codon is at nucleotides 2022-2024. The protein of SEQ ID NO:32 is predicted by the PSORT program to localize in the nucleus with a certainty of 0.8920. The program SignalP predicts that there is probably no signal peptide.

The encoded protein has 37 of 43 residues (86%) identical to, and 39 of 43 residues (90%) positive with, the 80 residue fragment of human epidermal growth factor receptor-related protein (ACC:Q04842).

NOV16 expression is downregulated in many tumor cell lines compared with the corresponding normal cell lines. (See Example 2, *infra)*

NOV16 proteins include the full protein disclosed as being encoded by the ORF described herein, as well as any mature protein arising therefrom as a result of posttranslational modifications. Thus the proteins of the invention encompass both the precursors and the active forms of the NOV16 protein.

### NOV17

NOV17 is a variant of NOV18 (discussed below), which was isolated from bone marrow. It is also found in osteosarcoma, thymus gland, fetal kidney, and lymph node. A NOV17 nucleic acid according to the invention includes the nucleotide sequence of SEQ ID NO:33, which is 2596 bp and includes an open reading frame encoding a polypeptide of 708 amino acid residues (SEQ ID NO:34). The start codon of this open reading frame is at nucleotides 289-291 and the stop codon is at nucleotides 2413-2415. The protein of SEQ ID NO:34 is predicted by the PSORT program to localize in the plasma membrane with a certainty of 0.6000. The program SignalP predicts that there is probably no signal peptide.

The encoded protein has 70 of 80 residues (87%) identical to, and 75 of 80 residues (93%) positive with, the 80 residue fragment of human epidermal growth factor receptor-related protein (ACC:Q04842).

NOV17 proteins include the full protein disclosed as being encoded by the ORF described herein, as well as any mature protein arising therefrom as a result of posttranslational modifications. Thus the proteins of the invention encompass both the precursors and the active forms of the NOV17 protein.

### NOV18

A NOV18 nucleic acid according to the invention includes the nucleotide sequence of SEQ ID NO:35. This nucleic acid 705 nucleotides in length and includes an open reading frame encoding a polypeptide of 137 amino acid residues (SEQ ID NO:36). The start codon of the open reading frame is at nucleotides 135-137, and the stop codon is at nucleotides 546-548. The protein of SEQ ID NO:36 is predicted by the PSORT program to localize in the plasma membrane with a certainty of 0.650. The program SignalP predicts that there is probably a signal peptide with the most likely cleavage site between residues 52 and 53: APS-ED.

The encoded protein has 25 of 73 residues (34%) identical to, and 36 of 73 residues (49%) positive with, the 488 residue human stromelysin-3 precursor (EC 3.4.24.-) (matrix metalloproteinase-11) (MMP-11) (ST3) (SL-3) protein (ACC:P24347).

NOV18 was isolated from the uterus. In addition NOV18 is found in fetal liver, bone marrow, uterus, fetal brain, and osteogenic sarcoma.

NOV18 proteins according to the invention include the full protein disclosed as being encoded by the ORF described herein, as well as any mature protein arising therefrom as a result of posttranslational modifications. Thus the proteins of the invention encompass both the precursors and the active forms of the NOV18 protein.

### NOV19

A NOV19 nucleic acid sequence according to the invention includes the nucleotide sequence of SEQ ID NO:37. This nucleic acid sequence is 1150 nucleotides in length and includes an open reading frame encoding a polypeptide of 156 amino acid residues (SEQ ID NO:38). The protein of SEQ ID NO:38 is predicted by the PSORT program to localize in the plasma membrane with a certainty of 0.6000. The program SignalP predicts that there is a moderate probability of a signal peptide with the most likely cleavage site between residues 58 and 59: ISA-YM.

The encoded protein has 40 of 112 residues (35%) identical to, and 61 of 112 residues (54%) positive with, the 152 residue human intestinal membrane A4 protein (differentiation-dependent protein A4) (ACC:Q04941).

NOV19 proteins according to the invention include the full protein disclosed as being encoded by the ORF described herein, as well as any mature proteins arising therefrom as a result of posttranslational modifications. Thus the proteins of the invention encompass both the precursors and the active forms of the NOV19 protein.

NOV19 sequences are expressed in thalamus and bone marrow.

### NOV20

A NOV20 nucleic acid according to the invention includes the nucleotide sequence (SEQ ID NO:39), which is 1611 nucleotides in length and includes an open reading frame encoding a polypeptide of 260 amino acid residues (SEQ ID NO:40). The start codon of the open reading frame is at nucleotides 505-507 and the stop codon is at nucleotides 1285-1287. The protein of SEQ ID NO:40 is predicted by the PSORT program to localize in the plasma membrane with a certainty of 0.4600. The program SignalP predicts that there is probably a signal peptide with the most likely cleavage site between residues 29 and 30: VVA-VP.

The encoded protein has 73 of 204 residues (35%) identical to, and 119 of 204 residues (58%) positive with, the 595 residue F40E10.6 protein from *Caenorhabditis elegans* (ACC:Q19985).

The expression of NOV20 is widely dispersed in many tissues, *e*.*g*., the placenta. NOV20 was isolated from lymph node tissue.

NOV20 proteins according to the invention include the full protein disclosed as being encoded by the ORF described herein, as well as any mature protein arising therefrom as a result of posttranslational modifications. Thus the proteins of the invention encompass both the precursors and the active forms of the NOV20 protein.

### NOVX Nucleic Acids

The novel nucleic acids of the invention include those that encode a NOVX or a NOVX-like protein, or biologically active portions thereof. The nucleic acids include nucleic acids encoding polypeptides that include the amino acid sequence of one or more of SEQ ID NO:2, 4, 6, 8, 10,12, 14, 16, 18, 20, 22, 24, 26, 28, 30, 32, 34, 36, 38, 40, 42, 44, and 46. The encoded polypeptides can thus include, e.g., the amino acid sequences of SEQ ID NO: 2, 4, 6, 8, 10, 12, 14 16, 18, 20, 22, 24, 26, 28, 30, 32, 34, 36, 38, 40, and 42.

In some embodiments, a nucleic acid encoding a polypeptide having the amino acid sequence of one or more of SEQ ID NO:2, 4, 6, 8, 10, 12, 14, 16, 18, 20, 22, 24, 26, 28, 30, 32, 34, 36, 38, 40, 42, 44, and 46 includes the nucleic acid sequence of any of SEQ ID NO:1, 3, 5, 7, 9, 11, 13, 15, 17, 19, 21, 23, 25, 27, 29, 31, 33, 35, 37, 39, 41, 43, and 45, or a fragment thereof. Additionally, the invention includes mutant or variant nucleic acids of any of SEQ ID NO:1, 3, 5, 7, 9, 11, 13, 15, 17, 19, 21, 23, 25, 27, 29, 31, 33, 35, 37, 39, 41, 43, and 45, or a fragment thereof, any of whose bases may be changed from the disclosed sequence while still encoding a protein that maintains its NOVX-like activities and physiological functions. The invention further includes the complement of the nucleic acid sequence of any of SEQ ID NO:1, 3, 5, 7, 9, 11, 13, 15, 17, 19, 21, 23, 25, 27, 29, 31, 33, 35, 37, 39, 41, 43, and 45, including fragments, derivatives, analogs and homolog thereof. The invention additionally includes nucleic acids or nucleic acid fragments, or complements thereto, whose structures include chemical modifications.

Also included are nucleic acid fragments sufficient for use as hybridization probes to identify NOVX-encoding nucleic acids (*e.g.*, NOVX mRNA) and fragments for use as polymerase chain reaction (PCR) primers for the amplification or mutation of NOVX nucleic acid molecules. As used herein, the term "nucleic acid molecule" is intended to include DNA molecules (*e*.*g*., cDNA or genomic DNA), RNA molecules (*e*.*g*., mRNA), analogs of the DNA or RNA generated using nucleotide analogs, and derivatives, fragments and homologs thereof. The nucleic acid molecule can be single-stranded or double-stranded, but preferably is double-stranded DNA.

"Probes" refer to nucleic acid sequences of variable length, preferably between at least about 10 nucleotides (nt), 100 nt, or as many as about, *e*.*g*., 6,000 nt, depending on use. Probes are used in the detection of identical, similar, or complementary nucleic acid sequences. Longer length probes are usually obtained from a natural or recombinant source, are highly specific and much slower to hybridize than oligomers. Probes may be single- or double-stranded and designed to have specificity in PCR, membrane-based hybridization technologies, or ELISA-like technologies.

An "isolated" nucleic acid molecule is one that is separated from other nucleic acid molecules that are present in the natural source of the nucleic acid. Examples of isolated nucleic acid molecules include, but are not limited to, recombinant DNA molecules contained in a vector, recombinant DNA molecules maintained in a heterologous host cell, partially or substantially purified nucleic acid molecules, and synthetic DNA or RNA molecules. Preferably, an "isolated" nucleic acid is free of sequences which naturally flank the nucleic acid (*i*.*e*., sequences located at the 5' and 3' ends of the nucleic acid) in the genomic DNA of the organism from which the nucleic acid is derived. For example, in various embodiments, the isolated NOVX nucleic acid molecule can contain less than about 50 kb, 25 kb, 5 kb, 4 kb, 3 kb, 2 kb, 1 kb, 0.5 kb or 0.1 kb of nucleotide sequences which naturally flank the nucleic acid molecule in genomic DNA of the cell from which the nucleic acid is derived. Moreover, an "isolated" nucleic acid molecule, such as a cDNA molecule, can be substantially free of other cellular material or culture medium when produced by recombinant techniques, or of chemical precursors or other chemicals when chemically synthesized.

A nucleic acid molecule of the present invention, *e.g.*, a nucleic acid molecule having the nucleotide sequence of SEQ ID NO:1, 3, 5, 7, 9, 11, 13, 15, 17, 19, 21, 23, 25, 27, 29, 31, 33, 35, 37, 39, 41, 43, and 45, or a complement of any of this nucleotide sequence, can be isolated using standard molecular biology techniques and the sequence information provided herein. Using all or a portion of the nucleic acid sequence of any of SEQ ID NO:1, 3, 5, 7, 9, 11, 13, 15, 17, 19, 21, 23, 25, 27, 29, 31, 33, 35, 37, 39, 41, 43, and 45 as a hybridization probe, NOVX nucleic acid sequences can be isolated using standard hybridization and cloning techniques (*e*.*g*., as described in Sambrook *et al*., eds., MOLECULAR CLONING: A LABORATORY MANUAL 2^{nd} Ed., Cold Spring Harbor Laboratory Press, Cold Spring Harbor, NY, 1989; and Ausubel, *et al*., eds., CURRENT PROTOCOLS IN MOLECULAR BIOLOGY, John Wiley & Sons, New York, NY, 1993.)

A nucleic acid of the invention can be amplified using cDNA, mRNA or alternatively, genomic DNA, as a template and appropriate oligonucleotide primers according to standard PCR amplification techniques. The nucleic acid so amplified can be cloned into an appropriate vector and characterized by DNA sequence analysis. Furthermore, oligonucleotides corresponding to NOVX nucleotide sequences can be prepared by standard synthetic techniques, *e*.*g*., using an automated DNA synthesizer.

As used herein, the term "oligonucleotide" refers to a series of linked nucleotide residues, which oligonucleotide has a sufficient number of nucleotide bases to be used in a PCR reaction. A short oligonucleotide sequence may be based on, or designed from, a genomic or cDNA sequence and is used to amplify, confirm, or reveal the presence of an identical, similar or complementary DNA or RNA in a particular cell or tissue. Oligonucleotides comprise portions of a nucleic acid sequence having about 10 nt, 50 nt, or 100 nt in length, preferably about 15 nt to 30 nt in length. In one embodiment, an oligonucleotide comprising a nucleic acid molecule less than 100 nt in length would further comprise at lease 6 contiguous nucleotides of any of SEQ ID NO:1, 3, 5, 7, 9, 11, 13, 15, 17, 19,21,23,25,27,29,31,33,35,37,39,41,43, and 45, or a complement thereof. Oligonucleotides may be chemically synthesized and may be used as probes.

In another embodiment, an isolated nucleic acid molecule of the invention comprises a nucleic acid molecule that is a complement of the nucleotide sequence shown in any of SEQ ID NO:1, 3, 5, 7, 9, 11, 13, 15, 17, 19, 21, 23, 25, 27, 29, 31, 33, 35, 37, 39, 41, 43, and 45. In another embodiment, an isolated nucleic acid molecule of the invention comprises a nucleic acid molecule that is a complement of the nucleotide sequence shown in any of SEQ ID NO:1, 3, 5, 7, 9, 11, 13, 15; 17, 19, 21, 23, 25, 27, 29, 31, 33, 35, 37, 39, 41, 43, and 45, or a portion of this nucleotide sequence. A nucleic acid molecule that is complementary to the nucleotide sequence shown in any of SEQ ID NO:1, 3, 5, 7, 9, 11, 13, 15, 17, 19, 21, 23, 25, 27, 29, 31, 33, 35, 37, 39, 41, 43, and 45 is one that is sufficiently complementary to the nucleotide sequence shown in of any of SEQ ID NO:1, 3, 5, 7, 9, 11, 13, 15, 17, 19, 21, 23, 25, 27, 29, 31, 33, 35, 37, 39, 41, 43, and 45 that it can hydrogen bond with little or no mismatches to the nucleotide sequence shown in of any of SEQ ID NO:1, 3, 5, 7, 9, 11, 13, 15, 17, 19, 21, 23, 25, 27, 29, 31, 33, 35, 37, 39, 41, 43, and 45, thereby forming a stable duplex.

As used herein, the term "complementary" refers to Watson-Crick or Hoogsteen base pairing between nucleotides units of a nucleic acid molecule, and the term "binding" means the physical or chemical interaction between two polypeptides or compounds or associated polypeptides or compounds or combinations thereof. Binding includes ionic, non-ionic, Von der Waals, hydrophobic interactions, etc. A physical interaction can be either direct or indirect. Indirect interactions may be through or due to the effects of another polypeptide or compound. Direct binding refers to interactions that do not take place through, or due to, the effect of another polypeptide or compound, but instead are without other substantial chemical intermediates.

Moreover, the nucleic acid molecule of the invention can comprise only a portion of the nucleic acid sequence of any of SEQ ID NO:1, 3, 5, 7, 9, 11, 13, 15, 17, 19, 21, 23, 25, 27, 29, 31, 33, 35, 37, 39, 41, 43, and 45, *e*.*g*., a fragment that can be used as a probe or primer, or a fragment encoding a biologically active portion of NOVX. Fragments provided herein are defined as sequences of at least 6 (contiguous) nucleic acids or at least 4 (contiguous) amino acids, a length sufficient to allow for specific hybridization in the case of nucleic acids or for specific recognition of an epitope in the case of amino acids, respectively, and are at most some portion less than a full length sequence. Fragments may be derived from any contiguous portion of a nucleic acid or amino acid sequence of choice. Derivatives are nucleic acid sequences or amino acid sequences formed from the native compounds either directly or by modification or partial substitution. Analogs are nucleic acid sequences or amino acid sequences that have a structure similar to, but not identical to, the native compound but differs from it in respect to certain components or side chains. Analogs may be synthetic or from a different evolutionary origin and may have a similar or opposite metabolic activity compared to wild type.

Derivatives and analogs may be full length or other than full length, if the derivative or analog contains a modified nucleic acid or amino acid, as described below. Derivatives or analogs of the nucleic acids or proteins of the invention include, but are not limited to, molecules comprising regions that are substantially homologous to the nucleic acids or proteins of the invention, in various embodiments, by at least about 70%, 80%, 85%, 90%, 95%, 98%, or even 99% identity (with a preferred identity of 80-99%) over a nucleic acid or amino acid sequence of identical size or when compared to an aligned sequence in which the alignment is done by a computer homology program known in the art, or whose encoding nucleic acid is capable of hybridizing to the complement of a sequence encoding the aforementioned proteins under stringent, moderately stringent, or low stringent conditions. See *e.g.* Ausubel, *et al*., CURRENT PROTOCOLS IN MOLECULAR BIOLOGY, John Wiley & Sons, New York, NY, 1993, and below. An exemplary program is the Gap program (Wisconsin Sequence Analysis Package, Version 8 for UNIX, Genetics Computer Group, University Research Park, Madison, WI) using the default settings, which uses the algorithm of Smith and Waterman (Adv. Appl. Math., 1981, 2: 482-489, which is incorporated herein by reference in its entirety).

A "homologous nucleic acid sequence" or "homologous amino acid sequence," or variations thereof, refer to sequences characterized by a homology at the nucleotide level or amino acid level as discussed above. Homologous nucleotide sequences encode those sequences coding for isoforms of NOVX polypeptide. Isoforms can be expressed in different tissues of the same organism as a result of, for example, alternative splicing of RNA. Alternatively, isoforms can be encoded by different genes. In the present invention, homologous nucleotide sequences include nucleotide sequences encoding for a NOVX polypeptide of species other than humans, including, but not limited to, mammals, and thus can include, *e.g.*, mouse, rat, rabbit, dog, cat cow, horse, and other organisms. Homologous. nucleotide sequences also include, but are not limited to, naturally occurring allelic variations and mutations of the nucleotide sequences set forth herein. A homologous nucleotide sequence does not, however, include the nucleotide sequence encoding human NOVX protein. Homologous nucleic acid sequences include those nucleic acid sequences that encode conservative amino acid substitutions (see below) in any of SEQ ID NO:2, 4, 6, 8, 10, 12, 14, 16, 18, 20, 22, 24, 26, 28, 30, 32, 34, 36, 38, 40, 42, 44, and 46 as well as a polypeptide having NOVX activity. Biological activities of the NOVX proteins are described below. A homologous amino acid sequence does not encode the amino acid sequence of a human NOVX polypeptide.

The nucleotide sequence determined from the cloning of the human NOVX gene allows for the generation of probes and primers designed for use in identifying the cell types disclosed and/or cloning NOVX homologues in other cell types, *e*.*g*., from other tissues, as well as NOVX homologues from other mammals. The probe/primer typically comprises a substantially purified oligonucleotide. The oligonucleotide typically comprises a region of nucleotide sequence that hybridizes under stringent conditions to at least about 12, 25, 50, 100, 150, 200, 250, 300, 350 or 400 or more consecutive sense strand nucleotide sequence of SEQ ID NO:1, 3, 5, 7, 9, 11, 13, 15, 17, 19, 21, 23, 25, 27, 29, 31, 33, 35, 37, 39, 41, 43, and 45; or an anti-sense strand nucleotide sequence of SEQ ID NO:1, 3, 5, 7, 9, 11, 13, 15, 17, 19, 21, 23, 25, 27, 29, 31, 33, 35, 37, 39, 41, 43, and 45; or of a naturally occurring mutant of SEQ ID NO:1, 3, 5, 7, 9, 11, 13, 15, 17, 19, 21, 23, 25, 27, 29, 31, 33, 35, 37, 39, 41, 43, and 45.

Probes based on the human NOVX nucleotide sequence can be used to detect transcripts or genomic sequences encoding the same or homologous proteins. In various embodiments, the probe further comprises a label group attached thereto, *e*.*g*., the label group can be a radioisotope, a fluorescent compound, an enzyme, or an enzyme co-factor. Such probes can be used as a part of a diagnostic test kit for identifying cells or tissue which misexpress a NOVX protein, such as by measuring a level of a NOVX-encoding nucleic acid in a sample of cells from a subject *e*.*g*., detecting NOVX mRNA levels or determining whether a genomic NOVX gene has been mutated or deleted.

"A polypeptide having a biologically active portion of NOVX" refers to polypeptides exhibiting activity similar, but not necessarily identical to, an activity of a polypeptide of the present invention, including mature forms, as measured in a particular biological assay, with or without dose dependency. A nucleic acid fragment encoding a "biologically active portion of NOVX" can be prepared by isolating a portion of SEQ ID NO:1, 3, 5, 7, 9, 11, 13, 15, 17, 19, 21, 23, 25, 27, 29, 31, 33, 35, 37, 39, 41, 43, and 45, that encodes a polypeptide having a NOVX biological activity (biological activities of the NOVX proteins are summarized in Table 1), expressing the encoded portion of NOVX protein (*e*.*g*., by recombinant expression *in vitro)* and assessing the activity of the encoded portion of NOVX.

### NOVX variants

The invention further encompasses nucleic acid molecules that differ from the disclosed NOVX nucleotide sequences due to degeneracy of the genetic code. These nucleic acids thus encode the same NOVX protein as that encoded by the nucleotide sequence shown in SEQ ID NO:1, 3, 5, 7, 9, 11, 13, 15, 17, 19, 21, 23, 25, 27, 29, 31, 33, 35, 37, 39, 41, 43, and 45. In another embodiment, an isolated nucleic acid molecule of the invention has a nucleotide sequence encoding a protein having an amino acid sequence shown in any of SEQ ID NO:2, 4, 6, 8, 10, 12, 14, 16, 18, 20, 22, 24, 26, 28, 30, 32, 34, 36, 38, 40, 42, 44, and 46.

In addition to the human NOVX nucleotide sequence shown in any of SEQ ID NO:1, 3, 5, 7, 9,11, 13, 15, 17, 19, 21, 23, 25, 27, 29, 31, 33, 35, 37, 39, 41, 43, and 45, it will be appreciated by those skilled in the art that DNA sequence polymorphisms that lead to changes in the amino acid sequences of NOVX may exist within a population (*e*.*g*., the human population). Such genetic polymorphisms in the NOVX gene may exist among individuals within a population due to natural allelic variation. As used herein, the terms "gene" and "recombinant gene" refer to nucleic acid molecules comprising an open reading frame encoding a NOVX protein, preferably a mammalian NOVX protein. Such natural allelic variations can typically result in 1-5% variance in the nucleotide sequence of the NOVX gene. Any and all such nucleotide variations and resulting amino acid polymorphisms in NOVX that are the result of natural allelic variation and that do not alter the functional activity of NOVX are intended to be within the scope of the invention.

Moreover, nucleic acid molecules encoding NOVX proteins from other species, and thus that have a nucleotide sequence that differs from the human sequence of any of SEQ ID NO:1, 3, 5, 7, 9, 11, 13, 15, 17, 19, 21, 23, 25, 27, 29, 31, 33, 35, 37, 39, 41, 43, and 45, are intended to be within the scope of the invention. Nucleic acid molecules corresponding to natural allelic variants and homologues of the NOVX cDNAs of the invention can be isolated based on their homology to the human NOVX nucleic acids disclosed herein using the human cDNAs, or a portion thereof, as a hybridization probe according to standard hybridization techniques under stringent hybridization conditions.

In another embodiment, an isolated nucleic acid molecule of the invention is at least 6 nucleotides in length and hybridizes under stringent conditions to the nucleic acid molecule comprising the nucleotide sequence of any of SEQ ID NO:1, 3, 5, 7, 9, 11, 13, 15, 17, 19, 21, 23, 25, 27, 29, 31, 33, 35, 37, 39, 41, 43, and 45. In another embodiment, the nucleic acid is at least 10, 25, 50, 100, 250, 500 or 750 nucleotides in length. In another embodiment, an isolated nucleic acid molecule of the invention hybridizes to the coding region. As used herein, the term "hybridizes under stringent conditions" is intended to describe conditions for hybridization and washing under which nucleotide sequences at least 60% homologous to each other typically remain hybridized to each other.

Homologs (*i*.*e*., nucleic acids encoding NOVX proteins derived from species other than human) or other related sequences (*e.g.*, paralogs) can be obtained by low, moderate or high stringency hybridization with all or a portion of the particular human sequence as a probe using methods well known in the art for nucleic acid hybridization and cloning.

As used herein, the phrase "stringent hybridization conditions" refers to conditions under which a probe, primer or oligonucleotide will hybridize to its target sequence, but to no other sequences. Stringent conditions are sequence-dependent and will be different in different circumstances. Longer sequences hybridize specifically at higher temperatures than shorter sequences. Generally, stringent conditions are selected to be about 5°C lower than the thermal melting point (Tm) for the specific sequence at a defined ionic strength and pH. The Tm is the temperature (under defined ionic strength, pH and nucleic acid concentration) at which 50% of the probes complementary to the target sequence hybridize to the target sequence at equilibrium. Since the target sequences are generally present at excess, at Tm, 50% of the probes are occupied at equilibrium. Typically, stringent conditions will be those in which the salt concentration is less than about 1.0 M sodium ion, typically about 0.01 to 1.0 M sodium ion (or other salts) at pH 7.0 to 8.3 and the temperature is at least about 30°C for short probes, primers or oligonucleotides (*e*.*g*., 10 nt to 50 nt) and at least about 60°C for longer probes, primers and oligonucleotides. Stringent conditions may also be achieved with the addition of destabilizing agents, such as formamide.

Stringent conditions are known to those skilled in the art and can be found in CURRENT PROTOCOLS IN MOLECULAR BIOLOGY, John Wiley & Sons, N.Y. (1989), 6.3.1-6.3.6. Preferably, the conditions are such that sequences at least about 65%, 70%, 75%, 85%, 90%, 95%, 98%, or 99% homologous to each other typically remain hybridized to each other. A non-limiting example of stringent hybridization conditions is hybridization in a high salt buffer comprising 6X SSC, 50 mM Tris-HCl (pH 7.5), 1 mM EDTA, 0.02% PVP, 0.02% Ficoll, 0.02% BSA, and 500 mg/ml denatured salmon sperm DNA at 65°C. This hybridization is followed by one or more washes in 0.2X SSC, 0.01% BSA at 50°C. An isolated nucleic acid molecule of the invention that hybridizes under stringent conditions to the sequence of any of SEQ ID NO:1, 3, 5, 7, 9, 11, 13, 15, 17, 19, 21, 23, 25, 27, 29, 31, 33, 35, 37, 39, 41, 43, and 45 corresponds to a naturally occurring nucleic acid molecule. As used herein, a "naturally-occurring" nucleic acid molecule refers to an RNA or DNA molecule having a nucleotide sequence that occurs in nature (*e*.*g*., encodes a natural protein).

In a second embodiment, a nucleic acid sequence that is hybridizable to the nucleic acid molecule comprising the nucleotide sequence of any of SEQ ID NO:1, 3, 5, 7, 9, 11, 13, 15,17, 19, 21, 23, 25, 27, 29, 31, 33, 35, 37, 39, 41, 43, and 45, or fragments, analogs or derivatives thereof, under conditions of moderate stringency is provided. A non-limiting example of moderate stringency hybridization conditions are hybridization in 6X SSC, 5X Denhardt's solution, 0.5% SDS and 100 mg/ml denatured salmon sperm DNA at 55°C, followed by one or more washes in 1X SSC, 0.1 % SDS at 37°C. Other conditions of moderate stringency that may be used are well known in the art. See, *e.g.*, Ausubel *et al*. (eds.), 1993, CURRENT PROTOCOLS IN MOLECULAR BIOLOGY, John Wiley & Sons, NY, and Kriegler, 1990, GENE TRANSFER AND EXPRESSION, A LABORATORY MANUAL, Stockton Press, NY.

In a third embodiment, a nucleic acid that is hybridizable to the nucleic acid molecule comprising the nucleotide sequence of any of SEQ ID NO:1, 3, 5, 7, 9, 11, 13, 15, 17, 19, 21, 23, 25, 27, 29, 31, 33, 35, 37, 39, 41, 43, and 45, or fragments, analogs or derivatives thereof, under conditions of low stringency, is provided. A non-limiting example of low stringency hybridization conditions are hybridization in 35% formamide, 5X SSC, 50 mM Tris-HCl (pH 7.5), 5 mM EDTA, 0.02% PVP, 0.02% Ficoll, 0.2% BSA, 100 mg/ml denatured salmon sperm DNA, 10% (wt/vol) dextran sulfate at 40°C, followed by one or more washes in 2X SSC, 25 mM Tris-HCl (pH 7.4), 5 mM EDTA, and 0.1% SDS at 50°C. Other conditions of low stringency that may be used are well known in the art (*e.g.*, as employed for cross-species hybridizations). See, *e.g.,* Ausubel *et al*. (eds.), 1993, CURRENT PROTOCOLS IN MOLECULAR BIOLOGY, John Wiley & Sons, NY, and Kriegler, 1990, GENE TRANSFER AND EXPRESSION, A LABORATORY MANUAL, Stockton Press, NY; Shilo and Weinberg, 1981, *Proc Natl Acad Sci USA 78*: 6789-6792.

### Conservative mutations

In addition to naturally-occurring allelic variants of the NOVX sequence that may exist in the population, the skilled artisan will further appreciate that changes can be introduced by mutation into the nucleotide sequence of any of SEQ ID NO:1, 3, 5, 7, 9, 11, 13, 15, 17, 19, 21, 23, 25, 27, 29, 31, 33, 35, 37, 39, 41, 43, and 45, thereby leading to changes in the amino acid sequence of the encoded NOVX protein, without altering the functional ability of the NOVX protein. For example, nucleotide substitutions leading to amino acid substitutions at "non-essential" amino acid residues can be made in the sequence of any of SEQ ID NO:1, 3, 5, 7, 9, 11, 13, 15, 17, 19, 21, 23, 25, 27, 29, 31, 33, 35, 37, 39, 41, 43, and 45. A "non-essential" amino acid residue is a residue that can be altered from the wild-type sequence ofNOVX without altering the biological activity, whereas an "essential" amino acid residue is required for biological activity. For example, amino acid residues that are conserved among the NOVX proteins of the present invention, are predicted to be particularly unamenable to alteration.

Amino acid residues that are conserved among members of a NOVX family members are predicted to be less amenable to alteration. For example, a NOVX protein according to the present invention can contain at least one domain (*e*.*g*., as shown in Table 1) that is a typically conserved region in a NOVX family member. As such, these conserved domains are not likely to be amenable to mutation. Other amino acid residues, however, (*e*.*g*., those that are not conserved or only semi-conserved among members of the NOVX family) may not be as essential for activity and thus are more likely to be amenable to alteration.

Another aspect of the invention pertains to nucleic acid molecules encoding NOVX proteins that contain changes in amino acid residues that are not essential for activity. Such NOVX proteins differ in amino acid sequence from any of any of SEQ ID NO:2, 4, 6, 8, 10, 12, 14, 16, 18, 20, 22, 24, 26, 28, 30, 32, 34, 36, 38, 40, 42, 44, and 46, yet retain biological activity. In one embodiment, the isolated nucleic acid molecule comprises a nucleotide sequence encoding a protein, wherein the protein comprises an amino acid sequence at least about 75% homologous to the amino acid sequence of any of SEQ ID NO:2, 4, 6, 8, 10, 12, 14, 16, 18, 20, 22, 24, 26, 28, 30, 32, 34, 36, 38, 40, 42, 44, and 46. Preferably, the protein encoded by the nucleic acid is at least about 80% homologous to any of SEQ ID NO:2, 4, 6, 8, 10, 12, 14, 16, 18, 20, 22, 24, 26, 28, 30, 32, 34, 36, 38, 40, 42, 44, and 46, more preferably at least about 90%, 95%, 98%, and most preferably at least about 99% homologous to any one of SEQ ID NO:2, 4, 6, 8, 10, 12, 14, 16, 18, 20, 22, 24, 26, 28, 30, 32, 34, 36, 38, 40, 42, 44, and 46.

An isolated nucleic acid molecule encoding a NOVX protein homologous to the protein of any of SEQ ID NO:2, 4, 6, 8, 10, 12, 14, 16, 18, 20, 22, 24, 26, 28, 30, 32, 34, 36, 3 8, 40, 42, 44, and 46 can be created by introducing one or more nucleotide substitutions, additions or deletions into the corresponding nucleotide sequence, *i*.*e*. SEQ ID NO:1, 3, 5, 7, 9, 11, 13, 15, 17, 19, 21, 23, 25, 27, 29, 31, 33, 35, 37, 39, 41, 43, and 45, such that one or more amino acid substitutions, additions or deletions are introduced into the encoded protein.

Mutations can be introduced into SEQ ID NO:1, 3, 5, 7, 9, 11, 13, 15, 17, 19, 21, 23, 25, 27, 29, 31, 33, 35, 37, 39, 41, 43, and 45 by standard techniques, such as site-directed mutagenesis and PCR-mediated mutagenesis. Preferably, conservative amino acid substitutions are made at one or more predicted non-essential amino acid residues. A "conservative amino acid substitution" is one in which the amino acid residue is replaced with an amino acid residue having a similar side chain. Families of amino acid residues having similar side chains have been defined in the art. These families include amino acids with basic side chains (*e*.*g*., lysine, arginine, histidine), acidic side chains *(e.g.,* aspartic acid, glutamic acid), uncharged polar side chains *(e.g.,* glycine, asparagine, glutamine, serine, threonine, tyrosine, cysteine), nonpolar side chains (*e.g.*, alanine, valine, leucine, isoleucine, proline, phenylalanine, methionine, tryptophan), beta-branched side chains (*e*.*g*., threonine, valine, isoleucine) and aromatic side chains (*e.g.*, tyrosine, phenylalanine, tryptophan, histidine). Thus, a predicted nonessential amino acid residue in NOVX is replaced with another amino acid residue from the same side chain family. Alternatively, in another embodiment, mutations can be introduced randomly along all or part of a NOVX coding sequence, such as by saturation mutagenesis, and the resultant mutants can be screened for NOVX biological activity to identify mutants that retain activity. Following mutagenesis of SEQ ID NO:1, 3, 5, 7, 9, 11, 13, 15, 17, 19, 21, 23, 25, 27, 29, 31, 33, 35, 37, 39, 41, 43, and 45, the encoded protein can be expressed by any recombinant technology known in the art and the activity of the protein can be determined.

In one embodiment, a mutant NOVX protein can be assayed for (1) the ability to form protein:protein interactions with other NOVX proteins, other cell-surface proteins, or biologically active portions thereof, (2) complex formation between a mutant NOVX protein and a NOVX receptor; (3) the ability of a mutant NOVX protein to bind to an intracellular target protein or biologically active portion thereof; (*e.g.*, avidin proteins); (4) the ability to bind BRA protein; or (5) the ability to specifically bind an anti-NOVX protein antibody.

### Antisense

Another aspect of the invention pertains to isolated antisense nucleic acid molecules that are hybridizable to or complementary to the nucleic acid molecule comprising the nucleotide sequence of SEQ ID NO:1, 3, 5, 7, 9, 11, 13, 15, 17, 19, 21, 23, 25, 27, 29, 31, 33, 35, 37, 39, 41, 43, and 45, or fragments, analogs or derivatives thereof. An "antisense" nucleic acid comprises a nucleotide sequence that is complementary to a "sense" nucleic acid encoding a protein, *e*.*g*., complementary to the coding strand of a double-stranded cDNA molecule or complementary to an mRNA sequence. In specific aspects, antisense nucleic acid molecules are provided that comprise a sequence complementary to at least about 10, 25, 50, 100, 250 or 500 nucleotides or an entire NOVX coding strand, or to only a portion thereof. Nucleic acid molecules encoding fragments, homologs, derivatives and analogs of a NOVX protein of any of SEQ ID NO:2, 4, 6, 8, 10, 12, 14, 16, 18, 20, 22, 24, 26, 28, 30, 32, 34, 36, 38, 40, 42, 44, and 46 or antisense nucleic acids complementary to a NOVX nucleic acid sequence of SEQ ID NO:1, 3, 5, 7, 9, 11, 13, 15, 17, 19, 21, 23, 25, 27, 29, 31, 33, 35, 37, 39, 41, 43, and 45 are additionally provided.

In one embodiment, an antisense nucleic acid molecule is antisense to a "coding region" of the coding strand of a nucleotide sequence encoding NOVX. The term "coding region" refers to the region of the nucleotide sequence comprising codons which are translated into amino acid residues *(e.g.,* the protein coding region of a human NOVX that corresponds to any of SEQ ID NO:2, 4, 6, 8, 10, 12, 14, 16, 18, 20, 22, 24, 26, 28, 30, 32, 34, 36, 38, 40, 42, 44, and 46). In another embodiment, the antisense nucleic acid molecule is antisense to a "noncoding region" of the coding strand of a nucleotide sequence encoding NOVX. The term "noncoding region" refers to 5' and 3' sequences which flank the coding region that are not translated into amino acids (*i*.*e*., also referred to as 5' and 3' untranslated regions).

Given the coding strand sequences encoding NOVX disclosed herein (*e*.*g*., SEQ ID NO:1, 3, 5, 7, 9, 11, 13, 15, 17, 19, 21, 23, 25, 27, 29, 31, 33, 35, 37, 39, 41, 43, and 45), antisense nucleic acids of the invention can be designed according to the rules of Watson and Crick or Hoogsteen base pairing. The antisense nucleic acid molecule can be complementary to the entire coding region of NOVX mRNA, but more preferably is an oligonucleotide that is antisense to only a portion of the coding or noncoding region of NOVX mRNA. For example, the antisense oligonucleotide can be complementary to the region surrounding the translation start site of NOVX mRNA. An antisense oligonucleotide can be, for example, about 5, 10, 15, 20, 25, 30, 35, 40, 45 or 50 nucleotides in length. An antisense nucleic acid of the invention can be constructed using chemical synthesis or enzymatic ligation reactions using procedures known in the art. For example, an antisense nucleic acid (*e.g*., an antisense oligonucleotide) can be chemically synthesized using naturally occurring nucleotides or variously modified nucleotides designed to increase the biological stability of the molecules or to increase the physical stability of the duplex formed between the antisense and sense nucleic acids, *e.g.*, phosphorothioate derivatives and acridine substituted nucleotides can be used.

Examples of modified nucleotides that can be used to generate the antisense nucleic acid include: 5-fluorouracil, 5-bromouracil, 5-chlorouracil, 5-iodouracil, hypoxarithine, xanthine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine. Alternatively, the antisense nucleic acid can be produced biologically using an expression vector into which a nucleic acid has been subcloned in an antisense orientation (*i*.*e*., RNA transcribed from the inserted nucleic acid will be of an antisense orientation to a target nucleic acid of interest, described further in the following subsection).

The antisense nucleic acid molecules of the invention are typically administered to a subject or generated *in situ* such that they hybridize with or bind to cellular mRNA and/or genomic DNA encoding a NOVX protein to thereby inhibit expression of the protein, *e*.*g*., by inhibiting transcription and/or translation. The hybridization can be by conventional nucleotide complementarity to form a stable duplex, or, for example, in the case of an antisense nucleic acid molecule that binds to DNA duplexes, through specific interactions in the major groove of the double helix. An example of a route of administration of antisense nucleic acid molecules of the invention includes direct injection at a tissue site. Alternatively, antisense nucleic acid molecules can be modified to target selected cells and then administered systemically. For example, for systemic administration, antisense molecules can be modified such that they specifically bind to receptors or antigens expressed on a selected cell surface, *e*.*g*., by linking the antisense nucleic acid molecules to peptides or antibodies that bind to cell surface receptors or antigens. The antisense nucleic acid molecules can also be delivered to cells using the vectors described herein. To achieve sufficient intracellular concentrations of antisense molecules, vector constructs in which the antisense nucleic acid molecule is placed under the control of a strong pol II or pol III promoter are preferred.

In yet another embodiment, the antisense nucleic acid molecule of the invention is an α-anomeric nucleic acid molecule. An α-anomeric nucleic acid molecule forms specific double-stranded hybrids with complementary RNA in which, contrary to the usual β-units, the strands run parallel to each other (Gaultier *et al*. (1987) *Nucleic Acids Res* 15: 6625-6641). The antisense nucleic acid molecule can also comprise a 2'-o-methylribonucleotide (Inoue *et al*. (1987) *Nucleic Acids Res* 15: 6131-6148) or a chimeric RNA -DNA analogue (Inoue *et al*. (1987) *FEBS Lett* 215: 327-330).

### Ribozymes and PNA moieties

Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject.

In still another embodiment, an antisense nucleic acid of the invention is a ribozyme. Ribozymes are catalytic RNA molecules with ribonuclease activity that are capable of cleaving a single-stranded nucleic acid, such as an mRNA, to which they have a complementary region. Thus, ribozymes (*e*.*g*., hammerhead ribozymes (described in Haselhoff and Gerlach (1988) *Nature* 334:585-591)) can be used to catalytically cleave NOVX mRNA transcripts to thereby inhibit translation of NOVX mRNA. A ribozyme having specificity for a NOVX-encoding nucleic acid can be designed based upon the nucleotide sequence of a NOVX DNA disclosed herein (*i*.*e*., SEQ ID NO:1, 3, 5, 7, 9, 11, 13, 15, 17, 19, 21, 23, 25, 27, 29, 31, 33, 35, 37, 39, 41, 43, and 45). For example, a derivative of a Tetrahymena L-19 IVS RNA can be constructed in which the nucleotide sequence of the active site is complementary to the nucleotide sequence to be cleaved in a NOVX-encoding mRNA. See, *e.g.,* Cech *et al*. U.S. Pat. No. 4,987,071; and Cech *et al*. U.S. Pat. No. 5,116,742. Alternatively, NOVX mRNA can be used to select a catalytic RNA having a specific ribonuclease activity from a pool of RNA molecules. See, *e*.*g*., Bartel *et al*., (1993) *Science* 261:1411-1418.

Alternatively, NOVX gene expression can be inhibited by targeting nucleotide sequences complementary to the regulatory region of the NOVX (*e*.*g*., the NOVX promoter and/or enhancers) to form triple helical structures that prevent transcription of the NOVX gene in target cells. See generally, Helene. (1991) *Anticancer Drug Des*. 6: 569-84; Helene. *et al*. (1992) *Ann. N*.*Y*. *Acad*. *Sci.* 660:27-36; and Maher (1992) *Bioassays* 14: 807-15.

In various embodiments, the nucleic acids of NOVX can be modified at the base moiety, sugar moiety or phosphate backbone to improve, *e.g*., the stability, hybridization, or solubility of the molecule. For example, the deoxyribose phosphate backbone of the nucleic acids can be modified to generate peptide nucleic acids (see Hyrup *et al*. (1996) *Bioorg Med Chem* 4: 5-23). As used herein, the terms "peptide nucleic acids" or "PNAs" refer to nucleic acid mimics, *e*.*g*., DNA mimics, in which the deoxyribose phosphate backbone is replaced by a pseudopeptide backbone and only the four natural nucleobases are retained. The neutral backbone of PNAs has been shown to allow for specific hybridization to DNA and RNA under conditions of low ionic strength. The synthesis of PNA oligomers can be performed using standard solid phase peptide synthesis protocols as described in Hyrup *et al*. (1996) above; Perry-O'Keefe *et al*. (1996) *PNAS* 93: 14670-675.

PNAs of NOVX can be used in therapeutic and diagnostic applications. For example, PNAs can be used as antisense or antigene agents for sequence-specific modulation of gene expression by, *e*.*g*., inducing transcription or translation arrest or inhibiting replication. PNAs of NOVX can also be used, *e.g.,* in the analysis of single base pair mutations in a gene by, *e*.*g*., PNA directed PCR clamping; as artificial restriction enzymes when used in combination with other enzymes, *e.g*., S1 nucleases (Hyrup B. (1996) above); or as probes or primers for DNA sequence and hybridization (Hyrup *et al*. (1996), above; Perry-O'Keefe (1996), above).

In another embodiment, PNAs of NOVX can be modified, *e.g.*, to enhance their stability or cellular uptake, by attaching lipophilic or other helper groups to PNA, by the formation of PNA-DNA chimeras, or by the use of liposomes or other techniques of drug delivery known in the art. For example, PNA-DNA chimeras of NOVX can be generated that may combine the advantageous properties of PNA and DNA. Such chimeras allow DNA recognition enzymes, *e.g.*, RNase H and DNA polymerases, to interact with the DNA portion while the PNA portion would provide high binding affinity and specificity. PNA-DNA chimeras can be linked using linkers of appropriate lengths selected in terms of base stacking, number of bonds between the nucleobases, and orientation (Hyrup (1996) above). The synthesis of PNA-DNA chimeras can be performed as described in Hyrup (1996) above and Finn *et al*. (1996) *Nucl Acids Res* 24: 3357-63. For example, a DNA chain can be synthesized on a solid support using standard phosphoramidite coupling chemistry, and modified nucleoside analogs, *e*.*g*., 5'-(4-methoxytrityl)amino-5'-deoxy-thymidine phosphoramidite, can be used between the PNA and the 5' end of DNA (Mag *et al*. (1989) *Nucl Acid Res* 17: 5973-88). PNA monomers are then coupled in a stepwise manner to produce a chimeric molecule with a 5' PNA segment and a 3' DNA segment (Finn *et al*. (1996) above). Alternatively, chimeric molecules can be synthesized with a 5' DNA segment and a 3' PNA segment. See, Petersen *et al*. (1975) *Bioorg Med Chem Lett* 5: 1119-11124.

In other embodiments, the oligonucleotide may include other appended groups such as peptides (*e*.*g*., for targeting host cell receptors *in vivo),* or agents facilitating transport across the cell membrane (see, *e*.*g*., Letsinger *et al*., 1989, *Proc. Natl. Acad*. *Sci. U*.*S*.*A*. 86:6553-6556; Lemaitre *et al*., 1987, Proc. *Natl*. *Acad*. *Sci.* 84:648-652; PCT Publication No. W088/09810) or the blood-brain barrier (see, *e*.*g*., PCT Publication No. W089/10134). In addition, oligonucleotides can be modified with hybridization triggered cleavage agents (See, *e.g.,* Krol *et al*., 1988, *BioTechniques* 6:958-976) or intercalating agents. (See, *e*.*g*., Zon, 1988, *Pharm. Res.* 5: 539-549). To this end, the oligonucleotide may be conjugated to another molecule, *e*.*g*., a peptide, a hybridization triggered cross-linking agent, a transport agent, a hybridization-triggered cleavage agent, etc.

### NOVX polypeptides

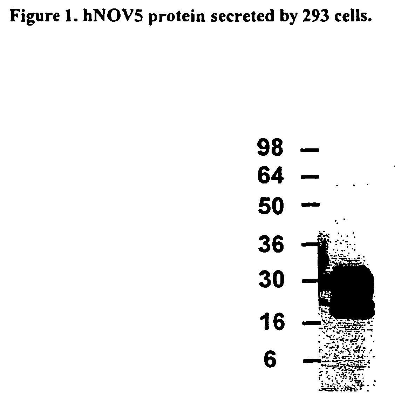

The novel protein of the invention includes the NOVX-like protein whose sequence is provided in any of SEQ ID NO:2, 4, 6, 8, 10, 12, 14, 16, 18, 20, 22, 24, 26, 28, 30, 32, 34, 36, 38, 40, 42, 44, and 46. The invention also includes a mutant or variant protein any of whose residues may be changed from the corresponding residue shown in FIG. 1 while still encoding a protein that maintains its NOVX-like activities and physiological functions, or a functional fragment thereof. For example, the invention includes the polypeptides encoded by the variant NOVX nucleic acids described above. In the mutant or variant protein, up to 20% or more of the residues may be so changed.

In general, a NOVX -like variant that preserves NOVX-like function includes any variant in which residues at a particular position in the sequence have been substituted by other amino acids, and further include the possibility of inserting an additional residue or residues between two residues of the parent protein as well as the possibility of deleting one or more residues from the parent sequence. Any amino acid substitution, insertion, or deletion is encompassed by the invention. In favorable circumstances, the substitution is a conservative substitution as defined above. Furthermore, without limiting the scope of the invention, positions of any of SEQ ID NO:2, 4, 6, 8, 10, 12, 14, 16, 18, 20, 22, 24, 26, 28, 30, 32, 34, 36, 38, 40, 42, 44, and 46 may be substitute such that a mutant or variant protein may include one or more substitutions

The invention also includes isolated NOVX proteins, and biologically active portions thereof, or derivatives, fragments, analogs or homologs thereof. Also provided are polypeptide fragments suitable for use as immunogens to raise anti-NOVX antibodies. In one embodiment, native NOVX proteins can be isolated from cells or tissue sources by an appropriate purification scheme using standard protein purification techniques. In another embodiment, NOVX proteins are produced by recombinant DNA techniques. Alternative to recombinant expression, a NOVX protein or polypeptide can be synthesized chemically using standard peptide synthesis techniques.

An "isolated" or "purified" protein or biologically active portion thereof is substantially free of cellular material or other contaminating proteins from the cell or tissue source from which the NOVX protein is derived, or substantially free from chemical precursors or other chemicals when chemically synthesized. The language "substantially free of cellular material" includes preparations of NOVX protein in which the protein is separated from cellular components of the cells from which it is isolated or recombinantly produced. In one embodiment, the language "substantially free of cellular material" includes preparations of NOVX protein having less than about 30% (by dry weight) of non-NOVX protein (also referred to herein as a "contaminating protein"), more preferably less than about 20% of non-NOVX protein, still more preferably less than about 10% of non-NOVX protein, and most preferably less than about 5% non-NOVX protein. When the NOVX protein or biologically active portion thereof is recombinantly produced, it is also preferably substantially free of culture medium, *i*.*e*., culture medium represents less than about 20%, more preferably less than about 10%, and most preferably less than about 5% of the volume of the protein preparation.

The language "substantially free of chemical precursors or other chemicals" includes preparations of NOVX protein in which the protein is separated from chemical precursors or other chemicals that are involved in the synthesis of the protein. In one embodiment, the language "substantially free of chemical precursors or other chemicals" includes preparations of NOVX protein having less than about 30% (by dry weight) of chemical precursors or non-NOVX chemicals, more preferably less than about 20% chemical precursors or non-NOVX chemicals, still more preferably less than about 10% chemical precursors or non-NOVX chemicals, and most preferably less than about 5% chemical precursors or non-NOVX chemicals.

Biologically active portions of a NOVX protein include peptides comprising amino acid sequences sufficiently homologous to or derived from the amino acid sequence of the NOVX protein, *e*.*g*., the amino acid sequence shown in SEQ ID NO:2 that include fewer amino acids than the full length NOVX proteins, and exhibit at least one activity of a NOVX protein. Typically, biologically active portions comprise a domain or motif with at least one activity of the NOVX protein. A biologically active portion of a NOVX protein can be a polypeptide, which is, for example, 10, 25, 50, 100 or more amino acids in length.

A biologically active portion of a NOVX protein of the present invention may contain at least one of the above-identified domains conserved between the FGF family of proteins. Moreover, other biologically active portions, in which other regions of the protein are deleted, can be prepared by recombinant techniques and evaluated for one or more of the functional activities of a native NOVX protein.

In an embodiment, the NOVX protein has an amino acid sequence shown in any of SEQ ID NO:2, 4, 6, 8, 10, 12, 14, 16, 18, 20, 22, 24, 26, 28, 30, 32, 34, 36, 38, 40, 42, 44, and 46. In other embodiments, the NOVX protein is substantially homologous to any of SEQ ID NO:2, 4, 6, 8, 10, 12, 14, 16, 18, 20, 22, 24, 26, 28, 30, 32, 34, 36, 38, 40, 42, 44, and 46 and retains the functional activity of the protein of any of SEQ ID NO:2, 4, 6, 8, 10, 12, 14, 16, 18, 20, 22, 24, 26, 28, 30, 32, 34, 36, 38, 40, 42, 44, and 46, yet differs in amino acid sequence due to natural allelic variation or mutagenesis, as described in detail below. Accordingly, in another embodiment, the NOVX protein is a protein that comprises an amino acid sequence at least about 45% homologous, and more preferably about 55, 65, 70, 75, 80, 85, 90, 95, 98 or even 99% homologous to the amino acid sequence of any of SEQ ID NO:2, 4, 6, 8, 10, 12, 14, 16, 18, 20, 22, 24, 26, 28, 30, 32, 34, 36, 38, 40, 42, 44, and 46 and retains the functional activity of the NOVX proteins of the corresponding polypeptide having the sequence of SEQ ID NO:2, 4, 6, 8, 10, 12, 14, 16, 18, 20, 22, 24, 26, 28, 30, 32, 34, 36, 38, 40, 42, 44, and 46.

### Determining homology between two or more sequences

To determine the percent homology of two amino acid sequences or of two nucleic acids, the sequences are aligned for optimal comparison purposes (*e*.*g*., gaps can be introduced in either of the sequences being compared for optimal alignment between the sequences). The amino acid residues or nucleotides at corresponding amino acid positions or nucleotide positions are then compared. When a position in the first sequence is occupied by the same amino acid residue or nucleotide as the corresponding position in the second sequence, then the molecules are homologous at that position (*i*.*e*., as used herein amino acid or nucleic acid "homology" is equivalent to amino acid or nucleic acid "identity").

The nucleic acid sequence homology may be determined as the degree of identity between two sequences. The homology may be determined using computer programs known in the art, such as GAP software provided in the GCG program package. See, *Needleman and Wunsch* 1970 *J Mol Biol* 48: 443-453. Using GCG GAP software with the following settings for nucleic acid sequence comparison: GAP creation penalty of 5.0 and GAP extension penalty of 0.3, the coding region of the analogous nucleic acid sequences referred to above exhibits a degree of identity preferably of at least 70%, 75%, 80%, 85%, 90%, 95%, 98%, or 99%, with the CDS (encoding) part of the DNA sequence shown in SEQ ID NO:1, 3, 5, 7, 9, 11, 13, 15, 17, 19, 21, 23, 25, 27, 29, 31, 33, 35, 37, 39, 41, 43, and 45.

The term "sequence identity" refers to the degree to which two polynucleotide or polypeptide sequences are identical on a residue-by-residue basis over a particular region of comparison. The term "percentage of sequence identity" is calculated by comparing two optimally aligned sequences over that region of comparison, determining the number of positions at which the identical nucleic acid base (*e*.*g*., A, T, C, G, U, or I, in the case of nucleic acids) occurs in both sequences to yield the number of matched positions, dividing the number of matched positions by the total number of positions in the region of comparison (*i*.*e*., the window size), and multiplying the result by 100 to yield the percentage of sequence identity. The term "substantial identity" as used herein denotes a characteristic of a polynucleotide sequence, wherein the polynucleotide comprises a sequence that has at least 80 percent sequence identity, preferably at least 85 percent identity and often 90 to 95 percent sequence identity, more usually at least 99 percent sequence identity as compared to a reference sequence over a comparison region. The term "percentage of positive residues" is calculated by comparing two optimally aligned sequences over that region of comparison, determining the number of positions at which the identical and conservative amino acid substitutions, as defined above, occur in both sequences to yield the number of matched positions, dividing the number of matched positions by the total number of positions in the region of comparison (*i*.*e*., the window size), and multiplying the result by 100 to yield the percentage of positive residues.

### Chimeric and fusion proteins

The invention also provides NOVX chimeric or fusion proteins. As used herein, a NOVX "chimeric protein" or "fusion protein" includes a NOVX polypeptide operatively linked to a non-NOVX polypeptide. A "NOVX polypeptide" refers to a polypeptide having an amino acid sequence corresponding to NOVX, whereas a "non-NOVX polypeptide" refers to a polypeptide having an amino acid sequence corresponding to a protein that is not substantially homologous to the NOVX protein, *e.g.*, a protein that is different from the NOVX protein and that is derived from the same or a different organism. Within a NOVX fusion protein the NOVX polypeptide can correspond to all or a portion of a NOVX protein. In one embodiment, a NOVX fusion protein comprises at least one biologically active portion of a NOVX protein. In another embodiment, a NOVX fusion protein comprises at least two biologically active portions of a NOVX protein. Within the fusion protein, the term "operatively linked" is intended to indicate that the NOVX polypeptide and the non-NOVX polypeptide are fused in-frame to each other. The non-NOVX polypeptide can be fused to the N-terminus or C-terminus of the NOVX polypeptide.

For example, in one embodiment a NOVX fusion protein comprises a NOVX polypeptide operably linked to the extracellular domain of a second protein. Such fusion proteins can be further utilized in screening assays for compounds that modulate NOVX activity (such assays are described in detail below).

In another embodiment, the fusion protein is a GST-NOVX fusion protein in which the NOVX sequences are fused to the C-terminus of the GST (i.e., glutathione S-transferase) sequences. Such fusion proteins can facilitate the purification of recombinant NOVX.

In yet another embodiment, the fusion protein is a NOVX protein containing a heterologous signal sequence at its N-terminus. For example, the native NOVX signal sequence can be removed and replaced with a signal sequence from another protein. In certain host cells (*e*.*g*., mammalian host cells), expression and/or secretion of NOVX can be increased through use of a heterologous signal sequence.

In another embodiment, the fusion protein is a NOVX-immunoglobulin fusion protein in which the NOVX sequences comprising one or more domains are fused to sequences derived from a member of the immunoglobulin protein family. The NOVX-immunoglobulin fusion proteins of the invention can be incorporated into pharmaceutical compositions and administered to a subject to inhibit an interaction between a NOVX ligand and a NOVX protein on the surface of a cell, to thereby suppress NOVX-mediated signal transduction *in vivo*. In one nonlimiting example, a contemplated NOVX ligand of the invention is a NOVX receptor. The NOVX-immunoglobulin fusion proteins can be used to modulate the bioavailability of a NOVX cognate ligand. Inhibition of the NOVX ligand/NOVX interaction may be useful therapeutically for both the treatment of proliferative and differentiative disorders, as well as modulating (*e.g*., promoting or inhibiting) cell survival. Moreover, the NOVX-immunoglobulin fusion proteins of the invention can be used as immunogens to produce anti-NOVX antibodies in a subject, to purify NOVX ligands, and in screening assays to identify molecules that inhibit the interaction of NOVX with a NOVX ligand.

A NOVX chimeric or fusion protein of the invention can be produced by standard recombinant DNA techniques. For example, DNA fragments coding for the different polypeptide sequences are ligated together in-frame in accordance with conventional techniques, *e.g.*, by employing blunt-ended or stagger-ended termini for ligation, restriction enzyme digestion to provide for appropriate termini, filling-in of cohesive ends as appropriate, alkaline phosphatase treatment to avoid undesirable joining, and enzymatic ligation. In another embodiment, the fusion gene can be synthesized by conventional techniques including automated DNA synthesizers. Alternatively, PCR amplification of gene fragments can be carried out using anchor primers that give rise to complementary overhangs between two consecutive gene fragments that can subsequently be annealed and reamplified to generate a chimeric gene sequence (see, for example, Ausubel et al. (eds.) CURRENT PROTOCOLS IN MOLECULAR BIOLOGY, John Wiley & Sons, 1992). Moreover, many expression vectors are commercially available that already encode a fusion moiety (*e.g*., a GST polypeptide). A NOVX-encoding nucleic acid can be cloned into such an expression vector such that the fusion moiety is linked in-frame to the NOVX protein.

### NOVX agonists and antagonists

The present invention also pertains to variants of the NOVX proteins that function as either NOVX agonists (mimetics) or as NOVX antagonists. Variants of the NOVX protein can be generated by mutagenesis, *e*.*g*., discrete point mutation or truncation of the NOVX protein. An agonist of the NOVX protein can retain substantially the same, or a subset of, the biological activities of the naturally occurring form of the NOVX protein. An antagonist of the NOVX protein can inhibit one or more of the activities of the naturally occurring form of the NOVX protein by, for example, competitively binding to a downstream or upstream member of a cellular signaling cascade which includes the NOVX protein. Thus, specific biological effects can be elicited by treatment with a variant of limited function. In one embodiment, treatment of a subject with a variant having a subset of the biological activities of the naturally occurring form of the protein has fewer side effects in a subject relative to treatment with the naturally occurring form of the NOVX proteins.

Variants of the NOVX protein that function as either NOVX agonists (mimetics) or as NOVX antagonists can be identified by screening combinatorial libraries of mutants, *e*.*g*., truncation mutants, of the NOVX protein for NOVX protein agonist or antagonist activity. In one embodiment, a variegated library of NOVX variants is generated by combinatorial mutagenesis at the nucleic acid level and is encoded by a variegated gene library. A variegated library of NOVX variants can be produced by, for example, enzymatically ligating a mixture of synthetic oligonucleotides into gene sequences such that a degenerate set of potential NOVX sequences is expressible as individual polypeptides, or alternatively, as a set of larger fusion proteins (*e*.*g*., for phage display) containing the set of NOVX sequences therein. There are a variety of methods which can be used to produce libraries of potential NOVX variants from a degenerate oligonucleotide sequence. Chemical synthesis of a degenerate gene sequence can be performed in an automatic DNA synthesizer, and the synthetic gene then ligated into an appropriate expression vector. Use of a degenerate set of genes allows for the provision, in one mixture, of all of the sequences encoding the desired set of potential NOVX sequences. Methods for synthesizing degenerate oligonucleotides are known in the art (see, *e*.*g*., Narang (1983) *Tetrahedron* 39:3; Itakura *et al*. (1984) *Annu Rev Biochem* 53:323; Itakura *et al*. (1984) *Science* 198:1056; Ike *et al*. (1983) *Nucl Acid Res* 11:477.

### Polypeptide libraries

In addition, libraries of fragments of the NOVX protein coding sequence can be used to generate a variegated population of NOVX fragments for screening and subsequent selection of variants of a NOVX protein. In one embodiment, a library of coding sequence fragments can be generated by treating a double stranded PCR fragment of a NOVX coding sequence with a nuclease under conditions wherein nicking occurs only about once per molecule, denaturing the double stranded DNA, renaturing the DNA to form double stranded DNA that can include sense/antisense pairs from different nicked products, removing single stranded portions from reformed duplexes by treatment with S1 nuclease, and ligating the resulting fragment library into an expression vector. By this method, an expression library can be derived which encodes N-terminal and internal fragments of various sizes of the NOVX protein.

Several techniques are known in the art for screening gene products of combinatorial libraries made by point mutations or truncation, and for screening cDNA libraries for gene products having a selected property. Such techniques are adaptable for rapid screening of the gene libraries generated by the combinatorial mutagenesis of NOVX proteins. The most widely used techniques, which are amenable to high throughput analysis, for screening large gene libraries typically include cloning the gene library into replicable expression vectors, transforming appropriate cells with the resulting library of vectors, and expressing the combinatorial genes under conditions in which detection of a desired activity facilitates isolation of the vector encoding the gene whose product was detected. Recrusive ensemble mutagenesis (REM), a new technique that enhances the frequency of functional mutants in the libraries, can be used in combination with the screening assays to identify NOVX variants (Arkin and Yourvan (1992) PNAS 89:7811-7815; Delgrave *et al*. (1993) Protein Engineering 6:327-331).

### Anti-NOVX Antibodies

The invention further encompasses antibodies and antibody fragments, such as F_{ab} or (F_{ab})₂, that bind immunospecifically to any of the proteins of the invention.

An isolated NOVX protein, or a portion or fragment thereof, can be used as an immunogen to generate antibodies that bind NOVX using standard techniques for polyclonal and monoclonal antibody preparation. Full-length NOVX protein can be used. Alternatively, the invention provides antigenic peptide fragments of NOVX for use as immunogens. The antigenic peptide of NOVX comprises at least 4 amino acid residues of the amino acid sequence shown in any of SEQ ID NO:2, 4, 6, 8, 10, 12, 14, 16, 18, 20, 22, 24, 26, 28, 30, 32, 34, 36, 38, 40, 42, 44, and 46. The antigenic peptide encompasses an epitope of NOVX such that an antibody raised against the peptide forms a specific immune complex with NOVX. The antigenic peptide may comprise at least 6 aa residues, at least 8 aa residues, at least 10 aa residues, at least 15 aa residues, at least 20 aa residues, or at least 30 aa residues. In one embodiment of the invention, the antigenic peptide comprises a polypeptide comprising at least 6 contiguous amino acids of any of SEQ ID NO:2, 4, 6, 8, 10, 12, 14, 16, 18, 20, 22, 24, 26, 28, 30, 32, 34, 36, 38, 40, 42, 44, and 46.

In an embodiment of the invention, epitopes encompassed by the antigenic peptide are regions of NOVX that are located on the surface of the protein, *e*.*g*., hydrophilic regions. As a means for targeting antibody production, hydropathy plots showing regions of hydrophilicity and hydrophobicity may be generated by any method well known in the art, including, for example, the Kyte Doolittle or the Hopp Woods methods, either with or without Fourier transformation. See, *e.g.,* Hopp and Woods, 1981, Proc. Nat. Acad. Sci. USA 78: 3824-3828; Kyte and Doolittle 1982, J. Mol. Biol. 157: 105-142, each incorporated herein by reference in their entirety.

As disclosed herein, a NOVX protein sequence of any of SEQ ID NO:2, 4, 6, 8, 10, 12, 14, 16, 18, 20, 22, 24, 26, 28, 30, 32, 34, 36, 38, 40, 42, 44, and 46, or derivatives, fragments, analogs or homologs thereof, may be utilized as immunogens in the generation of antibodies that immunospecifically-bind these protein components. The term "antibody" as used herein refers to immunoglobulin molecules and immunologically active portions of immunoglobulin molecules, i.e., molecules that contain an antigen binding site that specifically binds (immunoreacts with) an antigen, such as NOVX. Such antibodies include, but are not limited to, polyclonal, monoclonal, chimeric, single chain, F_{ab} and F_{(ab')2} fragments, and an F_{ab} expression library. In a specific embodiment, antibodies to human NOVX proteins are disclosed. Various procedures known within the art may be used for the production of polyclonal or monoclonal antibodies to a NOVX protein sequence of any of SEQ ID NO:2, 4, 6, 8, 10, 12, 14, 16, 18, 20, 22, 24, 26, 28, 30, 32, 34, 36, 38, 40, 42, 44, and 46 or derivative, fragment, analog or homolog thereof. Some of these proteins are discussed below.

For the production of polyclonal antibodies, various suitable host animals (*e*.*g*., rabbit, goat, mouse or other mammal) may be immunized by injection with the native protein, or a synthetic variant thereof, or a derivative of the foregoing. An appropriate immunogenic preparation can contain, for example, recombinantly expressed NOVX protein or a chemically synthesized NOVX polypeptide. The preparation can further include an adjuvant. Various adjuvants used to increase the immunological response include, but are not limited to, Freund's (complete and incomplete), mineral gels (*e*.*g*., aluminum hydroxide), surface active substances (*e.g.*, lysolecithin, pluronic polyols, polyanions, peptides, oil emulsions, dinitrophenol, etc.), human adjuvants such as *Bacille Calmette-Guerin* and *Corynebacterium parvum,* or similar immunostimulatory agents. If desired, the antibody molecules directed against NOVX can be isolated from the mammal (*e*.*g*., from the blood) and further purified by well known techniques, such as protein A chromatography to obtain the IgG fraction.

The term "monoclonal antibody" or "monoclonal antibody composition", as used herein, refers to a population of antibody molecules that contain only one species of an antigen binding site capable of inimunoreacting with a particular epitope of NOVX. A monoclonal antibody composition thus typically displays a single binding affinity for a particular NOVX protein with which it immunoreacts. For preparation of monoclonal antibodies directed towards a particular NOVX protein, or derivatives, fragments, analogs or homologs thereof, any technique that provides for the production of antibody molecules by continuous cell line culture may be utilized. Such techniques include, but are not limited to, the hybridoma technique (see Kohler & Milstein, 1975 *Nature* 256: 495-497); the trioma technique; the human B-cell hybridoma technique (see Kozbor, *et al*., 1983 *Immunol Today* 4: 72) and the EBV hybridoma technique to produce human monoclonal antibodies (see Cole, *et al*., 1985 In: MONOCLONAL ANTIBODIES AND CANCER THERAPY, Alan R. Liss, Inc., pp. 77-96). Human monoclonal antibodies may be utilized in the practice of the present invention and may be produced by using human hybridomas (see Cote, *et al*., 1983. *Proc Natl Acad Sci USA* 80: 2026-2030) or by transforming human B-cells with Epstein Barr Virus *in vitro* (see Cole, *et al*., 1985 In: MONOCLONAL ANTIBODIES AND CANCER THERAPY, Alan R. Liss, Inc., pp. 77-96). Each of the above citations are incorporated herein by reference in their entirety

According to the invention, techniques can be adapted for the production of single-chain antibodies specific to a NOVX protein (see *e.g*., U.S. Patent No. 4,946,778). In addition, methods can be adapted for the construction of F_{ab} expression libraries (see *e*.*g*., Huse, *et al*., 1989 *Science* 246: 1275-1281) to allow rapid and effective identification of monoclonal F_{ab} fragments with the desired specificity for a NOVX protein or derivatives, fragments, analogs or homologs thereof. Non-human antibodies can be "humanized" by techniques well known in the art. See *e*.*g*., U.S. Patent No. 5,225,539. Each of the above citations are incorporated herein by reference. Antibody fragments that contain the idiotypes to a NOVX protein may be produced by techniques known in the art including, but riot limited to: (*i*) an F_{(ab')2} fragment produced by pepsin digestion of an antibody molecule; (*ii*) an F_{ab} fragment generated by reducing the disulfide bridges of an F_{(ab')2} fragment; (*iii*) an F_{ab} fragment generated by the treatment of the antibody molecule with papain and a reducing agent and (*iv*) Fᵥ fragments.

Additionally, recombinant anti-NOVX antibodies, such as chimeric and humanized monoclonal antibodies, comprising both human and non-human portions, which can be made using standard recombinant DNA techniques, are within the scope of the invention. Such chimeric and humanized monoclonal antibodies can be produced by recombinant DNA techniques known in the art, for example using methods described in PCT International Application No. PCT/US86/02269; European Patent Application No. 184,187; European Patent Application No. 171,496; European Patent Application No. 173,494; PCT International Publication No. WO 86/01533; U.S. Pat. No. 4,816,567; European Patent Application No. 125,023; Better *et al*.(1988) *Science* 240:1041-1043; Liu *et al*. (1987) *PNAS* 84:3439-3443; Liu *et al*. (1987) *J Immunol*. 139:3521-3526; Sun *et al*. (1987) *PNAS* 84:214-218; Nishimura *et al*. (1987) *Cancer Res* 47:999-1005; Wood *et al*. (1985) *Nature* 314:446-449; Shaw *et al*. (1988), *J*. *Natl Cancer Inst* 80:1553-1559); Morrison(1985) *Science* 229:1202-1207; Oi *et al*. (1986) *BioTechniques* 4:214; U.S. Pat. No. 5,225,539; Jones *et al*. (1986) *Nature* 321:552-525; Verhoeyan *et al*. (1988) *Science* 239:1534; and Beidler *et al*. (1988) *J Immunol* 141:4053-4060. Each of the above citations are incorporated herein by reference.

In one embodiment, methods for the screening of antibodies that possess the desired specificity include, but are not limited to, enzyme-linked immunosorbent assay (ELISA) and other immunologically-mediated techniques known within the art. In a specific embodiment, selection of antibodies that are specific to a particular domain of a NOVX protein is facilitated by generation of hybridomas that bind to the fragment of a NOVX protein possessing such a domain. Antibodies that are specific for one or more domains within a NOVX protein, *e*.*g*., the domain spanning the first fifty amino-terminal residues specific to NOVX when compared to FGF-9, or derivatives, fragments, analogs or homologs thereof, are also provided herein.

Anti-NOVX antibodies may be used in methods known within the art relating to the localization and/or quantitation of a NOVX protein (*e*.*g*., for use in measuring levels of the NOVX protein within appropriate physiological samples, for use in diagnostic methods, for use in imaging the protein, and the like). In a given embodiment, antibodies for NOVX proteins, or derivatives, fragments, analogs or homologs thereof, that contain the antibody derived binding domain, are utilized as pharmacologically-active compounds [hereinafter "Therapeutics"].

An anti-NOVX antibody (*e*.*g*., monoclonal antibody) can be used to isolate NOVX by standard techniques, such as affinity chromatography or immunoprecipitation. An anti-NOVX antibody can facilitate the purification of natural NOVX from cells and of recombinantly produced NOVX expressed in host cells. Moreover, an anti-NOVX antibody can be used to detect NOVX protein (*e*.*g*., in a cellular lysate or cell supernatant) in order to evaluate the abundance and pattern of expression of the NOVX protein. Anti-NOVX antibodies can be used diagnostically to monitor protein levels in tissue as part of a clinical testing procedure, *e.g.,* to, for example, determine the efficacy of a given treatment regimen. Detection can be facilitated by coupling (*i*.*e*., physically linking) the antibody to a detectable substance. Examples of detectable substances include various enzymes, prosthetic groups, fluorescent materials, luminescent materials, bioluminescent materials, and radioactive materials. Examples of suitable enzymes include horseradish peroxidase, alkaline phosphatase, β-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin, and examples of suitable radioactive material include ¹²⁵I, ¹³¹I, ³⁵S or ³H.

### NOVX Recombinant Vectors and Host Cells

Another aspect of the invention pertains to vectors, preferably expression vectors, containing a nucleic acid encoding NOVX protein, or derivatives, fragments, analogs or homologs thereof. As used herein, the term "vector" refers to a nucleic acid molecule capable of transporting another nucleic acid to which it has been linked. One type of vector is a "plasmid", which refers to a circular double stranded DNA loop into which additional DNA segments can be ligated. Another type of vector is a viral vector, wherein additional DNA segments can be ligated into the viral genome. Certain vectors are capable of autonomous replication in a host cell into which they are introduced (*e*.*g*., bacterial vectors having a bacterial origin of replication and episomal mammalian vectors). Other vectors (*e*.*g*., non-episomal mammalian vectors) are integrated into the genome of a host cell upon introduction into the host cell, and thereby are replicated along with the host genome. Moreover, certain vectors are capable of directing the expression of genes to which they are operatively linked. Such vectors are referred to herein as "expression vectors". In general, expression vectors of utility in recombinant DNA techniques are often in the form ofplasmids. In the present specification, "plasmid" and "vector" can be used interchangeably as the plasmid is the most commonly used form of vector. However, the invention is intended to include such other forms of expression vectors, such as viral vectors (*e*.*g*., replication defective retroviruses, adenoviruses and adeno-associated viruses), which serve equivalent functions.

The recombinant expression vectors of the invention comprise a nucleic acid of the invention in a form suitable for expression of the nucleic acid in a host cell, which means that the recombinant expression vectors include one or more regulatory sequences, selected on the basis of the host cells to be used for expression, that is operatively linked to the nucleic acid sequence to be expressed. Within a recombinant expression vector, "operably linked" is intended to mean that the nucleotide sequence of interest is linked to the regulatory sequence(s) in a manner that allows for expression of the nucleotide sequence (*e*.*g*., in an *in vitro* transcription/translation system or in a host cell when the vector is introduced into the host cell). The term "regulatory sequence" is intended to includes promoters, enhancers and other expression control elements (*e*.*g*., polyadenylation signals). Such regulatory sequences are described, for example, in Goeddel; GENE EXPRESSION TECHNOLOGY: METHODS IN ENZYMOLOGY 185, Academic Press, San Diego, Calif. (1990). Regulatory sequences include those that direct constitutive expression of a nucleotide sequence in many types of host cell and those that direct expression of the nucleotide sequence only in certain host cells (*e*.*g*., tissue-specific regulatory sequences). It will be appreciated by those skilled in the art that the design of the expression vector can depend on such factors as the choice of the host cell to be transformed, the level of expression of protein desired, etc. The expression vectors of the invention can be introduced into host cells to thereby produce proteins or peptides, including fusion proteins or peptides, encoded by nucleic acids as described herein (*e*.*g*., NOVX proteins, mutant forms of NOVX, fusion proteins, etc.).

The recombinant expression vectors of the invention can be designed for expression of NOVX in prokaryotic or eukaryotic cells. For example, NOVX can be expressed in bacterial cells such as *E. coli,* insect cells (using baculovirus expression vectors) yeast cells or mammalian cells. Suitable host cells are discussed further in Goeddel, GENE EXPRESSION TECHNOLOGY: METHODS IN ENZYMOLOGY 185, Academic Press, San Diego, Calif. (1990). Alternatively, the recombinant expression vector can be transcribed and translated *in vitro*, for example using T7 promoter regulatory sequences and T7 polymerase.

Expression of proteins in prokaryotes is most often carried out in *E. coli* with vectors containing constitutive or inducible promoters directing the expression of either fusion or non-fusion proteins. Fusion vectors add a number of amino acids to a protein encoded therein, usually to the amino terminus of the recombinant protein. Such fusion vectors typically serve three purposes: (1) to increase expression of recombinant protein; (2) to increase the solubility of the recombinant protein; and (3) to aid in the purification of the recombinant protein by acting as a ligand in affinity purification. Often, in fusion expression vectors, a proteolytic cleavage site is introduced at the junction of the fusion moiety and the recombinant protein to enable separation of the recombinant protein from the fusion moiety subsequent to purification of the fusion protein. Such enzymes, and their cognate recognition sequences, include Factor Xa, thrombin and enterokinase. Typical fusion expression vectors include pGEX (Pharmacia Biotech Inc; Smith and Johnson (1988) *Gene* 67:31-40), pMAL (New England Biolabs, Beverly, Mass.) and pRIT5 (Pharmacia, Piscataway, N.J.) that fuse glutathione S-transferase (GST), maltose E binding protein, or protein A, respectively, to the target recombinant protein.

Examples of suitable inducible non-fusion *E. coli* expression vectors include pTrc (Amrann *et al*., (1988) *Gene* 69:301-315) and pET 11d (Studier *et al*., GENE EXPRESSION TECHNOLOGY: METHODS IN ENZYMOLOGY 185, Academic Press, San Diego, Calif. (1990) 60-89).

One strategy to maximize recombinant protein expression in *E. coli* is to express the protein in a host bacteria with an impaired capacity to proteolytically cleave the recombinant protein. See, Gottesman, GENE EXPRESSION TECHNOLOGY: METHODS IN ENZYMOLOGY 185, Academic Press, San Diego, Calif. (1990) 119-128. Another strategy is to alter the nucleic acid sequence of the nucleic acid to be inserted into an expression vector so that the individual codons for each amino acid are those preferentially utilized in *E*. *coli* (Wada *et al*., (1992) *Nucleic Acids Res.* 20:2111-2118). Such alteration of nucleic acid sequences of the invention can be carried out by standard DNA synthesis techniques.

In another embodiment, the NOVX expression vector is a yeast expression vector. Examples of vectors for expression in yeast *S. cerivisae* include pYepSec1 (Baldari, *et al*., (1987) *EMBO J6:229-234),* pMFa (Kurjan and Herskowitz, (1982) *Cell* 30:933-943), pJRY88 (Schultz *et al*., (1987) *Gene* 54:113-123), pYES2 (Invitrogen Corporation, San Diego, Calif.), and picZ (In Vitrogen Corp, San Diego, Calif.).

Alternatively, NOVX can be expressed in insect cells using baculovirus expression vectors. Baculovirus vectors available for expression of proteins in cultured insect cells (*e*.*g*., SF9 cells) include the pAc series (Smith *et al*. (1983) *Mol Cell Biol* 3:2156-2165) and the pVL series (Lucklow and Summers (1989) *Virology* 170:31-39).

In yet another embodiment, a nucleic acid of the invention is expressed in mammalian cells using a mammalian expression vector. Examples of mammalian expression vectors include pCDM8 (Seed (1987) *Nature* 329:840) and pMT2PC (Kaufman *et al*. (1987) *EMBO J* 6: 187-195). When used in mammalian cells, the expression vector's control functions are often provided by viral regulatory elements. For example, commonly used promoters are derived from polyoma, Adenovirus 2, cytomegalovirus and Simian Virus 40. For other suitable expression systems for both prokaryotic and eukaryotic cells. See, *e*.*g*., Chapters 16 and 17 of Sambrook *et al*., MOLECULAR CLONING: A LABORATORY MANUAL. 2nd ed., Cold Spring Harbor Laboratory, Cold Spring Harbor Laboratory Press, Cold Spring Harbor, N.Y., 1989.

In another embodiment, the recombinant mammalian expression vector is capable of directing expression of the nucleic acid preferentially in a particular cell type (*e*.*g*., tissue-specific regulatory elements are used to express the nucleic acid). Tissue-specific regulatory elements are known in the art. Non-limiting examples of suitable tissue-specific promoters include the albumin promoter (liver-specific; Pinkert *et al*. (1987) *Genes Dev* 1:268-277), lymphoid-specific promoters (Calame and Eaton (1988) *Adv Immunol* 43:235-275), in particular promoters of T cell receptors (Winoto and Baltimore (1989) *EMBO J* 8:729-733) and immunoglobulins (Banerji *et al*. (1983) *Cell* 33:729-740; Queen and Baltimore (1983) *Cell* 33:741-748), neuron-specific promoters (*e*.*g*., the neurofilament promoter; Byrne and Ruddle (1989) *PNAS* 86:5473-5477), pancreas-specific promoters (Edlund *et al*. (1985) *Science* 230:912-916), and mammary gland-specific promoters (*e*.*g*., milk whey promoter; U.S. Pat. No. 4,873,316 and European Application Publication No. 264,166). Developmentally-regulated promoters are also encompassed, *e*.*g*., the murine hox promoters (Kessel and Gruss (1990) *Science* 249:374-379) and the α-fetoprotein promoter (Campes and Tilghman (1989) *Genes Dev* 3:537-546).

The invention further provides a recombinant expression vector comprising a DNA molecule of the invention cloned into the expression vector in an antisense orientation. That is, the DNA molecule is operatively linked to a regulatory sequence in a manner that allows for expression (by transcription of the DNA molecule) of an RNA molecule that is antisense to NOVX mRNA. Regulatory sequences operatively linked to a nucleic acid cloned in the antisense orientation can be chosen that direct the continuous expression of the antisense RNA molecule in a variety of cell types, for instance viral promoters and/or enhancers, or regulatory sequences can be chosen that direct constitutive, tissue specific or cell type specific expression of antisense RNA. The antisense expression vector can be in the form of a recombinant plasmid, phagemid or attenuated virus in which antisense nucleic acids are produced under the control of a high efficiency regulatory region, the activity of which can be determined by the cell type into which the vector is introduced. For a discussion of the regulation of gene expression using antisense genes see Weintraub *et al*., "Antisense RNA as a molecular tool for genetic analysis," Reviews--Trends in Genetics, Vol. 1(1) 1986.

Another aspect of the invention pertains to host cells into which a recombinant expression vector of the invention has been introduced. The terms "host cell" and "recombinant host cell" are used interchangeably herein. It is understood that such terms refer not only to the particular subject cell but to the progeny or potential progeny of such a cell. Because certain modifications may occur in succeeding generations due to either mutation or environmental influences, such progeny may not, in fact, be identical to the parent cell, but are still included within the scope of the term as used herein.

A host cell can be any prokaryotic or eukaryotic cell. For example, NOVX protein can be expressed in bacterial cells such as *E. coli*, insect cells, yeast or mammalian cells (such as Chinese hamster ovary cells (CHO) or COS cells). Other suitable host cells are known to those skilled in the art.

Vector DNA can be introduced into prokaryotic or eukaryotic cells via conventional transformation or transfection techniques. As used herein, the terms "transformation" and "transfection" are intended to refer to a variety of art-recognized techniques for introducing foreign nucleic acid (*e*.*g*., DNA) into a host cell, including calcium phosphate or calcium chloride co-precipitation, DEAE-dextran-mediated transfection, lipofection, or electroporation. Suitable methods for transforming or transfecting host cells can be found in Sambrook, *et al*. (MOLECULAR CLONING: A LABORATORY MANUAL. 2nd ed., Cold Spring Harbor Laboratory, Cold Spring Harbor Laboratory Press, Cold Spring Harbor, N.Y., 1989), and other laboratory manuals.

For stable transfection of mammalian cells, it is known that, depending upon the expression vector and transfection technique used, only a small fraction of cells may integrate the foreign DNA into their genome. In order to identify and select these integrants, a gene that encodes a selectable marker (*e*.*g*., resistance to antibiotics) is generally introduced into the host cells along with the gene of interest. Various selectable markers include those that confer resistance to drugs, such as G418, hygromycin and methotrexate. Nucleic acid encoding a selectable marker can be introduced into a host cell on the same vector as that encoding NOVX or can be introduced on a separate vector. Cells stably transfected with the introduced nucleic acid can be identified by drug selection (*e*.*g*., cells that have incorporated the selectable marker gene will survive, while the other cells die).

A host cell of the invention, such as a prokaryotic or eukaryotic host cell in culture, can be used to produce (*i*.*e*., express) NOVX protein. Accordingly, the invention further provides methods for producing NOVX protein using the host cells of the invention. In one embodiment, the method comprises culturing the host cell of invention (into which a recombinant expression vector encoding NOVX has been introduced) in a suitable medium such that NOVX protein is produced. In another embodiment, the method further comprises isolating NOVX from the medium or the host cell.

### Transgenic animals

The host cells of the invention can also be used to produce nonhuman transgenic animals. For example, in one embodiment, a host cell of the invention is a fertilized oocyte or an embryonic stem cell into which NOVX-coding sequences have been introduced. Such host cells can then be used to create non-human transgenic animals in which exogenous NOVX sequences have been introduced into their genome or homologous recombinant animals in which endogenous NOVX sequences have been altered. Such animals are useful for studying the function and/or activity of NOVX and for identifying and/or evaluating modulators of NOVX activity. As used herein, a "transgenic animal" is a non-human animal, preferably a mammal, more preferably a rodent such as a rat or mouse, in which one or more of the cells of the animal includes a transgene. Other examples of transgenic animals include non-human primates, sheep, dogs, cows, goats, chickens, amphibians, etc. A transgene is exogenous DNA that is integrated into the genome of a cell from which a transgenic animal develops and that remains in the genome of the mature animal, thereby directing the expression of an encoded gene product in one or more cell types or tissues of the transgenic animal. As used herein, a "homologous recombinant animal" is a non-human animal, preferably a mammal, more preferably a mouse, in which an endogenous NOVX gene has been altered by homologous recombination between the endogenous gene and an exogenous DNA molecule introduced into a cell of the animal, *e.g.*, an embryonic cell of the animal, prior to development of the animal.

A transgenic animal of the invention can be created by introducing NOVX-encoding nucleic acid into the male pronuclei of a fertilized oocyte, *e.g*., by microinjection, retroviral infection, and allowing the oocyte to develop in a pseudopregnant female foster animal. The human NOVX DNA sequence of SEQ ID NO:1, 3, 5, 7, 9, 11, 13, 15, 17, 19, 21, 23, 25, 27, 29, 31, 33, 35, 37, 39, 41, 43, and 45 can be introduced as a transgene into the genome of a non-human animal. Alternatively, a nonhuman homologue of the human NOVX gene, such as a mouse NOVX gene, can be isolated based on hybridization to the human NOVX cDNA (described further above) and used as a transgene. Intronic sequences and polyadenylation signals can also be included in the transgene to increase the efficiency of expression of the transgene. A tissue-specific regulatory sequence(s) can be operably linked to the NOVX transgene to direct expression of NOVX protein to particular cells. Methods for generating transgenic animals via embryo manipulation and microinjection, particularly animals such as mice, have become conventional in the art and are described, for example, in U.S. Pat. Nos. 4,736,866; 4,870,009; and 4,873,191; and Hogan 1986, In: MANIPULATING THE MOUSE EMBRYO, Cold Spring Harbor Laboratory Press, Cold Spring Harbor, N.Y. Similar methods are used for production of other transgenic animals. A transgenic founder animal can be identified based upon the presence of the NOVX transgene in its genome and/or expression of NOVX mRNA in tissues or cells of the animals. A transgenic founder animal can then be used to breed additional animals carrying the transgene. Moreover, transgenic animals carrying a transgene encoding NOVX can further be bred to other transgenic animals carrying other transgenes.

To create a homologous recombinant animal, a vector is prepared which contains at least a portion of a NOVX gene into which a deletion, addition or substitution has been introduced to thereby alter, *e.g.*, functionally disrupt, the NOVX gene. The NOVX gene can be a human gene (*e.g.*, SEQ ID NO:1, 3, 5, 7, 9, 11, 13, 15, 17, 19, 21, 23, 25, 27, 29, 31, 33, 35, 37, 39, 41, 43, and 45), but more preferably, is a non-human homologue of a human NOVX gene. For example, a mouse homologue of human NOVX gene of SEQ ID NO:1, 3, 5, 7, 9, 11, 13, 15, 17, 19, 21, 23, 25, 27, 29, 31, 33, 35, 37, 39, 41, 43, and 45 can be used to construct a homologous recombination vector suitable for altering an endogenous NOVX gene in the mouse genome. In one embodiment, the vector is designed such that, upon homologous recombination, the endogenous NOVX gene is functionally disrupted (*i*.*e*., no longer encodes a functional protein; also referred to as a "knock out" vector).

Alternatively, the vector can be designed such that, upon homologous recombination, the endogenous NOVX gene is mutated or otherwise altered but still encodes functional protein (*e*.*g*., the upstream regulatory region can be altered to thereby alter the expression of the endogenous NOVX protein). In the homologous recombination vector, the altered portion of the NOVX gene is flanked at its 5' and 3' ends by additional nucleic acid of the NOVX gene to allow for homologous recombination to occur between the exogenous NOVX gene carried by the vector and an endogenous NOVX gene in an embryonic stem cell. The additional flanking NOVX nucleic acid is of sufficient length for successful homologous recombination with the endogenous gene. Typically, several kilobases of flanking DNA (both at the 5' and 3' ends) are included in the vector. See *e.g*., Thomas *et al*. (1987) *Cell* 51:503 for a description of homologous recombination vectors. The vector is introduced into an embryonic stem cell line (*e*.*g*., by electroporation) and cells in which the introduced NOVX gene has homologously recombined with the endogenous NOVX gene are selected (see *e.g.,* Li *et al*. (1992) *Cell* 69:915).

The selected cells are then injected into a blastocyst of an animal (*e*.*g*., a mouse) to form aggregation chimeras. See *e*.*g*., Bradley 1987, In: TERATOCARCINOMAS AND EMBRYONIC STEM CELLS: A PRACTICAL APPROACH, Robertson, ed. IRL, Oxford, pp. 113-152. A chimeric embryo can then be implanted into a suitable pseudopregnant female foster animal and the embryo brought to term. Progeny harboring the homologously recombined DNA in their germ cells can be used to breed animals in which all cells of the animal contain the homologously recombined DNA by germline transmission of the transgene. Methods for constructing homologous recombination vectors and homologous recombinant animals are described further in Bradley (1991) *Curr Opin Biotechnol* 2:823-829; PCT International Publication Nos.: WO 90/11354; WO 91/01140; WO 92/0968; and WO 93/04169.

In another embodiment, transgenic non-humans animals can be produced that contain selected systems that allow for regulated expression of the transgene. One example of such a system is the cre/loxP recombinase system of bacteriophage P1. For a description of the cre/loxP recombinase system, *see, e.g.,* Lakso *et al*. (1992) *PNAS* 89:6232-6236. Another example of a recombinase system is the FLP recombinase system of *Saccharomyces cerevisiae* (O'Gorman *et al*. (1991) *Science* 251:1351-1355. If a cre/loxP recombinase system is used to regulate expression of the transgene, animals containing transgenes encoding both the Cre recombinase and a selected protein are required. Such animals can be provided through the construction of "double" transgenic animals, *e.g.*, by mating two transgenic animals, one containing a transgene encoding a selected protein and the other containing a transgene encoding a recombinase.

Clones of the non-human transgenic animals described herein can also be produced according to the methods described in Wilmut *et al*. (1997) *Nature* 385:810-813. In brief, a cell, *e*.*g*., a somatic cell, from the transgenic animal can be isolated and induced to exit the growth cycle and enter G₀ phase. The quiescent cell can then be fused, *e*.*g*., through the use of electrical pulses, to an enucleated oocyte from an animal of the same species from which the quiescent cell is isolated. The reconstructed oocyte is then cultured such that it develops to morula or blastocyte and then transferred to pseudopregnant female foster animal. The offspring borne of this female foster animal will be a clone of the animal from which the cell, *e.g.*, the somatic cell, is isolated.

### Pharmaceutical Compositions

The NOVX nucleic acid molecules, NOVX proteins, and anti-NOVX antibodies (also referred to herein as "active compounds") of the invention, and derivatives, fragments, analogs and homologs thereof, can be incorporated into pharmaceutical compositions suitable for administration. Such compositions typically comprise the nucleic acid molecule, protein, or antibody and a pharmaceutically acceptable carrier. As used herein, "pharmaceutically acceptable carrier" is intended to include any and all solvents, dispersion media, coatings, antibacterial and antifungal agents, isotonic and absorption delaying agents, and the like, compatible with pharmaceutical administration. Suitable carriers are described in the most recent edition of Remington's Pharmaceutical Sciences, a standard reference text in the field, which is incorporated herein by reference. Preferred examples of such carriers or diluents include, but are not limited to, water, saline, finger's solutions, dextrose solution, and 5% human serum albumin. Liposomes and non-aqueous vehicles such as fixed oils may also be used. The use of such media and agents for pharmaceutically active substances is well known in the art. Except insofar as any conventional media or agent is incompatible with the active compound, use thereof in the compositions is contemplated. Supplementary active compounds can also be incorporated into the compositions.

A pharmaceutical composition of the invention is formulated to be compatible with its intended route of administration. Examples of routes of administration include parenteral, *e.g.*, intravenous, intradermal, subcutaneous, oral (*e.g*., inhalation), transdermal (topical), transmucosal, and rectal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates, and agents for the adjustment of tonicity such as sodium chloride or dextrose. The pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL™ (BASF, Parsippany, N.J.) or phosphate buffered saline (PBS). In all cases, the composition must be sterile and should be fluid to the extent that easy syringeability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as manitol, sorbitol, sodium chloride in the composition. Prolonged absorption of the injectable compositions can be brought about by including in the composition an agent which delays absorption, for example, aluminum monostearate and gelatin.

Sterile injectable solutions can be prepared by incorporating the active compound (*e.g*., a NOVX protein or anti-NOVX antibody) in the required amount in an appropriate solvent with one or a combination of ingredients enumerated above, as required, followed by filtered sterilization. Generally, dispersions are prepared by incorporating the active compound into a sterile vehicle that contains a basic dispersion medium and the required other ingredients from those enumerated above. In the case of sterile powders for the preparation of sterile injectable solutions, methods of preparation are vacuum drying and freeze-drying that yields a powder of the active ingredient plus any additional desired ingredient from a previously sterile-filtered solution thereof.

Oral compositions generally include an inert diluent or an edible carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

For administration by inhalation, the compounds are delivered in the form of an aerosol spray from pressured container or dispenser which contains a suitable propellant, *e*.*g*., a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

The compounds can also be prepared in the form of suppositories (*e.g*., with conventional suppository bases such as cocoa butter and other glycerides) or retention enemas for rectal delivery.

In one embodiment, the active compounds are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. Pat. No. 4,522,811.

It is especially advantageous to formulate oral or parenteral compositions in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier. The specification for the dosage unit forms of the invention are dictated by and directly dependent on the unique characteristics of the active compound and the particular therapeutic effect to be achieved.

The nucleic acid molecules of the invention can be inserted into vectors and used as gene therapy vectors. Gene therapy vectors can be delivered to a subject by any of a number of routes, *e.g*., as described in U.S. Patent Nos. 5,703,055. Delivery can thus also include, *e.g.*, intravenous injection, local administration (see U.S. Pat. No. 5,328,470) or stereotactic injection (see *e*.*g*., Chen *et al*. (1994) *PNAS* 91:3054-3057). The pharmaceutical preparation of the gene therapy vector can include the gene therapy vector in an acceptable diluent, or can comprise a slow release matrix in which the gene delivery vehicle is imbedded. Alternatively, where the complete gene delivery vector can be produced intact from recombinant cells, *e.g*., retroviral vectors, the pharmaceutical preparation can include one or more cells that produce the gene delivery system.

The pharmaceutical compositions can be included in a container, pack, or dispenser together with instructions for administration.

### Additional Uses and Methods of the Invention

The nucleic acid molecules, proteins, protein homologues, and antibodies described herein can be used in one or more of the following methods: (a) screening assays; (b) detection assays (*e*.*g*., chromosomal mapping, cell and tissue typing, forensic biology), (c) predictive medicine (*e*.*g*., diagnostic assays, prognostic assays, monitoring clinical trials, and phannacogenomics); and (d) methods of treatment (*e*.*g*., therapeutic and prophylactic).

The isolated nucleic acid molecules of the invention can be used to express NOVX protein (*e*.*g*., via a recombinant expression vector in a host cell in gene therapy applications), to detect NOVX mRNA (*e*.*g*., in a biological sample) or a genetic lesion in a NOVX gene, and to modulate NOVX activity, as described further below. In addition, the NOVX proteins can be used to screen drugs or compounds that modulate the NOVX activity or expression as well as to treat disorders characterized by insufficient or excessive production of NOVX protein, for example proliferative or differentiative disorders, or production of NOVX protein forms that have decreased or aberrant activity compared to NOVX wild type protein. In addition, the anti-NOVX antibodies of the invention can be used to detect and isolate NOVX proteins and modulate NOVX activity.

This invention further pertains to novel agents identified by the above described screening assays and uses thereof for treatments as described herein.

### Screening Assays

The invention provides a method (also referred to herein as a "screening assay") for identifying modulators, *i*.*e*., candidate or test compounds or agents *(e.g.,* peptides, peptidomimetics, small molecules or other drugs) that bind to NOVX proteins or have a stimulatory or inhibitory effect on, for example, NOVX expression or NOVX activity.

In one embodiment, the invention provides assays for screening candidate or test compounds which bind to or modulate the activity of a NOVX protein or polypeptide or biologically active portion thereof. The test compounds of the present invention can be obtained using any of the numerous approaches in combinatorial library methods known in the art, including: biological libraries; spatially addressable parallel solid phase or solution phase libraries; synthetic library methods requiring deconvolution; the "one-bead one-compound" library method; and synthetic library methods using affinity chromatography selection. The biological library approach is limited to peptide libraries, while the other four approaches are applicable to peptide, non-peptide oligomer or small molecule libraries of compounds (Lam (1997) *Anticancer Drug Des* 12:145).

Examples of methods for the synthesis of molecular libraries can be found in the art, for example in: DeWitt *et al*. (1993) *Proc Natl Acad Sci U*.*S*.*A*. 90:6909; Erb *et al*. (1994) *Proc Natl Acad Sci U*.*S*.*A*. 91:11422; Zuckermann *et al*. (1994) *J Med Chem* 37:2678; Cho *et al*. (1993) *Science* 261:1303; Carrell *et al*. (1994) *Angew Chem Int Ed Engl* 33:2059; Carell *et al*. (1994) *Angew Chem Int Ed Engl* 33:2061; and Gallop *et al*. (1994) *J Med Chem* 37:1233.

Libraries of compounds may be presented in solution (*e*.*g*., Houghten (1992) *Biotechniques* 13:412-421 ), or on beads (Lam (1991) *Nature* 354:82-84), on chips (Fodor (1993) *Nature* 364:555-556), bacteria (Ladner U.S. Pat. No. 5,223,409), spores (Ladner USP '409), plasmids (Cull *et al*. (1992) *Proc Natl Acad Sci USA* 89:1865-1869) or on phage (Scott and Smith (1990) *Science* 249:386-390; Devlin (1990) *Science* 249:404-406; Cwirla *et al*. (1990) *Proc Natl Acad Sci U*.*S*.*A*. 87:6378-6382; Felici (1991) *J Mol Biol* 222:301-310; Ladner above.).

In one embodiment, an assay is a cell-based assay in which a cell which expresses a membrane-bound form of NOVX protein, or a biologically active portion thereof, on the cell surface is contacted with a test compound and the ability of the test compound to bind to a NOVX protein determined. The cell, for example, can of mammalian origin or a yeast cell. Determining the ability of the test compound to bind to the NOVX protein can be accomplished, for example, by coupling the test compound with a radioisotope or enzymatic label such that binding of the test compound to the NOVX protein or biologically active portion thereof can be determined by detecting the labeled compound in a complex. For example, test compounds can be labeled with ¹²⁵I, ³⁵S, ¹⁴C, or ³H, either directly or indirectly, and the radioisotope detected by direct counting of radioemission or by scintillation counting. Alternatively, test compounds can be enzymatically labeled with, for example, horseradish peroxidase, alkaline phosphatase, or luciferase, and the enzymatic label detected by determination of conversion of an appropriate substrate to product. In one embodiment, the assay comprises contacting a cell which expresses a membrane-bound form of NOVX protein, or a biologically active portion thereof, on the cell surface with a known compound which binds NOVX to form an assay mixture, contacting the assay mixture with a test compound, and determining the ability of the test compound to interact with a NOVX protein, wherein determining the ability of the test compound to interact with a NOVX protein comprises determining the ability of the test compound to preferentially bind to NOVX or a biologically active portion thereof as compared to the known compound.

In another embodiment, an assay is a cell-based assay comprising contacting a cell expressing a membrane-bound form of NOVX protein, or a biologically active portion thereof, on the cell surface with a test compound and determining the ability of the test compound to modulate (*e*.*g*., stimulate or inhibit) the activity of the NOVX protein or biologically active portion thereof. Determining the ability of the test compound to modulate the activity of NOVX or a biologically active portion thereof can be accomplished, for example, by determining the ability of the NOVX protein to bind to or interact with a NOVX target molecule. As used herein, a "target molecule" is a molecule with which a NOVX protein binds or interacts in nature, for example, a molecule on the surface of a cell which expresses a NOVX interacting protein, a molecule on the surface of a second cell, a molecule in the extracellular milieu, a molecule associated with the internal surface of a cell membrane or a cytoplasmic molecule. A NOVX target molecule can be a non-NOVX molecule or a NOVX protein or polypeptide of the present invention. In one embodiment, a NOVX target molecule is a component of a signal transduction pathway that facilitates transduction of an extracellular signal (*e*.*g*., a signal generated by binding of a compound to a membrane-bound NOVX molecule) through the cell membrane and into the cell. The target, for example, can be a second intercellular protein that has catalytic activity or a protein that facilitates the association of downstream signaling molecules with NOVX.

Determining the ability of the NOVX protein to bind to or interact with a NOVX target molecule can be accomplished by one of the methods described above for determining direct binding. In one embodiment, determining the ability of the NOVX protein to bind to or interact with a NOVX target molecule can be accomplished by determining the activity of the target molecule. For example, the activity of the target molecule can be determined by detecting induction of a cellular second messenger of the target (*i*.*e*. intracellular Ca²⁺, diacylglycerol, IP₃, etc.), detecting catalytic/enzymatic activity of the target an appropriate substrate, detecting the induction of a reporter gene (comprising a NOVX-responsive regulatory element operatively linked to a nucleic acid encoding a detectable marker, *e.g.*, luciferase), or detecting a cellular response, for example, cell survival, cellular differentiation, or cell proliferation.

In yet another embodiment, an assay of the present invention is a cell-free assay comprising contacting a NOVX protein or biologically active portion thereof with a test compound and determining the ability of the test compound to bind to the NOVX protein or biologically active portion thereof. Binding of the test compound to the NOVX protein can be determined either directly or indirectly as described above. In one embodiment, the assay comprises contacting the NOVX protein or biologically active portion thereof with a known compound which binds NOVX to form an assay mixture, contacting the assay mixture with a test compound, and determining the ability of the test compound to interact with a NOVX protein, wherein determining the ability of the test compound to interact with a NOVX protein comprises determining the ability of the test compound to preferentially bind to NOVX or biologically active portion thereof as compared to the known compound.

In another embodiment, an assay is a cell-free assay comprising contacting NOVX protein or biologically active portion thereof with a test compound and determining the ability of the test compound to modulate (*e.g.*, stimulate or inhibit) the activity of the NOVX protein or biologically active portion thereof. Determining the ability of the test compound to modulate the activity of NOVX can be accomplished, for example, by determining the ability of the NOVX protein to bind to a NOVX target molecule by one of the methods described above for determining direct binding. In an alternative embodiment, determining the ability of the test compound to modulate the activity of NOVX can be accomplished by determining the ability of the NOVX protein further modulate a NOVX target molecule. For example, the catalytic/enzymatic activity of the target molecule on an appropriate substrate can be determined as previously described.

In yet another embodiment, the cell-free assay comprises contacting the NOVX protein or biologically active portion thereof with a known compound which binds NOVX to form an assay mixture, contacting the assay mixture with a test compound, and determining the ability of the test compound to interact with a NOVX protein, wherein determining the ability of the test compound to interact with a NOVX protein comprises determining the ability of the NOVX protein to preferentially bind to or modulate the activity of a NOVX target molecule.

The cell-free assays of the present invention are amenable to use of both the soluble form or the membrane-bound form of NOVX. In the case of cell-free assays comprising the membrane-bound form of NOVX, it may be desirable to utilize a solubilizing agent such that the membrane-bound form of NOVX is maintained in solution. Examples of such solubilizing agents include non-ionic detergents such as n-octylglucoside, n-dodecylglucoside, n-dodecylmaltoside, octanoyl-N-methylglucamide, decanoyl-N-methylglucamide, Triton® X-100, Triton® X-114, Thesit®, Isotridecypoly(ethylene glycol ether)ₙ, N-dodecyl-N,N-dimethyl-3-ammonio-1-propane sulfonate, 3-(3-cholamidopropyl)dimethylamminiol-1-propane sulfonate (CHAPS), or 3-(3-cholamidopropyl)dimethylamminiol-2-hydroxy-1-propane sulfonate (CHAPSO).

In more than one embodiment of the above assay methods of the present invention, it may be desirable to immobilize either NOVX or its target molecule to facilitate separation of complexed from uncomplexed forms of one or both of the proteins, as well as to accommodate automation of the assay. Binding of a test compound to NOVX, or interaction of NOVX with a target molecule in the presence and absence of a candidate compound, can be accomplished in any vessel suitable for containing the reactants. Examples of such vessels include microtiter plates, test tubes, and micro-centrifuge tubes. In one embodiment, a fusion protein can be provided that adds a domain that allows one or both of the proteins to be bound to a matrix. For example, GST-NOVX fusion proteins or GST-target fusion proteins can be adsorbed onto glutathione sepharose beads (Sigma Chemical, St. Louis, MO) or glutathione derivatized microtiter plates, that are then combined with the test compound or the test compound and either the non-adsorbed target protein or NOVX protein, and the mixture is incubated under conditions conducive to complex formation (*e*.*g*., at physiological conditions for salt and pH). Following incubation, the beads or microtiter plate wells are washed to remove any unbound components, the matrix immobilized in the case of beads, complex determined either directly or indirectly, for example, as described above. Alternatively, the complexes can be dissociated from the matrix, and the level of NOVX binding or activity determined using standard techniques.

Other techniques for immobilizing proteins on matrices can also be used in the screening assays of the invention. For example, either NOVX or its target molecule can be immobilized utilizing conjugation of biotin and streptavidin. Biotinylated NOVX or target molecules can be prepared from biotin-NHS (N-hydroxy-succinimide) using techniques well known in the art (*e*.*g*., biotinylation kit, Pierce Chemicals, Rockford, Ill.), and immobilized in the wells of streptavidin-coated 96 well plates (Pierce Chemical). Alternatively, antibodies reactive with NOVX or target molecules, but which do not interfere with binding of the NOVX protein to its target molecule, can be derivatized to the wells of the plate, and unbound target or NOVX trapped in the wells by antibody conjugation. Methods for detecting such complexes, in addition to those described above for the GST-immobilized complexes, include immunodetection of complexes using antibodies reactive with the NOVX or target molecule, as well as enzyme-linked assays that rely on detecting an enzymatic activity associated with the NOVX or target molecule.

In another embodiment, modulators of NOVX expression are identified in a method wherein a cell is contacted with a candidate compound and the expression of NOVX mRNA or protein in the cell is determined. The level of expression of NOVX mRNA or protein in the presence of the candidate compound is compared to the level of expression of NOVX mRNA or protein in the absence of the candidate compound. The candidate compound can then be identified as a modulator of NOVX expression based on this comparison. For example, when expression of NOVX mRNA or protein is greater (statistically significantly greater) in the presence of the candidate compound than in its absence, the candidate compound is identified as a stimulator of NOVX mRNA or protein expression. Alternatively, when expression of NOVX mRNA or protein is less (statistically significantly less) in the presence of the candidate compound than in its absence, the candidate compound is identified as an inhibitor of NOVX mRNA or protein expression. The level of NOVX mRNA or protein expression in the cells can be determined by methods described herein for detecting NOVX mRNA or protein.

In yet another aspect of the invention, the NOVX proteins can be used as "bait proteins" in a two-hybrid assay or three hybrid assay (see, *e.g*., U.S. Pat. No. 5,283,317; Zervos *et al*. (1993) Cell 72:223-232; Madura *et al*. (1993) J Biol Chem 268:12046-12054; Bartel *et al*. (1993) Biotechniques 14:920-924; Iwabuchi *et al*. (1993) Oncogene 8:1693-1696; and Brent WO94/10300), to identify other proteins that bind to or interact with NOVX ("NOVX-binding proteins" or "NOVX-bp") and modulate NOVX activity. Such NOVX-binding proteins are also likely to be involved in the propagation of signals by the NOVX proteins as, for example, upstream or downstream elements of the NOVX pathway.

The two-hybrid system is based on the modular nature of most transcription factors, which consist of separable DNA-binding and activation domains. Briefly, the assay utilizes two different DNA constructs. In one construct, the gene that codes for NOVX is fused to a gene encoding the DNA binding domain of a known transcription factor (*e*.*g*., GAL-4). In the other construct, a DNA sequence, from a library of DNA sequences, that encodes an unidentified protein ("prey" or "sample") is fused to a gene that codes for the activation domain of the known transcription factor. If the "bait" and the "prey" proteins are able to interact, *in vivo,* forming a NOVX-dependent complex, the DNA-binding and activation domains of the transcription factor are brought into close proximity. This proximity allows transcription of a reporter gene (*e.g*., LacZ) that is operably linked to a transcriptional regulatory site responsive to the transcription factor. Expression of the reporter gene can be detected and cell colonies containing the functional transcription factor can be isolated and used to obtain the cloned gene that encodes the protein which interacts with NOVX.

This invention further pertains to novel agents identified by the above-described screening assays and uses thereof for treatments as described herein.

### Detection Assays

Portions or fragments of the cDNA sequences identified herein (and the corresponding complete gene sequences) can be used in numerous ways as polynucleotide reagents. For example, these sequences can be used to: (i) map their respective genes on a chromosome; and, thus, locate gene regions associated with genetic disease; (ii) identify an individual from a minute biological sample (tissue typing); and (iii) aid in forensic identification of a biological sample.

The NOVX sequences of the present invention can also be used to identify individuals from minute biological samples. In this technique, an individual's genomic DNA is digested with one or more restriction enzymes, and probed on a Southern blot to yield unique bands for identification. The sequences of the present invention are useful as additional DNA markers for RFLP ("restriction fragment length polymorphisms," described in U.S. Pat. No. 5,272,057).

Furthermore, the sequences of the present invention can be used to provide an alternative technique that determines the actual base-by-base DNA sequence of selected portions of an individual's genome. Thus, the NOVX sequences described herein can be used to prepare two PCR primers from the 5' and 3' ends of the sequences. These primers can then be used to amplify an individual's DNA and subsequently sequence it.

Panels of corresponding DNA sequences from individuals, prepared in this manner, can provide unique individual identifications, as each individual will have a unique set of such DNA sequences due to allelic differences. The sequences of the present invention can be used to obtain such identification sequences from individuals and from tissue. The NOVX sequences of the invention uniquely represent portions of the human genome. Allelic variation occurs to some degree in the coding regions of these sequences, and to a greater degree in the noncoding regions. It is estimated that allelic variation between individual humans occurs with a frequency of about once per each 500 bases. Much of the allelic variation is due to single nucleotide polymorphisms (SNPs), which include restriction fragment length polymorphisms (RFLPs).

Each of the sequences described herein can, to some degree, be used as a standard against which DNA from an individual can be compared for identification purposes. Because greater numbers of polymorphisms occur in the noncoding regions, fewer sequences are necessary to differentiate individuals. The noncoding sequences of SEQ ID NO:1, 3, 5, 7, 9, 11, 13, 15, 17, 19, 21, 23, 25, 27, 29, 31, 33, 35, 37, 39, 41, 43, and 45, as described above, can comfortably provide positive individual identification with a panel of perhaps 10 to 1,000 primers that each yield a noncoding amplified sequence of 100 bases. If predicted coding sequences are used, a more appropriate number of primers for positive individual identification would be 500-2,000.

### Predictive Medicine

The present invention also pertains to the field of predictive medicine in which diagnostic assays, prognostic assays, pharmacogenomics, and monitoring clinical trials are used for prognostic (predictive) purposes to thereby treat an individual prophylactically. Accordingly, one aspect of the present invention relates to diagnostic assays for determining NOVX protein and/or nucleic acid expression as well as NOVX activity, in the context of a biological sample *(e.g.,* blood, serum, cells, tissue) to thereby determine whether an individual is afflicted with a disease or disorder, or is at risk of developing a disorder, associated with aberrant NOVX expression or activity. The invention also provides for prognostic (or predictive) assays for determining whether an individual is at risk of developing a disorder associated with NOVX protein, nucleic acid expression or activity. For example, mutations in a NOVX gene can be assayed in a biological sample. Such assays can be used for prognostic or predictive purpose to thereby prophylactically treat an individual prior to the onset of a disorder characterized by or associated with NOVX protein, nucleic acid expression or activity.

Another aspect of the invention provides methods for determining NOVX protein, nucleic acid expression or NOVX activity in an individual to thereby select appropriate therapeutic or prophylactic agents for that individual (referred to herein as "pharmacogenomics"). Pharmacogenomics allows for the selection of agents (*e.g.*, drugs) for therapeutic or prophylactic treatment of an individual based on the genotype of the individual (*e.g*., the genotype of the individual examined to determine the ability of the individual to respond to a particular agent.)

Yet another aspect of the invention pertains to monitoring the influence of agents (*e*.*g*., drugs, compounds) on the expression or activity of NOVX in clinical trials.

### Use of Partial NOVX Sequences in Forensic Biology

DNA-based identification techniques can also be used in forensic biology. Forensic biology is a scientific field employing genetic typing of biological evidence found at a crime scene as a means for positively identifying, for example, a perpetrator of a crime. To make such an identification, PCR technology can be used to amplify DNA sequences taken from very small biological samples such as tissues, *e.g*., hair or skin, or body fluids, *e.g*., blood, saliva, or semen found at a crime scene. The amplified sequence can then be compared to a standard, thereby allowing identification of the origin of the biological sample.

The sequences of the present invention can be used to provide polynucleotide reagents, *e*.*g*., PCR primers, targeted to specific loci in the human genome, that can enhance the reliability of DNA-based forensic identifications by, for example, providing another "identification marker" (*i*.*e*. another DNA sequence that is unique to a particular individual). As mentioned above, actual base sequence information can be used for identification as an accurate alternative to patterns formed by restriction enzyme generated fragments. Sequences targeted to noncoding regions of SEQ ID NOs: 1, 3, 5, 7, 9, 11, 13, 15, 17, 19, 21, 23, 25, 27, 29, 31, 33, 35, 37, 39, 41, 43, and 45 are particularly appropriate for this use as greater numbers of polymorphisms occur in the noncoding regions, making it easier to differentiate individuals using this technique. Examples of polynucleotide reagents include the NOVX sequences or portions thereof, *e*.*g*., fragments derived from the noncoding regions of one or more of SEQ ID NO:1, 3, 5, 7, 9, 11, 13, 15, 17, 19, 21, 23, 25, 27, 29, 31, 33, 35, 37, 39, 41, 43, and 45, having a length of at least 20 bases, preferably at least 30 bases.

The NOVX sequences described herein can further be used to provide polynucleotide reagents, *e.g*., labeled or label-able probes that can be used, for example, in an *in situ* hybridization technique, to identify a specific tissue, *e.g.*, brain tissue, etc. This can be very useful in cases where a forensic pathologist is presented with a tissue of unknown origin. Panels of such NOVX probes can be used to identify tissue by species and/or by organ type.

In a similar fashion, these reagents, *e.g.,* NOVX primers or probes can be used to screen tissue culture for contamination (*i*.*e*. screen for the presence of a mixture of different types of cells in a culture).

### Predictive Medicine

The present invention also pertains to the field of predictive medicine in which diagnostic assays, prognostic assays, pharmacogenomics, and monitoring clinical trials are used for prognostic (predictive) purposes to thereby treat an individual prophylactically. Accordingly, one aspect of the present invention relates to diagnostic assays for determining NOVX protein and/or nucleic acid expression as well as NOVX activity, in the context of a biological sample (*e.g.,* blood, serum, cells, tissue) to thereby determine whether an individual is afflicted with a disease or disorder, or is at risk of developing a disorder, associated with aberrant NOVX expression or activity. The invention also provides for prognostic (or predictive) assays for determining whether an individual is at risk of developing a disorder associated with NOVX protein, nucleic acid expression or activity. For example, mutations in a NOVX gene can be assayed in a biological sample. Such assays can be used for prognostic or predictive purpose to thereby prophylactically treat an individual prior to the onset of a disorder characterized by or associated with NOVX protein, nucleic acid expression or activity.

Another aspect of the invention provides methods for determining NOVX protein, nucleic acid expression or NOVX activity in an individual to thereby select appropriate therapeutic or prophylactic agents for that individual (referred to herein as "pharmacogenomics"). Pharmacogenomics allows for the selection of agents (*e.g.*, drugs) for therapeutic or prophylactic treatment of an individual based on the genotype of the individual (*e.g.,* the genotype of the individual examined to determine the ability of the individual to respond to a particular agent.)

Yet another aspect of the invention pertains to monitoring the influence of agents (*e.g*., drugs, compounds) on the expression or activity of NOVX in clinical trials.

These and other agents are described in further detail in the following sections.

### Diagnostic Assays

Other conditions in which proliferation of cells plays a role include tumors, restenosis, psoriasis, Dupuytren's contracture, diabetic complications, Kaposi's sarcoma and rheumatoid arthritis.

A NOVX polypeptide may be used to identify an interacting polypeptide a sample or tissue. The method comprises contacting the sample or tissue with NOVX, allowing formation of a complex between the NOVX polypeptide and the interacting polypeptide, and detecting the complex, if present.

The proteins of the invention may be used to stimulate production of antibodies specifically binding the proteins. Such antibodies may be used in immunodiagnostic procedures to detect the occurrence of the protein in a sample. The proteins of the invention may be used to stimulate cell growth and cell proliferation in conditions in which such growth would be favorable. An example would be to counteract toxic side effects of chemotherapeutic agents on, for example, hematopoiesis and platelet formation, linings of the gastrointestinal tract, and hair follicles. They may also be used to stimulate new cell growth in neurological disorders including, for example, Alzheimer's disease. Alternatively, antagonistic treatments may be administered in which an antibody specifically binding the NOVX -like proteins of the invention would abrogate the specific growth-inducing effects of the proteins. Such antibodies may be useful, for example, in the treatment of proliferative disorders including various tumors and benign hyperplasias.

Polynucleotides or oligonucleotides corresponding to any one portion of the NOVX nucleic acids of SEQ ID NO:1, 3, 5, 7, 9, 11, 13, 15, 17, 19, 21, 23, 25, 27, 29, 31, 33, 35, 37, 39, 41, 43, and 45 may be used to detect DNA containing a corresponding NOV gene, or detect the expression of a corresponding NOVX gene, or NOVX-like gene. For example, a NOVX nucleic acid expressed in a particular cell or tissue, as noted in Table 1, can be used to identify the presence of that particular cell type.

An exemplary method for detecting the presence or absence of NOVX in a biological sample involves obtaining a biological sample from a test subject and contacting the biological sample with a compound or an agent capable of detecting NOVX protein or nucleic acid (*e*.*g*., mRNA, genomic DNA) that encodes NOVX protein such that the presence of NOVX is detected in the biological sample. An agent for detecting NOVX mRNA or genomic DNA is a labeled nucleic acid probe capable of hybridizing to NOVX mRNA or genomic DNA. The nucleic acid probe can be, for example, a full-length NOVX nucleic acid, such as the nucleic acid of SEQ ID NO:1, 3, 5, 7, 9, 11, 13, 15, 17, 19, 21, 23, 25, 27, 29, 31, 33, 35, 37, 39, 41, 43, and 45, or a portion thereof, such as an oligonucleotide of at least 15, 30, 50, 100, 250 or 500 nucleotides in length and sufficient to specifically hybridize under stringent conditions to NOVX mRNA or genomic DNA, as described above. Other suitable probes for use in the diagnostic assays of the invention are described herein.

An agent for detecting NOVX protein is an antibody capable of binding to NOVX protein, preferably an antibody with a detectable label. Antibodies can be polyclonal, or more preferably, monoclonal. An intact antibody, or a fragment thereof (*e*.*g*., Fab or F(ab')₂) can be used. The term "labeled", with regard to the probe or antibody, is intended to encompass direct labeling of the probe or antibody by coupling (*i*.*e*., physically linking) a detectable substance to the probe or antibody, as well as indirect labeling of the probe or antibody by reactivity with another reagent that is directly labeled. Examples of indirect labeling include detection of a primary antibody using a fluorescently labeled secondary antibody and end-labeling of a DNA probe with biotin such that it can be detected with fluorescently labeled streptavidin. The term "biological sample" is intended to include tissues, cells and biological fluids isolated from a subject, as well as tissues, cells and fluids present within a subject. That is, the detection method of the invention can be used to detect NOVX mRNA, protein, or genomic DNA in a biological sample *in vitro* as well as *in vivo.* For example, *in vitro* techniques for detection of NOVX mRNA include Northern hybridizations and *in situ* hybridizations. *In vitro* techniques for detection of NOVX protein include enzyme linked immunosorbent assays (ELISAs), Western blots, immunoprecipitations and immunofluorescence. *In vitro* techniques for detection of NOVX genomic DNA include Southern hybridizations. Furthermore, *in vivo* techniques for detection of NOVX protein include introducing into a subject a labeled anti-NOVX antibody. For example, the antibody can be labeled with a radioactive marker whose presence and location in a subject can be detected by standard imaging techniques.

In one embodiment, the biological sample contains protein molecules from the test subject. Alternatively, the biological sample can contain mRNA molecules from the test subject or genomic DNA molecules from the test subject. A preferred biological sample is a peripheral blood leukocyte sample isolated by conventional means from a subject.

In another embodiment, the methods further involve obtaining a control biological sample from a control subject, contacting the control sample with a compound or agent capable of detecting NOVX protein, mRNA, or genomic DNA, such that the presence of NOVX protein, mRNA or genomic DNA is detected in the biological sample, and comparing the presence of NOVX protein, mRNA or genomic DNA in the control sample with the presence of NOVX protein, mRNA or genomic DNA in the test sample.

The invention also encompasses kits for detecting the presence of NOVX in a biological sample. For example, the kit can comprise: a labeled compound or agent capable of detecting NOVX protein or mRNA in a biological sample; means for determining the amount of NOVX in the sample; and means for comparing the amount of NOVX in the sample with a standard. The compound or agent can be packaged in a suitable container. The kit can further comprise instructions for using the kit to detect NOVX protein or nucleic acid.

### Prognostic Assays

The diagnostic methods described herein can furthermore be utilized to identify subjects having or at risk of developing a disease or disorder associated with aberrant NOVX expression or activity. For example, the assays described herein, such as the preceding diagnostic assays or the following assays, can be utilized to identify a subject having or at risk of developing a disorder associated with NOVX protein, nucleic acid expression or activity in, *e.g.*, proliferative or differentiative disorders such as hyperplasias, tumors, restenosis, psoriasis, Dupuytren's contracture, diabetic complications, or rheumatoid arthritis, etc.; and glia-associated disorders such as cerebral lesions, diabetic neuropathies, cerebral edema, senile dementia, Alzheimer's disease, etc. Alternatively, the prognostic assays can be utilized to identify a subject having or at risk for developing a disease or disorder. Thus, the present invention provides a method for identifying a disease or disorder associated with aberrant NOVX expression or activity in which a test sample is obtained from a subject and NOVX protein or nucleic acid (*e*.*g*., mRNA, genomic DNA) is detected, wherein the presence of NOVX protein or nucleic acid is diagnostic for a subject having or at risk of developing a disease or disorder associated with aberrant NOVX expression or activity. As used herein, a "test sample" refers to a biological sample obtained from a subject of interest. For example, a test sample can be a biological fluid (*e.g*., serum), cell sample, or tissue.

Furthermore, the prognostic assays described herein can be used to determine whether a subject can be administered an agent (*e.g.*, an agonist, antagonist, peptidomimetic, protein, peptide, nucleic acid, small molecule, or other drug candidate) to treat a disease or disorder associated with aberrant NOVX expression or activity. For example, such methods can be used to determine whether a subject can be effectively treated with an agent for a disorder, such as a proliferative disorder, differentiative disorder, glia-associated disorders, etc. Thus, the present invention provides methods for determining whether a subject can be effectively treated with an agent for a disorder associated with aberrant NOVX expression or activity in which a test sample is obtained and NOVX protein or nucleic acid is detected (*e*.*g*., wherein the presence of NOVX protein or nucleic acid is diagnostic for a subject that can be administered the agent to treat a disorder associated with aberrant NOVX expression or activity.)

The methods of the invention can also be used to detect genetic lesions in a NOVX gene, thereby determining if a subject with the lesioned gene is at risk for, or suffers from, a proliferative disorder, differentiative disorder, glia-associated disorder, etc. In various embodiments, the methods include detecting, in a sample of cells from the subject, the presence or absence of a genetic lesion characterized by at least one of an alteration affecting the integrity of a gene encoding a NOVX-protein, or the mis-expression of the NOVX gene. For example, such genetic lesions can be detected by ascertaining the existence of at least one of (1) a deletion of one or more nucleotides from a NOVX gene; (2) an addition of one or more nucleotides to a NOVX gene; (3) a substitution of one or more nucleotides of a NOVX gene, (4) a chromosomal rearrangement of a NOVX gene; (5) an alteration in the level of a messenger RNA transcript of a NOVX gene, (6) aberrant modification of a NOVX gene, such as of the methylation pattern of the genomic DNA, (7) the presence of a non-wild type splicing pattern of a messenger RNA transcript of a NOVX gene, (8) a non-wild type level of a NOVX-protein, (9) allelic loss of a NOVX gene, and (10) inappropriate post-translational modification of a NOVX-protein. As described herein, there are a large number of assay techniques known in the art which can be used for detecting lesions in a NOVX gene. A preferred biological sample is a peripheral blood leukocyte sample isolated by conventional means from a subject. However, any biological sample containing nucleated cells may be used, including, for example, buccal mucosal cells.

In certain embodiments, detection of the lesion involves the use of a probe/primer in a polymerase chain reaction (PCR) (see, *e.g.*, U.S. Pat. Nos. 4,683,195 and 4,683,202), such as anchor PCR or RACE PCR, or, alternatively, in a ligation chain reaction (LCR) (see, *e*.*g*., Landegran *et al*. (1988) *Science* 241:1077-1080; and Nakazawa *et al*. (1994) *PNAS* 91:360-364), the latter of which can be particularly useful for detecting point mutations in the NOVX-gene (see Abravaya *et al*. (1995) *Nucl Acids Res* 23:675-682). This method can include the steps of collecting a sample of cells from a patient, isolating nucleic acid (*e*.*g*., genomic, mRNA or both) from the cells of the sample, contacting the nucleic acid sample with one or more primers that specifically hybridize to a NOVX gene under conditions such that hybridization and amplification of the NOVX gene (if present) occurs, and detecting the presence or absence of an amplification product, or detecting the size of the amplification product and comparing the length to a control sample. It is anticipated that PCR and/or LCR may be desirable to use as a preliminary amplification step in conjunction with any of the techniques used for detecting mutations described herein.

Alternative amplification methods include: self sustained sequence replication (Guatelli *et al*., 1990, *Proc Natl Acad Sci USA* 87:1874-1878), transcriptional amplification system (Kwoh, *et al*., 1989, *Proc Natl Acad Sci USA* 86:1173-1177), Q-Beta Replicase (Lizardi *et al*, 1988, *BioTechnology* 6:1197), or any other nucleic acid amplification method, followed by the detection of the amplified molecules using techniques well known to those of skill in the art. These detection schemes are especially useful for the detection of nucleic acid molecules if such molecules are present in very low numbers.

In an alternative embodiment, mutations in a NOVX gene from a sample cell can be identified by alterations in restriction enzyme cleavage patterns. For example, sample and control DNA is isolated, amplified (optionally), digested with one or more restriction endonucleases, and fragment length sizes are determined by gel electrophoresis and compared. Differences in fragment length sizes between sample and control DNA indicates mutations in the sample DNA. Moreover, the use of sequence specific ribozymes (see, for example, U.S. Pat. No. 5,493,531) can be used to score for the presence of specific mutations by development or loss of a ribozyme cleavage site.

In other embodiments, genetic mutations in NOVX can be identified by hybridizing a sample and control nucleic acids, *e*.*g*., DNA or RNA, to high density arrays containing hundreds or thousands of oligonucleotides probes (Cronin *et al*. (1996) *Human Mutation 7:* 244-255; Kozal *et al*. (1996) *Nature Medicine* 2: 753-759). For example, genetic mutations in NOVX can be identified in two dimensional arrays containing light-generated DNA probes as described in Cronin *et al*. above. Briefly, a first hybridization array of probes can be used to scan through long stretches of DNA in a sample and control to identify base changes between the sequences by making linear arrays of sequential overlapping probes. This step allows the identification of point mutations. This step is followed by a second hybridization array that allows the characterization of specific mutations by using smaller, specialized probe arrays complementary to all variants or mutations detected. Each mutation array is composed of parallel probe sets, one complementary to the wild-type gene and the other complementary to the mutant gene.

In yet another embodiment, any of a variety of sequencing reactions known in the art can be used to directly sequence the NOVX gene and detect mutations by comparing the sequence of the sample NOVX with the corresponding wild-type (control) sequence. Examples of sequencing reactions include those based on techniques developed by Maxim and Gilbert (1977) *PNAS* 74:560 or Sanger (1977) *PNAS* 74:5463. It is also contemplated that any of a variety of automated sequencing procedures can be utilized when performing the diagnostic assays (Naeve *et al*., (1995) *Biotechniques* 19:448), including sequencing by mass spectrometry (see, *e.g.,* PCT International Publ. No. WO 94/16101; Cohen *et al*. (1996) *Adv Chromatogr* 36:127-162; and Griffin *et al*. (1993) *Appl Biochem Biotechnol* 38:147-159).

Other methods for detecting mutations in the NOVX gene include methods in which protection from cleavage agents is used to detect mismatched bases in RNA/RNA or RNA/DNA heteroduplexes (Myers *et al*. (1985) *Science* 230:1242). In general, the art technique of "mismatch cleavage" starts by providing heteroduplexes of formed by hybridizing (labeled) RNA or DNA containing the wild-type NOVX sequence with potentially mutant RNA or DNA obtained from a tissue sample. The double-stranded duplexes are treated with an agent that cleaves single-stranded regions of the duplex such as which will exist due to basepair mismatches between the control and sample strands. For instance, RNA/DNA duplexes can be treated with RNase and DNA/DNA hybrids treated with S1 nuclease to enzymatically digesting the mismatched regions. In other embodiments, either DNA/DNA or RNA/DNA duplexes can be treated with hydroxylamine or osmium tetroxide and with piperidine in order to digest mismatched regions. After digestion of the mismatched regions, the resulting material is then separated by size on denaturing polyacrylamide gels to determine the site of mutation. See, for example, Cotton *et al* (1988) *Proc Natl Acad Sci USA* 85:4397; Saleeba *et al* (1992) *Methods Enzymol* 217:286-295. In an embodiment, the control DNA or RNA can be labeled for detection.

In still another embodiment, the mismatch cleavage reaction employs one or more proteins that recognize mismatched base pairs in double-stranded DNA (so called "DNA mismatch repair" enzymes) in defined systems for detecting and mapping point mutations in NOVX cDNAs obtained from samples of cells. For example, the mutY enzyme of *E*. *coli* cleaves A at G/A mismatches and the thymidine DNA glycosylase from HeLa cells cleaves T at G/T mismatches (Hsu *et al*. (1994) *Carcinogenesis* 15:1657-1662). According to an exemplary embodiment, a probe based on a NOVX sequence, *e*.*g*., a wild-type NOVX sequence, is hybridized to a cDNA or other DNA product from a test cell(s). The duplex is treated with a DNA mismatch repair enzyme, and the cleavage products, if any, can be detected from electrophoresis protocols or the like. See, for example, U.S. Pat. No. 5,459,039.

In other embodiments, alterations in electrophoretic mobility will be used to identify mutations in NOVX genes. For example, single strand conformation polymorphism (SSCP) may be used to detect differences in electrophoretic mobility between mutant and wild type nucleic acids (Orita *et al*. (1989) *Proc Natl Acad Sci USA*: 86:2766, see also Cotton (1993) *Mutat Res* 285:125-144; Hayashi (1992) *Genet Anal Tech Appl* 9:73-79). Single-stranded DNA fragments of sample and control NOVX nucleic acids will be denatured and allowed to renature. The secondary structure of single-stranded nucleic acids varies according to sequence, the resulting alteration in electrophoretic mobility enables the detection of even a single base change. The DNA fragments may be labeled or detected with labeled probes. The sensitivity of the assay may be enhanced by using RNA, rather than DNA, in which the secondary structure is more sensitive to a change in sequence. In one embodiment, the subject method utilizes heteroduplex analysis to separate double stranded heteroduplex molecules on the basis of changes in electrophoretic mobility. See, *e*.*g*., Keen *et al*. (1991) *Trends Genet* 7:5.

In yet another embodiment the movement of mutant or wild-type fragments in polyacrylamide gels containing a gradient of denaturant is assayed using denaturing gradient gel electrophoresis (DGGE). See, *e.g.*, Myers *et al* (1985) *Nature* 313:495. When DGGE is used as the method of analysis, DNA will be modified to insure that it does not completely denature, for example by adding a GC clamp of approximately 40 bp of high-melting-GC-rich DNA by PCR. In a further embodiment, a temperature gradient is used in place of a denaturing gradient to identify differences in the mobility of control and sample DNA. See, *e.g*., Rosenbaum and Reissner (1987) *Biophys Chem* 265:12753.

Examples of other techniques for detecting point mutations include, but are not limited to, selective oligonucleotide hybridization, selective amplification, or selective primer extension. For example, oligonucleotide primers may be prepared in which the known mutation is placed centrally and then hybridized to target DNA under conditions that permit hybridization only if a perfect match is found. See, *e*.*g*., Saiki *et al*. (1986) *Nature* 324:163); Saiki *et al*. (1989) *Proc Natl Acad*. *Sci USA* 86:6230. Such allele specific oligonucleotides are hybridized to PCR amplified target DNA or a number of different mutations when the oligonucleotides are attached to the hybridizing membrane and hybridized with labeled target DNA.

Alternatively, allele specific amplification technology that depends on selective PCR amplification may be used in conjunction with the instant invention. Oligonucleotides used as primers for specific amplification may carry the mutation of interest in the center of the molecule (so that amplification depends on differential hybridization) (Gibbs *et al*. (1989) *Nucleic Acids Res* 17:2437-2448) or at the extreme 3' end of one primer where, under appropriate conditions, mismatch can prevent, or reduce polymerase extension (Prossner (1993) *Tibtech* 11:238). In addition it may be desirable to introduce a novel restriction site in the region of the mutation to create cleavage-based detection. See, *e.g.,* Gasparini *et al* (1992) *Mol Cell Probes* 6:1. It is anticipated that in certain embodiments amplification may also be performed using Taq ligase for amplification. See, *e.g.*, Barany (1991) *Proc Natl Acad Sci USA* 88:189. In such cases, ligation will occur only if there is a perfect match at the 3' end of the 5' sequence, making it possible to detect the presence of a known mutation at a specific site by looking for the presence or absence of amplification.

The methods described herein may be performed, for example, by utilizing pre-packaged diagnostic kits comprising at least one probe nucleic acid or antibody reagent described herein, which may be conveniently used, *e.g.*, in clinical settings to diagnose patients exhibiting symptoms or family history of a disease or illness involving a NOVX gene.

Furthermore, any cell type or tissue, preferably peripheral blood leukocytes, in which NOVX is expressed may be utilized in the prognostic assays described herein. However, any biological sample containing nucleated cells may be used, including, for example, buccal mucosal cells.

### Pharmacogenomics

Agents, or modulators that have a stimulatory or inhibitory effect on NOVX activity (*e*.*g*., NOVX gene expression), as identified by a screening assay described herein can be administered to individuals to treat (prophylactically or therapeutically) disorders (*e*.*g*., neurological, cancer-related or gestational disorders) associated with aberrant NOVX activity. In conjunction with such treatment, the pharmacogenomics (*i*.*e*., the study of the relationship between an individual's genotype and that individual's response to a foreign compound or drug) of the individual may be considered. Differences in metabolism of therapeutics can lead to severe toxicity or therapeutic failure by altering the relation between dose and blood concentration of the pharmacologically active drug. Thus, the pharmacogenomics of the individual permits the selection of effective agents (*e*.*g*., drugs) for prophylactic or therapeutic treatments based on a consideration of the individual's genotype. Such pharmacogenomics can further be used to determine appropriate dosages and therapeutic regimens. Accordingly, the activity of NOVX protein, expression of NOVX nucleic acid, or mutation content of NOVX genes in an individual can be determined to thereby select appropriate agent(s) for therapeutic or prophylactic treatment of the individual.

Pharmacogenomics deals with clinically significant hereditary variations in the response to drugs due to altered drug disposition and abnormal action in affected persons. See *e.g*., Eichelbaum, 1996, *Clin Exp Pharmacol Physiol,* 23:983-985 and Linder, 1997, *Clin Chem,* 43:254-266. In general, two types of pharmacogenetic conditions can be differentiated. Genetic conditions transmitted as a single factor altering the way drugs act on the body (altered drug action) or genetic conditions transmitted as single factors altering the way the body acts on drugs (altered drug metabolism). These pharmacogenetic conditions can occur either as rare defects or as polymorphisms. For example, glucose-6-phosphate dehydrogenase (G6PD) deficiency is a common inherited enzymopathy in which the main clinical complication is haemolysis after ingestion of oxidant drugs (anti-malarials, sulfonamides, analgesics, nitrofurans) and consumption of fava beans.

As an illustrative embodiment, the activity of drug metabolizing enzymes is a major determinant of both the intensity and duration of drug action. The discovery of genetic polymorphisms of drug metabolizing enzymes (*e.g*., N-acetyltransferase 2 (NAT 2) and cytochrome P450 enzymes CYP2D6 and CYP2C19) has provided an explanation as to why some patients do not obtain the expected drug effects or show exaggerated drug response and serious toxicity after taking the standard and safe dose of a drug. These polymorphisms are expressed in two phenotypes in the population, the extensive metabolizer (EM) and poor metabolizer (PM). The prevalence of PM is different among different populations. For example, the gene coding for CYP2D6 is highly polymorphic and several mutations have been identified in PM, which all lead to the absence of functional CYP2D6. Poor metabolizers of CYP2D6 and CYP2C19 quite frequently experience exaggerated drug response and side effects when they receive standard doses. If a metabolite is the active therapeutic moiety, PM show no therapeutic response, as demonstrated for the analgesic effect of codeine mediated by its CYP2D6-formed metabolite morphine. The other extreme are the so called ultra-rapid metabolizers who do not respond to standard doses. Recently, the molecular basis of ultra-rapid metabolism has been identified to be due to CYP2D6 gene amplification.

Thus, the activity of NOVX protein, expression of NOVX nucleic acid, or mutation content of NOVX genes in an individual can be determined to thereby select appropriate agent(s) for therapeutic or prophylactic treatment of the individual. In addition, pharmacogenetic studies can be used to apply genotyping of polymorphic alleles encoding drug-metabolizing enzymes to the identification of an individual's drug responsiveness phenotype. This knowledge, when applied to dosing or drug selection, can avoid adverse reactions or therapeutic failure and thus enhance therapeutic or prophylactic efficiency when treating a subject with a NOVX modulator, such as a modulator identified by one of the exemplary screening assays described herein.

### Monitoring Clinical Efficacy

Monitoring the influence of agents (*e.g.*, drugs, compounds) on the expression or activity of NOVX (*e.g*., the ability to modulate aberrant cell proliferation and/or differentiation) can be applied in basic drug screening and in clinical trials. For example the effectiveness of an agent determined by a screening assay as described herein to increase NOVX gene expression, protein levels, or upregulate NOVX activity, can be monitored in clinical trials of subjects exhibiting decreased NOVX gene expression, protein levels, or downregulated NOVX activity. Alternatively, the effectiveness of an agent determined by a screening assay to decrease NOVX gene expression, protein levels, or downregulate NOVX activity, can be monitored in clinical trials of subjects exhibiting increased NOVX gene expression, protein levels, or upregulated NOVX activity. In such clinical trials, the expression or activity of NOVX and, preferably, other genes that have been implicated in, for example, a proliferative or neurological disorder, can be used as a "read out" or marker of the responsiveness of a particular cell.

For example, genes, including NOVX, that are modulated in cells by treatment with an agent (*e*.*g*., compound, drug or small molecule) that modulates NOVX activity (*e*.*g*., identified in a screening assay as described herein) can be identified. Thus, to study the effect of agents on cellular proliferation disorders, for example, in a clinical trial, cells can be isolated and RNA prepared and analyzed for the levels of expression of NOVX and other genes implicated in the disorder. The levels of gene expression (*i*.*e*., a gene expression pattern) can be quantified by Northern blot analysis or RT-PCR, as described herein, or alternatively by measuring the amount of protein produced, by one of the methods as described herein, or by measuring the levels of activity of NOVX or other genes. In this way, the gene expression pattern can serve as a marker, indicative of the physiological response of the cells to the agent. Accordingly, this response state may be determined before, and at various points during, treatment of the individual with the agent.

In one embodiment, the invention provides a method for monitoring the effectiveness of treatment of a subject with an agent (*e*.*g*., an agonist, antagonist, protein, peptide, nucleic acid, peptidomimetic, small molecule, or other drug candidate identified by the screening assays described herein) comprising the steps of (*i*) obtaining a pre-administration sample from a subject prior to administration of the agent; (*ii*) detecting the level of expression of a NOVX protein, mRNA, or genomic DNA in the preadministration sample; (*iii*) obtaining one or more post-administration samples from the subject; (*iv*) detecting the level of expression or activity of the NOVX protein, mRNA, or genomic DNA in the post-administration samples; (*v*) comparing the level of expression or activity of the NOVX protein, mRNA, or genomic DNA in the pre-administration sample with the NOVX protein, mRNA, or genomic DNA in the post administration sample or samples; and (*vi*) altering the administration of the agent to the subject accordingly. For example, increased administration of the agent may be desirable to increase the expression or activity of NOVX to higher levels than detected, *i.e.*, to increase the effectiveness of the agent. Alternatively, decreased administration of the agent may be desirable to decrease expression or activity of NOVX to lower levels than detected, *i*.*e*., to decrease the effectiveness of the agent.

### Methods of Treatment

The present invention provides for both prophylactic and therapeutic methods of treating a subject at risk of (or susceptible to) a disorder or having a disorder associated with aberrant NOVX expression or activity.

Diseases and disorders that are characterized by increased (relative to a subject not suffering from the disease or disorder) levels or biological activity may be treated with Therapeutics that antagonize (*i.e*., reduce or inhibit) activity. Therapeutics that antagonize activity may be administered in a therapeutic or prophylactic manner. Therapeutics that may be utilized include, but are not limited to, (*i*) a NOVX polypeptide, or analogs, derivatives, fragments or homologs thereof; (*ii*) antibodies to a NOVX peptide; (*iii*) nucleic acids encoding a NOVX peptide; (*iv*) administration of antisense nucleic acid and nucleic acids that are "dysfunctional" (*i*.*e*., due to a heterologous insertion within the coding sequences of coding sequences to a NOVX peptide) that are utilized to "knockout" endogenous function of a NOVX peptide by homologous recombination (see, *e.g.,* Capecchi, 1989, *Science* 244: 1288-1292); or (*v*) modulators (*i*.*e*., inhibitors, agonists and antagonists, including additional peptide mimetic of the invention or antibodies specific to a peptide of the invention) that alter the interaction between a NOVX peptide and its binding partner.

Diseases and disorders that are characterized by decreased (relative to a subject not suffering from the disease or disorder) levels or biological activity may be treated with Therapeutics that increase (*i.e*., are agonists to) activity. Therapeutics that upregulate activity may be administered in a therapeutic or prophylactic manner. Therapeutics that may be utilized include, but are not limited to, a NOVX peptide, or analogs, derivatives, fragments or homologs thereof; or an agonist that increases bioavailability.

Increased or decreased levels can be readily detected by quantifying peptide and/or RNA, by obtaining a patient tissue sample (*e.g*., from biopsy tissue) and assaying it *in vitro* for RNA or peptide levels, structure and/or activity of the expressed peptides (or mRNAs of a NOVX peptide). Methods that are well-known within the art include, but are not limited to, immunoassays (*e*.*g*., by Western blot analysis, immunoprecipitation followed by sodium dodecyl sulfate (SDS) polyacrylamide gel electrophoresis, immunocytochemistry, etc.) and/or hybridization assays to detect expression of mRNAs (*e*.*g*., Northern assays, dot blots, *in situ* hybridization, etc.).

In one aspect, the invention provides a method for preventing, in a subject, a disease or condition associated with an aberrant NOVX expression or activity, by administering to the subject an agent that modulates NOVX expression or at least one NOVX activity. Subjects at risk for a disease that is caused or contributed to by aberrant NOVX expression or activity can be identified by, for example, any or a combination of diagnostic or prognostic assays as described herein. Administration of a prophylactic agent can occur prior to the manifestation of symptoms characteristic of the NOVX aberrancy, such that a disease or disorder is prevented or, alternatively, delayed in its progression. Depending on the type of NOVX aberrancy, for example, a NOVX agonist or NOVX antagonist agent can be used for treating the subject. The appropriate agent can be determined based on screening assays described herein.

Another aspect of the invention pertains to methods of modulating NOVX expression or activity for therapeutic purposes. The modulatory method of the invention involves contacting a cell with an agent that modulates one or more of the activities of NOVX protein activity associated with the cell. An agent that modulates NOVX protein activity can be an agent as described herein, such as a nucleic acid or a protein, a naturally-occurring cognate ligand of a NOVX protein, a peptide, a NOVX peptidomimetic, or other small molecule. In one embodiment, the agent stimulates one or more NOVX protein activity. Examples of such stimulatory agents include active NOVX protein and a nucleic acid molecule encoding NOVX that has been introduced into the cell. In another embodiment, the agent inhibits one or more NOVX protein activity. Examples of such inhibitory agents include antisense NOVX nucleic acid molecules and anti-NOVX antibodies. These modulatory methods can be performed *in vitro* (*e*.*g*., by culturing the cell with the agent) or, alternatively, *in vivo* (*e*.*g*., by administering the agent to a subject). As such, the present invention provides methods of treating an individual afflicted with a disease or disorder characterized by aberrant expression or activity of a NOVX protein or nucleic acid molecule. In one embodiment, the method involves administering an agent (*e.g.,* an agent identified by a screening assay described herein), or combination of agents that modulates (*e*.*g*., upregulates or downregulates) NOVX expression or activity. In another embodiment, the method involves administering a NOVX protein or nucleic acid molecule as therapy to compensate for reduced or aberrant NOVX expression or activity.

### Determination of the Biological Effect of a Therapeutic

In various embodiments of the present invention, suitable *in vitro* or *in vivo* assays are utilized to determine the effect of a specific Therapeutic and whether its administration is indicated for treatment of the affected tissue.

In various specific embodiments, *in vitro* assays may be performed with representative cells of the type(s) involved in the patient's disorder, to determine if a given Therapeutic exerts the desired effect upon the cell type(s). Compounds for use in therapy may be tested in suitable animal model systems including, but not limited to rats, mice, chicken, cows, monkeys, rabbits, and the like, prior to testing in human subjects. Similarly, for *in vivo* testing, any of the animal model system known in the art may be used prior to administration to human subjects.

### Malignancies

Some of the NOVX proteins may be involved in the regulation of cell proliferation. Accordingly, Therapeutics of the present invention may be useful in the therapeutic or prophylactic treatment of diseases or disorders that are associated with cell hyperproliferation and/or loss of control of cell proliferation (*e.g.*, cancers, malignancies and tumors). For a review of such hyperproliferation disorders, see *e*.*g*., Fishman, *et al*., 1985. MEDICINE, 2nd ed., J.B. Lippincott Co., Philadelphia, PA.

Therapeutics of the present invention may be assayed by any method known within the art for efficacy in treating or preventing malignancies and related disorders. Such assays include, but are not limited to, *in vitro* assays utilizing transformed cells or cells derived from the patient's tumor, as well as *in vivo* assays using animal models of cancer or malignancies. Potentially effective Therapeutics are those that, for example, inhibit the proliferation of tumor-derived or transformed cells in culture or cause a regression of tumors in animal models, in comparison to the controls.

In the practice of the present invention, once a malignancy or cancer has been shown to be amenable to treatment by modulating (*i.e*., inhibiting, antagonizing or agonizing) activity, that cancer or malignancy may subsequently be treated or prevented by the administration of a Therapeutic that serves to modulate protein function.

### Premalignant conditions

The Therapeutics of the present invention that are effective in the therapeutic or prophylactic treatment of cancer or malignancies may also be administered for the treatment of pre-malignant conditions and/or to prevent the progression of a pre-malignancy to a neoplastic or malignant state. Such prophylactic or therapeutic use is indicated in conditions known or suspected of preceding progression to neoplasia or cancer, in particular, where non-neoplastic cell growth consisting of hyperplasia, metaplasia or, most particularly, dysplasia has occurred. For a review of such abnormal cell growth see *e.g*., Robbins & Angell, 1976. BASIC PATHOLOGY, 2nd ed., W.B. Saunders Co., Philadelphia, PA.

Hyperplasia is a form of controlled cell proliferation involving an increase in cell number in a tissue or organ, without significant alteration in its structure or function. For example, it has been demonstrated that endometrial hyperplasia often precedes endometrial cancer. Metaplasia is a form of controlled cell growth in which one type of mature or fully differentiated cell substitutes for another type of mature cell. Metaplasia may occur in epithelial or connective tissue cells. Dysplasia is generally considered a precursor of cancer, and is found mainly in the epithelia. Dysplasia is the most disorderly form of non-neoplastic cell growth, and involves a loss in individual cell uniformity and in the architectural orientation of cells. Dysplasia characteristically occurs where there exists chronic irritation or inflammation, and is often found in the cervix, respiratory passages, oral cavity, and gall bladder.

Alternatively, or in addition to the presence of abnormal cell growth characterized as hyperplasia, metaplasia, or dysplasia, the presence of one or more characteristics of a transformed or malignant phenotype displayed either *in vivo* or *in vitro* within a cell sample derived from a patient, is indicative of the desirability of prophylactic/therapeutic administration of a Therapeutic that possesses the ability to modulate activity of An aforementioned protein. Characteristics of a transformed phenotype include, but are not limited to: (*i*) morphological changes; (*ii*) looser substratum attachment; (*iii*) loss of cell-to-cell contact inhibition; (*iv*) loss of anchorage dependence; (*v*) protease release; (*vi*) increased sugar transport; (*vii*) decreased serum requirement; (*viii*) expression of fetal antigens, (*ix*) disappearance of the 250 kDal cell-surface protein, and the like. See *e*.*g*., Richards, *et al*., 1986. MOLECULAR PATHOLOGY, W.B. Saunders Co., Philadelphia, PA.

In a specific embodiment of the present invention, a patient that exhibits one or more of the following predisposing factors for malignancy is treated by administration of an effective amount of a Therapeutic: (*i*) a chromosomal translocation associated with a malignancy (*e*.*g*., the Philadelphia chromosome (*bcr*/*abl*) for chronic myelogenous leukemia and t(14;18) for follicular lymphoma, etc.); (*ii*) familial polyposis or Gardner's syndrome (possible forerunners of colon cancer); (*iii*) monoclonal gammopathy of undetermined significance (a possible precursor of multiple myeloma) and (*iv*) a first degree kinship with persons having a cancer or pre-cancerous disease showing a Mendelian (genetic) inheritance pattern *(e.g.,* familial polyposis of the colon, Gardner's syndrome, hereditary exostosis, polyendocrine adenomatosis, Peutz-Jeghers syndrome, neurofibromatosis of Von Recklinghausen, medullary thyroid carcinoma with amyloid production and pheochromocytoma, retinoblastoma, carotid body tumor, cutaneous melanocarcinoma, intraocular melanocarcinoma, xeroderma pigmentosum, ataxia telangiectasia, Chediak-Higashi syndrome, albinism, Fanconi's aplastic anemia and Bloom's syndrome).

In another embodiment, a Therapeutic of the present invention is administered to a human patient to prevent the progression to breast, colon, lung, pancreatic, or uterine cancer, or melanoma or sarcoma.

### Hyperproliferative and dysproliferative disorders

In one embodiment of the present invention, a Therapeutic is administered in the therapeutic or prophylactic treatment of hyperproliferative or benign dysproliferative disorders. The efficacy in treating or preventing hyperproliferative diseases or disorders of a Therapeutic of the present invention may be assayed by any method known within the art. Such assays include *in vitro* cell proliferation assays, *in vitro* or *in vivo* assays using animal models of hyperproliferative diseases or disorders, or the like. Potentially effective Therapeutics may, for example, promote cell proliferation in culture or cause growth or cell proliferation in animal models in comparison to controls.

Specific embodiments of the present invention are directed to the treatment or prevention of cirrhosis of the liver (a condition in which scarring has overtaken normal liver regeneration processes); treatment of keloid (hypertrophic scar) formation causing disfiguring of the skin in which the scarring process interferes with normal renewal; psoriasis (a common skin condition characterized by excessive proliferation of the skin and delay in proper cell fate determination); benign tumors; fibrocystic conditions and tissue hypertrophy (*e.g*., benign prostatic hypertrophy).

### Neurodegenerative disorders

Some of the NOVX proteins may be found in cell types have been implicated in the deregulation of cellular maturation and apoptosis, which are both characteristic of neurodegenerative disease. Accordingly, Therapeutics of the invention, particularly but not limited to those that modulate (or supply) activity of an aforementioned protein, may be effective in treating or preventing neurodegenerative disease. Therapeutics of the present invention that modulate the activity of an aforementioned protein involved in neurodegenerative disorders can be assayed by any method known in the art for efficacy in treating or preventing such neurodegenerative diseases and disorders. Such assays include *in vitro* assays for regulated cell maturation or inhibition of apoptosis or *in vivo* assays using animal models of neurodegenerative diseases or disorders, or any of the assays described below. Potentially effective Therapeutics, for example but not by way of limitation, promote regulated cell maturation and prevent cell apoptosis in culture, or reduce neurodegeneration in animal models in comparison to controls.

Once a neurodegenerative disease or disorder has been shown to be amenable to treatment by modulation activity, that neurodegenerative disease or disorder can be treated or prevented by administration of a Therapeutic that modulates activity. Such diseases include all degenerative disorders involved with aging, especially osteoarthritis and neurodegenerative disorders.

### Disorders related to organ transplantation

Some NOVX can be associated with disorders related to organ transplantation, in particular but not limited to organ rejection. Therapeutics of the invention, particularly those that modulate (or supply) activity, may be effective in treating or preventing diseases or disorders related to organ transplantation. Therapeutics of the invention (particularly Therapeutics that modulate the levels or activity of an aforementioned protein) can be assayed by any method known in the art for efficacy in treating or preventing such diseases and disorders related to organ transplantation. Such assays include *in vitro* assays for using cell culture models as described below, or *in vivo* assays using animal models of diseases and disorders related to organ transplantation, see *e*.*g*., below. Potentially effective Therapeutics, for example but not by way of limitation, reduce immune rejection responses in animal models in comparison to controls.

Accordingly, once diseases and disorders related to organ transplantation are shown to be amenable to treatment by modulation of activity, such diseases or disorders can be treated or prevented by administration of a Therapeutic that modulates activity.

### Cardiovascular Disease

NOVX may be implicated in cardiovascular disorders, including in atherosclerotic plaque formation. Diseases such as cardiovascular disease, including cerebral thrombosis or hemorrhage, ischemic heart or renal disease, peripheral vascular disease, or thrombosis of other major vessel, and other diseases, including diabetes mellitus, hypertension, hypothyroidism, cholesterol ester storage disease, systemic lupus erythematosus, homocysteinemia, and familial protein or lipid processing diseases, and the like, are either directly or indirectly associated with atherosclerosis. Accordingly, Therapeutics of the invention, particularly those that modulate (or supply) activity or formation may be effective in treating or preventing atherosclerosis-associated diseases or disorders. Therapeutics of the invention (particularly Therapeutics that modulate the levels or activity) can be assayed by any method known in the art, including those described below, for efficacy in treating or preventing such diseases and disorders.

A vast array of animal and cell culture models exist for processes involved in atherosclerosis. A limited and non-exclusive list of animal models includes knockout mice for premature atherosclerosis (Kurabayashi and Yazaki, 1996, Int. Angiol. 15: 187-194), transgenic mouse models of atherosclerosis (Kappel *et al*., 1994, FASEB J. 8: 583-592), antisense oligonucleotide treatment of animal models (Callow, 1995, Curr. Opin. Cardiol. 10: 569-576), transgenic rabbit models for atherosclerosis (Taylor, 1997, Ann. N.Y. Acad. Sci 811: 146-152), hypercholesterolemic animal models (Rosenfeld, 1996, Diabetes Res. Clin. Pract. 30 Suppl.: 1-11), hyperlipidemic mice (Paigen *et al*., 1994, Curr. Opin. Lipidol. 5: 258-264), and inhibition of lipoxygenase in animals (Sigal *et al*., 1994, Ann. N.Y. Acad. Sci. 714: 211-224). In addition, *in vitro* cell models include but are not limited to monocytes exposed to low density lipoprotein (Frostegard *et al*., 1996, Atherosclerosis 121: 93-103), cloned vascular smooth muscle cells (Suttles *et al*., 1995, Exp. Cell Res. 218: 331-338), endothelial cell-derived chemoattractant exposed T cells (Katz *et al*., 1994, J. Leukoc. Biol. 55: 567-573), cultured human aortic endothelial cells (Farber *et al*., 1992, Am. J. Physiol. 262: H1088-1085), and foam cell cultures (Libby *et al*., 1996, Curr Opin Lipidol 7: 330-335). Potentially effective Therapeutics, for example but not by way of limitation, reduce foam cell formation in cell culture models, or reduce atherosclerotic plaque formation in hypercholesterolemic mouse models of atherosclerosis in comparison to controls.

Accordingly, once an atherosclerosis-associated disease or disorder has been shown to be amenable to treatment by modulation of activity or formation, that disease or disorder can be treated or prevented by administration of a Therapeutic that modulates activity.

### Cytokine and Cell Proliferation/Differentiation Activity

A NOVX protein of the present invention may exhibit cytokine, cell proliferation (either inducing or inhibiting) or cell differentiation (either inducing or inhibiting) activity or may induce production of other cytokines in certain cell populations. Many protein factors discovered to date, including all known cytokines, have exhibited activity in one or more factor dependent cell proliferation assays, and hence the assays serve as a convenient confirmation of cytokine activity. The activity of a protein of the present invention is evidenced by any one of a number of routine factor dependent cell proliferation assays for cell lines including, without limitation, 32D, DA2, DA1G, T10, B9, B9/11, BaF3, MC9/G, M+ (preB M+), 2E8, RB5, DA1, 123, T1165, HT2, CTLL2, TF-1, Mo7e and CMK.

The activity of a protein of the invention may, among other means, be measured by the following methods: Assays for T-cell or thymocyte proliferation include without limitation those described in: CURRENT PROTOCOLS IN IMMUNOLOGY, Ed by Coligan *et al*., Greene Publishing Associates and Wiley-Interscience (Chapter 3 and Chapter 7); Takai *et al*., *J Immunol* 137:3494-3500, 1986; Bertagnoili *et al*., *J Immunol* 145:1706-1712, 1990; Bertagnolli *et al*., *Cell Immunol* 133:327-341, 1991; Bertagnolli, *et al*., *J Immunol* 149:3778-3783, 1992; Bowman *et al*., *J Immunol* 152:1756-1761, 1994.

Assays for cytokine production and/or proliferation of spleen cells, lymph node cells or thymocytes include, without limitation, those described by Kruisbeek and Shevach, In: CURRENT PROTOCOLS IN IMMUNOLOGY. Coligan *et al*., eds. Vol 1, pp. 3.12.1-14, John Wiley and Sons, Toronto 1994; and by Schreiber, In: CURRENT PROTOCOLS IN IMMUNOLOGY. Coligan eds. Vol 1 pp. 6.8.1-8, John Wiley and Sons, Toronto 1994.

Assays for proliferation and differentiation of hematopoietic and lymphopoietic cells include, without limitation, those described by Bottomly *et al*., In: CURRENT PROTOCOLS IN IMMUNOLOGY. Coligan *et al*., eds. Vol 1 pp. 6.3.1-6.3.12, John Wiley and Sons, Toronto 1991; deVries *et al*., *J Exp Med* 173:1205-1211, 1991; Moreau *et al*., *Nature* 336:690-692, 1988; Greenberger *et al*., *Proc Natl Acad Sci U*.*S*.*A*. 80:2931-2938, 1983; Nordan, In: CURRENT PROTOCOLS IN IMMUNOLOGY. Coligan *et al*., eds. Vol 1 pp. 6.6.1-5, John Wiley and Sons, Toronto 1991; Smith *et al*., *Proc Natl Acad Sci U*.*S*.*A*. 83:1857-1861, 1986; Measurement of human Interleukin 11-Bennett, *et al.* In: CURRENT PROTOCOLS IN IMMUNOLOGY. Coligan *et al*., eds. Vol 1 pp. 6.15.1 John Wiley and Sons, Toronto 1991; Ciarletta, *et al*., In: CURRENT PROTOCOLS IN IMMUNOLOGY. Coligan *et al*., eds. Vol 1 pp. 6.13.1, John Wiley and Sons, Toronto 1991.

Assays for T-cell clone responses to antigens (which will identify, among others, proteins that affect APC-T cell interactions as well as direct T-cell effects by measuring proliferation and cytokine production) include, without limitation, those described In: CURRENT PROTOCOLS IN IMMUNOLOGY. Coligan *et al*., eds., Greene Publishing Associates and Wiley-Interscience (Chapter 3, Chapter 6, Chapter 7); Weinberger *et al*., *Proc Natl Acad Sci USA* 77:6091-6095, 1980; Weinberger *et al*., *Eur J Immun* 11:405-411, 1981; Takai *et al*., *J Immunol* 137:3494-3500, 1986; Takai *et al*., *J Immunol* 140:508-512, 1988.

### Immune Stimulating or Suppressing Activity

A NOVX protein of the present invention may also exhibit immune stimulating or immune suppressing activity, including without limitation the activities for which assays are described herein. A protein may be useful in the treatment of various immune deficiencies and disorders (including severe combined immunodeficiency (SCID)), *e*.*g*., in regulating (up or down) growth and proliferation of T and/or B lymphocytes, as well as effecting the cytolytic activity of NK cells and other cell populations. These immune deficiencies may be genetic or be caused by vital (*e.g.,* HIV) as well as bacterial or fungal infections, or may result from autoimmune disorders. More specifically, infectious diseases causes by vital, bacterial, fungal or other infection may be treatable using a protein of the present invention, including infections by HIV, hepatitis viruses, herpesviruses, mycobacteria, Leishmania species., malaria species. and various fungal infections such as candidiasis. Of course, in this regard, a protein of the present invention may also be useful where a boost to the immune system generally may be desirable, *i*.*e*., in the treatment of cancer.

Autoimmune disorders which may be treated using a protein of the present invention include, for example, connective tissue disease, multiple sclerosis, systemic lupus erythematosus, rheumatoid arthritis, autoimmune pulmonary inflammation, Guillain-Barre syndrome, autoimmune thyroiditis, insulin dependent diabetes mellitus, myasthenia gravis, graft-versus-host disease and autoimmune inflammatory eye disease. Such a protein of the present invention may also to be useful in the treatment of allergic reactions and conditions, such as asthma (particularly allergic asthma) or other respiratory problems. Other conditions, in which immune suppression is desired (including, for example, organ transplantation), may also be treatable using a protein of the present invention.

Using the proteins of the invention it may also be possible to immune responses, in a number of ways. Down regulation may be in the form of inhibiting or blocking an immune response already in progress or may involve preventing the induction of an immune response. The functions of activated T cells may be inhibited by suppressing T cell responses or by inducing specific tolerance in T cells, or both. Immunosuppression of T cell responses is generally an active, non-antigen-specific, process which requires continuous exposure of the T cells to the suppressive agent. Tolerance, which involves inducing non-responsiveness or energy in T cells, is distinguishable from immunosuppression in that it is generally antigen-specific and persists after exposure to the tolerizing agent has ceased. Operationally, tolerance can be demonstrated by the lack of a T cell response upon re-exposure to specific antigen in the absence of the tolerizing agent.

Down regulating or preventing one or more antigen functions (including without limitation B lymphocyte antigen functions (such as, for example, B7), *e*.*g*., preventing high level lymphokine synthesis by activated T cells, will be useful in situations of tissue, skin and organ transplantation and in graft-versus-host disease (GVHD). For example, blockage of T cell function should result in reduced tissue destruction in tissue transplantation. Typically, in tissue transplants, rejection of the transplant is initiated through its recognition as foreign by T cells, followed by an immune reaction that destroys the transplant. The administration of a molecule which inhibits or blocks interaction of a B7 lymphocyte antigen with its natural ligand(s) on immune cells (such as a soluble, monomeric form of a peptide having B7-2 activity alone or in conjunction with a monomeric form of a peptide having an activity of another B lymphocyte antigen (*e.g*., B7-1, B7-3) or blocking antibody), prior to transplantation can lead to the binding of the molecule to the natural ligand(s) on the immune cells without transmitting the corresponding costimulatory signal. Blocking B lymphocyte antigen function in this matter prevents cytokine synthesis by immune cells, such as T cells, and thus acts as an immunosuppressant. Moreover, the lack of costimulation may also be sufficient to energize the T cells, thereby inducing tolerance in a subject. Induction of long-term tolerance by B lymphocyte antigen-blocking reagents may avoid the necessity of repeated administration of these blocking reagents. To achieve sufficient immunosuppression or tolerance in a subject, it may also be necessary to block the function of B lymphocyte antigens.

The efficacy of particular blocking reagents in preventing organ transplant rejection or GVHD can be assessed using animal models that are predictive of efficacy in humans. Examples of appropriate systems which can be used include allogeneic cardiac grafts in rats and xenogeneic pancreatic islet cell grafts in mice, both of which have been used to examine the immunosuppressive effects of CTLA4Ig fusion proteins *in vivo* as described in Lenschow *et al*., Science 257:789-792 (1992) and Turka *et al*., Proc Natl Acad Sci USA, 89:11102-11105 (1992). In addition, murine models of GVHD (see Paul ed., FUNDAMENTAL IMMUNOLOGY, Raven Press, New York, 1989, pp. 846-847) can be used to determine the effect of blocking B lymphocyte antigen function *in vivo* on the development of that disease.

Blocking antigen function may also be therapeutically useful for treating autoimmune diseases. Many autoimmune disorders are the result of inappropriate activation of T cells that are reactive against self tissue and which promote the production of cytokines and auto-antibodies involved in the pathology of the diseases. Preventing the activation of autoreactive T cells may reduce or eliminate disease symptoms. Administration of reagents which block costimulation of T cells by disrupting receptor:ligand interactions ofB lymphocyte antigens can be used to inhibit T cell activation and prevent production of auto-antibodies or T cell-derived cytokines which may be involved in the disease process. Additionally, blocking reagents may induce antigen-specific tolerance of autoreactive T cells which could lead to long-term relief from the disease. The efficacy of blocking reagents in preventing or alleviating autoimmune disorders can be determined using a number of well-characterized animal models of human autoimmune diseases. Examples include murine experimental autoimmune encephalitis, systemic lupus erythematosis in MRL/lpr/lpr mice or NZB hybrid mice, murine autoimmune collagen arthritis, diabetes mellitus in NOD mice and BB rats, and murine experimental myasthenia gravis (see Paul ed., FUNDAMENTAL IMMUNOLOGY, Raven Press, New York, 1989, pp. 840-856).

Upregulation of an antigen function (preferably a B lymphocyte antigen function), as a means of up regulating immune responses, may also be useful in therapy. Upregulation of immune responses may be in the form of enhancing an existing immune response or eliciting an initial immune response. For example, enhancing an immune response through stimulating B lymphocyte antigen function may be useful in cases of viral infection. In addition, systemic vital diseases such as influenza, the common cold, and encephalitis might be alleviated by the administration of stimulatory forms of B lymphocyte antigens systemically.

Alternatively, anti-viral immune responses may be enhanced in an infected patient by removing T cells from the patient, costimulating the T cells *in vitro* with viral antigen-pulsed APCs either expressing a peptide of the present invention or together with a stimulatory form of a soluble peptide of the present invention and reintroducing the *in vitro* activated T cells into the patient. Another method of enhancing anti-vital immune responses would be to isolate infected cells from a patient, transfect them with a nucleic acid encoding a protein of the present invention as described herein such that the cells express all or a portion of the protein on their surface, and reintroduce the transfected cells into the patient. The infected cells would now be capable of delivering a costimulatory signal to, and thereby activate, T cells *in vivo*.

In another application, up regulation or enhancement of antigen function (preferably B lymphocyte antigen function) may be useful in the induction of tumor immunity. Tumor cells (*e.g*., sarcoma, melanoma, lymphoma, leukemia, neuroblastoma, carcinoma) transfected with a nucleic acid encoding at least one peptide of the present invention can be administered to a subject to overcome tumor-specific tolerance in the subject. If desired, the tumor cell can be transfected to express a combination of peptides. For example, tumor cells obtained from a patient can be transfected *ex vivo* with an expression vector directing the expression of a peptide having B7-2-like activity alone, or in conjunction with a peptide having B7-1-like activity and/or B7-3-like activity. The transfected tumor cells are returned to the patient to result in expression of the peptides on the surface of the transfected cell. Alternatively, gene therapy techniques can be used to target a tumor cell for transfection *in vivo.*

The presence of the peptide of the present invention having the activity of a B lymphocyte antigen(s) on the surface of the tumor cell provides the necessary costimulation signal to T cells to induce a T cell mediated immune response against the transfected tumor cells. In addition, tumor cells which lack MHC class I or MHC class II molecules, or which fail to reexpress sufficient amounts of MHC class I or MHC class II molecules, can be transfected with nucleic acid encoding all or a portion of (*e.g.,* a cytoplasmic-domain truncated portion) of an MHC class Iα chain protein and β₂ microglobulin protein or an MHC class II a chain protein and an MHC class II β chain protein to thereby express MHC class I or MHC class II proteins on the cell surface. Expression of the appropriate class I or class II MHC in conjunction with a peptide having the activity of a B lymphocyte antigen (*e*.*g*., B7-1, B7-2, B7-3) induces a T cell mediated immune response against the transfected tumor cell. Optionally, a gene encoding an antisense construct which blocks expression of an MHC class II associated protein, such as the invariant chain, can also be cotransfected with a DNA encoding a peptide having the activity of a B lymphocyte antigen to promote presentation of tumor associated antigens and induce tumor specific immunity. Thus, the induction of a T cell mediated immune response in a human subject may be sufficient to overcome tumor-specific tolerance in the subject.

The activity of a protein of the invention may, among other means, be measured by the following methods: Suitable assays for thymocyte or splenocyte cytotoxicity include, without limitation, those described In: CURRENT PROTOCOLS IN IMMUNOLOGY. Coligan *et al*., eds. Greene Publishing Associates and Wiley-Interscience (Chapter 3, Chapter 7); Herrmann *et al*., *Proc Natl Acad Sci USA* 78:2488-2492, 1981; Herrmann *et al*., *J Immunol* 128:1968-1974, 1982; Handa *et al*., *J Immunol* 135:1564-1572, 1985; Takai *et al*., *J Immunol* 137:3494-3500, 1986; Takai *et al*., *J Immunol* 140:508-512, 1988; Herrmann *et al*., *Proc Natl Acad Sci USA* 78:2488-2492, 1981; Herrmann *et al*., *J Immunol* 128:1968-1974, 1982; Handa *et al*., *J Immunol* 135:1564-1572, 1985; Takai *et al*., *J Immunol* 137:3494-3500, 1986; Bowman *et al*., *J Virology* 61:1992-1998; Takai *et al*., *J Immunol* 140:508-512, 1988; Bertagnolli *et al*., *Cell Immunol* 133:327-341, 1991; Brown *et al*., *J Immunol* 153:3079-3092, 1994.

Assays for T-cell-dependent immunoglobulin responses and isotype switching (which will identify, among others, proteins that modulate T-cell dependent antibody responses and that affect Th1/Th2 profiles) include, without limitation, those described in: Maliszewski, *J Immunol* 144:3028-3033, 1990; and Mond and Brunswick In: CURRENT PROTOCOLS IN IMMUNOLOGY. Coligan *et al*., (eds.) Vol 1 pp. 3.8.1-3.8.16, John Wiley and Sons, Toronto 1994.

Mixed·lymphocyte reaction (MLR) assays (which will identify, among others, proteins that generate predominantly Th1 and CTL responses) include, without limitation, those described In: CURRENT PROTOCOLS IN IMMUNOLOGY. Coligan *et al*., eds. Greene Publishing Associates and Wiley-Interscience (Chapter 3, Chapter 7); Takai *et al*., *J Immunol* 137:3494-3500, 1986; Takai *et al*., *J Immunol* 140:508-512, 1988; Bertagnolli *et al*., *J Immunol* 149:3778-3783, 1992.

Dendritic cell-dependent assays (which will identify, among others, proteins expressed by dendritic cells that activate naive T-cells) include, without limitation, those described in: Guery *et al*., *J Immunol* 134:536-544, 1995; Inaba *et al*., *J Exp Med* 173:549-559, 1991; Macatonia *et al*., *J Immunol* 154:5071-5079, 1995; Porgador *et al*., *J Exp Med* 182:255-260, 1995; Nair *et al*., *J Virol* 67:4062-4069, 1993; Huang *et al*., *Science* 264:961-965, 1994; Macatonia *et al*., *J Exp Med* 169:1255-1264, 1989; Bhardwaj *et al*., *J Clin Investig* 94:797-807, 1994; and Inaba *et al*., *J Exp Med* 172:631-640, 1990.

Assays for lymphocyte survival/apoptosis (which will identify, among others, proteins that prevent apoptosis after superantigen induction and proteins that regulate lymphocyte homeostasis) include, without limitation, those described in: Darzynkiewicz *et al*., *Cytometry* 13:795-808, 1992; Gorczyca *et al*., *Leukemia* 7:659-670, 1993; Gorczyca *et al*., *Cancer Res* 53:1945-1951, 1993; Itoh *et al*., *Cell* 66:233-243, 1991; Zacharchuk, *J Immunol* 145:4037-4045, 1990; Zamai *et al*., *Cytometry* 14:891-897, 1993; *Gorczyca et al*., *Internat J Oncol* 1:639-648, 1992.

Assays for proteins that influence early steps of T-cell commitment and development include, without limitation, those described in: Antica *et al*., *Blood* 84:111-117, 1994; Fine *et al*., *Cell Immunol* 155: 111-122, 1994; Galy *et al*., *Blood* 85:2770-2778, 1995; Toki *et al*., *Proc Nat Acad Sci USA* 88:7548-7551, 1991.

### Hematopoiesis Regulating Activity

A NOVX protein of the present invention may be useful in regulation of hematopoiesis and, consequently, in the treatment of myeloid or lymphoid cell deficiencies. Even marginal biological activity in support of colony forming cells or of factor-dependent cell lines indicates involvement in regulating hematopoiesis, *e*.*g*. in supporting the growth and proliferation of erythroid progenitor cells alone or in combination with other cytokines, thereby indicating utility, for example, in treating various anemias or for use in conjunction with irradiation/chemotherapy to stimulate the production of erythroid precursors and/or erythroid cells; in supporting the growth and proliferation of myeloid cells such as granulocytes and monocytes/macrophages (*i*.*e*., traditional CSF activity) useful, for example, in conjunction with chemotherapy to prevent or treat consequent myelo-suppression; in supporting the growth and proliferation of megakaryocytes and consequently of platelets thereby allowing prevention or treatment of various platelet disorders such as thrombocytopenia, and generally for use in place of or complimentary to platelet transfusions; and/or in supporting the growth and proliferation of hematopoietic stem cells which are capable of maturing to any and all of the above-mentioned hematopoietic cells and therefore find therapeutic utility in various stem cell disorders (such as those usually treated with transplantation, including, without limitation, aplastic anemia and paroxysmal nocturnal hemoglobinuria), as well as in repopulating the stem cell compartment post irradiation/chemotherapy, either in-vivo or ex-vivo (i.e., in conjunction with bone marrow transplantation or with peripheral progenitor cell transplantation (homologous or heterologous)) as normal cells or genetically manipulated for gene therapy.

The activity of a protein of the invention may, among other means, be measured by the following methods:
Suitable assays for proliferation and differentiation of various hematopoietic lines are cited above.
Assays for embryonic stem cell differentiation (which will identify, among others, proteins that influence embryonic differentiation hematopoiesis) include, without limitation, those described in: Johansson *et al*. *Cellular Biology* 15:141-151, 1995; Keller *et al*., *Mol*. *Cell. Biol*. 13:473-486, 1993; McClanahan *et al*., *Blood* 81:2903-2915, 1993.
Assays for stem cell survival and differentiation (which will identify, among others, proteins that regulate lympho-hematopoiesis) include, without limitation, those described in: Methylcellulose colony forming assays, Freshney, In: CULTURE OF HEMATOPOIETIC CELLS. Freshney, *et al*. (eds.) Vol pp. 265-268, Wiley-Liss, Inc., New York, N.Y 1994; Hirayama *et al*., *Proc Natl Acad Sci USA* 89:5907-5911, 1992; McNiece and Briddeli, In: CULTURE OF HEMATOPOIETIC CELLS. Freshney, *et al*. (eds.) Vol pp. 23-39, Wiley-Liss, Inc., New York, N.Y. 1994; Neben *et al*., *Exp Hematol* 22:353-359, 1994; Ploemacher, In: CULTURE OF HEMATOPOIETIC CELLS. Freshney, *et al*. eds. Vol pp. 1-21, Wiley-Liss, Inc., New York, N.Y. 1994; Spoonceret *al*., In: CULTURE OF HEMATOPOIETIC CELLS. Freshhey, *et al*., (eds.) Vol pp. 163-179, Wiley-Liss, Inc., New York, N.Y. 1994; Sutherland, In: CULTURE OF HEMATOPOIETIC CELLS. Freshney, *et al*., (eds.) Vol pp. 139-162, Wiley-Liss, Inc., New York, N.Y. 1994.

### Tissue Growth Activity

A NOVX protein of the present invention also may have utility in compositions used for bone, cartilage, tendon, ligament and/or nerve tissue growth or regeneration, as well as for wound healing and tissue repair and replacement, and in the treatment of burns, incisions and ulcers.

A protein of the present invention, which induces cartilage and/or bone growth in circumstances where bone is not normally formed, has application in the healing of bone fractures and cartilage damage or defects in humans and other animals. Such a preparation employing a protein of the invention may have prophylactic use in closed as well as open fracture reduction and also in the improved fixation of artificial joints. *De novo* bone formation induced by an osteogenic agent contributes to the repair of congenital, trauma induced, or oncologic resection induced craniofacial defects, and also is useful in cosmetic plastic surgery.

A protein of this invention may also be used in the treatment of periodontal disease, and in other tooth repair processes. Such agents may provide an environment to attract bone-forming cells, stimulate growth of bone-forming cells or induce differentiation of progenitors of bone-forming cells. A protein of the invention may also be useful in the treatment of osteoporosis or osteoarthritis, such as through stimulation of bone and/or cartilage repair or by blocking inflammation or processes of tissue destruction (collagenase activity, osteoclast activity, etc.) mediated by inflammatory processes.

Another category of tissue regeneration activity that may be attributable to the protein of the present invention is tendon/ligament formation. A protein of the present invention, which induces tendon/ligament-like tissue or other tissue formation in circumstances where such tissue is not normally formed, has application in the healing of tendon or ligament tears, deformities and other tendon or ligament defects in humans and other animals. Such a preparation employing a tendon/ligament-like tissue inducing protein may have prophylactic use in preventing damage to tendon or ligament tissue, as well as use in the improved fixation of tendon or ligament to bone or other tissues, and in repairing defects to tendon or ligament tissue. *De novo* tendon/ligament-like tissue formation induced by a composition of the present invention contributes to the repair of congenital, trauma induced, or other tendon or ligament defects of other origin, and is also useful in cosmetic plastic surgery for attachment or repair of tendons or ligaments. The compositions of the present invention may provide an environment to attract tendon- or ligament-forming cells, stimulate growth of tendon- or ligament-forming cells, induce differentiation of progenitors of tendon- or ligament-forming cells, or induce growth of tendon/ligament cells or progenitors *ex vivo* for return *in vivo* to effect tissue repair. The compositions of the invention may also be useful in the treatment of tendonitis, carpal tunnel syndrome and other tendon or ligament defects. The compositions may also include an appropriate matrix and/or sequestering agent as a career as is well known in the art.

The protein of the present invention may also be useful for proliferation of neural cells and for regeneration of nerve and brain tissue, *i*.*e*. for the treatment of central and peripheral nervous system diseases and neuropathies, as well as mechanical and traumatic disorders, which involve degeneration, death or trauma to neural cells or nerve tissue. More specifically, a protein may be used in the treatment of diseases of the peripheral nervous system, such as peripheral nerve injuries, peripheral neuropathy and localized neuropathies, and central nervous system diseases, such as Alzheimer's, Parkinson's disease, Huntington's disease, amyotrophic lateral sclerosis, and Shy-Drager syndrome. Further conditions which may be treated in accordance with the present invention include mechanical and traumatic disorders, such as spinal cord disorders, head trauma and cerebrovascular diseases such as stroke. Peripheral neuropathies resulting from chemotherapy or other medical therapies may also be treatable using a protein of the invention.

Proteins of the invention may also be useful to promote better or faster closure of non-healing wounds, including without limitation pressure ulcers, ulcers associated with vascular insufficiency, surgical and traumatic wounds, and the like.

It is expected that a protein of the present invention may also exhibit activity for generation or regeneration of other tissues, such as organs (including, for example, pancreas, liver, intestine, kidney, skin, endothelium), muscle (smooth, skeletal or cardiac) and vascular (including vascular endothelium) tissue, or for promoting the growth of cells comprising such tissues. Part of the desired effects may be by inhibition or modulation of fibrotic scarring to allow normal tissue to regenerate. A protein of the invention may also exhibit angiogenic activity.

A protein of the present invention may also be useful for gut protection or regeneration and treatment of lung or liver fibrosis, reperfusion injury in various tissues, and conditions resulting from systemic cytokine damage.

A protein of the present invention may also be useful for promoting or inhibiting differentiation of tissues described above from precursor tissues or cells; or for inhibiting the growth of tissues described above.

The activity of a protein of the invention may, among other means, be measured by the following methods:
Assays for tissue generation activity include, without limitation, those described in: International Patent Publication No. WO95/16035 (bone, cartilage, tendon); International Patent Publication No. WO95/05846 (nerve, neuronal); International Patent Publication No. WO91/07491 (skin, endothelium).
Assays for wound healing activity include, without limitation, those described in: Winter, EPIDERMAL WOUND HEALING, pp. 71-112 (Maibach and Rovee, eds.), Year Book Medical Publishers, Inc., Chicago, as modified by Eaglstein and Menz, *J*. *Invest. Dermatol* 71:382-84 (1978).

### Activin/Inbibin Activity

A NOVX protein of the present invention may also exhibit activin- or inhibin-related activities. Inhibins are characterized by their ability to inhibit the release of follicle stimulating hormone (FSH), while activins and are characterized by their ability to stimulate the release of follicle stimulating hormone (FSH). Thus, a protein of the present invention, alone or in heterodimers with a member of the inhibin a family, may be useful as a contraceptive based on the ability of inhibins to decrease fertility in female mammals and decrease spermatogenesis in male mammals. Administration of sufficient amounts of other inhibins can induce infertility in these mammals. Alternatively, the protein of the invention, as a homodimer or as a heterodimer with other protein subunits of the inhibin-b group, may be useful as a fertility inducing therapeutic, based upon the ability of activin molecules in stimulating FSH release from cells of the anterior pituitary. See, for example, U.S. Pat. No. 4,798,885. A protein of the invention may also be useful for advancement of the onset of fertility in sexually immature mammals, so as to increase the lifetime reproductive performance of domestic animals such as cows, sheep and pigs.

The activity of a protein of the invention may, among other means, be measured by the following methods:
Assays for activin/inhibin activity include, without limitation, those described in: Vale *et al*., *Endocrinology* 91:562-572, 1972; Ling *et al*., *Nature* 321:779-782, 1986; Vale *et al*., *Nature* 321:776-779, 1986; Mason *et al*., *Nature* 318:659-663, 1985; Forage *et al*., *Proc Natl Acad Sci USA* 83:3091-3095, 1986.

### Chemotactic/Chemokinetic Activity

A NOVX protein of the present invention may have chemotactic or chemokinetic activity (*e.g.*, act as a chemokine) for mammalian cells, including, for example, monocytes, fibroblasts, neutrophils, T-cells, mast cells, eosinophils, epithelial and/or endothelial cells. Chemotactic and chemokinetic proteins can be used to mobilize or attract a desired cell population to a desired site of action. Chemotactic or chemokinetic proteins provide particular advantages in treatment of wounds and other trauma to tissues, as well as in treatment of localized infections. For example, attraction of lymphocytes, monocytes or neutrophils to tumors or sites of infection may result in improved immune responses against the tumor or infecting agent.

A protein or peptide has chemotactic activity for a particular cell population if it can stimulate, directly or indirectly, the directed orientation or movement of such cell population. Preferably, the protein or peptide has the ability to directly stimulate directed movement of cells. Whether a particular protein has chemotactic activity for a population of cells can be readily determined by employing such protein or peptide in any known assay for cell chemotaxis.

The activity of a protein of the invention may, among other means, be measured by following methods:
Assays for chemotactic activity (which will identify proteins that induce or prevent chemotaxis) consist of assays that measure the ability of a protein to induce the migration of cells across a membrane as well as the ability of a protein to induce the adhesion of one cell population to another cell population. Suitable assays for movement and adhesion include, without limitation, those described in: CURRENT PROTOCOLS IN IMMUNOLOGY, Coligan *et al*., eds. (Chapter 6.12, MEASUREMENT OF ALPHA AND BETA CHEMOKINES 6.12.1-6.12.28); Taub *et al*. *J Clin Invest* 95:1370-1376, 1995; Lind *et al*. *APMIS* 103:140-146, 1995; Muller *et al*., *Eur J Immunol* 25: 1744-1748; Gruber *et al*. *J Immunol* 152:5860-5867*,* 1994; Johnston *et al*., *J Immunol* 153: 1762-1768, 1994.

### Hemostatic and Thrombolytic Activity

A NOVX protein of the invention may also exhibit hemostatic or thrombolytic activity. As a result, such a protein is expected to be useful in treatment of various coagulation disorders (including hereditary disorders, such as hemophilias) or to enhance coagulation and other hemostatic events in treating wounds resulting from trauma, surgery or other causes. A protein of the invention may also be useful for dissolving or inhibiting formation of thromboses and for treatment and prevention of conditions resulting therefrom (such as, for example, infarction of cardiac and central nervous system vessels (*e*.*g*., stroke).

The activity of a protein of the invention may, among other means, be measured by the following methods:
Assay for hemostatic and thrombolytic activity include, without limitation, those described in: Linet *et al*., *J*. *Clin*. *Pharmacol.* 26:131-140, 1986; Burdick *et al*., *Thrombosis* *Res.* 45:413-419, 1987; Humphrey *et al*., *Fibrinolysis* 5:71-79 (1991); Schaub, *Prostaglandins* 35:467-474, 1988.

### Receptor/Ligand Activity

A NOVX protein of the present invention may also demonstrate activity as receptors, receptor ligands or inhibitors or agonists of receptor/ligand interactions. Examples of such receptors and ligands include, without limitation, cytokine receptors and their ligands, receptor kinases and their ligands, receptor phosphatases and their ligands, receptors involved in cell-cell interactions and their ligands (including without limitation, cellular adhesion molecules (such as selectins, integrins and their ligands) and receptor/ligand pairs involved in antigen presentation, antigen recognition and development of cellular and humoral immune responses). Receptors and ligands are also useful for screening of potential peptide or small molecule inhibitors of the relevant receptor/ligand interaction. A protein of the present invention (including, without limitation, fragments of receptors and ligands) may themselves be useful as inhibitors of receptor/ligand interactions.

The activity of a protein of the invention may, among other means, be measured by the following methods:
Suitable assays for receptor-ligand activity include without limitation those described in: CURRENT PROTOCOLS IN IMMUNOLOGY, Ed by Coligan, *et al*., Greene Publishing Associates and Wiley-Interscience (Chapter 7.28, Measurement of Cellular Adhesion under static conditions 7.28.1-7.28.22), Takai *et al*., *Proc Natl Acad Sci USA* 84:6864-6868, 1987; Bierer *et al*., *J*. *Exp. Med.* 168:1145-1156, 1988; Rosenstein *et al*., *J*. *Exp*. *Med.* 169:149-160 1989; Stoltenborg *et al*., *J Immunol Methods* 175:59-68, 1994; Stitt *et al*., *Cell* 80:661-670, 1995.

### Anti-Inflammatory Activity

NOVX proteins of the present invention may also exhibit anti-inflammatory activity. The anti-inflammatory activity may be achieved by providing a stimulus to cells involved in the inflammatory response, by inhibiting or promoting cell-cell interactions (such as, for example, cell adhesion), by inhibiting or promoting chemotaxis of cells involved in the inflammatory process, inhibiting or promoting cell extravasation, or by stimulating or suppressing production of other factors which more directly inhibit or promote an inflammatory response. Proteins exhibiting such activities can be used to treat inflammatory conditions including chronic or acute conditions), including without limitation inflammation associated with infection (such as septic shock, sepsis or systemic inflammatory response syndrome (SIRS)), ischemia-reperfusion injury, endotoxin lethality, arthritis, complement-mediated hyperacute rejection, nephritis, cytokine or chemokine-induced lung injury, inflammatory bowel disease, Crohn's disease or resulting from over production of cytokines such as TNF or IL-1. Proteins of the invention may also be useful to treat anaphylaxis and hypersensitivity to an antigenic substance or material.

### Tumor Inhibition Activity

In addition to the activities described above for immunological treatment or prevention of tumors, a NOVX protein of the invention may exhibit other anti-tumor activities. A protein may inhibit tumor growth directly or indirectly (such as, for example, via ADCC). A protein may exhibit its tumor inhibitory activity by acting on tumor tissue or tumor precursor tissue, by inhibiting formation of tissues necessary to support tumor growth (such as, for example, by inhibiting angiogenesis), by causing production of other factors, agents or cell types which inhibit tumor growth, or by suppressing, eliminating or inhibiting factors, agents or cell types which promote tumor growth.

### Other Activities

A NOVX protein of the invention may also exhibit one or more of the following additional activities or effects: inhibiting the growth, infection or function of, or killing, infectious agents, including, without limitation, bacteria, viruses, fungi and other parasites; effecting (suppressing or enhancing) bodily characteristics, including, without limitation, height, weight, hair color, eye color, skin, fat to lean ratio or other tissue pigmentation, or organ or body part size or shape (such as, for example, breast augmentation or diminution, change in bone form or shape); effecting biorhythms or circadian cycles or rhythms; effecting the fertility of male or female subjects; effecting the metabolism, catabolism, anabolism, processing, utilization, storage or elimination of dietary fat, lipid, protein, carbohydrate, vitamins, minerals, cofactors or other nutritional factors or component(s); effecting behavioral characteristics, including, without limitation, appetite, libido, stress, cognition (including cognitive disorders), depression (including depressive disorders) and violent behaviors; providing analgesic effects or other pain reducing effects; promoting differentiation and growth of embryonic stem cells in lineages other than hematopoietic lineages; hormonal or endocrine activity; in the case of enzymes, correcting deficiencies of the enzyme and treating deficiency-related diseases; treatment ofhyperproliferative disorders (such as, for example, psoriasis); immunoglobulin-like activity (such as, for example, the ability to bind antigens or complement); and the ability to act as an antigen in a vaccine composition to raise an immune response against such protein or another material or entity which is cross-reactive with such protein.

Neural disorders in general include Parkinson's disease, Alzheimer's disease, Huntington's disease, multiple sclerosis, amyotrophic lateral sclerosis (ALS), peripheral neuropathy, tumors of the nervous system, exposure to neurotoxins, acute brain injury, peripheral nerve trauma or injury, and other neuropathies, epilepsy, and/or tremors.

### EXAMPLES

### Example 1. Chromosomal location of NOV6

Radiation hybrid mapping using human chromosome markers was carried out for many of the clones described herein. The procedure used to obtain these results is analogous to that described in Steen, RG *et al*., Genome Research 1999 (Published Online on May 21, 1999) Vol. 9, AP1-AP8, 1999. A panel of 93 cell clones containing randomized radiation-induced human chromosomal fragments was screened in 96 well plates using PCR primers designed to identify the sought clones in a unique fashion. Using this procedure, a NOV6 nucleic acid according to the invention was localized chromosome 11 at a map distance of -0.7 cR from WI-4920 and -3.90 cR from WI-1421.

### Example 2. Quantitative tissue expression analysis of NOVX nucleic acids

The quantitative expression of various clones containning NOVX nucleic acids was assessed in 41 normal and 55 tumor samples (see Table 4) by real time quantitative PCR (TAQMAN® analysis). In Table 4, the following abbreviations are used:
ca. = carcinoma,
* = established from metastasis,
met = metastasis,
s cell var= small cell variant,
non-s = non-sm =non-small,
squam = squamous,
pl. eff = pl effusion = pleural effusion,
glio = glioma,
astro = astrocytoma, and
neuro = neuroblastoma.

First, 96 RNA samples were normalized to β-actin and GAPDH. RNA (∼50 ng total or ∼1 ng polyA+) was converted to cDNA using the TAQMAN® Reverse Transcription Reagents Kit (PE Biosystems, Foster City, CA; cat # N808-0234) and random hexamers according to the manufacturer's protocol. Reactions were performed in 20 ul and incubated for 30 min. at 48°C. cDNA (5 ul) was then transferred to a separate plate for the TAQMAN® reaction using β-actin and GAPDH TAQMAN® Assay Reagents (PE Biosystems; cat. #'s 4310881E and 4310884E, respectively) and TAQMAN® universal PCR Master Mix (PE Biosystems; cat # 4304447) according to the manufacturer's protocol. Reactions were performed in 25 ul using the following parameters: 2 min. at 50°C; 10 min. at 95°C; 15 sec. at 95°C/l min. at 60°C (40 cycles). Results were recorded as CT values (cycle at which a given sample crosses a threshold level of fluorescence) using a log scale, with the difference in RNA concentration between a given sample and the sample with the lowest CT value being represented as 2 to the power of delta CT. The percent relative expression is then obtained by taking the reciprocal of this RNA difference and multiplying by 100. The average CT values obtained for β-actin and GAPDH were used to normalize RNA samples. The RNA sample generating the highest CT value required no further diluting, while all other samples were diluted relative to this sample according to their β-actin /GAPDH average CT values.

Normalized RNA (5 ul) was converted to cDNA and analyzed via TAQMAN® using One Step RT-PCR Master Mix Reagents (PE Biosystems; cat. # 4309169) and gene-specific primers according to the manufacturer's instructions. Probes and primers were designed for each assay according to Perkin Elmer Biosystem's *Primer Express* Software package (version I for Apple Computer's Macintosh Power PC) using the sequence of the respective clones as input. Default settings were used for reaction conditions, and the following parameters were set before selecting primers: primer concentration = 250 nM, primer melting temperature (Tₘ) range = 58°-60° C, primer optimal Tm = 59° C, maximum primer difference = 2° C, probe does not have 5' G, probe Tₘ must be 10° C greater than primer Tₘ, amplicon size 75 bp to 100 bp. The probes and primers selected (see below) were synthesized by Synthegen (Houston, TX, USA). Probes were double purified by HPLC to remove uncoupled dye and evaluated by mass spectroscopy to verify coupling of reporter and quencher dyes to the 5' and 3' ends of the probe, respectively. Their final concentrations were: forward and reverse primers, 900 nM each, and probe, 200nM.

PCR conditions: Normalized RNA from each tissue and each cell line was spotted in each well of a 96 well PCR plate (Perkin Elmer Biosystems). PCR cocktails including two probes (NOVX-specific and another gene-specific probe multiplexed with the NOVX probe) were set up using 1X TaqMan™ PCR Master Mix for the PE Biosystems 7700, with 5 mM MgCl2, dNTPs (dA, G, C, U at 1:1:1:2 ratios), 0.25 U/ml AmpliTaq Gold™ (PE Biosystems), and 0.4 U/µl RNase inhibitor, and 0.25 U/µl reverse transcriptase. Reverse transcription was performed at 48° C for 30 minutes followed by amplification/PCR cycles as follows: 95° C 10 min, then 40 cycles of 95° C for 15 seconds, 60° C for 1 minute.

The primer-probe sets employed in the expression analysis of each clone, and a summary of the results, are given below.

NOV2
Ag 142 (F): 5'-AAAAAGGAGGAGTCAAACGTGTCT-3' (SEQ ID NO:47)
Ag 142 (R): 5'-GGTCAAGCGCAGCTTTGC-3' (SEQ ID NO:48)
Ag 142 (P): FAM-5'-CCCATCGACCACATCCTCCTCCAG-3'-TAMRA (SEQ ID NO:49)

The results in Table 4 indicate that NOV2 is preferentially expressed in several cancer cell lines, with the highest in metastasized prostate cancer.

NOV5
Ag 89 (F): 5'-TAAAGAGAATCTTCCCCTGGAAGAG-3' (SEQ ID NO:50)
Ag 89 (R): 5'-GGCCTCCCCAGAGTATGGA-3' (SEQ ID NO:51)
Ag 89 (P): TET-5'-CATGCACAGATACCAGAAGGCAGCCAA-3'-TAMRA (SEQ ID
NO:52)

The results show high expression in mammary gland (Table 4).

NOV7
Ag 6(F): 5'-GGATGCATGCTCCAAAGAAGA-3' (SEQ ID NO:53)
Ag 6(R): 5'-CTCACCCACTGCTGTCTCCA-3' (SEQ ID NO:54)
Ag 6(P):-FAM-5'-CTGCCCAGGTGGCCGTCACTC-3'-TAMRA (SEQ ID NO:55)

The results indicate high expression in a number of normal cell lines, including brain, pancreas, placenta and testis, and cancer cell lines, including colon, lung and prostate metastasis (see Table 4).

NOV8
Ag7(F): 5'-GCTCCCCAATCTGGTCTCCTAC-3' (SEQ ID NO:56)
Ag7(R): 5'-GATGGGCTTGAACTGGAAAGAG-3' (SEQ ID NO:57)
Ag7(P):-FAM-5'-CTCTCTGTGTGCCACCCATGCTGG-3'-TAMRA (SEQ ID NO:58)

The results show that NOV8 is broadly expressed to varying extents in most normal and cancer tissues examined, including lung cancer, in the tissue panel (see Table 4).

NOV16 and NOV17
Ag 145 (F): 5'-CGCACAGTCACATGGTCGA-3' (SEQ ID NO:59)
Ag 145 (R): 5'-CAGGGCGACGTTGTGACAG-3' (SEQ ID NO:60)
Ag 145 (P): FAM-5'-TAGTTTCCGAAGCCCCAGTATCCCACC-3'-TAMRA (SEQ ID
NO:61)

These nucleic acid sequences are downregulated in many tumor cell lines compared with the cognate normal tissue (see Table 4). These results suggest these tumor types can be treated, or prevented, by increasing the activity or expression of these NOVX nucleic acids or encoded polypeptides.

NOV11
Ag 47b (F): 5'-GAACGCCGGAGCATACAGA-3' (SEQ ID NO:62)
Ag 47b (R): 5'-GATGCCACAGGCCCACA-3' (SEQ ID NO:63)
Ag 47b (P): TET-5'-CCAGGTACTGCACAAACACGGCTTCAT-3'-TAMRA (SEQ ID
NO:64)

The results show that NOV11 is broadly expressed in brain and central nervous system cells, with higher expression found in cancer cell lines than in normal cells (see Table 4). It is also expressed in breast cancer cells, among others.

NOV10
Ag 159 (F): 5'-AACCGCCCCGAAATTCTC-3' (SEQ ID NO:65)
Ag 159 (R): 5'-CTGGGACATTTTTCTGAGCCTT-3' (SEQ ID NO:66)
Ag 159 (P): FAM-5'-CCCTGGCACCGTGTCCGCTT-3'-TAMRA (SEQ ID NO:67)

The results show that NOV10 is widely expressed in most cell lines examined (see Table 4). High expression was found, for example, in melanoma, breast cancer, colon cancer, liver cancer, and ovarian cancer.

NOV12
Ag 43 (F): 5'-AAATCGCAAGACATTCACTGTCA-3' (SEQ ID NO:68)
Ag 43 (R): 5'-CCGCCACTCCATCATCACT-3' (SEQ ID NO:69)
Ag 43 (P): TET-5'-CAGCACACTGGACTTCCGAGTGGACC-3'-TAMRA (SEQ ID
NO:70)

It was found that NOV 12 is highly expressed specifically in brain and large cell lung cancer.

NOV20

Ag 119 (F): 5'-GCAGTACAACCGGGTAGATGC-3' (SEQ ID NO:71)
Ag 119 (R): 5'-GCCTCTCAGGGTGCTATTGG-3' (SEQ ID NO:72)
Ag 119 (P): FAM-5'-GAGCAGTGCAGGCAACATCCTTTCTTCT-3'-TAMRA (SEQ ID
NO:73)

The results also show that NOV20 is widely expressed in many tissues. The highest expression level is found in the testis.

**Table 4.**

| Real Time Expression Analysis of Clones of the Invention | | | | | | | | | |
|---|---|---|---|---|---|---|---|---|---|
| | **Percent Relative Expression** | | | | | | | | |
| **Cell Line** | **NOV2** | **NOV5** | **NOV7** | **NOV8** | **NOV16 &17** | **NOV11** | **NOV10** | **NOV12** | **NOV20** |
| Endothelial cells | 2.3 | 0.0 | 1.4 | 3.4 | 0.9 | 0.0 | 0.4 | 0.0 | 5.3 |
| Endothelial cells (treated) | 3.2 | 0.0 | 1.8 | 3.0 | 6.7 | 0.0 | 0.1 | 0.0 | 5.5 |
| Pancreas | 8.8 | 1.4 | 60.7 | 11.3 | 12.0 | 0.0 | 8.2 | 0.1 | 18.2 |
| Pancreatic ca. CAPAN 2 | 3.0 | 0.0 | 1.4 | 11.2 | 9.7 | 0.0 | 10.3 | 0.0 | 15.2 |
| Adipose | 7.4 | 0.0 | 14.6 | 57.4 | 8.9 | 0.0 | 88.3 | 1.4 | 11.6 |
| Adrenal gland | 3.4 | 0.0 | 12.1 | 4.1 | 7.2 | 0.0 | 48.0 | 0.4 | 24.8 |
| Thyroid | 1.0 | 0.0 | 2.9 | 9.9 | 1.0 | 0.0 | 12.0 | 0.1 | 42.3 |
| Salivary gland | 2.1 | 0.0 | 32.3 | 8.1 | 2.3 | 0.0 | 11.3 | 0.2 | 21.9 |
| Pituitary gland | 0.4 | 0.0 | 5.8 | 5.5 | 1.1 | 0.0 | 5.0 | 0.1 | 6.4 |
| Brain (fetal) | 1.4 | 0.0 | 35.6 | 9.3 | 1.4 | 21.8 | 2.5 | 8.0 | 10.7 |
| Brain (whole) | 2.1 | 0.0 | 56.6 | 26.2 | 1.0 | 32.5 | 11.3 | 0.0 | 31.9 |
| Brain (amygdala) | 1.3 | 0.0 | 24.7 | 11.3 | 0.0 | 12.2 | 28.9 | 14.3 | 11.7 |
| Brain (cerebellum) | 3.0 | 0.0 | 100.0 | 40.1 | 0.0 | 14.3 | 28.7 | 100.0 | 52.1 |
| Brain (hippocampus) | 2.3 | 0.0 | 66.9 | 30.6 | 0.2 | 15.6 | 20.6 | 40.1 | 19.9 |
| Brain (hypothalamus) | 2.0 | 0.0 | 27.9 | 12.2 | 3.5 | 0.0 | 15.4 | 3.5 | 14.9 |
| Brain (substantia nigra) | 2.8 | 0.0 | 28.7 | 21.2 | 0.0 | 3.8 | 23.8 | 12.6 | 15.5 |
| Brain (thalamus) | 10.0 | 0.0 | 36.6 | 17.3 | 0.3 | 9.1 | 31.4 | 10.0 | 22.4 |
| Spinal cord | 3.3 | 0.0 | 9.4 | 14.7 | 0.1 | 1.1 | 12.2 | 2.1 | 16.2 |
| CNS ca. (glio/astro) U87-MG | 1.2 | 0.0 | 2.1 | 11.7 | 0.0 | 3.0 | 24.0 | 0.0 | 24.8 |
| CNS ca. (glio/astro) U-118-MG | 2.1 | 0.0 | 1.9 | 14.3 | 17.2 | 0.1 | 16.0 | 0.0 | 12.4 |
| CNS ca. (astro) SW1783 | 1.4 | 0.0 | 1.1 | 7.2 | 0.1 | 36.1 | 8.2 | 0.0 | 7.0 |
| CNS ca.* (neuro; met ) SK-N-AS | 2.5 | 0.0 | 8.5 | 6.7 | 0.0 | 0.0 | 34.9 | 1.4 | 23.7 |
| CNS ca. (astro) SF-539 | 1.9 | 0.0 | 3.3 | 13.1 | 5.8 | 48.3 | 11.0 | 0.0 | 6.6 |
| CNS ca. (astro) SNB-75 | 0.3 | 0.0 | 5.9 | 12.7 | 0.2 | 50.3 | 5.9 | 0.0 | 9.7 |
| CNS ca. (glio) SNB-19 | 2.1 | 0.0 | 7.2 | 20.0 | 8.5 | 100.0 | 16.6 | 0.0 | 32.3 |
| CNS ca. (glio) U251 | 0.9 | 0.0 | 4.5 | 5.8 | 2.5 | 41.5 | 10.2 | 0.0 | 9.2 |
| CNS ca. (glio) SF-295 | 4.4 | 0.0 | 3.1 | 17.6 | 2.5 | 8.7 | 30.4 | 0.0 | 20.0 |
| Heart | 0.7 | 0.0 | 24.8 | 12.0 | 25.5 | 39.8 | 16.6 | 0.4 | 13.4 |
| Skeletal muscle | 1.7 | 0.0 | 10.0 | 4.5 | 6.0 | 0.0 | 6.3 | 1.1 | 11.8 |
| Bone marrow | 2.7 | 0.0 | 2.8 | 4.2 | 0.0 | 0.0 | 5.8 | 0.1 | 6.0 |
| Thymus | 2.1 | 0.0 | 9.3 | 38.2 | 0.7 | 17.2 | 32.3 | 0.2 | 34.6 |
| Spleen | 3.6 | 0.0 | 11.3 | 13.2 | 34.9 | 0.0 | 14.6 | 0.0 | 8.7 |
| Lymph node | 9.7 | 0.0 | 8.5 | 22.7 | 0.0 | 0.0 | 10.1 | 0.0 | 12.8 |
| Colon (ascending) | 1.8 | 0.0 | 11.1 | 8.9 | 1.8 | 4.5 | 9.6 | 0.7 | 19.3 |
| Stomach | 2.7 | 0.0 | 12.8 | 13.6 | 8.1 | 0.2 | 7.3 | 1.7 | 20.3 |
| Small intestine | 2.2 | 0.0 | 17.4 | 9.3 | 2.1 | 0.0 | 4.5 | 0.6 | 12.9 |
| Colon ca. SW480 | 0.7 | 0.0 | 3.1 | 6.0 | 0.0 | 1.4 | 34.2 | 0.0 | 3.3 |
| Colon ca.* (SW480 met)SW620 | 4.1 | 0.0 | 4.4 | 7.7 | 0.0 | 0.0 | 16.7 | 0.0 | 8.9 |
| Colon ca. HT29 | 0.7 | 0.0 | 7.2 | 8.2 | 1.7 | 0.0 | 24.3 | 0.0 | 5.6 |
| Colon ca. HCT-116 | 20.2 | 0.0 | 36.9 | 100.0 | 0.0 | 0.0 | 15.4 | 0.0 | 20.2 |
| Colon ca. CaCo-2 | 0.8 | 0.0 | 6.5 | 11.2 | 9.3 | 0.0 | 14.8 | 0.0 | 12.2 |
| Colon ca. HCT-15 | 1.6 | 0.0 | 5.7 | 29.1 | 0.4 | 0.0 | 28.5 | 0.0 | 13.9 |
| Colon ca. HCC-2998 | 2.3 | 0.0 | 4.9 | 21.2 | 0.0 | 0.0 | 58.2 | 0.0 | 13.2 |
| Gastric ca.* (liver met) NCI-N87 | 6.0 | 0.0 | 4.2 | 51.1 | 19.2 | 0.0 | 39.0 | 0.0 | 36.3 |
| Bladder | 1.7 | 0.0 | 9.3 | 3.1 | 29.9 | 0.1 | 13.7 | 0.9 | 8.0 |
| Trachea | 2.0 | 0.0 | 18.0 | 21.3 | 4.3 | 0.0 | 5.1 | 0.6 | 13.9 |
| Kidney | 1.5 | 0.0 | 6.0 | 12.2 | 52.5 | 1.0 | 31.0 | 0.0 | 18.9 |
| Kidney (fetal) | 1.7 | 0.0 | 13.5 | 12.3 | 17.0 | 2.7 | 13.3 | 0.1 | 26.2 |
| Renal ca. 786-0 | 17.0 | 0.0 | 4.7 | 12.1 | 1.1 | 12.2 | 16.3 | 0.0 | 11.8 |
| Renal ca. A498 | 9.1 | 0.1 | 3.6 | 12.2 | 2.3 | 0.0 | 13.4 | 0.0 | 18.9 |
| Renal ca. RXF 393 | 9.0 | 0.0 | 3.0 | 12.9 | 0.0 | 15.2 | 2.1 | 0.0 | 4.2 |
| Renal ca. ACHN | 0.9 | 0.0 | 2.1 | 5.0 | 2.4 | 0.0 | 2.1 | 0.0 | 5.1 |
| Renal ca. UO-31 | 3.4 | 0.0 | 2.6 | 5.6 | 3.2 | 0.2 | 2.9 | 0.0 | 12.1 |
| Renal ca. TK-10 | 4.3 | 0.0 | 3.1 | 5.1 | 21.5 | 0.0 | 20.4 | 0.0 | 16.0 |
| Liver | 0.9 | 0.0 | 10.2 | 14.3 | 11.6 | 1.9 | 6.9 | 0.1 | 20.4 |
| Liver (fetal) | 0.8 | 0.0 | 3.4 | 6.7 | 0.6 | 0.0 | 12.6 | 0.0 | 8.5 |
| Liver ca. (hepatoblast) HepG2 | 3.2 | 0.0 | 9.2 | 4.1 | 13.9 | 0.0 | 100.0 | 0.0 | 15.7 |
| Lung | 2.4 | 0.0 | 3.5 | 7.9 | 1.9 | 0.0 | 2.5 | 0.0 | 2.8 |
| Lung (fetal) | 1.3 | 0.0 | 8.8 | 10.8 | 0.3 | 0.0 | 1.0 | 0.1 | 12.9 |
| Lung ca. (small cell) LX-1 | 9.1 | 0.0 | 6.6 | 14.3 | 0.0 | 0.0 | 30.6 | 0.0 | 6.7 |
| Lung ca. (small cell) NCI-H69 | 0.4 | 0.0 | 8.2 | 36.6 | 0.0 | 2.9 | 31.2 | 1.0 | 11.6 |
| Lung ca. (s.cell var.) SHP-77 | 2.7 | 0.0 | 44.4 | 80.1 | 0.0 | 0.0 | 11.9 | 8.8 | 35.4 |
| Lung ca. (large cell) NCI-H460 | 15.4 | 0.0 | 30.6 | 70.2 | 34.9 | 0.3 | 42.0 | 93.3 | 24.0 |
| Lung ca. (non-sm. cell) A549 | 1.8 | 0.0 | 2.9 | 35.4 | 7.3 | 0.0 | 29.5 | 0.0 | 12.3 |
| Lung ca. (non-s.cell) NCI-H23 | 3.4 | 0.0 | 8.2 | 17.8 | 0.0 | 0.0 | 20.7 | 0.9 | 12.2 |
| Lung ca (non-s.cell) HOP-62 | 7.5 | 0.0 | 3.7 | 6.1 | 3.9 | 6.7 | 8.4 | 0.0 | 9.1 |
| Lung ca. (non-s.cl) NCI-H522 | 1.4 | 0.0 | 9.7 | 33.4 | 0.1 | 4.8 | 69.7 | 0.3 | 37.9 |
| Lung ca. (squam.) SW 900 | 1.9 | 0.0 | 17.1 | 36.6 | 40.6 | 0.0 | 45.1 | 0.6 | 33.4 |
| Lung ca. (squam.) NCI-H596 | 0.3 | 0.0 | 10.7 | 4.3 | 0.0 | 3.0 | 19.8 | 1.0 | 9.0 |
| Mammary gland | 5.7 | 100.0 | 20.2 | 35.6 | 46.3 | 10.6 | 25.0 | 0.7 | 22.1 |
| Breast ca.* (pl. effusion) MCF-7 | 1.7 | 0.0 | 51.4 | 62.4 | 0.1 | 0.0 | 46.7 | 0.0 | 52.1 |
| Breast ca.* (pl.ef) MDA-MB-231 | 1.3 | 0.0 | 1.1 | 5.3 | 0.1 | 0.0 | 29.5 | 0.0 | 9.2 |
| Breast ca.* (pl. effusion) T47D | 4.4 | 0.0 | 3.5 | 19.5 | 0.0 | 31.4 | 82.4 | 0.0 | 43.2 |
| Breast ca. BT-549 | 1.4 | 0.0 | 4.8 | 42.0 | 3.1 | 28.3 | 13.9 | 0.4 | 13.6 |
| Breast ca. MDA-N | 1.7 | 0.0 | 4.4 | 10.5 | 0.1 | 0.0 | 19.8 | 0.0 | 16.7 |
| Ovary | 1.6 | 0.0 | 4.9 | 8.0 | 5.4 | 0.2 | 16.4 | 0.1 | 6.7 |
| Ovarian ca. OVCAR-3 | 4.6 | 0.0 | 8.5 | 22.2 | 0.1 | 0.0 | 7.4 | 0.3 | 13.7 |
| Ovarian ca. OVCAR-4 | 1.0 | 0.0 | 1.2 | 42.3 | 5.8 | 0.0 | 14.2 | 0.0 | 13.3 |
| Ovarian ca. OVCAR-5 | 4.3 | 0.0 | 17.0 | 23.2 | 23.2 | 36.1 | 30.6 | 0.0 | 13.8 |
| Ovarian ca. OVCAR-8 | 2.8 | 0.0 | 5.8 | 11.2 | 5.4 | 0.2 | 95.9 | 0.1 | 23.3 |
| Ovarian ca. IGROV-1 | 1.1 | 0.0 | 4.2 | 4.6 | 13.2 | 0.0 | 6.5 | 0.0 | 9.0 |
| Ovarian ca.* (ascites) SK-OV-3 | 0.8 | 0.0 | 1.6 | 6.6 | 18.2 | 0.0 | 10.3 | 0.0 | 7.4 |
| Myometrium | 2.3 | 0.0 | 6.8 | 7.0 | 5.8 | 2.4 | 15.5 | 0.2 | 12.9 |
| Uterus | 1.0 | 0.0 | 6.3 | 12.6 | 100.0 | 2.9 | 36.6 | 0.1 | 33.7 |
| Placenta | 2.3 | 0.0 | 24.5 | 45.4 | 2.5 | 0.0 | 8.0 | 0.1 | 28.1 |
| Prostate | 0.9 | 0.0 | 19.2 | 10.2 | 17.0 | 1.7 | 17.6 | 0.5 | 17.9 |
| Prostate ca.* (bone met) PC-3 | 100.0 | 0.0 | 54.0 | 87.7 | 58.6 | 0.0 | 93.3 | 6.6 | 15.7 |
| Testis | 2.6 | 0.0 | 24.1 | 25.9 | 6.3 | 26.4 | 55.1 | 1.3 | 100.0 |
| Melanoma Hs688(A).T | 0.1 | 0.0 | 1.4 | 8.4 | 0.0 | 28.9 | 2.4 | 0.0 | 8.1 |
| Melanoma* (met) Hs688(B).T | 0.3 | 0.0 | 0.7 | 3.2 | 0.6 | 9.2 | 2.1 | 0.0 | 6.4 |
| Melanoma UACC-62 | 0.5 | 0.0 | 1.8 | 5.2 | 0.0 | 0.0 | 44.1 | 0.0 | 1.9 |
| Melanoma M14 | 2.5 | 0.0 | 12.4 | 11.5 | 0.0 | 0.0 | 7.5 | 0.0 | 7.8 |
| Melanoma LOX IMVI | 8.0 | 0.0 | 6.7 | 36.6 | 0.0 | 2.7 | 14.0 | 0.0 | 20.9 |
| Melanoma* (met) SK-MEL-5 | 3.9 | 0.0 | 12.8 | 8.3 | 0.0 | 0.0 | 12.1 | 0.1 | 11.0 |
| Melanoma SK-MEL-28 | 2.8 | 0.0 | 9.9 | 9.2 | 0.0 | 0.0 | 100.0 | 0.0 | 10.0 |
| Melanoma UACC-257 | 1.2 | 0.0 | 5.1 | 3.9 | 100.0 | 0.0 | 100.0 | 0.0 | 9.3 |

### Example 3. Molecular cloning of a NOV4 nucleic acid (Clone 3189601)

The following oligonucleotide primer pairs were designed to PCR amplify a full length cDNA clone coding for the 152 amino acid residue protein encoded by a NOV4 nucleic acid:

3189601 F-Forward: CGTC GGA TCC ATG CCA CAT CTG TAT ATA GAT GGG GTT
TTT CC (SEQ ID NO:93)
3189601 F-Reverse: GGTG GTC GAC TTA ATG GTG ATG GTG ATG ATG GTG GCT
CGG GGA TGT TTC CCC GTT (SEQ ID NO:74)

The forward primer includes an in-frame BamHI restriction site and the reverse primer contains an in-frame SalI restriction site.

PCR reactions were set up using 5 ng-human testis cDNA template, 1 microM of each of the 3189601 F-Forward and 3189601 F-Reverse primers, 5 micromoles dNTP (Clontech Laboratories, Palo Alto CA) and 1 microliter of 50xAdvantage-HF 2 polymerase (Clontech Laboratories, Palo Alto CA) in 50 microliters. The following reaction conditions were used:
a) 96°C 3 minutes
b) 96 °C 30 seconds denaturation
c) 70 °C 30 seconds, primer annealing. This temperature was gradually decreased by 1oC/cycle
d) 72 °C 1 minute extension.
   Repeat steps b-d 10 times
e) 96 °C 30 seconds denaturation
f) 60 °C 30 seconds annealing
g) 72 °C 1 minute extension
   Repeat steps e-g 25 times
h) 72 °C 5 minutes final extension

A single amplified product of approximately 300 bp was detected by agarose gel electrophoresis. The product was isolated, digested with BamHI and SalI restriction enzymes, and ligated directly into the pMelBac and pSecTag2 expression vectors (Invitrogen, Carlsbad CA). The DNA sequence of the cloned inserts were determined as an ORF coding for a 102 amino acid long polypeptide. The cloned constructs are called pMelBac-cg3189601-S3 and pSecTag2-cg3189601-S5, respectively.

The nucleotide sequence of the inserts in pMelBac-cg3189601-S3 and pSecTag2-cg3189601-S5 were found to be identical to each other, but different from that of SEQ ID NO:7. The difference includes a gap and an insertion that, respectively, disrupt and restore the reading frame for the encoded protein. The sequences of the gene fragment (SEQ ID NO:75) and the encoded polypeptide (SEQ ID NO:76) are shown below:

Clone 3189601A

1 ATGCCACATCTGTATATAGATGGGGTTTTTCCAATACAGCTGGTT
M P H L Y I D G V F P I Q L V
46 CGTGATAAACTGCATGAAACTCCTGCCGTCCTGCGCCTGCTGGGG
R D K L H E T P A V L R L L G
91 CCTCCAGGCAAGGCCACGTGGGGTTGGGGGTGGGGCTGGTCCTTC
P P G K A T W G W G W G W S F
136 TCCCTCCCCAGGCCTGTGTTCTTGGGGCTGCTCCCATGCAGACAG
S L P R P V F L G L L P C R Q
181 GATCACCTAACAGAGATGGAAGCCAGGGCATGGATGGGGCTTTGG
D H L T E M E A R A W M G L W
226 GTCCTCGAGGTTGGACCCCAGCTTCTTGCCACCTTCCCCTCCGGG
V L E V G P Q L L A T F P S G
271 CAGTCAGCTCTCCATCCATCCCCCTCTTTAATCTA (SEQ ID NO:75)
Q S A L H P S P S L I (SEQ ID NO:76)

### Example 4. Molecular cloning of a NOV21 nucleic acid (Clone 3211101.0.120)

The predicted mature extracellular domain of a NOV21 nucleic acid present in clone 3211101.0.120 was cloned. The cloned domain encoded residues 26 to 149. Other regions of the polypeptide include the predicted signal peptide (residues 1-25) and the transmembrane domain (residues 150 and 169).

Oligonucleotide primers used to amplify the extracellular domain included a forward primer, which includes an BamHI restriction site, and a reverse primer, which contained an Xhol restriction site. The sequences of the primers are the following:

3211101 MatForward:	GGATCC GAA GTT GAG AAA TCC TCA GAT GGT (SEQ
ID NO: 77)

3211101 MatReverse:	CTCGAG AGG GTT GTA CTC TGT CAC CAT GTG (SEQ
ID NO: 78)

PCR reactions were set up using 5 ng human pancreas cDNA template, 1 microM of each of the 3211101 MatForward and 3211101 Mat Reverse primers. The remaining conditions and steps were the same as those employed in Example BA.

A single amplified product of approximately 450 bp was detected by agarose gel electrophoresis. The product was isolated and ligated into the pCR2.1 vector (Invitrogen, Carlsbad CA). The DNA sequence of the cloned insert was verified as an ORF coding for the mature, extracellular domain of NOV21 from residues 26 to 149. The construct is called pCR2.1-3211101-S219-3C. The sequence is identical to that in the ORF of clone NOV21.

### Example 5. Molecular cloning of a NOV6 nucleic cid (Clone 3218715)

Oligonucleotide primers were prepared to PCR amplify a DNA segment coding for the full-length cgNOV6 protein of 393 residues. The forward primer includes a BamHI restriction site and the reverse primer contains an XhoI restriction site. The sequences of the primers are the following:

3218715 F-TOPO-Forward: GGA TCC ACC ATG CGG ACA CTC TTC AAC CTC
CTC TGG (SEQ ID NO:79)
3218715 F-TOPO-Reverse: CTC GAG GAG CAG GTC GTA GAA GTA GTC CAG
G (SEQ ID NO:80).

PCR reactions were set up using 5 ng human testis and fetal brain cDNA templates and 1 microM of each of the 3218715 F-TOPO-Forward and 3218715 F-TOPO-Reverse primers. The remaining conditions were the same as used in Example 3 except for the following steps:
d) 72°C 2 minutes extension;
g) 72°C 2 minutes extension

A single amplified product of approximately 1.2 kbp was detected by agarose gel electrophoresis. The product was isolated, and ligated directly into the pcDNA3.1-TOPO-V5-His expression vector (Invitrogen, Carlsbad CA). The DNA sequences of the cloned inserts were determined to be identical to the sequence of the corresponding segment of SEQ ID NO:11. The construct is called as pcDNA3.1-TOPO-cg-3218715.

### Example 6. Molecular cloning of the extracellular domain of a NOV9 nucleic acid (Clone

### 3540000)

Oligonucleotide primers were designed to PCR amplify a DNA segment coding for the extracellular domain ofNOV9 from residues 138-410. The forward primer includes a BamHI restriction site and the reverse primer contains an XhoI restriction site. The sequences of the primers are the following:

3540000 C-Forward: CGTC GGA TCC TAT GTC AAG TGC CGT CTC AAC GTG
CTG CTC TGG TAC (SEQ ID NO:81)
3540000 C-Reverse: CGTC CTC GAG TTA ATG GTG ATG GTG ATG ATG CAT
ATC ATC CTT GGA CAC CAG GCA G (SEQ ID NO:82)

PCR reactions were set up using 5 ng human placenta cDNA templates, and 1 microM of each of the 3540000 C-Forward and 3540000 C-Reverse primers. The remaining conditions and procedures were the same as employed in Example 3.

A single amplified product of approximately 800 bp was detected by agarose gel electrophoresis. The product was isolated, digested with BamHI and XhoI restriction enzymes and ligated into the pSecTag2 expression vector (Invitrogen, Carlsbad CA). The DNA sequence of the cloned insert was determined as an ORF coding for a 273 amino acid long polypeptide as expected. The construct was named pSecTag2-cg3540000-S22A. The nucleotide sequence is identical to the corresponding segment of SEQ ID NO:17.

### Example 7. Molecular cloninit of a NOV12 nucleic acid (Clone 10219646.0.58)

The predicted open reading frame of a NOV12 nucleic acid according to the invention encodes a novel 404 residue protein. The encoded protein is predicted to be a Type I transmembrane protein with a signal peptide from residues 1 to 25. Oligonucleotide primers were designed to PCR amplify a DNA segment, coding for the mature form of the extracellular domain, from residues 25-333. The forward primer includes a BamHI restriction site and the reverse primer contains a SalI restriction site. The sequences of the primers are the following:

10219646 MatF:		GGATCCAAGAATAAAGTTAAAGGCAGC		(SEQ ID
NO:83)

10219646 Reverse:    GTCGACGCCAGCCAAAGCATTAGGATCATGCAC    (SEQ ID
NO:84)

PCR reactions were set up using 5 ng cDNA template consisting of equal portions of human testis, fetal brain, mammary, skeletal muscle derived cDNA, and 1 microM of each of 10219646 MatF and 10219646 Reverse primers. The remaining conditions and steps were the same as those used in Example 3.

An amplified product of approximately 400 bp was detected by agarose gel electrophoresis. The product was isolated and ligated into the pCR2.1 vector (Invitrogen Corp, Carlsbad). The DNA sequence of the cloned insert was determined as an ORF coding for a 309 amino acid long polypeptide, as expected. The construct was named pCR2.1-cg10219646-S344-5B, and its sequence is identical the corresponding segment in SEQ ID NO 23

### Example 8. Molecular cloning of a NOV18 nucleic acid (clone 3726392)

Oligonucleotide primers were designed to PCR amplify a DNA segment coding for a 137 residue NOV18 protein. The forward primer included a BamHI restriction site and the consensus Kozak sequence CCACC. The reverse primer contained an XhoI restriction site. The primers had the following sequences:

3726392 F-Forward: CGGGATCCACCATGTCAAGCCCTGCTTCCACCTGCATAG
(SEQ ID NO:85)
3726392 F-Reverse: CGCTCGAGACTGAATGGATACATGAAAAGAAAGGAACAAA
GAGGTG (SEQ ID NO:86)

PCR reactions were set up using 5 ng cDNA template consisting of equal portions of human testis, fetal brain, mammary, skeletal muscle derived cDNA, and 1 microM of each of 3726392 F-Forward and 3726392 F-Reverse primers. The other conditions and steps were the same as described in Example 3.

An amplified product of approximately 400 bp was detected by agarose gel electrophoresis. The product was isolated, digested with BamHI and XhoI restriction enzymes, and ligated into the BIgHis baculovirus expression vector (see Example 9, below). The DNA sequence of the cloned insert was determined as an open reading frame encoding a 137 amino acid long polypeptide. The construct was named BIgHis-cg3726392-#2. The nucleotide sequence of the construct was found to be identical to the coding sequence in SEQ ID NO:35.

### Example 9. Construction of expression vector pBIgHis

An expression vector, named pBIgHis, was constructed for expressing NOVX nucleic acid sequences. To construct the pBIgHis expression vector, oligonucleotide primers were designed to amplify the Fc fragment of the human immunoglobulin heavy chain. The forward primer was 5'-CCGCTCGAGTGAGCCCAAATCTTGTGACAAA (SEQ ID NO:87), and the reverse primer was

5'-GCTCTAGACTTTTACCCGGGACAGGGAG (SEQ ID NO: 88).

PCR was initiated by heating 25 ul Mix 1 (75 pmoles primers, 4 ug adult testis cDNA, 5 umoles dNTPs) and 25 ul Mix 2 [1 unit Fidelity Expand polymerase (Boehringer Mannheim), 5 ul 1 0X Fidelity Expand Buffer] separately at 96°C for 20 seconds. Mixes 1 and 2 were then pooled, and the following PCR cycling parameters were used: 96°C, 3 min (1 cycle); 96°C, 30 sec, 55°C,1 min, 68°C, 2 min (10 cycles); 96°C, 30 sec, 60°C, 1 min, 68°C, 2 min (20 cycles); 72°C, 7 min (1 cycle). After PCR, a single DNA fragment of approximately 0.75 kb was obtained. The DNA fragment was digested with XhoI and XbaI restriction enzymes and cloned into the pCDNA3.1 VSHis(B) expression vector (Invitrogen, Carlsbad, CA). This vector is named as pCDNA3.1 Ig and contains Fc fragment fused to V5 epitope and 6xHis tag. At the next step a recombinant TEV protease cleavage site was introduced to the N-terminus of the Fc fragment. First, two oligonucleotides were designed, 5'-AATTCTGCAGCGAAAACCTGTATTTTCAGGGT (SEQ ID NO: 89) and

5'-TCGAACCCTGAAAATACAGGTTTTCGCTGCAG (SEQ ID NO: 90).

These two oligonucleotides were annealed and purified using 20% polyacrylamide gel and ligated into EcoRI and XhoI digested pCDNA3.1Ig. The resulting plasmid was then cut with PstI and PmeI to release a DNA fragment of approximately 0.9 kb, which was ligated into pBlueBac4.5 (Invitrogen, Carlsbad, CA) digested with PstI and SmaI. The resulting plasmid construct was named pBIgHis. The Fc fragment was verified by sequence analysis.

### Example 10. Construction of the mammalian expression vector pCEP4/Sec

An expression vector, named pCEP4/Sec, was constructed to express NOVX nucleic acids in mammalian cells.

To construct pCEP4/Sec, the oligonucleotide primers, 
pSec-V5-His Forward:          CTCGTC CTCGAG GGT AAG CCT ATC CCT AAC
                                                    (SEQ ID NO:91) and

pSec-V5-His Reverse:CTCGTC GGGCCCCTGATCAGCGGGTTTAAAC
                                                    (SEQ ID NO:92)
 were designed to amplify a fragment from the pcDNA3.1-V5His (Invitrogen, Carlsbad, CA) expression vector. The PCR product was digested with XhoI and ApaI and ligated into the XhoI/ApaI digested pSecTag2 B vector (Invitrogen, Carlsbad CA). The correct structure of the resulting vector, pSecV5His, was verified by DNA sequence analysis. The vector pSecV5His was digested with PmeI and Nhel, and the PmeI-NheI fragment was ligated into the BamHI/Klenow and NheI treated vector pCEP4 (Invitrogen, Carlsbad, CA). The resulting vector was named pCEP4/Sec expression vector. This vector allows heterologous protein expression and secretion by fusing any protein to the Ig kappa chain signal peptide. Detection and purification of the expressed protein are aided by the presence of the V5 epitope tag and 6xHis tag at the C-terminus (Invitrogen, Carlsbad, CA).

### Example 11. Expression of NOV5 in human embryonic kidney 293 cells.

The BamHI-XhoI fragment containing a NOV5 sequence was isolated from pCR2.1-3211101-S219-3C (described in Example 4) and subcloned into the vector pCEP4/Sec to generate expression vector pCEP4/Sec-3211101. The pCEP4/Sec-3211101 vector was transfected into 293 cells using the LipofectaminePlus reagent following the manufacturer instructions (Gibco/BRL/Lefe Technologies, Rockville, MD). The cell pellet and supernatant were harvested 72 hours after transfection and examined for hNOV5 expression by Western blotting (reducing conditions) with an anti-V5 antibody. FIG. 1 shows that NOV5 is expressed as 30 and 20 kDa proteins secreted by 293 cells. These appear to represent the expected polypeptide product glycosylated to greater and lesser extents.

### Example 12. Expression and secretion of NOV5 by E. coli

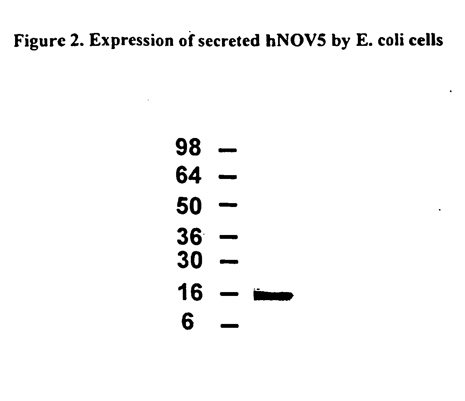

The vector pBADgIII (InVitrogen Inc., Carlsbad, CA) was digested with BamHI and XhoI restriction enzymes. The BamHI-XhoI fragment containing the NOV5 sequence was isolated from pCR2.1-3211101-S219-3C and subcloned into the vector pBADgIII to generate expression vector pBADgIII-3211101. The resulting vector was confirmed by restriction analysis and sequencing and was named as pBADgIII-3211101. In this vector, hNOV5 was fused to the 6xHis tag at its C-terminus. The plasmid pBADgIII-3211101 was then transformed into the *E*. *coli* expression host BL21 (DE3, pLys) (Novagen, Madison, WI) and the expression induction of protein NOV5 was carried out according to the manufacturer's instructions. After induction, total cells were harvested, and proteins were analyzed by Western blotting using anti-HisGly antibody (Invitrogen, Carlsbad, CA). Fig. 2 shows hNOV5 was expressed as a 16 kDa protein secreted by E. coli cells. This apparent molecular weight is consistent with the size of the polypeptide predicted by the amino acid sequence of SEQ ID NO: 10.

### Example 13. Expression of NOV6 in human embryonic kidney 293 cells

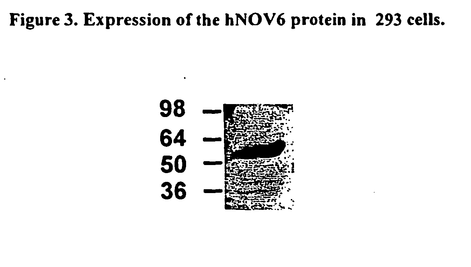

The pcDNA3.1-TOPO-cg-3218715 vector (see Example 5) was transfected into 293 cells using the LipofectaminePlus reagent following the manufacturer instructions (Gibco/BRL). The cell pellet and supernatant were harvested 72 hours after transfection and examined for hNOV6 expression by Western blotting (reducing conditions) with an anti-V5 antibody. Fig. 3 shows that hNOV6 is expressed as a 60 kDa protein in 293 cells. It is believed that this apparent molecular weight corresponds to a glycosylated form of the NOV6 polypeptide. The expressed protein was not detected in the cell supernatent.

### EQUIVALENTS

From the foregoing detailed description of the specific embodiments of the invention, it should be apparent that particular novel compositions and methods involving nucleic acids, polypeptides, antibodies, detection and treatment have been described. Although these particular embodiments have been disclosed herein in detail, this has been done by way of example for purposes of illustration only, and is not intended to be limiting with respect to the scope of the appended claims that follow. In particular, it is contemplated by the inventors that various substitutions, alterations, and modifications may be made as a matter of routine for a person of ordinary skill in the art to the invention without departing from the spirit and scope of the invention as defined by the claims. Indeed, various modifications of the invention in addition to those described herein will become apparent to those skilled in the art from the foregoing description and accompanying figures. Such modifications are intended to fall within the scope of the appended claims.

## Claims

1. An isolated polypeptide comprising an amino acid sequence selected from the group consisting of:
a) a mature form of an amino acid sequence selected from the group consisting of SEQ ID NOs: 22, 32, and 34;
b) a variant of a mature form of an amino acid sequence selected from the group consisting of SEQ ID NOs: 22, 32, and 34; wherein one or more amino acid residues in said variant differs from the amino acid sequence of said mature form, provided that said variant differs in no more than 15% of the amino acid residues from the amino acid sequence of said mature form;
c) an amino acid sequence selected from the group consisting of SEQ ID NOs: 22, 32, and 34; and
d) a variant of an amino acid sequence selected from the group consisting of SEQ ID NOs: 22, 32, and 34; wherein one or more amino acid residues in said variant differs from the amino acid sequence of said mature form, provided that said variant differs in no more than 15% of amino acid residues from said amino acid sequence.

2. The polypeptide of Claim 1, wherein said polypeptide comprises the amino acid sequence of a naturally occurring allelic variant of an amino acid sequence selected from the group consisting of SEQ ID NOs: 22, 32, and 34.

3. The polypeptide of Claim 2, wherein said allelic variant comprises an amino acid sequence that is the translation of a nucleic acid sequence differing by a single nucleotide from a nucleic acid sequence selected from the group consisting of SEQ ID NOs: 21, 31, and 33.

4. The polypeptide of Claim 1, wherein the amino acid sequence of said variant comprises a conservative amino acid substitution.

5. An isolated nucleic acid molecule comprising a nucleic acid sequence encoding a polypeptide comprising an amino acid sequence selected from the group consisting of:
a) a mature form of an amino acid sequence selected from the group consisting of SEQ ID NOs: 22, 32, and 34;
b) a variant of a mature form of an amino acid sequence selected from the group consisting of SEQ ID NOs: 22, 32, and 34; wherein one or more amino acid residues in said variant differs from the amino acid sequence of said mature form, provided that said variant differs in no more than 15% of the amino acid residues from the amino acid sequence of said mature form;
c) an amino acid sequence selected from the group consisting of SEQ ID NOs: 22, 32, and 34;
d) a variant of an amino acid sequence selected from the group consisting of SEQ ID NOs: 22, 32, and 34; wherein one or more amino acid residues in said variant differs from the amino acid sequence of said mature form, provided that said variant differs in no more than 15% of amino acid residues from said amino acid sequence;
e) a nucleic acid fragment encoding at least a portion of a polypeptide comprising an amino acid sequence chosen from the group consisting of SEQ ID NOs: 22, 32, and 34, or a variant of said polypeptide, wherein one or more amino acid residues in said variant differs from the amino acid sequence of said mature form, provided that said variant differs in no more than 15% of amino acid residues from said amino acid sequence; and
f) a nucleic acid molecule comprising the complement of a), b), c), d), or e).

6. The nucleic acid molecule of Claim 5, wherein the nucleic acid molecule comprises the nucleotide sequence of a naturally occurring allelic nucleic acid variant.

7. The nucleic acid molecule of Claim 5, wherein the nucleic acid molecule encodes a polypeptide comprising the amino acid sequence of a naturally occurring polypeptide variant.

8. The nucleic acid molecule of Claim 5, wherein the nucleic acid molecule differs by a single nucleotide from a nucleic acid sequence selected from the group consisting of SEQ ID NOs: 21, 31, and 33.

9. The nucleic acid molecule of Claim 5, wherein said nucleic acid molecule comprises a nucleotide sequence selected from the group consisting of
a) a nucleotide sequence selected from the group consisting of SEQ ID NOs: 21, 31, and 33;
b) a nucleotide sequence differing by one or more nucleotides from a nucleotide sequence selected from the group consisting of SEQ ID NOs: 21, 31, and 33, provided that no more than 20% of the nucleotides differ from said nucleotide sequence;
c) a nucleic acid fragment of a); and
d) a nucleic acid fragment of b).

10. The nucleic acid molecule of Claim 5, wherein said nucleic acid molecule hybridizes under stringent conditions to a nucleotide sequence chosen from the group consisting of SEQ ID NOs: 21, 31, and 33, or a complement of said nucleotide sequence.

11. The nucleic acid molecule of Claim 5, wherein the nucleic acid molecule comprises a nucleotide sequence selected from the group consisting of
a) a first nucleotide sequence comprising a coding sequence differing by one or more nucleotide sequences from a coding sequence encoding said amino acid sequence, provided that more than 20% of the nucleotides in the coding sequence in said first nucleotide sequence differ from said coding sequence;
b) an isolated second polynucleotide that is a complement of the first polynucleotide; and
c) a nucleic acid fragment of a) or b).

12. A vector comprising the nucleic acid molecule of Claim 11.

13. The vector of Claim 12, further comprising a promoter operably linked to said nucleic acid molecule.

14. A cell comprising the vector of Claim 12.

15. An antibody that binds immunospecifically to the polypeptide of Claim 1.

16. The antibody of Claim 15, wherein said antibody is a monoclonal antibody.

17. The antibody of Claim 15, wherein the antibody is a humanized antibody.

18. A method for determining the presence or amount of the polypeptide of Claim 1 in a sample, the method comprising:
(a) providing the sample;
(b) contacting the sample with an antibody that binds immunospecifically to the polypeptide; and
(c) determining the presence or amount of antibody bound to said polypeptide, thereby determining the presence or amount of polypeptide in said sample.

19. A method for determining the presence or amount of the nucleic acid molecule of Claim 5 in a sample, the method comprising:
(a) providing the sample;
(b) contacting the sample with a probe that binds to said nucleic acid molecule; and
(c) determining the presence or amount of the probe bound to said nucleic acid molecule,
thereby determining the presence or amount of the nucleic acid molecule in said sample.

20. A method of identifying an agent that binds to a polypeptide of Claim 1, the method comprising:
(a) contacting said polypeptide with said agent; and
(b) determining whether said agent binds to said polypeptide.

21. A method for identifying an agent that modulates the expression or activity of the polypeptide of Claim 1, the method comprising:
(a) providing a cell expressing said polypeptide;
(b) contacting the cell with said agent, and
(c) determining whether the agent modulates expression or activity of said polypeptide,
whereby an alteration in expression or activity of said peptide indicates said agent modulates expression or activity of said polypeptide.

22. A method for modulating the activity of the polypeptide of Claim 1, the method comprising contacting a cell sample expressing the polypeptide of said claim with a compound that binds to said polypeptide in an amount sufficient to modulate the activity of the polypeptide.

23. Use of a polypeptide according to Claim 1, for the manufacture of a medicament for treating or preventing a NOVX-associated disorder in a subject.

24. Use according to Claim 23, wherein said subject is a human.

25. Use of the nucleic acid of Claim 5 for the manufacture of a medicament for treating or preventing a NOVX-associated disorder in a subject.

26. Use according to Claim 25, wherein said subject is a human.

27. Use of an antibody according to Claim 15 for the manufacture of a medicament for treating or preventing a NOVX-associated disorder in a subject.

28. Use according to Claim 27, wherein the subject is a human.

29. A pharmaceutical composition comprising the polypeptide of Claim 1 and a pharmaceutically acceptable carrier.

30. A pharmaceutical composition comprising the nucleic acid molecule of Claim 5 and a pharmaceutically acceptable carrier.

31. A pharmaceutical composition comprising the antibody of Claim 15 and a pharmaceutically acceptable carrier.

32. A kit comprising in one or more containers, the pharmaceutical composition of Claim 29.

33. A kit comprising in one or more containers, the pharmaceutical composition of Claim 30.

34. A kit comprising in one or more containers, the pharmaceutical composition of Claim 31.

35. A method for determining the presence of or predisposition to a disease associated with altered levels of the polypeptide of Claim 1 in a first mammalian subject, the method comprising:
a) measuring the level of expression of the polypeptide in a sample from the first mammalian subject; and
b) comparing the amount of said polypeptide in the sample of step (a) to the amount of the polypeptide present in a control sample from a second mammalian subject known not to have, or not to be predisposed to, said disease,
wherein an alteration in the expression level of the polypeptide in the first subject as compared to the control sample indicates the presence of or predisposition to said disease.

36. A method for determining the presence of or predisposition to a disease associated with altered levels of the nucleic acid molecule of Claim 5 in a first mammalian subject, the method comprising:
a) measuring the amount of the nucleic acid in a sample from the first mammalian subject; and
b) comparing the amount of said nucleic acid in the sample of step (a) to the amount of the nucleic acid present in a control sample from a second mammalian subject known not to have or not to be predisposed to, said disease;
wherein an alteration in the level of the nucleic acid in the first subject as compared to the control sample indicates the presence of or predisposition to said disease.

37. Use of a polypeptide having an amino acid sequence at least 95% identical to a polypeptide comprising an amino acid sequence of at least one of SEQ ID NOs: 22, 32, and 34, or a biologically active fragment thereof, for the manufacture of a medicament for treating a pathological state in a mammal.

38. Use of an antibody according to Claim 15 for the manufacture of a medicament for treating a pathological state in a mammal.
